# EUROPEAN PATENT SPECIFICATION

(11) **EP 1 618 127 B1**
(45) Date of publication and mention of the grant of the patent: **10.12.2014**
(21) Application number: 04750188.7
(22) Date of filing: 09.04.2004
(51) Int. Cl.: C07K 14/165

(54) **Immunogenic composition comprising a spike protein of the SARS coronavirus**
Immunogenische Zusammensetzung einer Spikeprotein des SARS Coronavirus
Composé immunogénique d'une protéine spike du coronavirus de la SARS

(30) Priority: 10.04.2003 US 462218 P; 11.04.2003 US 462465 P; 12.04.2003 US 462418 P; 13.04.2003 US 462748 P; 14.04.2003 US 463109 P; 15.04.2003 US 463460 P; 16.04.2003 US 463668 P; 17.04.2003 US 463983 P; 18.04.2003 US 463971 P; 22.04.2003 US 464899 P; 22.04.2003 US 464838 P; 23.04.2003 US 465273 P; 24.04.2003 US 465535 P; 05.05.2003 US 468312 P; 22.05.2003 US 473144 P; 14.08.2003 US 495024 P; 23.09.2003 US 505652 P; 11.10.2003 US 510781 P; 11.12.2003 US 529464 P; 12.01.2004 US 536177 P; 07.04.2004 US 560757 P
(43) Date of publication of application: 25.01.2006
(73) Proprietor: Novartis Vaccines and Diagnostics, Inc., Emeryville, CA 94608 (US)
(72) Inventor: RAPPUOLI, Rino, Emeryville, CA 94662-8097 (US); MASIGNANI, Vega, Emeryville, CA 94662-8097 (US); STADLER, Konrad, Emeryville, CA 94662-8097 (US); GREGERSEN, Jens-Peter, Emeryville, CA 94662-8097 (US); CHIEN, David, P.O. Box 8097 Emeryville, CA 94662-8097 (US); HAN, Jang, P.O. Box 8097 Emeryville, CA 94662-8097 (US); POLO, John, P.O. Box 8097 Emeryville, CA 94662-8097 (US); WEINER, Amy, P.O. Box 8097 Emeryville, CA 94662-8097 (US); HOUGHTON, Michael, Emeryville, CA 94662-8097 (US); SONG, Hyun, Chul, Emeryville, CA 94662-8097 (US); SEO, Mi, Young, Emeryville, CA 94662-8097 (US); DONNELLY, John, J., Emeryville, CA 94662-8097 (US); KLENK, Hans, Dieter, Emeryville, CA 94662-8097 (US); VALIANTE, Nicholas, Emeryville, CA 94662-8097 (US)
(74) Representative: Marshall, Cameron John
(86) International application number: PCT/US2004/011710
(87) International publication number: WO 2004/092360

(56) References cited:
- WO-A-2004/085455
- WO-A-2004/085633
- WO-A-2004/085650
- WO-A-2004/091524
- WO-A-2004/092208
- WO-A-2004/096842
- WO-A2-2004/089983
- PEIRIS J S M ET AL: "Coronavirus as a possible cause of severe acute respiratory syndrome." LANCET. 19 APR 2003, vol. 361, no. 9366, 8 April 2003 (2003-04-08), pages 1319-1325, XP004421148 ISSN: 0140-6736 cited in the application
- DROSTEN CHRISTIAN ET AL: "Identification of a novel coronavirus in patients with severe acute respiratory syndrome." NEW ENGLAND JOURNAL OF MEDICINE, vol. 348, no. 20, 10 April 2003 (2003-04-10), pages 1967-1976, XP002305233 ISSN: 0028-4793 cited in the application -& WORLD HEALTH ORGANIZATION, [Online] 25 March 2003 (2003-03-25), XP002319429 Retrieved from the Internet: URL:(http://www.who.int/csr/sars/primers/e n)> [retrieved on 2005-02-10]
- DATABASE GENBANK [Online] NATIONAL CENTER FOR BIO; 14 April 2003 (2003-04-14), BCCA GENOME SCIENCES CENTER BRITISH COLUMBIA: "SARS coronavirus Tor2" XP002319430 retrieved from NCBI Database accession no. AY274119 -& MARRA M A ET AL: "The genome sequence of the SARS-associated coronavirus" SCIENCE, AMERICAN ASSOCIATION FOR THE ADVANCEMENT OF SCIENCE,, US, vol. 300, no. 5624, 30 May 2003 (2003-05-30), pages 1399-1404, XP002269483 ISSN: 0036-8075
- ROTA PAUL A ET AL: "Characterization of a novel coronavirus associated with severe acute respiratory syndrome" SCIENCE, AMERICAN ASSOCIATION FOR THE ADVANCEMENT OF SCIENCE,, US, vol. 300, no. 5624, 30 May 2003 (2003-05-30), pages 1394-1399, XP002288938 ISSN: 0036-8075
- KSIAZEK T.G. ET AL.: "A new coronavirus associated with severe acute respiratory syndrome", NEW ENGLAND J MED, vol. 348, no. 20, 2003, pages 1953-1966,
- DATABASE EMBL ACCESSION NUMBER AY323976, 25 June 2003 CONG LM ET AL: 'SARS coronavirus ZJ01 isolate spike glycoprotein'

## Description

### FIELD OF THE INVENTION

The invention relates two nucleic acids and proteins from Severe Acute Respiratory Syndrome (SARS) Virus. These nucleic acids and proteins can be used in the preparation and manufacture of vaccine formulations for the treatment or prevention of SARS. Also disclosed are diagnostic reagents, kits (comprising such reagents) and methods which can be used to diagnose or identify the presence or absence of a SARS virus in a biological sample. Also disclosed are methods for the treatment or prevention of SARS utilizing small molecule viral inhibitors and combinations of small molecule viral inhibitors and kits for the treament of SARS.

### BACKGROUND OF THE INVENTION

An outbreak of a virulent respiratory virus, now known as Severe Acute Respiratory Syndrome (SARS), was identified in Hong Kong, China and a number of other countries around the world in 2003. Patients typically had symptoms including fever, dry cough, dyspnea, headache, and hypoxemia. Isolates of the SARS virus appear to have homology with at least the RNA polymerase gene of several known coronaviruses. A phylogenetic analysis of this homology is presented in Peiris et al., "Coronavirus as a possible cause of severe acute respiratory syndrome", Lancet, published online April 8, 2003 at http://image.thelancet.com/extras/03art3477web.pdf. Other sequenced fragments of the SARS virus genome appear to overlap with the open reading frame 1b of coronaviruses. See, Drosten et al., "Identification of a Novel Coronavirus in Patients with Severe Acute Respiratory Syndrome", New England Journal of Medicine, published online at lettp://www.nejin.org on April 10, 2003.

The Genome Science Center in British Colombia, Canada published on its website *(http:*///*www.bcgsc.ca*/*bioinfo*/*SARS*/*)* a draft genome assembly of 29,736 base pairs of a virus believed to be a SARS virus, referred to as the TOR2 isolate.

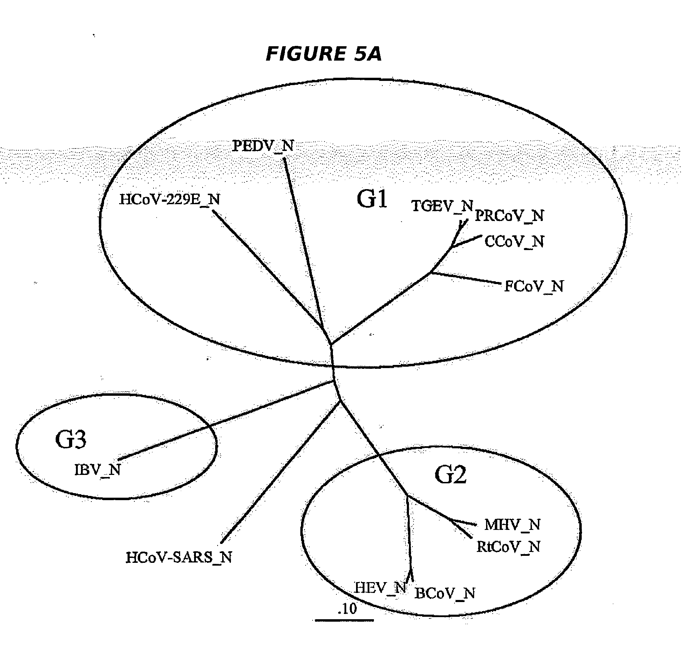

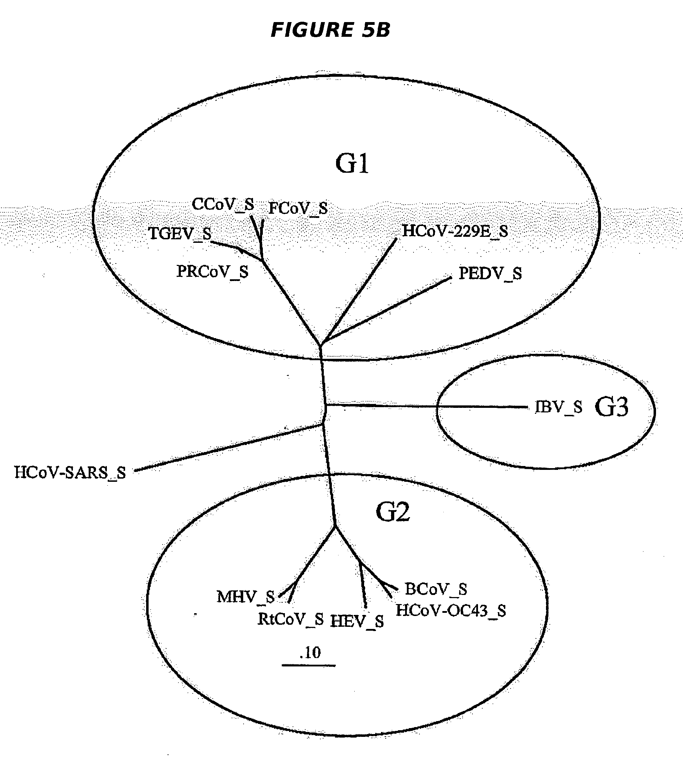

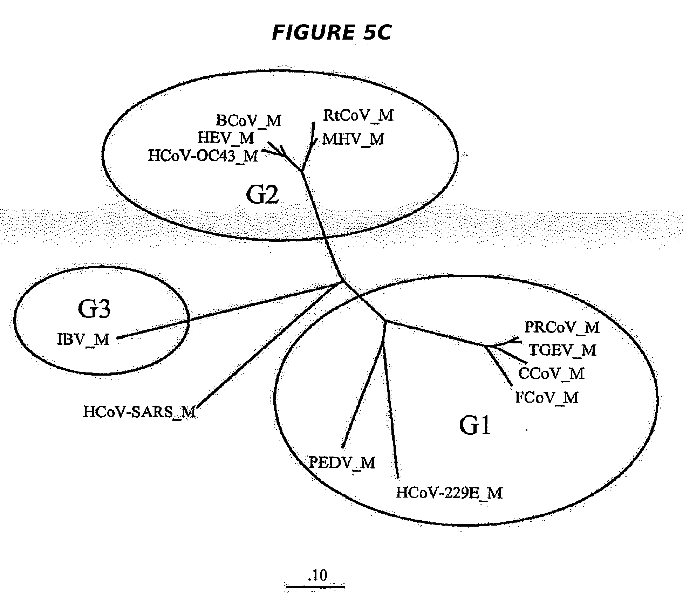

The Centers for Disease Control (CDC) published a nucleotide sequence of a SARS-CoV strain on its website (*http:*//*www.cdc.gov*/*ncidod*/*sars*/*pdf*/*nucleoseq.pdf*). The CDC has also published a phylogenetic tree of the predicted N, S and M proteins (attached as FIGURE 5). This tree places the SARS virus outside any of the previously known coronavirus groups.

WO 2004/089983 discloses an isolated essentially mammalian positive-sense single stranded RNA virus (SARS) within the group of coronaviruses and components thereof. Accession Number AY323976 (DATABASE EMBL) discloses the SARS coronavirus ZJ01 isolate spike glycoprotein. Ksiazek et al., 2003 (New England J Med, Vol. 348, pages 1953-1966) discloses a new coronavirus associated with severe acute respiratory syndrome.

There is a growing need for prophylactic or therapeutic vaccines against the SARS virus.

### SUMMARY OF THE INVENTION

The invention provides an immunogenic composition comprising an isolated or purified polypeptide comprising: the Severe Acute Respiratory Syndrome (SARS) virus Spike polypeptide amino acid sequence SEQ ID NO: 6042, or an amino acid sequence having >80% sequence identity to SEQ ID NO: 6042, or a fragment of at least 10 consecutive amino acids of SEQ ID NO: 6042; with the proviso that the polypeptide is not MFIFLLFLTLTSGSDLDRCTTFDDVQAP (as disclosed in WO 2004/089983).

The invention also provides an immunogenic composition comprising an isolated or purified polypeptide comprising amino acid sequence SEQ ID NO: 6042, or an amino acid sequence having >80% sequence identity to SEQ ID NO: 6042, or a fragment of at least 10 consecutive amino acids of SEQ ID NO: 6042; and further comprising an adjuvant. Further embodiments are defined in the appended claims.

### BRIEF DESCRIPTION OF THE DRAWINGS

FIGURE 1: Schematic of coronavirus genome organization.
FIGURE 2: Schematic of coronavirus ORF1a/ORF1b gene products.
FIGURE 3: Alignment of coronavirus polypeptide sequences (including,
FIGURE 4: Alignment of spike (S) polypeptide sequences, taken from Figure 3, in the region of the junction of the S1 and the S2 domains, and protease cleavage site for selected coronaviruses.
FIGURE 5: CDC phylogenetic tree of SARS-CoV strain (Clustalx 1.82, neighbor-joining tree).
Figure 5A shows coronavirus N protein analysis, Figure 5B shows coronavirus S protein analysis, and Figure 5C shows coronavirus M protein analysis.

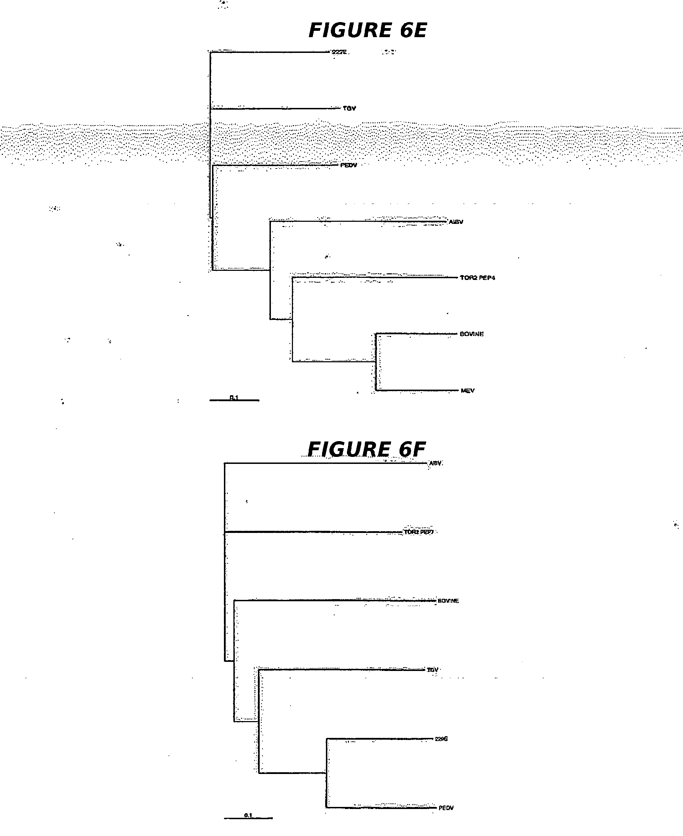

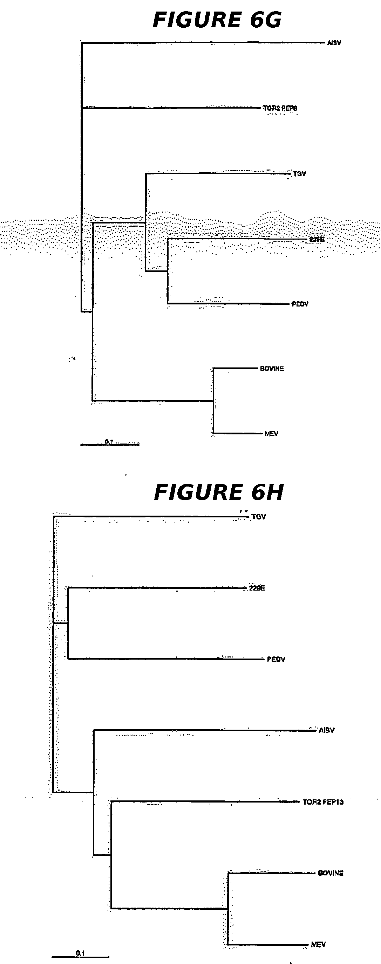

FIGURE 6: Labels 229E: human coronavirus; MEV: murine hepatitis virus; TGV: transmissible gastroenteritis virus; AIBV: avian infectious bronchitis virus; BOVINE: Bovine coronavirus; PEDV: porcine epidemic diarrhea virus.
FIGURE 7: Coiled-coil prediction for SEQ ID NO: 6042, using Coils program (Figure 7A) or LeanCoil (Figure 7B).

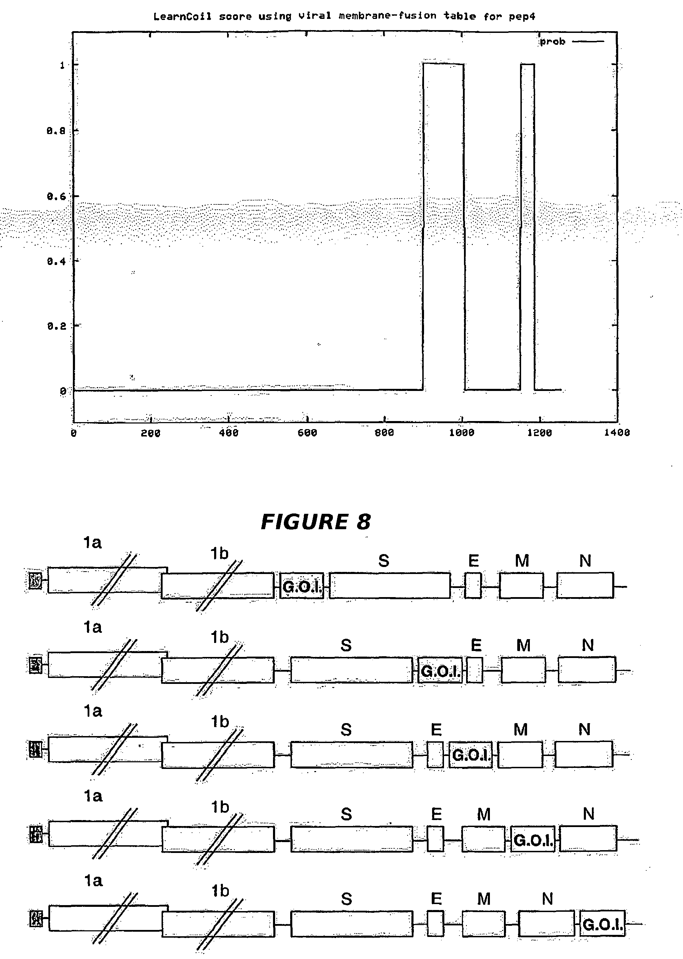

FIGURE 8: Example of insertion of a reporter gene-of-interest at a site between existing SARS virus genes. Small nonstructural gene products are not depicted schematically.

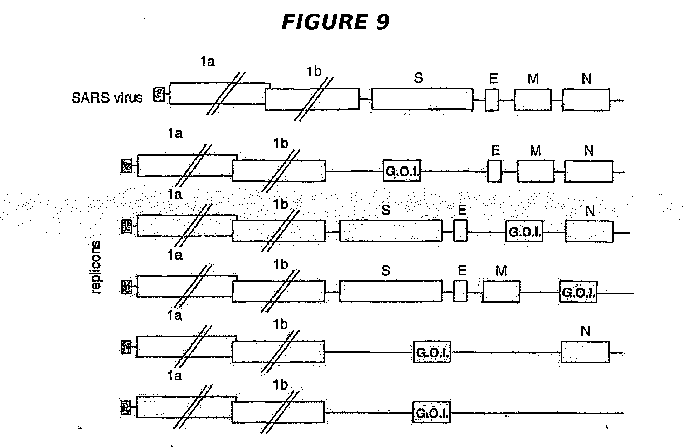

FIGURE 9: Schematic depicting representative examples of SARS virus replicons. Small nonstructural gene products are not depicted schematically.

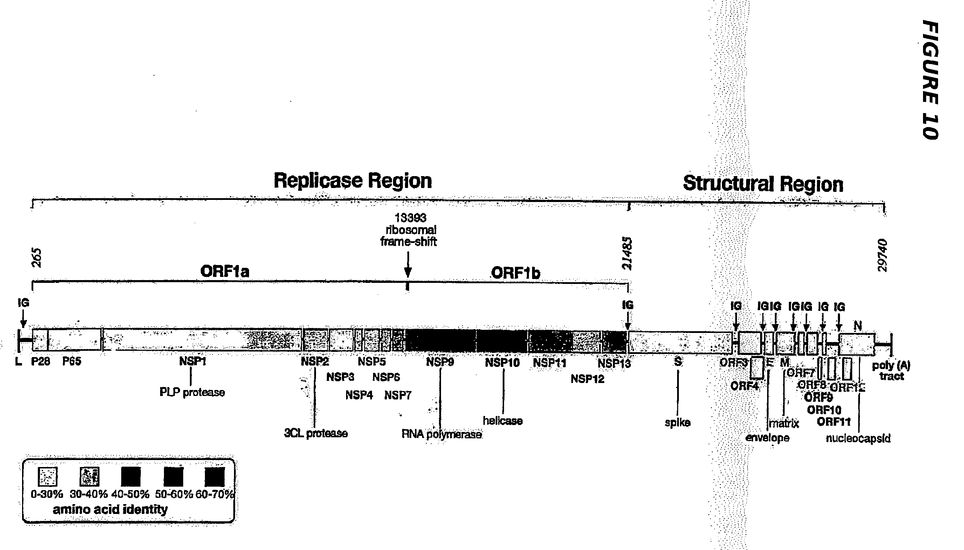

FIGURE 10: Genome organization of SARS coronavirus. Replicase and structural regions are shown, along with the predicted products of cleavage within ORF1a and ORF1b. The position of the 5' RNA leader sequence (L), the 3' poly(A) tract and the ribosomal frame-shift consensus between ORF1a and ORF1b are also indicated. Each box represent a protein product. They are shaded according to the level of amino acid identity with corresponding proteins of other coronaviruses. The SARS-specific genes are white. Positions of the 9 SARS-specific six-base IG sequences (5'-ACGAAC-3') are indicated by arrows.

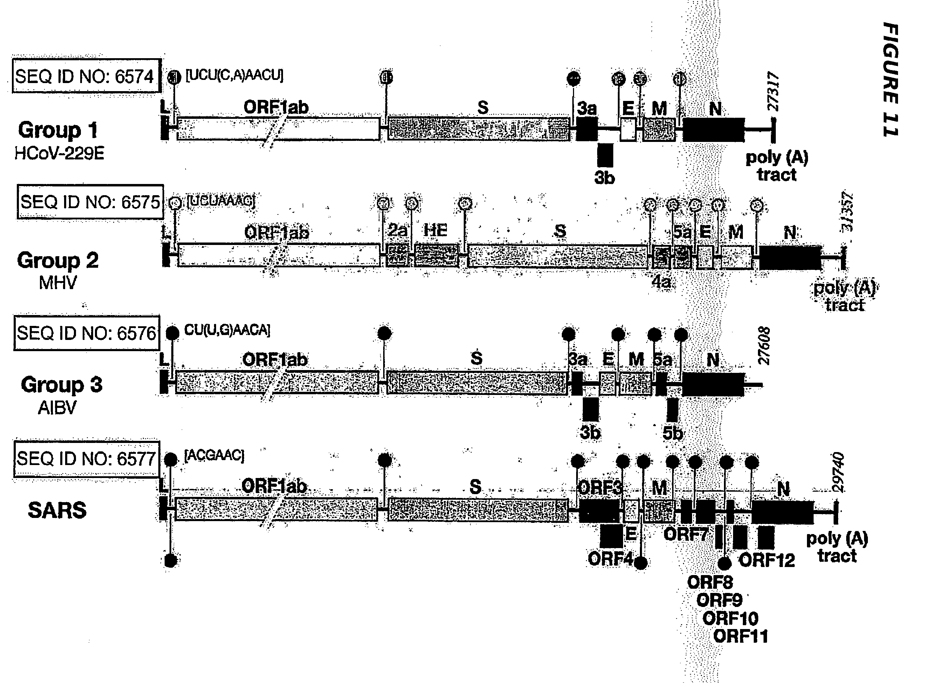

FIGURE 11: Genome organization of Coronaviruses representative of group 1 (HCoV-229E, accession number: AF304460), group 2 (mouse hepatitis virus MHV, accession number: NC_001846), group 3 (avian infectious bronchitis virus AIBV, accession number: NC_001451) and SARS coronavirus. Other completely sequenced coronaviruses used in this study are available at the following accession numbers: porcine epidemic diarrhea virus (PEDV), AF353511; transmissible gastroenteritis virus (TGV), NC_002306; Bovine coronavirus (BCoV): AF220295. Red boxes represent group-specific genes. The position of the leader RNA sequence and poly(A) tract is also indicated in genomes where they are reported. The position of specific IG sequences is indicated by circles of different shades. In the SARS genome, we also find three IG sequences specific for group 2 coronavirus.
FIGURE 12: Topological model predicted for the spike protein anchored to the viral membrane. Structural and predicted functional domains are indicated. The N-terminal region (S1) is predicted to contain the receptor binding domain. Two coiled coil regions within the S2 domain, partially superimposed to leucine zipper motifs are presumably involved in oligomerization. The hydrophobic domain is responsible for membrane anchoring.

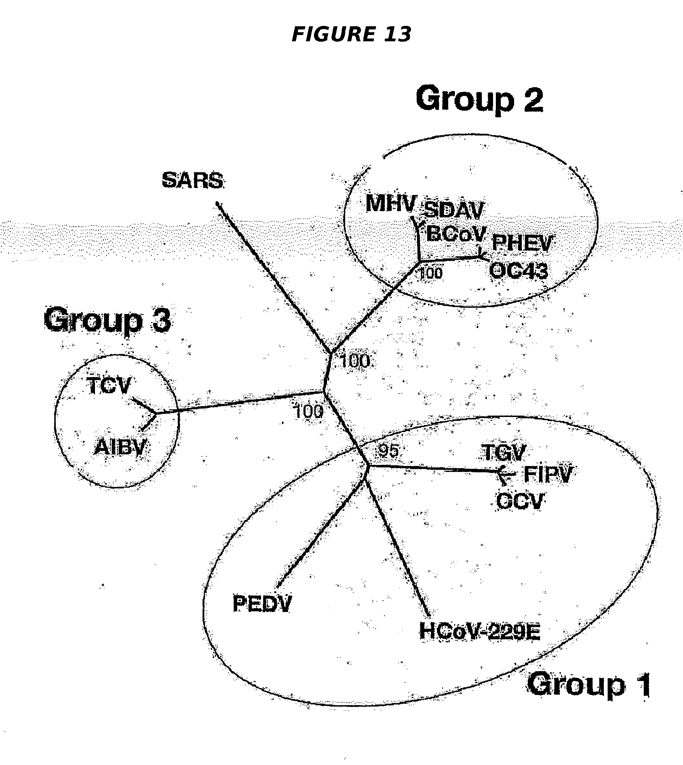

FIGURE 13: Phylogenetic tree obtained from the multiple sequence alignment of a 922 bp internal region of the *pol* gene from 12 coronaviruses and SARS. Numbers at the nodes represent the result of a bootstrap analysis and strongly support the branches. Sequences not available within the complete coronavirus genomes have been retrieved from GenBank at the following accession numbers: hemagglutinating encephalomyelitis virus of swine (PHEV), AF124988, Human OC43 virus (OC43), AF124989, canine coronavirus (CCV), AF124986, feline infectious peritonitis virus (FIPV), AF124987, turkey coronavirus (TCV), AF124991, syaloacryoadenitis virus of rats (SDAV), AF124990.

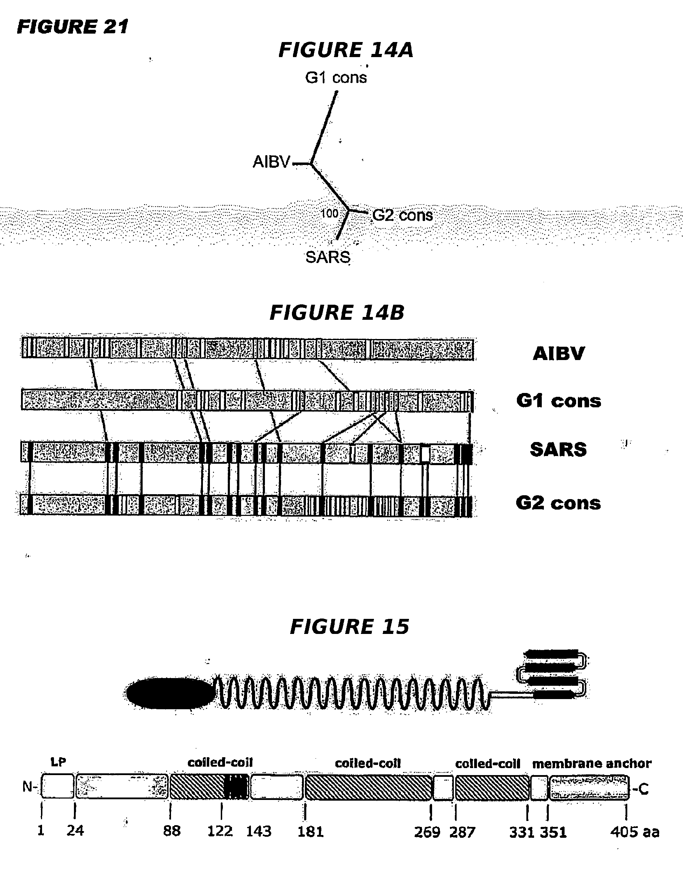

FIGURE 14: 14A. Unrooted tree obtained from the alignment of consensus sequences of the group I and group II S1 domain of spike proteins (G1_cons and G2_cons) with those of a group 3 spike (AIBV) and the spike of SARS virus. The number indicates the result of a bootstrap analysis. The sequences used to generate the consensus profile from group 1 are: HcoV-229E, accession number P15423; porcine epidemic diarrhea virus (PEDV), acc no: NP_598310; transmissible gastroenteritis virus (TGV), acc no: NP_058424; Canine coronavirus (CCV), acc no: S41453; porcine respiratory virus (PRV), acc no: S24284; feline infectious peritonitis virus (FIPV), acc no: VGIH79. The sequences used to generate the consensus profile from group 2 are: mouse hepatitis virus (MHV), acc no: NP_045300; Bovine coronavirus (BCoV), acc no: NP_150077; Human coronavirus OC43, acc no: P36334; hemagglutinating encephalomyelitis virus of swine (PHEV), acc no: AAL80031; for group 3, only the sequence of the spike protein of avian infectious bronchitis virus (AIBV), acc no: AAO34396 was used. 14B: Schematic representation of cysteine positions in S1 domains of group 1,2 and 3, compared to the SARS spike. Horizontal bars represent the S1 amino acid sequences (in the case of SARS and AIBV) or the consensus profiles (generated from group 1, G1_cons, and from group 2, G2_cons). The length of the bars are not to scale. Relative cysteine positions are indicated by rectangle bars. Only cysteines perfectly conserved within each consensus are reported. Lines connect cysteines conserved between the SARS S1 domain and the consensus sequences as shown.
FIGURE 15: illustration of a Neisseria Adhesin A protein (NadA).

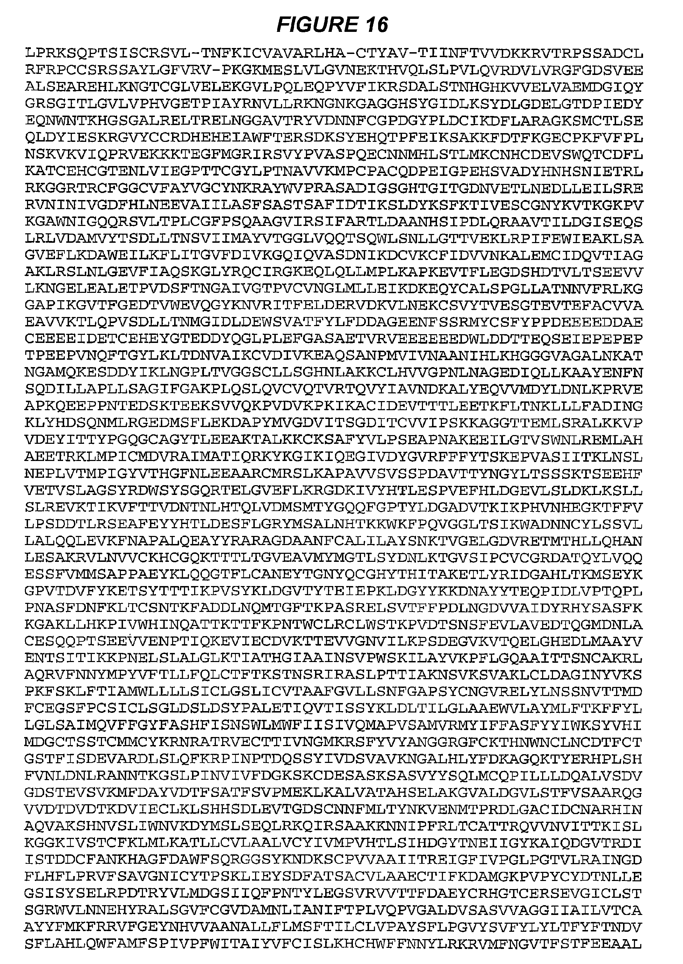

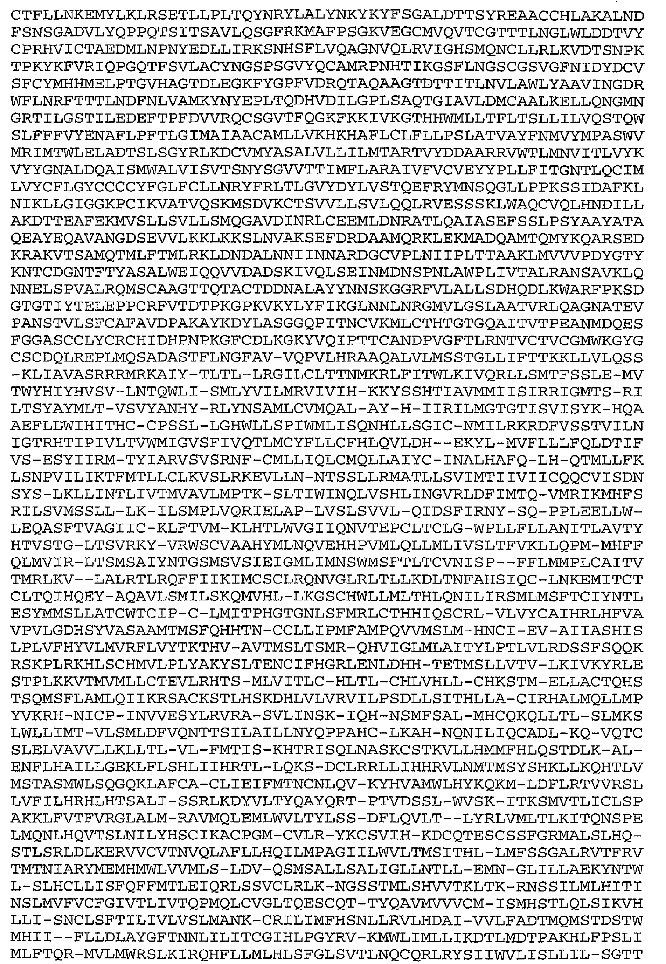

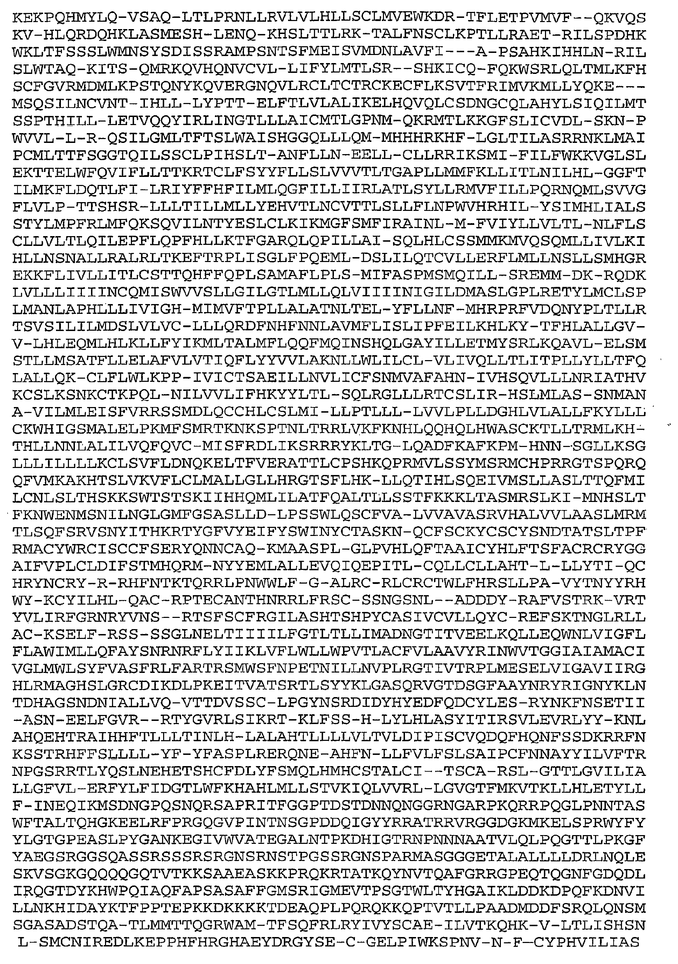

FIGURE 16: Raw translation from SARS coronavirus genome (reading frame +1).

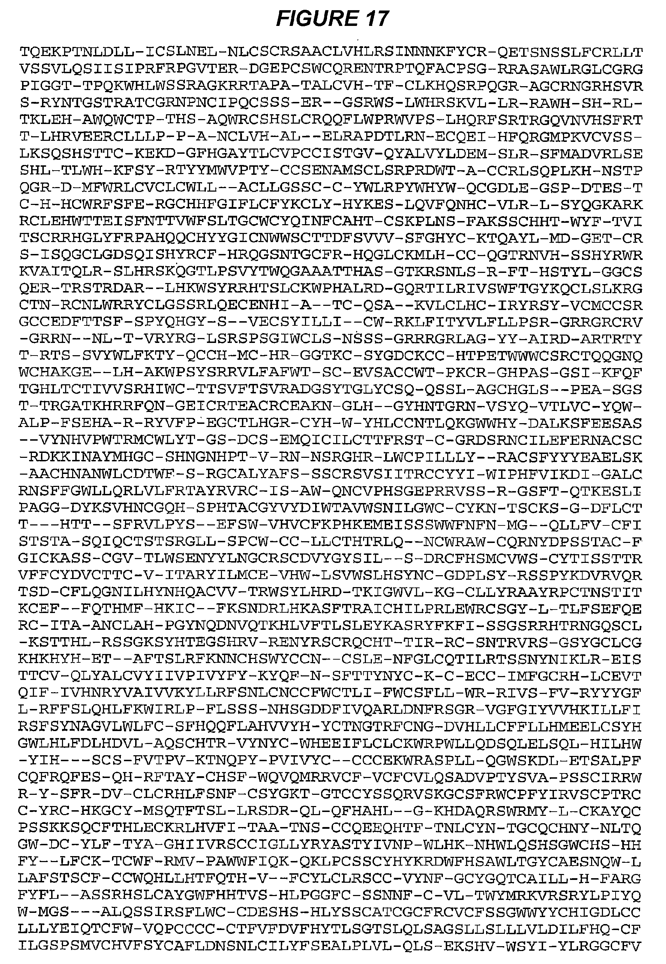

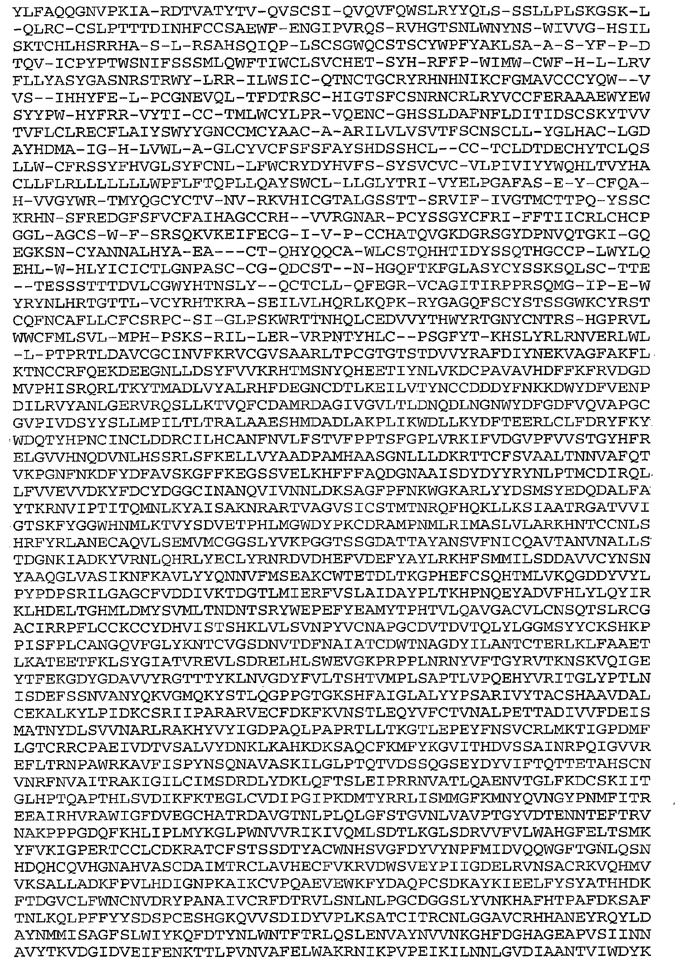

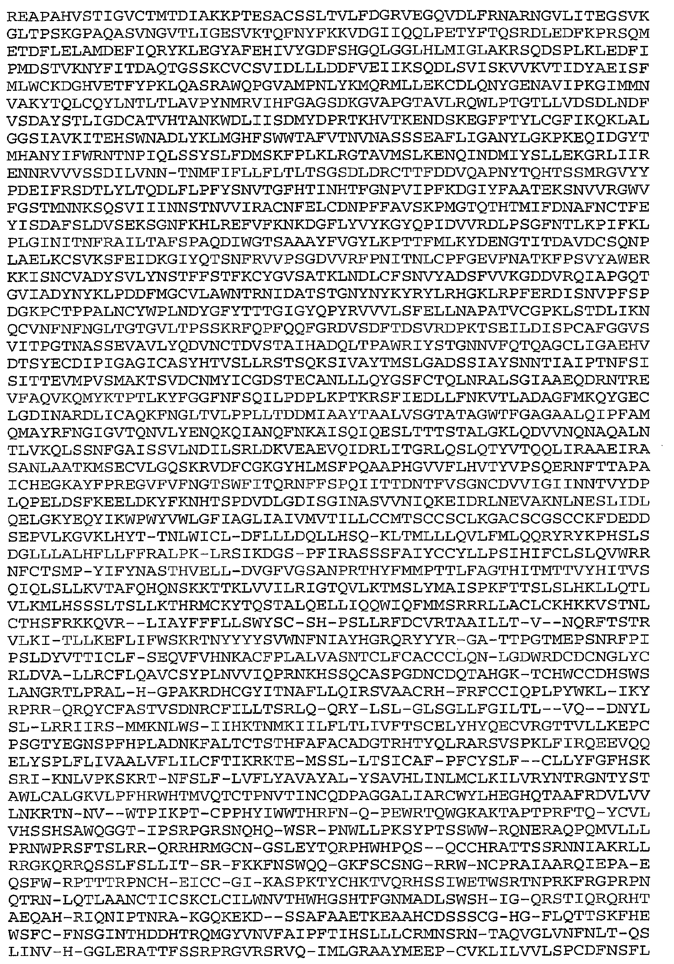

FIGURE 17: Raw translation from SARS coronavirus genome (reading frame +3)
FIGURE 18: 1b and Spike open reading frames, separated by *.

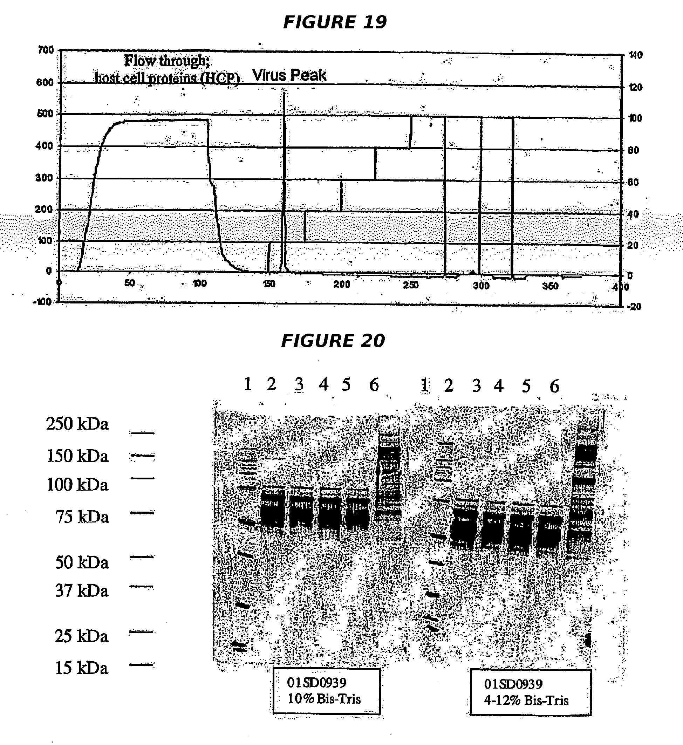

FIGURE 19: chromatogram of the capture step of SARS coronavirus on Matrix Cellufine Sulfate Superformance 150/10. Analysis was on 100ml coronoavirus harvest. The left Y axis shows absorbance at 280nm. The right Y axis shows the gradient (%B). The X axis shows the volume (ml).
FIGURE 20: Silver-stained MCS chromatography fractions. Lanes are: (1) marker; (2) coronavirus vero cell harvest; (3) coronavirus vero cell harvest, after 0.65µm filtration; (4) flowthrough; (5) wash; (6) 20% peak (virus peak). Lanes were loaded with 1 µg of test protein.

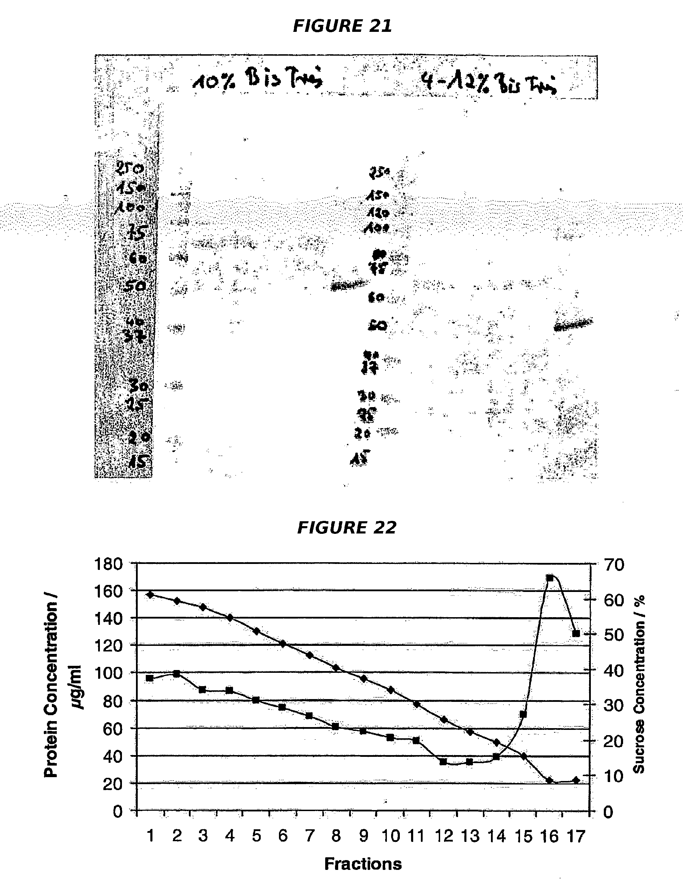

FIGURE 21: Western Blot of MCS chromatography fractions. Lanes are as described for Fig. 20.
FIGURE 22: Linear density gradient ultracentrifugation, 15-60% sucrose (SW28, 2 hours, 20000 rpm). The graph shows protein concentration (■) and sucrose concentration (◆). 23

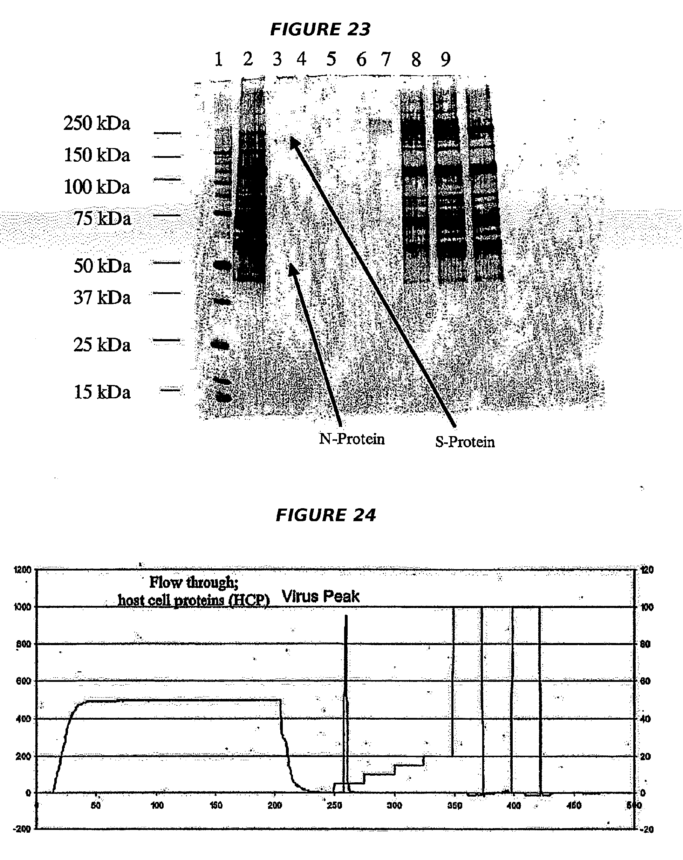

FIGURE 23: Silver-stained density gradient fractions on NuPage 4-12% Bis-Tris-Ge (Novex), reduced conditions, heated for 10 minutes at 70°C. Lanes are: (1) marker; (2) 20% peak MCS; (3) density gradient fraction 11; (4) density gradient fraction 12; (5) density gradient fraction 13; (6) density gradient fraction 14; (7) density gradient fraction 15; (8) density gradient fraction 16; (9) density gradient fraction 17. The bulk of proteins was in fractions 15 to 17. Lanes 2, 8 and 9 were loaded with 1µg protein.
FIGURE 24: Chromatogram of the Capture Step of SARS coronavirus on MCS. Details are as for Figure 19, except that 200ml harvest was used.

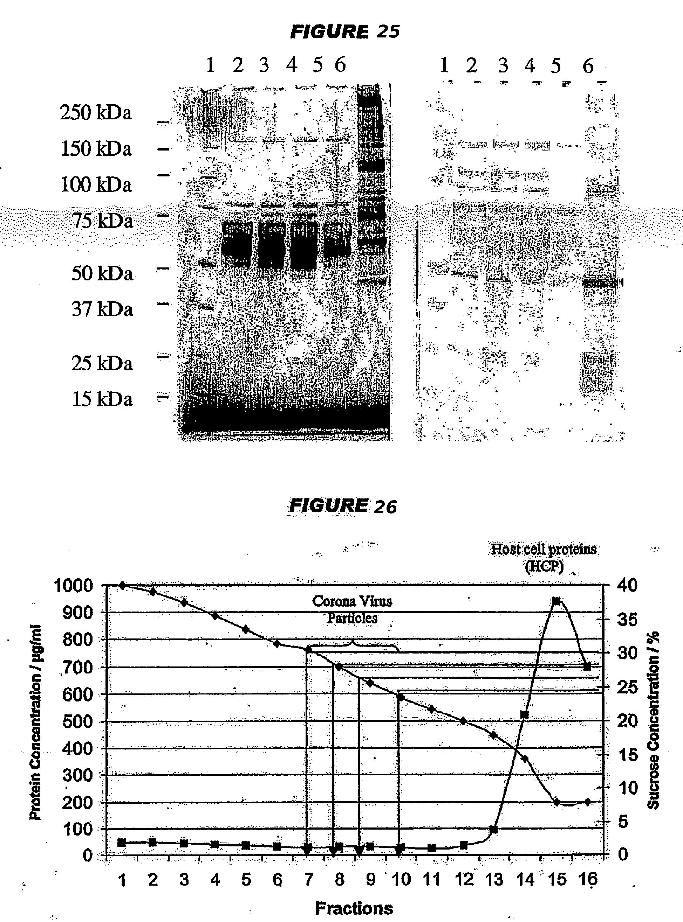

FIGURE 25: Silverstain (left) and Western Blot (right) of chromatographic fractions. Lanes are as described for Figures 20 and 21, except that lane (6) is the 5% peak. Treatment before SDS-PAGE was at room temperature for 30 minutes.
FIGURE 26, Density Gradient Ultracentrifugation, 15-40% sucrose (SW28, 2 hours, 20000 rpm). The graph shows protein concentration (■) and sucrose concentration (◆).

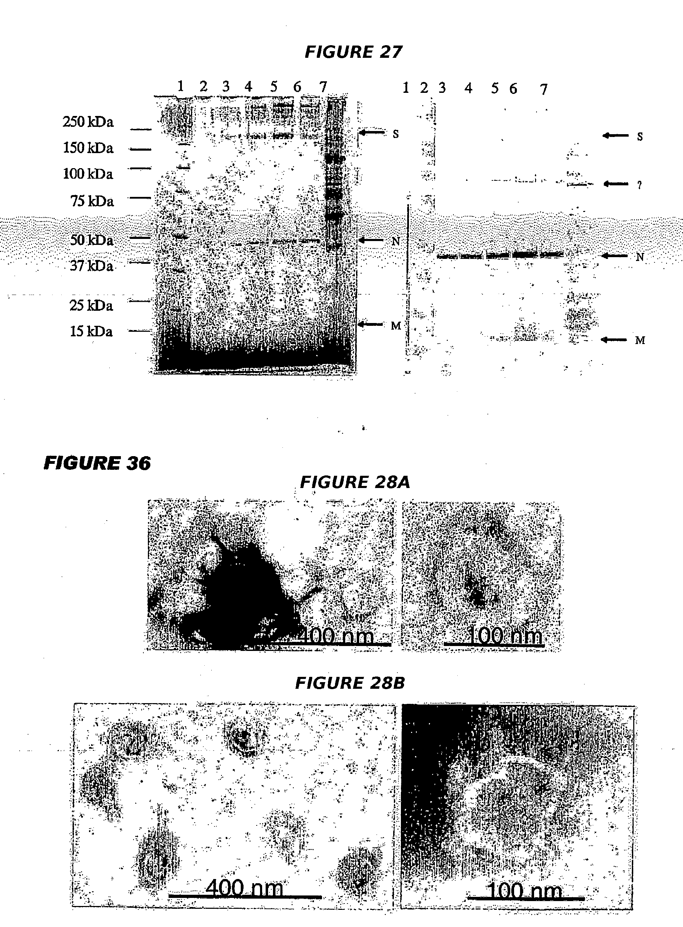

FIGURE 27: Silverstain (left) and Western Blot (right) of Density Gradient Ultracentrifugation fractions on NuPage 4-12% Bis-Tris-Ge (Novex), reduced conditions. Lanes are: (1) marker; (2) density gradient fraction 6; (3) density gradient fraction 7; (4) density gradient fraction 8; (5) density gradient fraction 9; (6) density gradient fraction 10; (7) density gradient fraction 15. Fractions 7-10 (lanes 3-6) contained pure coronavirus proteins. The bulk of impurities was in fraction 15 (lane 7). Lanes 2, 8 and 9 were loaded with ∼1µg protein. Treatment before SDS-PAGE was at room temperature for 30 minutes.

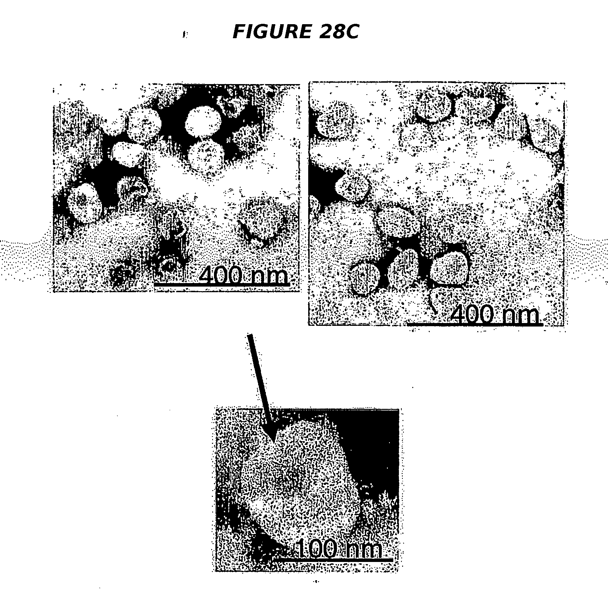

FIGURE 28: EM picture of Density Gradient Fractions 8=10. Figure 28A shows fraction 8; Figure 28B shows fraction 9; Figure 28C shows fraction 10.

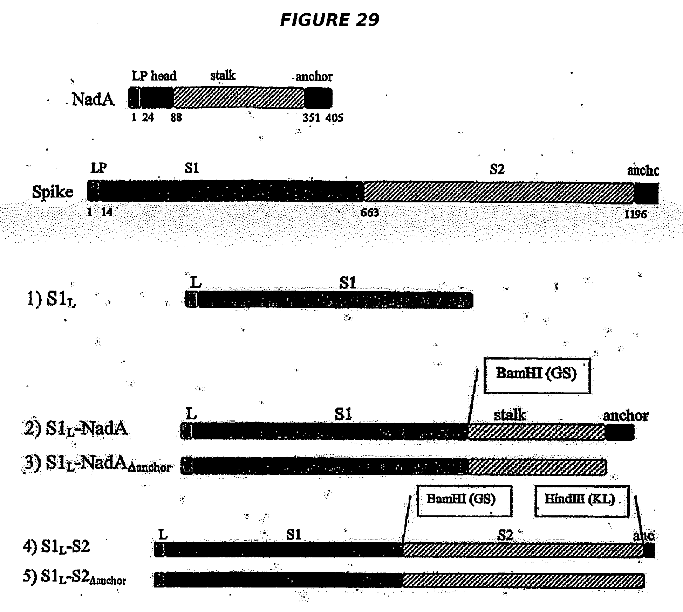

FIGURE 29: Spike/NadA fusion constructs.

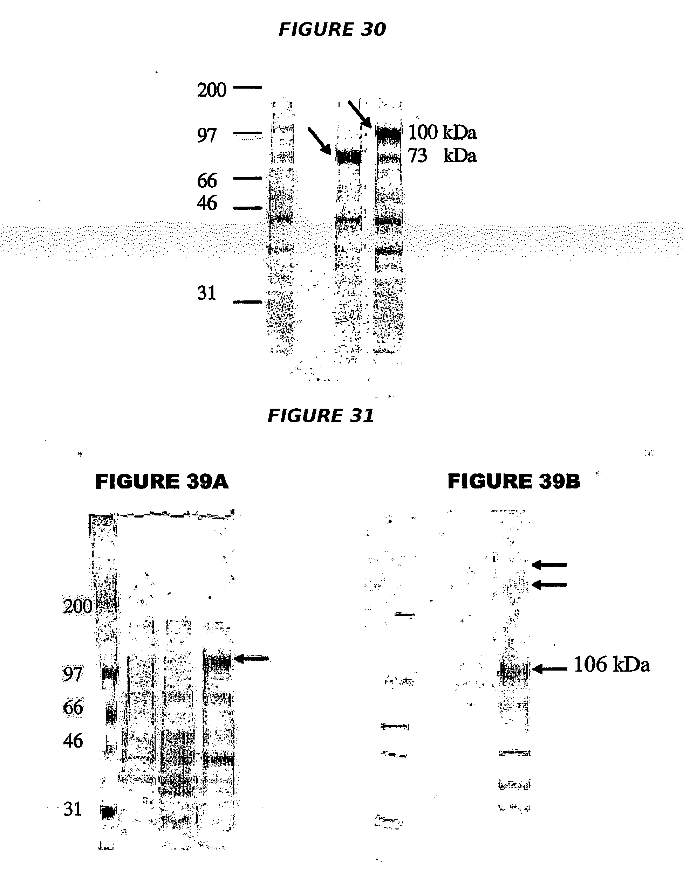

FIGURES 30 and 31: Results of the expression in *E.coli* of S1_{L}, S1_{L}-NadA and S1_{L}-NadΔ_{anchor}. Figure 30 shows SDS-PAGE analysis of total lysates from BL21(DE3)/pET, BL21(DE3)/pETS1_{L} and BL21(DE3)/pET-S1_{L}-Nad_{Δanchor}. The bands are indicated by an arrow, and the three lanes are, from left to right: BL21(DE3)/pET; BL21(DE3)/pET-S1_{L}; BL21(DE3)/pETS1_{L}-NadA_{Δanhor}. Figure 31 shows (39A) SDS-PAGE and (39B) western blot analyses of total lysates from BL21(DE3)/pET, BL21(DE3)/pET-S1_{L}-NadA (grown under un-induced condition) and BL21(DE3)/pET-S1_{L}-NadA (grown under induced condition). The bands are indicated by an arrow, and lanes are, from left to right: BL21(DE3)/pET; BL21(DE3)/pET-S1_{L}-NadA; BL21(DE3)/pET-S1_{L}-NadA. The western blot shows the presence of oligomeric forms of the protein.

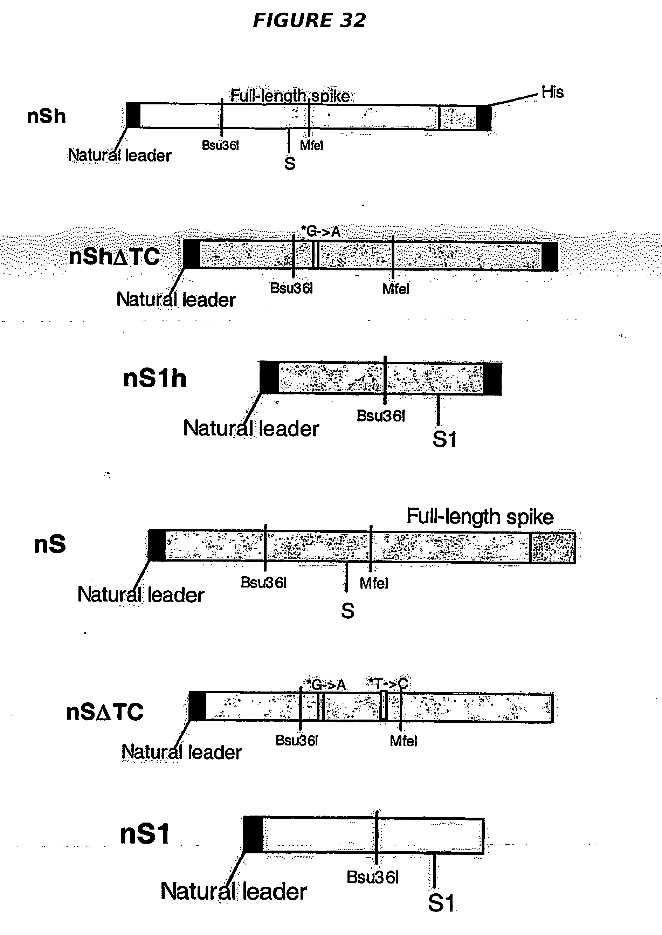

FIGURE 32: Schematic of SARS Spike clones.

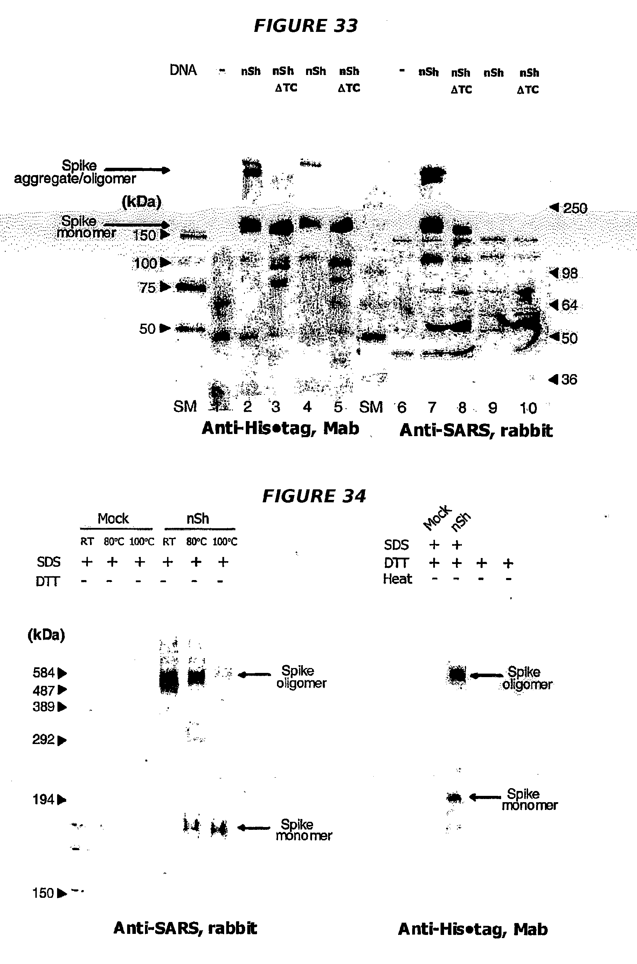

FIGURE 33: Transient Expression of SARS Spike Proteins (western blot of COS7 cell lysate). Each lane of the 4-20% TG SDS gel was loaded with 20µg cell lysate (total 1.2mg). The labeling antibodies are shown.
FIGURE 34: Western blot analyses of COS7 cell lysates on 4% TG SDS gel showing oligomerization state of intracellular S molecules.

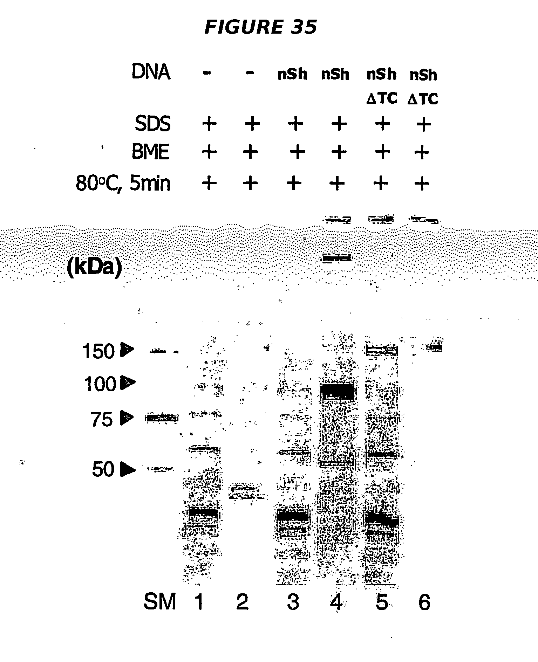

FIGURE 35: Western blot analyses of COS7 cell lysates on 4-20% TG SDS gel showing Transient Expression of SARS Spike Proteins. Lanes are: (1) mock, AF; (2) mock, DF; (3) nSh, AF; (4) nSh, DF; (5) nShΔTC, AF; (6) nShΔTC, DF. Each lane was loaded with 5µl of each sample, 400µl total. The blot was labeled with antibody against the His-tagged protein.

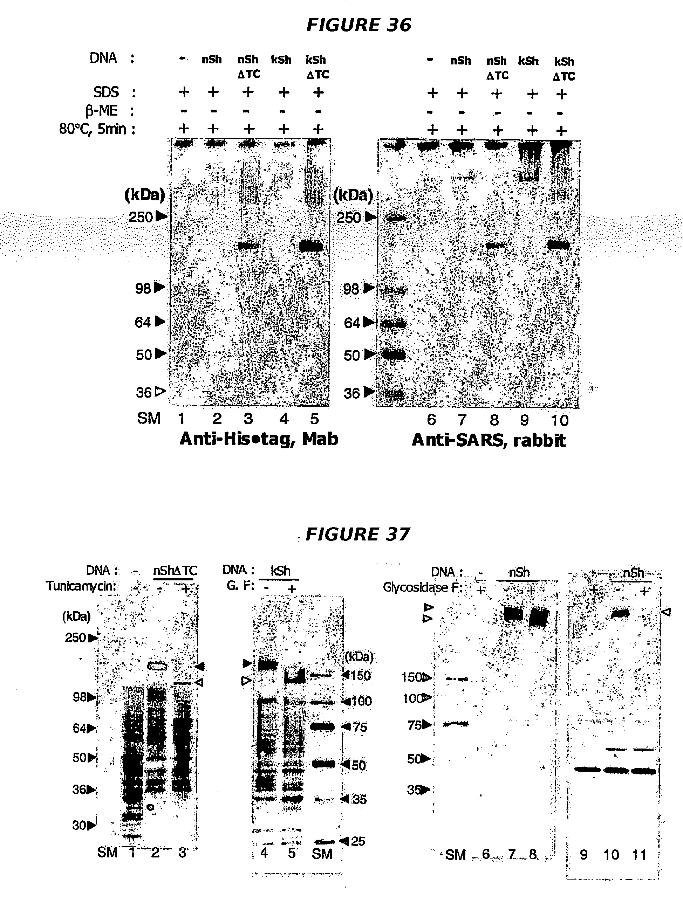

FIGURE 36: Western blot analyses of COS7 cell medium on 4-20% TG SDS gel showing Transient Expression of SARS Spike Proteins. Truncated spike protein is secreted. Spike proteins were purified from the culture medium (from a 10cm plate), first by a ConA column and then finally by His•tag Magnetic beads. Each lane was loaded with one third of the material.
FIGURE 37: Western blot analyses of COS7 cell lysates on 4-20% TG SDS gel showing glycoslation of SARS spike proteins. In the two left-hand blots (lanes 1-5), samples were boiled in SDS and β-mercaptoethanol; in the two right-hand blots (lanes 6-11), samples were in SDS only, with no boiling. Lanes 1-8 were labeled with a monoclonal raised against the His-tag protein; labes 9-11 were labeled with rabbit anti-SARS antibody.

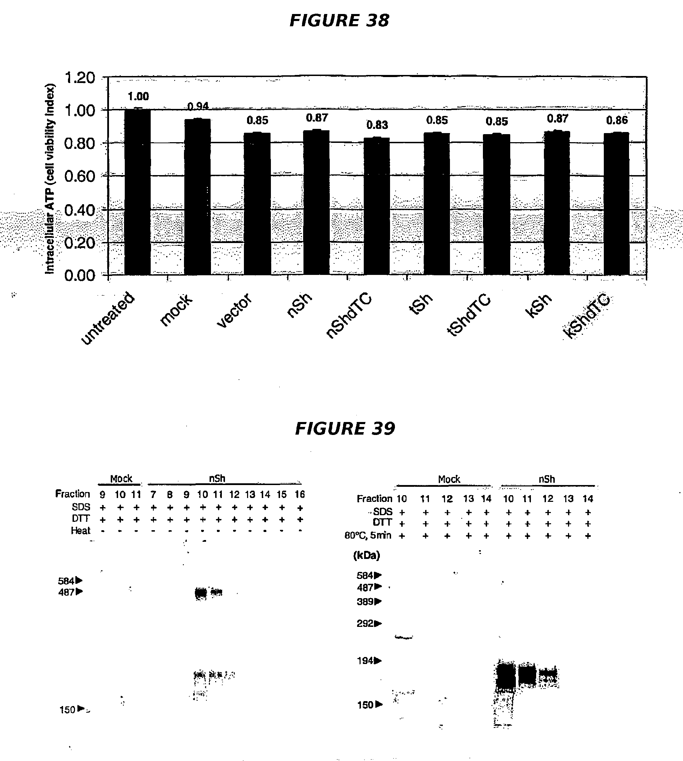

FIGURE 38: Effect of SARS spike protein expression on cell viability.
FIGURE 39: Western blot analyses of COS7 cell lysates on 4% TG SDS gels showing oligomerization state of intracellular spike molecules. Blots were labeled with anti-His-tag mAb.
The membrane fraction of COS7 cell lysate was fractionated by a sizing column before loading the lanes. Fractions 7 to 14 show bands with kDa values of: 71000, 1400, 898, 572, 365,232, 148 and 99, respectively.
FIGURE 40: Fractionation of cells into aqueous and detergent fractions.
FIGURE 41: Schematic of constructs for use in OMV preparation.
FIGURE 42: SARS HR1 and HR2 constructs.
FIGURE 43: Vaccine protection froms SARS in Balb/c mouse model.
FIGURE 44: Expressed on Spike protein in transfected 293 cell lysates (52A) or COS7 cell culture supernatants (44B). Proteins were separated on 4-20% TG SDS gels. The label was anti-His-tag, except for the right-hand three lanes of 44B, where the label was rabbit anti-SARS serum. In Figure 44A, the left-hand three lanes were treated with DTT and were boiled, but neither treatment was used for the right-hand three lanes. In Figure 44B, no DTT was used, but all lanes were heated to 80°C for 5 minutes.
FIGURE 45: Western blot of Spike proteins expressed in COS7 cells. Proteins were incubated at room temperature (RT), 80°C or 100°C to check for any effect on molecular weight. FIGURE 46 shows similar experiments on SARS virions.
FIGURE 47: Results of a pulse chase experiment, showing expression and processing of SARS spike protein following infection with alphavirus replicon particles. Cells were treated with or without EndoH as shown.
FIGURE 48: Effect of heating on Spike protein trimers.
FIGURE 49: Coomassie blue-stained gel of yeast-expressed proteins. Lanes are: 1-SeeBlue Standard (10µl); 2-pAB24 gbl (20µg); 3-SARS Spike S1 c.1 gbl (20µg); 4-SARS Spike S1 c.2 gbl (20µg); 5-See Blue Standard (10µl); 6-pAB24 ip (5µl); 7-SARS Spike S1 c.1 (5µl); 8-SARS Spike S1 c.2 (5µl).
FIGURES 50 to 56: Schematics of preparation of yeast expression constructs.
FIGURES 57 to 58: Yeast-expressed sequences for Spike.

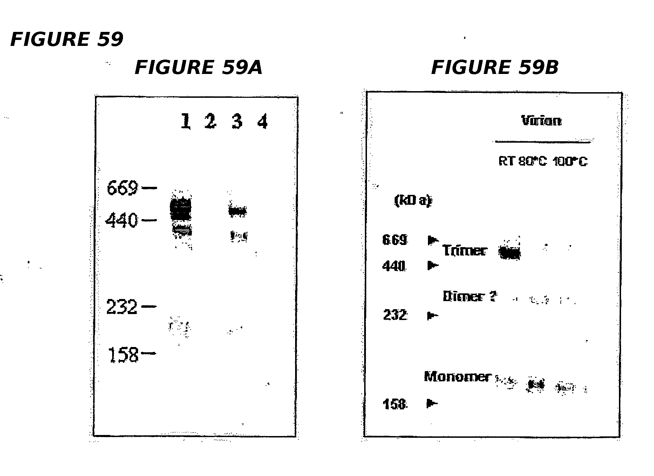

FIGURE 59: Western blots showing expression of SARS spike protein from alphavirus replicon particles and replicon RNA. Figure 59A was run under non-reducing conditions and at room temperature (*i.e.* no heating), with lanes: (1) VEE/SIN-spike infection; (2) VEE/SIN-GFP infection; (3) Replicon-spike RNA transfection; (4) Replicon-GFP RNA transfection. Figure 51B was run with SARS virions at different temperatures, as shown.
FIGURE 60: induction of antibody responses in mice. Vaccine groups are: (1) Inactivated SARS Virus; (2) Truncated Recombinant Spike Protein; (3) Full length Spike: DNA+DNA:PLG+ Alphavirus; (4) full Spike: Alphavirus particles only.
FIGURE 61: Binding of human monoclonal antibody S3.2 to purified truncated Spike protein. The X-axis shows antibody concentration, and the Y-axis shows ELISA absorbance. The interpolation result is 2158.13.
FIGURE 62: Geometric mean ELISA titers of antibodies induced by the SARS-CoV spike protein delivered as different vaccines (left to right: inactivated virus; 3µg truncated spike protein; 75µg DNA encoding truncated spike protein.
FIGURE 63: Neutralization titers after immunization with (left) nSdΔTC protein or (right) DNA encoding nSdΔTC, delivered on PLG.
FIGURE 64: Correlation between the spike antigen binding and neutralizing antibodies
FIGURE 65: Western blot of CHO cell lines expressing Spike protein in full-length form (left) or in truncated form (right). Proteins were separated by 4-12% SDS-PAGE, with boiling in DTT and staining by polyclonal serum.
FIGURE 66: Structural components of SARS-CoV spike glycoprotein and expression construct. L denotes leader peptide (residues 1-13), TM the transmembrane, and Cy the cytoplasmic tail segments. The hexa-His tags are not shown.

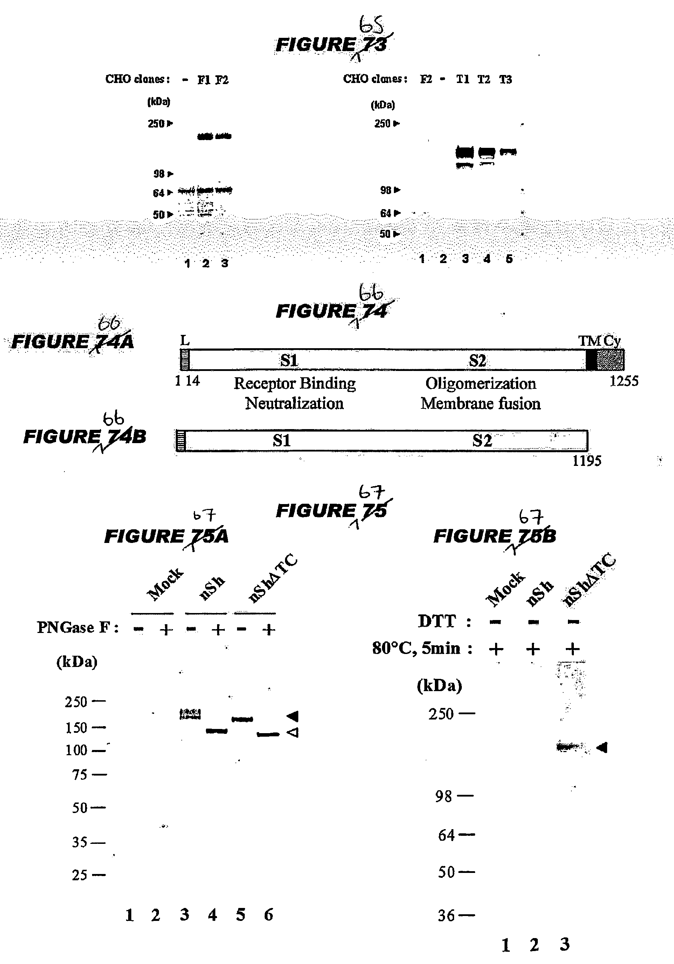

FIGURE 67: Western blot analysis of SARS spike proteins expressed in COS7 cells. In Figure 67A, COS7 cells were transfected with indicated plasmid constructs and the expressed proteins in cell lysates 48 hr post-transfection were analysed by SDS-PAGE (4-20% polyacrylamide) in reducing and denaturing condtions, with proteins visualized by anti-histidine Mab. In Figure 67B, proteins were collected from cell culture medium 48 hr post-transfection and purified first by a ConA column and then by His-tag magnetic beads. Purifed proteins were analysed by SDS-PAGE (4-20% polyacrylamide) and were visualized by anti-SARS rabbit serum.
FIGURE 68: Endo H sensitivity of C-terminal truncated spike protein (SΔ) found in cell lysate (lanes 1,2) and culture medium (lanes 3,4). Positions of internal SΔ protein and secreted SΔ protein are marked with arrow heads.
FIGURE 69: Oligomeric status of the SARS spike protein. Recombinant S protein oligomer in COS7 cells transfected with the full-length spike construct (nSh). The cell lysates were treated with DTT and/or heat as indicated above each lane. The different forms of S protein in treated and untreated samples were visualized by SDS-PAGE (4% polyacrylamide) and Western blot analysis using anti-histidine MAb.
FIGURE 70: Effect of heat denaturation on the oligomeric status of recombinant S protein in the absence of DTT. The COS7 cell lysates were heated before the electrophoresis as indicated and the S proteins were visualized as described fogi Figure 69.
FIGURE 71: Effect of heat denaturation on the oligomeric status of spike protein in SARS virion particles. SARS-CoV were grown in Vero cells, purified and solubilized from the virion particles by SDS, heat-denatured as indicated and visualized as described in Figure 69, except that rabbit antiserum against the purified virus was used as a probe.
FIGURE 72: Analysis of the oligomeric status of SARS virion spike protein by cross-linking experiment. Solubilized SARS virion proteins were treated with DMS. Both untreated (-) and DMS treated (+) virion proteins were heat denatured in the absence of DTT and visualized by 4% PAGE followed by silver staining.
FIGURES 73 & 74: Analysis of the oligomeric status of truncated spike protein by heat denaturation. Truncated spike protein within COS7 cell lysates (73) or secreted into culture medium (74) were heat denatured as indicated in the absence of DTT and visualized by Western blot analysis.
FIGURE 75: Reactivity of deglycosylated full-length spike oligomer with conformational and non-conformational antibody. The full-length recombinant spike oligomer was partially deglycosylated with PNGase F in non denaturating condition and visualized by Western blot analysis using anti-histidine Mab (lane 1,2,3) or rabbit antiserum against purified SARS CoV (lane 4,5,6).

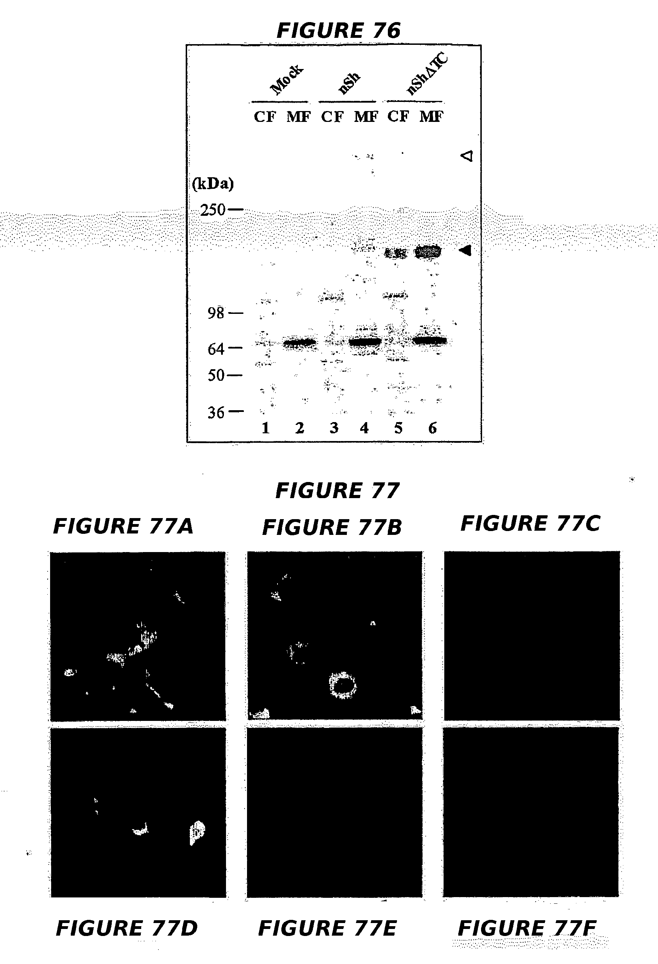

FIGURE 76: Localization of expressed SARS spike proteins in fractionated COS7 cell lysate visualized by western blot. Cells were transfected with indicated plasmids and lysed with Dounce homogeniser in hypotonic buffer 48 hr post transfection. Cell lysate was centrifuged to obtain soluble cytosol and insoluble membrane fraction that was further solublized by 4% Triton X-100. Proteins were heated with SDS at 80 C and analysed by SDS-PAGE (4-20% polyacrylamide) in reducing condtion. Proteins were visualized by anti-histidine Mab. The cytosol fractions were loaded in lanes 1, 3, and 5 and the membrane fractions were loaded in lanes 2, 4, and 6.
FIGURE 77: Intracellular and surface expression of recombinant full-length (A,D) or truncated (B,E) spike protein in COS7 cells. The cells were fixed at 48 hrs posttransfection and either treated with detergent (Cytofix/perm, BD Biosciences) for intracellular immunofluorescence (A,B,C) or with 2% paraformaldehyde for cell surface immunofluorescence observation (D,E,F) at x40 magnifcation. Mock transfected cells (C,F) were included as controls.
FIGURES 78-97: SDS-PAGE od *E.coli* expressed proteins. Tot = total protein; Sol = soluble protein fraction. Labels are protein names (Tables 26-30).
FIGURE 98. Immunofluorescence after administration of vector encoding optimsed N antigen.
FIGURE 99: Immunofluorescence of (A) native and (B) codon-optimsed M sequences.
FIGURE 100: Immunofluorescence of (A) native and (B) codon optimsed E sequences.
FIGURES 101-103: Western blots of Vero cells using rabbit antibodies obtained after immunization with spike proteins expressed in *E.coli.*
FIGURE 104: Spike protein expression in 293 cells. Lanes: (M) Markers; (1) Mock transfected; (2,6) cells expressing nS protein, lysate; (3,7) cells expressing nSdTC protein, lysate; (4,8) cells expressing nS protein, supernatant; (5,9) (4) cells expressing nSdTC protein, supernatant. Staining antibody: (2 to 5) mouse serum obtained after DNA immunization; (6 to 9) rabbit serum obtained after immunization with whole killed virus.

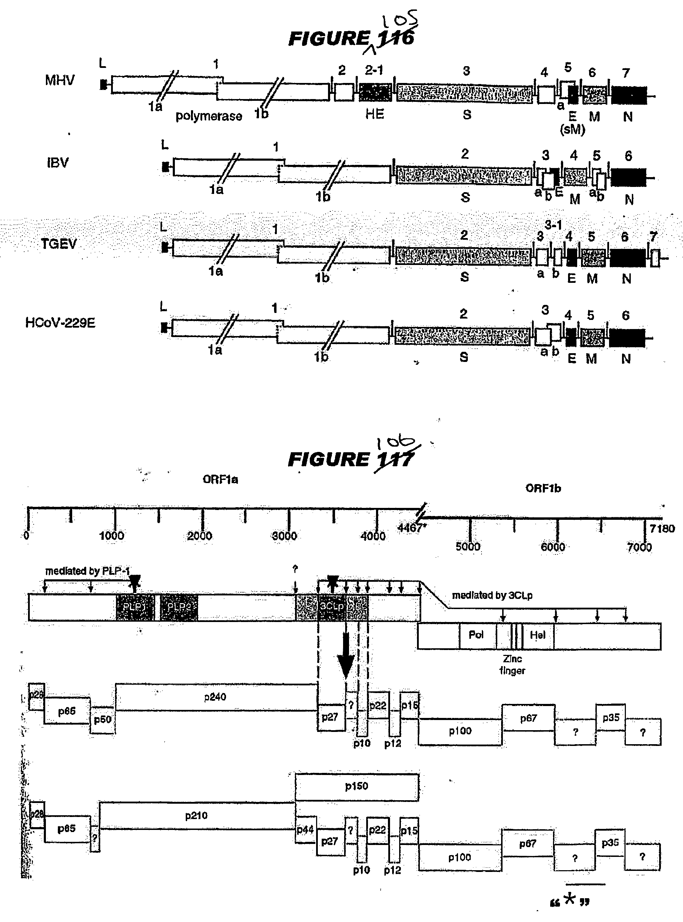

FIGURE 105: Schematic of coronavirus genome organization.
FIGURE 106: Schematic of coronavirus ORF1a/ORF1b gene products, including "*" region.

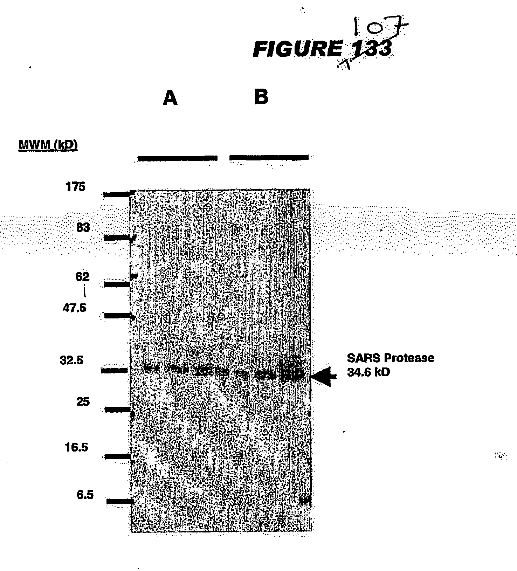

FIGURE 107: Western Blot of SARS protease purification fractions.

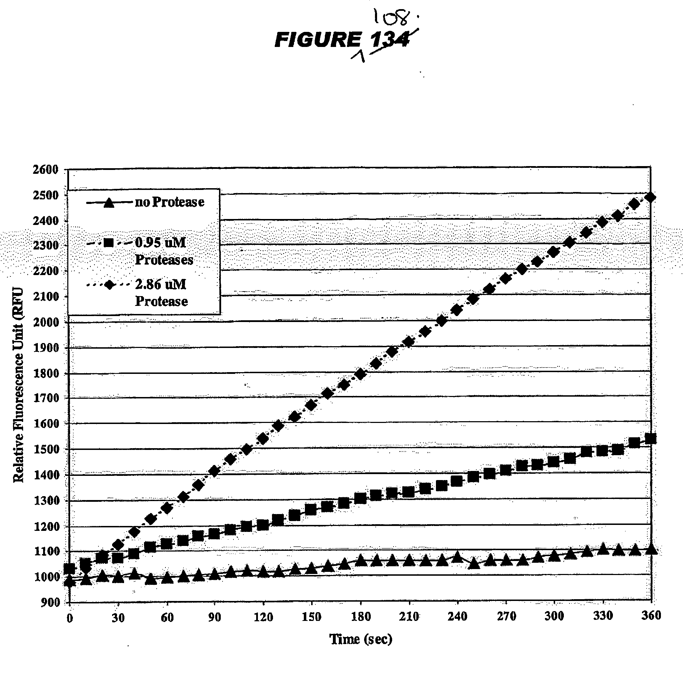

FIGURE 108: Cleavage of DABCYL-EDANS (a fluorescent tagged peptide with a SARS protease cleavage site) by SARS protease at different concentrations. The graph shows activity/concentration correlations with no protease (◆), 0.95 uM protease (■) and 2.86 uM protease (●).

In the event of a discrepancy between a sequence in the sequence listing and a sequence in the drawings, the drawings should take precedence.

### DETAILED DESCRIPTION OF THE INVENTION

The practice of the present invention will employ, unless otherwise indicated, conventional methods of chemistry, biochemistry, molecular biology, immunology and pharmacology, within the skill of the art. Such techniques are explained fully in the literature. See, *e.g.,* Remington's Pharmaceutical Sciences, Mack Publishing Company, Easton, Pa., 19th Edition (1995); Methods In Enzymology (S. Colowick and N. Kaplan, eds., Academic Press, Inc.); and Handbook of Experimental Immunology, Vols. I-IV (D.M. Weir and C.C. Blackwell, eds., 1986, Blackwell Scientific Publications); Sambrook, et al., Molecular Cloning: A Laboratory Manual (2nd Edition, 1989); Handbook of Surface and Colloidal Chemistry (Birdi, K.S. ed., CRC Press, 1997); Short Protocols in Molecular Biology, 4th ed. (Ausubel et al. eds., 1999, John Wiley & Sons); Molecular Biology Techniques: An Intensive Laboratory Course, (Ream et al., eds., 1998, Academic Press); PCR (Introduction to Biotechniques Series), 2nd ed. (Newton & Graham eds., 1997, Springer Verlag); Peters and Dalrymple, Fields Virology (2d ed), Fields et al. (eds.), B.N. Raven Press, New York, NY.

Severe Acute Respiratory Syndrome (SARS) virus has recently been identified as a new viral species. The SARS viral species includes the following isolates.
- two virus isolates described in Peiris et al. "Coronavirus as a possible cause of severe acute respiratory syndrome" Lancet published online at http://image.thelancet.com/extras/03art3477web.pdf on April 8 2003, and the sequences deposited with GenBank at accession number AY268070.
- the isolates and viral sequences described in Drosten et al., "Identification of a Novel Coronavirus in Patients with Severe Acute Respiratory Syndrome", New England Journal of Medicine, published online at http://www.nejm.org on April 10, 2003.
- the isolates and viral sequences described on the website of the WHO network on March 25 and 24, 2003.
- the isolates and viral sequences described in Tsang et al., "A Cluster of Cases of Severe Acute Respiratory Syndrome in Hong Kong" New England Journal of Medicine, published online at http://www.nejm.org on March 31,2003.
- the isolates and viral sequences described in Poutanen et al., "Identification of Severe Acute Respiratory Syndrome in Canada" New England Journal of Medicine, published online at http://www.nejm.org on March 31, 2003.

As described in the *Lancet* article, a 646 base pair polynucleotide from the SARS virus has weak homology to viruses of the family *Cornoaviridae.* The *Lancet* article further reports that a deduced amino acid sequence (of 215 amino acids) from this sequence has about 57% sequence homology to the RNA polymerase of bovine coronavirus and murine hepatitis virus. Phylogenetic analysis of the protein sequences are also presented in the *Lancet* article showing that the polymerase sequence is most closely related to the group II coronaviruses.

Additional SARS viral isolates can be identified, isolated and/or sequenced by virologists skilled in the art. Virologists can readily identify new viral isolates as a SARS virus. Criteria which a virologist may use to identify new SARS isolates include: sequence homology of the new isolate to known SARS viral isolates; similar genomic organization of the new viral isolate to known SARS viral isolates; immunological (serologic) similarity or identity with known SARS viral isolates; pathology; and similarity of virion morphology with known SARS viral isolates; and similarity of infected cell morphology as that caused by known SARS viral isolates (visualized, for instance, by electron microscopy).

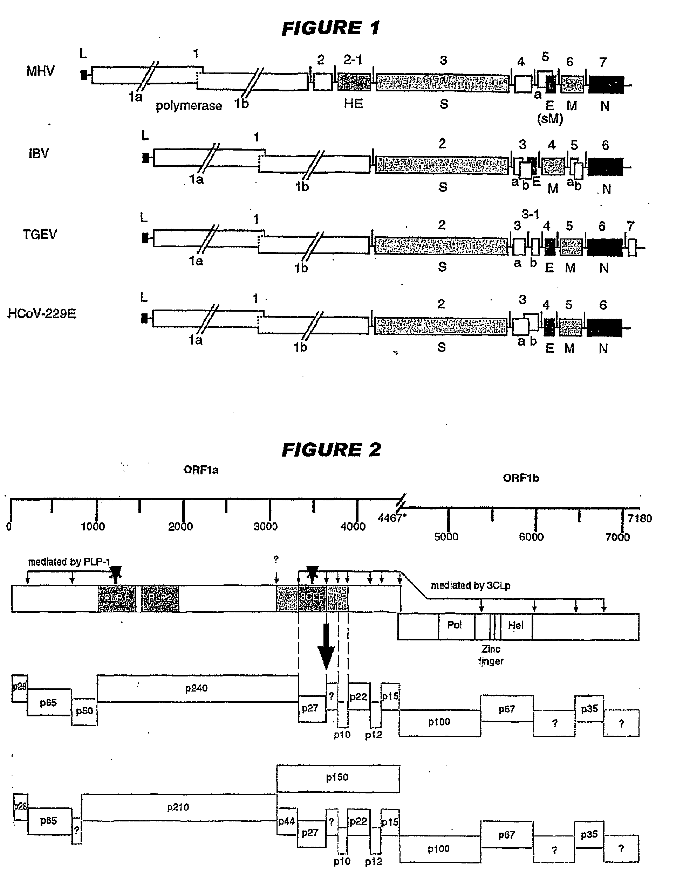

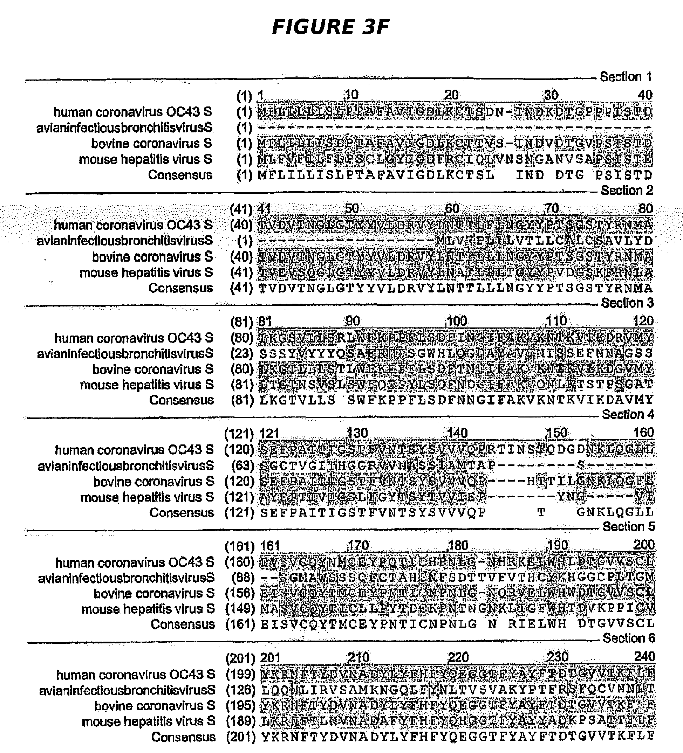

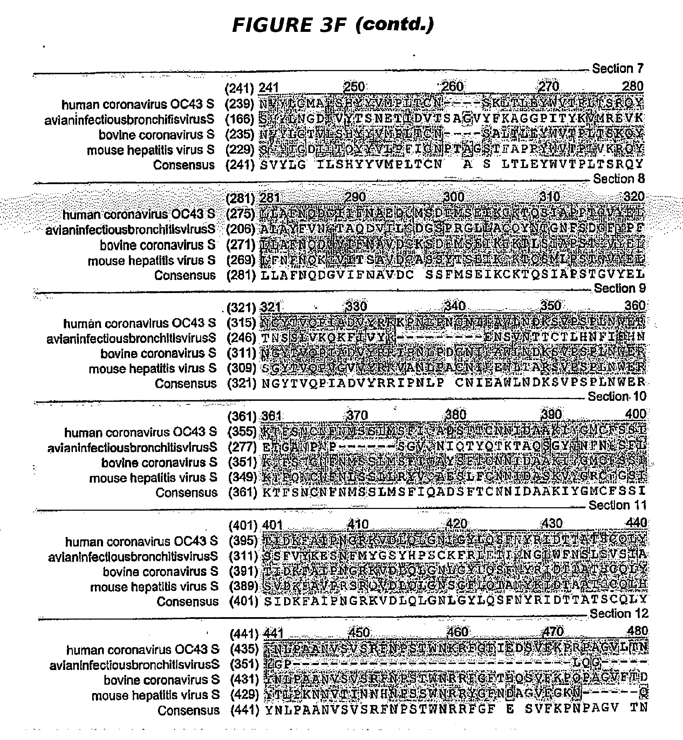

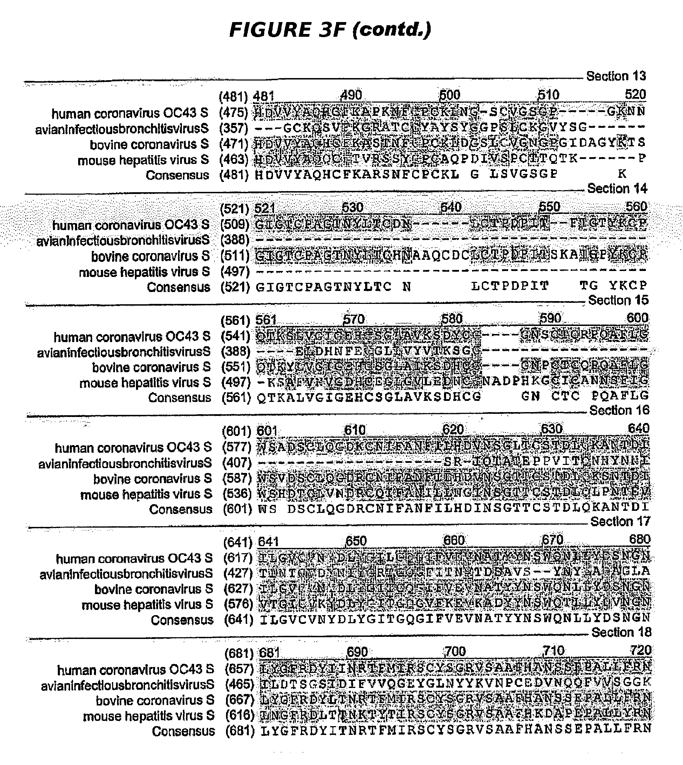

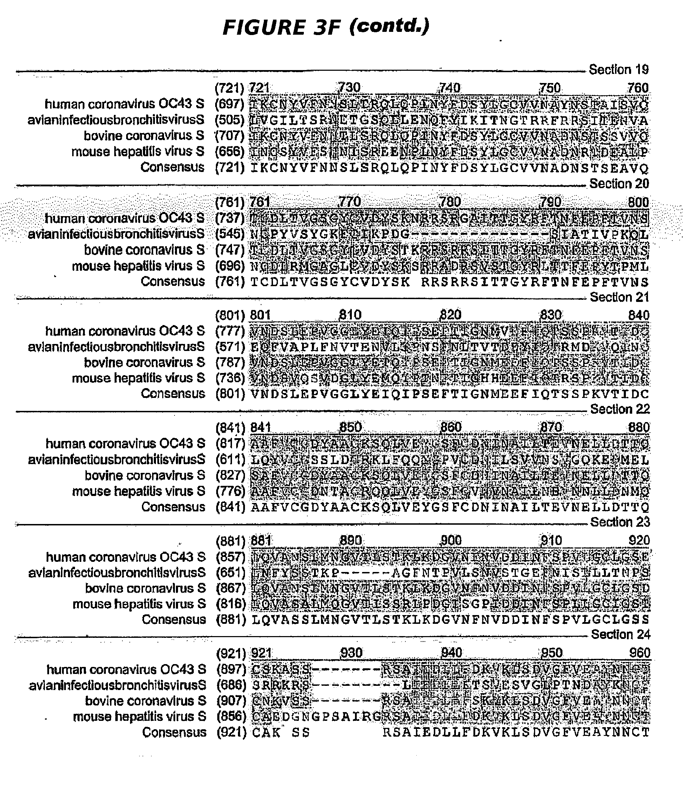

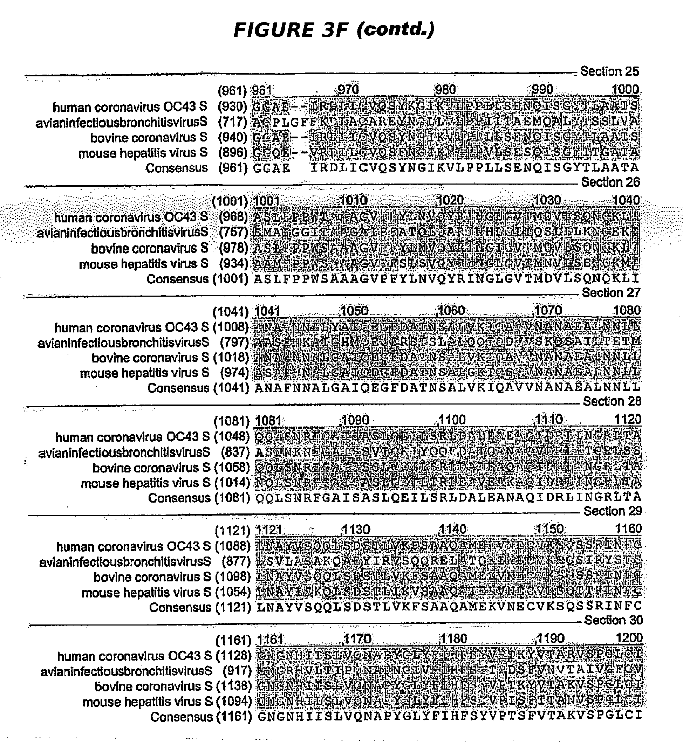

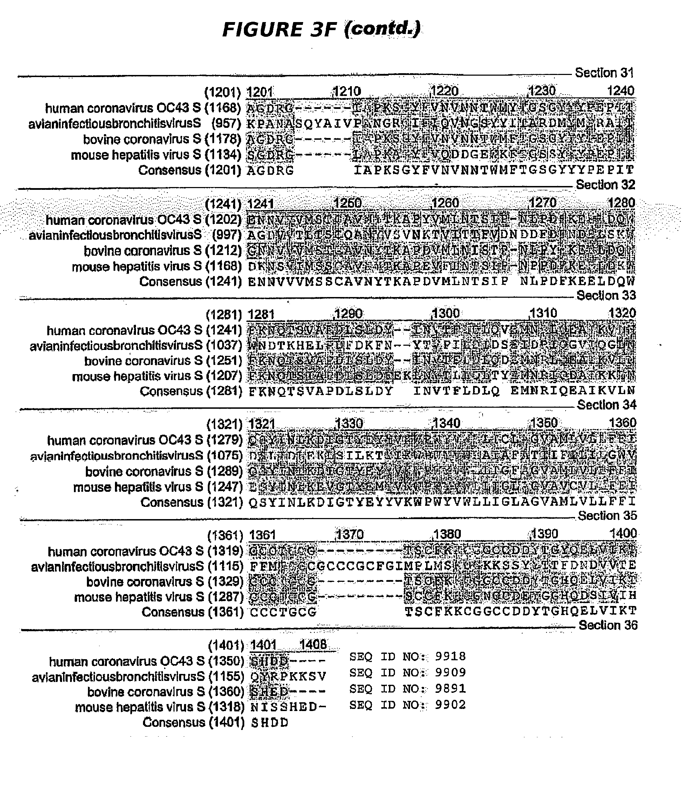

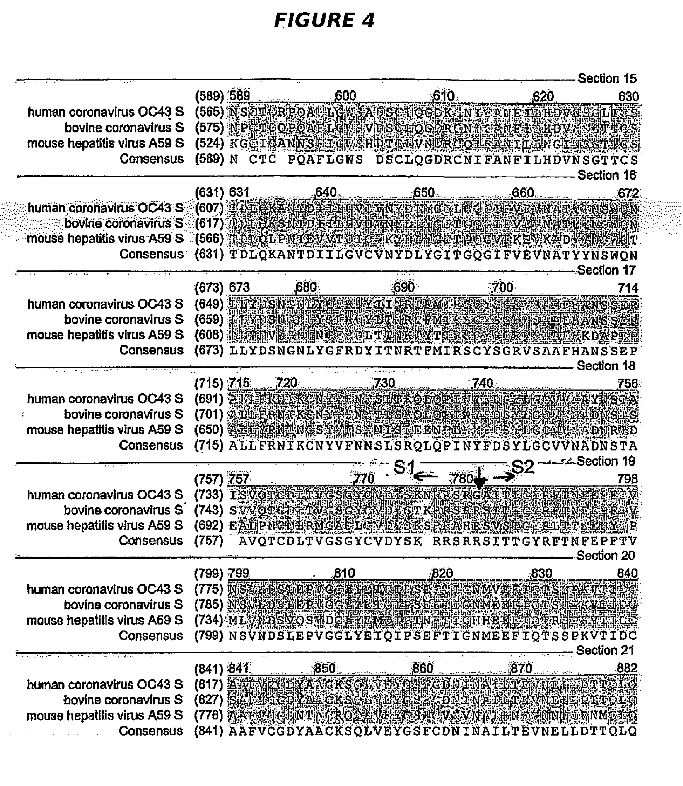

New SARS isolates may also be identified by a percent homology of 99%, 95%, 92%, 90%, 85%, or 80% homology of the polynucleotide sequence for specific genomic regions for the new virus with the polynucleotide sequence for specific genomic regions of the known SARS viruses. Additionally, new SARS isolates may be identified by a percent homology of 99%, 95%, 92%, 90%, 85%, or 80% homology of the polypeptide sequence encoded by the polynucleotide of specific genomic regions of the new SARS virus to the polypeptide sequence encoded by the polynucleotides of specific regions of the known SARS virus. These genomic regions may include regions (*e.g*., gene products) which are typically in common among numerous coronaviruses, as well as group specific regions (*e.g*., antigenic groups), such as, for example, any one of the following genomic regions which could be readily identified by a virologist skilled in the art: 5'untranslated region (UTR), leader sequence, ORF1a, ORF1b, nonstructural protein 2 (NS2), hemagglutinin-esterase glycoprotein (HE) (also referred to as E3), spike glycoprotein (S) (also referred to as E2), ORF3a, ORF3b, ORF3x, nonstructural protein 4 (NS4), envelope (small membrane) protein (E) (also referred to as sM), membrane glycoprotein (M) (also referred to as E1), ORF5a, ORF5b, nucleocapsid phosphoprotein (N), ORF7a, ORF7b, intergenic sequences, 3'UTR, or RNA dependent RNA polymerase (pol). The SARS virus may have identifiable genomic regions with one or more the above-identified genomic regions. A SARS Viral antigen includes a protein encoded by any one of these genomic regions. A SARS viral antigen may be a protein or a fragment thereof, which is highly conserved With coronaviruses. A SARS viral antigen may be a protein or fragment thereof, which is specific to the SARS virus (as compared to known cornaviruses). (See, Figures 1-4).

One skilled in the art could also recognize electron microscopy of a SARS virus infected mammalian cell. Electron microscopy of SARS infected cells are shown in the *Lancet* paper. As discussed in the paper, electron microscopy of negative stained (3% potassium phosphotungstate, pH 7.0) ultracentrifuged cell-culture extracts of SARS infected fetal rhesus kidney cells show the presence of pleomorphic enveloped virus particles of around 80-90 nm (range 70-130 nm) in diameter with surface morphology compatible with a coronavirus (see *Lancet* paper, Figure 1). Thin-section electron microscopy of infected cells reveals virus particles of 55-90 nm diameter within smooth walled vesicles in the cytoplasm (see *Lancet* paper, Figure 2B). Electron microscopy can also be used to observe virus particles at the cell surface. Electron microscopy of a human lung biopsy sample depicts similar viral morphology. See *Lancet* paper Figure 2A.

### I. SARS POLYPEPTIDES

The invention relates to proteins from SARS virus. Such polypeptides are exemplified further below.

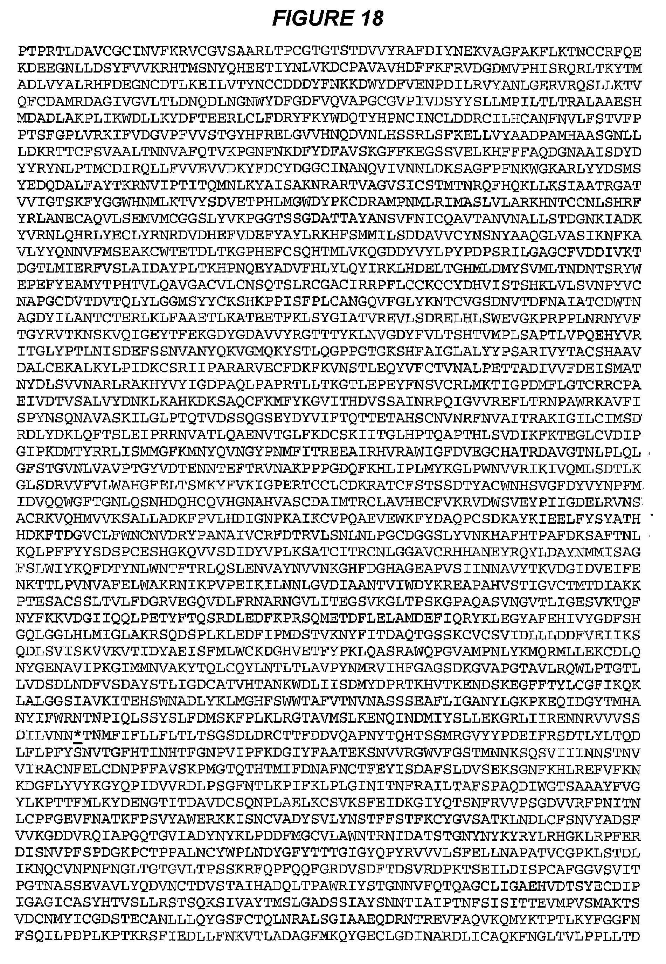

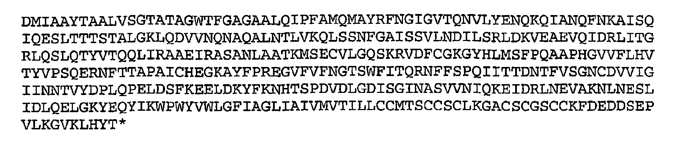

The two Amino acid sequences within Figure 18, separated by a *, are SEQ ID NOS: 7188 & 7189.

SEQ ID NO: 7188 contains an open reading frame comprising SEQ ID NO: 6042. The invention includes a polypeptide comprising SEQ ID NO: 6042. The invention includes a polypeptide comprising an amino acid sequence having sequence identity to SEQ ID NO: 6042. The invention includes a fragment of a polypeptide comprising SEQ ID NO: 6042. The invention includes an immunogenic composition comprising a polypeptide comprising SEQ ID NO: 6042, or a fragment thereof. SEQ ID NO: 6042 demonstrates functional homology to a coronavirus spike protein.

Predicted transmembrane regions of SEQ ID NO: 6042 are identified below.

### Predicted Transmembrane helices of SEQ ID NO: 6042

The sequence positions in brackets denominate the core region. Only scores above 500 are considered significant.

| Inside to outside helices : 18 found | | | | Outside to inside helices : 13 found | | | |
|---|---|---|---|---|---|---|---|
| *from* | *to* | *score* | *center* | *from* | *to* | *score* | *center* |
| 1 ( 1) | 16 ( 16) | 959 | 9 | 1 ( 1) | 17 ( 17) | 684 | 10 |
| 233 ( 237) | 257 ( 252) | 905 | 244 | 222 ( 222) | 240 ( 237) | 238 | 229 |
| 345 ( 347) | 364 ( 361) | 490 | 354 | 244 ( 247) | 264 ( 264) | 613 | 254 |
| 345 ( 354) | 369 ( 369) | 420 | 362 | 349 ( 355) | 369 ( 369) | 314 | 362 |
| 497 ( 497) | 513 ( 513) | 239 | 506 | 496 ( 496) | 511 ( 511) | 488 | 503 |
| 573 ( 573) | 588 ( 588) | 811 | 580 | 573 ( 573) | 591 ( 591) | 712 | 581 |
| 645 ( 648) | 666 ( 663) | 302 | 656 | 650 ( 652) | 666 ( 666) | 474 | 659 |
| 690 ( 696) | 714 ( 711) | 428 | 704 | 674 ( 679) | 702 ( 696) | 190 | 686 |
| 857 ( 860) | 882 ( 874) | 1508 | 867 | 691 ( 696) | 713 ( 711) | 210 | 704 |
| 1031 (1031) | 1046 (1046) | 446 | 1039 | 866 ( 868) | 886 ( 886) | 1172 | 876 |
| 1199 (1203) | 1219 (1217) | 2667 | 1210 | 1198 (1201) | 1215 (1215) | 3221 | 1208 |

SEQ ID NO: 6042, the spike protein, is a surface exposed polypeptide. Primers for amplifying the gene for spike protein and fragments thereof, such as fragments encoding the soluble ectodomain, include SEQ ID NOS: 9753-9763 (Xiao *et al.* (2003) *Biochem Biophys Res Comm* 312:1159-1164).

Further characterization of SEQ ID NO: 6042 is set forth below.

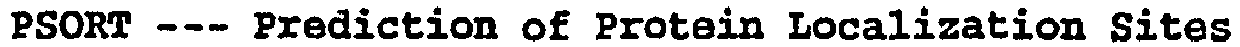

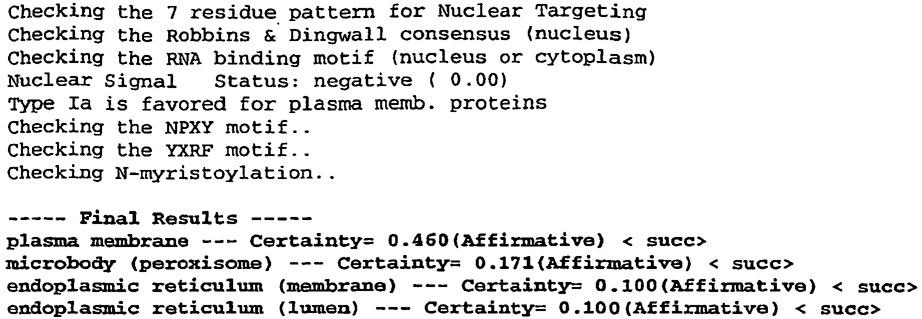

SEQ ID NO: 6042 appears to have a N-terminus signaling region, followed by a surface exposed region, followed by a transmembrane region followed by a C-terminus cytoplasmic domain region.

Two oligopeptide fragments of SEQ ID NO: 6042 that are able to elicit anti-spike antibodies are SEQ ID NOS: 7398 & 7399, as described (with additional C-terminus cysteines) by Xiao et al. (2003) Biochem Biophys Res Comm 312:1159-1164. C-terminal truncations of spike protein, with removal of part of the cytoplasmic region, or removal upto and including the transmembrane region, are described by Yang et al. (2004) Nature 428:561-564.

A variant of SEQ ID NO: 6042 is SEQ ID NO: 9962. Compared to SEQ ID NO: 6042, this sequence has Ser at residue 581 instead of Ala, and has Phe at residue 1152 instead of Leu.

The spike protein of coronaviruses may be cleaved into two separate chains into S1 and S2. The chains may remain associated together to form a dimer or a trimer.

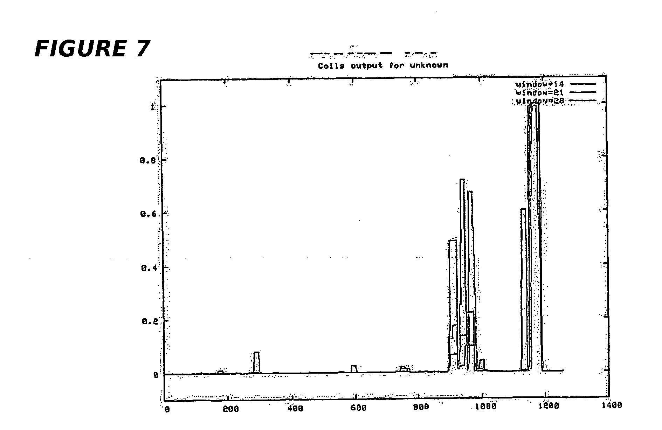

The spike protein appears to form an alpha-helical structure in the transmembrane region of the protein, preferably in the S2 domain. This alpha-helical structure is thought to associate with at least two additional spike proteins to form a trimer. Helical or coiled regions of the spike protein are identified below. Predicted coiled-coils of SEQ ID NO: 6042 (spike protein) are at amino acids 900-1005 and 1151-1185 (see Figure 7).

The spike protein is believed to play an integral role in fusion and infection of Coronaviruses with mammalian host cells. Analysis of coronavirus spike proteins as well as similar surface proteins in other viruses has identified at least two structural motifs, typically located within the S2 domain, associated with this fusion event: heptad repeats (HR) and membrane fusion peptides.

At least two 4,3 hydrophobic heptad repeat (HR) domains are typically found in the ectodomain of the S2 domain of Coronaviruses. One heptad repeat region (HR1) is typically located adjacent to a fusion peptide while a second heptad region (HR2) is typically located near the C-terminus of the S2 domain, close to the transmembrane anchor. Heptad repeats are characteristic of coiled-coil structures and the heptad repeats found in viral surface proteins (such as coronavirus spike protein) are thought to form bundled helix structures which are involved in viral entry. *See* Bosch et al., J. Virology (2003) 77:8801-8811 (Figure 1B of this reference illustrates an alignment of the HR1 and HR2 regions of five coronaviruses along with SARS, annotated "HCov-SARS").

Heptad repeats generally contain a repeating structure of seven amino acids, designated *a-b-c-d-e-f-g*, where hydrophobic sidechains of residues *a* and *d* typically form an apolar stripe, and electrostatic interactions are found in residues *e* and *g*. Position *a* is most frequently Leu, Ile or Ala and position *d* is usually Leu or Ala. Residues *e* and *g* are often Glu or Gln, with Arg and Lys also prominent at position *g*. Charged residues are common to positions *b, c* and *f* as these residues may be in contact with solvent. Exceptions to these general parameters are known. For instance Pro residues are sometimes found within the heptad.

The HR1 and HR2 sequences of an MHV strain have been postulated to assemble into a thermostable, oligomeric, alphahelical rold-like complex, with the HR1 and HR2 helices oriented in an antiparallel manner. *Id*. In this same study, HR2 was asserted to be a strong inhitibor of both virus entry into the cell and cell-cell fusion.

HR1 and HR2 sequences have been identified in the SARS virus genome. The SARS virus HR1 region comprises approximately amino acids 879 to 1005 of SEQ ID NO: 6042 or fragments thereof capable of forming at least one alpha-helical turn. Preferably, said fragments comprise at least 7 (*e.g.*, at least 14, 21, 28, 35, 42, 49 or 56) amino acid residues.

A preferred fragment of HR1 comprises amino acid residues 879 to 980 of SEQ ID NO: 6042. This preferred fragment is SEQ ID NO: 7193.

Another preferred fragment of HR1 comprises amino acid residues 901 to 1005 of SEQ ID NO: 6042. This preferred fragment is SEQ ID NO: 7194.

The SARS virus HR2 region comprises approximately amino acids 1144 to 1201 of SEQ ID NO: 6042, or fragments thereof capable of forming at least one alpha-helical turn. Preferably, said fragments comprise at least 7 (*e.g.*, at least 14, 21, 28, 35, 42, 49 or 56) amino acid residues. A preferred fragment of HR2 comprises amino acids 1144 to 1195 of SEQ ID NO: 6042. This preferred fragment is SEQ ID NO: 7196.

Membrane Fusion peptides sequences within the spike protein are also believed to participate in fusion (and infection) of the virus with a host cell. Fusion peptides generally comprise about 16 to 26 amino acid residues which are conserved within viral families. These Membrane Fusion peptides are relatively hydrophobic and generally show an asymmetric distribution of hydrophobitiy when modeled into an alpha helix. They are also generally rich in alanine and glycine.

At least three hydrophobic Membrane Fusion peptide regions have been identified within coronaviruses (PEP1, PEP2, and PEP3). *See*, Luo et al., "Roles in Cell-Cell Fusion of Two Conserved Hydrophobic Regions in the Murine Coronavirus Spike Protein", Virology (1998) 244:483-494. Figure 1 of this paper shows an alignment of Membrane Fusion peptide sequences of Mouse Hepatitis Viris, Bovine Corona Virus, Feline Infectious Peritonitis Virus, Transmissible Gastroenteritis Virus and Infectious Bronchitis Virus. *See also*, Bosch et al., "The Coronavirus Spike Protein is a Class I Virus Fusion Protein: Structural and Functional Characterization of the Fusion Core Complex" Journal of Virology (2003) 77(16):8801-8811.

PEP1 (SEQ ID NO: 7197), PEP2 (SEQ ID NO: 7198) and PEP3 (SEQ ID NO: 7199) sequences within the SARS spike protein have been identified.

The coronavirus spike proteins (and other similar surface viral proteins) are thought to undergo a conformational change upon receptor binding to the target cell membrane. One or more of the hydrophobic Membrane Fusion peptides are thought to become exposed and inserted into the target membrane as a result of this conformational change. The free energy released upon subsequent refolding of the spike protein to its most stable conformation is believed to play a role in the merger of the viral and cellular membranes.

One or more SARS HR sequences, preferably HR2, or a fragment thereof may be used to inhibit viral entry and membrane fusion with a target mammalian host cell.

As discussed in more detail later in the specification, Applicants have identified structural similarities between the SARS spike protein and the surface protein of *Neisseria meningitidis*, NadA (and other similar bacterial adhesion proteins). Another means of facilitating bacterial expression of HR sequences includes the addition of the stalk and/or anchor sequences of a NadA-like protein to the C-terminus of the recombinant HR sequences. Recombinant sequences containing the bacterial anchor sequence may preferably be prepared in outer membrane vesicles (the preparation of which is discussed in more detail later in the application). Recombinant sequences missing the bacterial anchor sequences may be secreted and isolated from the supernatant.

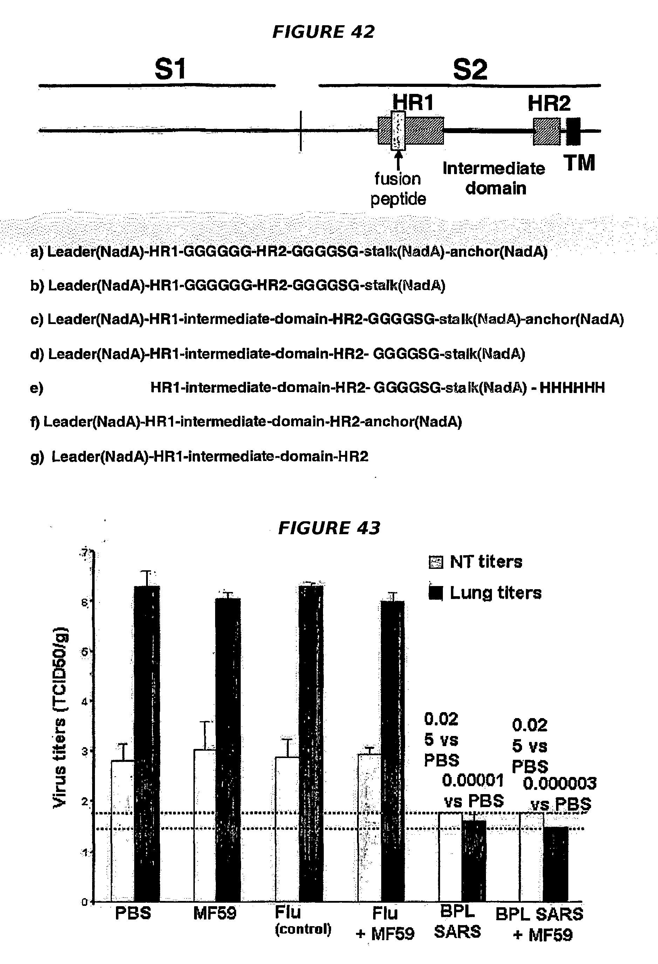

Examples of constructs which may be used in bacterial expression systems are shown in FIGURE 42.
Leader NadA (1-29) - HR1 (879-980) - 6Xgly - HR2 (1144-1195) - stalk+anchor NadA (88-405)
Leader NadA (1-29) - HR1 (879-980) - 6Xgly - HR2 (1144-1196) - stalk NadA (88-351)
Leader NadA (1-29) - HR1 - HR2 (879-1196) - stalk+anchor NadA (88-405)
Leader NadA (1-29) - HR1 - -HR2(879-1196)-stalk NadA (88-351)
HR1 - HR2 (879-1196)-stalk NadA (88-351)-6xhis
Leader NadA (1-29) - H1 - HR2 (879-1196)-anchor NadA (351-405)
Leader NadA (1-29)-HR1-HR2 (879-1196)

Administration of one of more of these Membrane Fusion sequences may also interfere with the ability of a coronavirus to fuse to a host cell membrane.

Two or more of the SARS Membrane Fusion peptides may be linked together.

The spike protein may be recombinantly produced. the alpha-helical portion of the spike protein is associated with the cell membrane. Preferably, the spike proteins form a trimer within the transmembrane region of attachment.

Predicted N-glycosylation sites of SEQ ID NO: 6042 are identified below:

| Position | | Potential | Jury agreement | NGlyc result |
|---|---|---|---|---|
| 29 NYTQ | SEQ ID NO: 7207 | 0.7751 | (9/9) | +++ |
| 65 NVTG | SEQ ID NO: 7208 | 0.8090 | (9/9) | +++ |
| 109 NKSQ | SEQ ID NO: 7209 | 0.6081 | (7/9) | + |
| 119 NSTN | SEQ ID NO: 7210 | 0.7039 | (9/9) | ++ |
| 158 NCTF | SEQ ID NO: 7211 | 0.5808 | (7/9) | + |
| 227 NITN | SEQ ID NO: 7212 | 0.7518 | (9/9) | +++ |
| 269 NGTI | SEQ ID NO: 7213 | 0.6910 | (9/9) | ++ |
| 318 NITN | SEQ ID NO: 7214 | 0.6414 | (9/9) | ++ |
| 330 NATK | SEQ ID NO: 7215 | 0.6063 | (8/9) | + |
| 357 NSTF | SEQ ID NO: 7216 | 0.5746 | (8/9) | + |
| 589 NASS | SEQ ID NO: 7217 | 0.5778 | (6/9) | + |
| 602 NCTD | SEQ ID NO: 7218 | 0.6882 | (9/9) | ++ |
| 699 NFSI | SEQ ID NO: 7219 | 0.5357 | (7/9) | + |
| 783 NFSQ | SEQ ID NO: 7220 | 0.6348 | (9/9) | ++ |
| 1080 NGTS | SEQ ID NO: 7221 | 0.5806 | (7/9) | + |
| 1116 NNTV | SEQ ID NO: 7222 | 0.5106 | (5/9) | + |
| 1176 NESL | SEQ ID NO: 7223 | 0.6796 | (9/9) | ++ |

Predicted O-glycosylation sites are identified below:

| Residue No. | | Potential | Threshold Assignment | |
|---|---|---|---|---|
| Thr | 698 | 0.8922 | 0.7696 | T |
| Thr | 706 | 0.9598 | 0.7870 | T |
| Thr | 922 | 0.9141 | 0.7338 | T |
| Ser | 36 | 0.8906 | 0.7264 | S |
| Ser | 703 | 0.8412 | 0.7676 | S |

Predicted phosphorylation sites of SEQ ID NO: 6042 are Ser-346, Tyr-195, and Tyr-723.

Expression and functional characterization of the spike glycoprotein has been described by Xiao et al. (2003) Biochem Biophys Res Comm 312:1159-1164.

The terms "polypeptide", "protein" and "amino acid sequence" as used herein generally refer to a polymer of amino acid residues and are not limited to a minimum length of the product. Thus, peptides, oligopeptides,:dimers, mulimers, and the like, are included within the definition. Both full-length proteins and fragments thereof are encompassed by the definition.

Polypeptides of the invention can be prepared in many ways *e.g.* by chemical synthesis (at least in part), by digesting longer polypeptides using proteases, by translation from RNA, by purification from cell culture (*e.g.* from recombinant expression), from the organism itself (*e.g.* after viral culture, or direct from patients), from a cell line source *etc.* A preferred method for production of peptides <40 amino acids long involves *in vitro* chemical synthesis (Bodanszky (1993) Principles of Peptide Synthesis (ISBN: 0387564314); Fields et al. (1997) Methods in Enzymology 289: Solid-Phase Peptide Synthesis. ISBN: 0121821900). Solid-phase peptide synthesis is particularly preferred, such as methods based on t-Boc or Fmoc (Chan & White (2000) Fmoc Solid Phase Peptide Synthesis ISBN: 0199637245) chemistry. Enzymatic synthesis (Kullmann (1987) Enzymatic Peptide Synthesis. ISBN: 0849368413) may also be used in part or in full. As an alternative to chemical synthesis, biological synthesis may be used *e.g.* the polypeptides may be produced by translation. This may be carried out in vitro or in vivo. Biological methods are in general restricted to the production of polypeptides based on L-amino acids, but manipulation of translation machinery (*e.g.* of aminoacyl tRNA molecules) can be used to allow the introduction of D-amino acids (or of other non natural amino acids, such as iodotyrosine or methylphenylalanine, azidohomoalanine, *etc.*) (Ibba (1996) Biotechnol Genet Eng Rev 13:197-216.). Where D-amino acids are included, however, it is preferred to use chemical synthesis. Polypeptides of the disclosure may have covalent modifications at the C-terminus and/or N-terminus, particularly where they are for *in vivo* administration *e.g* by attachment of acetyl or carboxamide, as in the Fuzeon™ product.

Reference to polypeptides and the like also includes derivatives of the amino acid sequences of the disclosure. Such derivatives can include postexpression modifications of the polypeptide, for example, glycosylation, acetylation, phosphorylation, and the like. Amino acid derivatives can also include modifications to the native sequence, such as deletions, additions and substitutions (generally conservative in nature), so long as the protein maintains the desired activity. These modifications may be deliberate, as through site-directed mutagenesis, or may be accidental, such as through mutations of hosts which produce the proteins or errors due to PCR amplification. Furthermore, modifications may be made that have one or more of the following effects: reducing toxicity; facilitating cell processing (*e.g.*, secretion; antigen presentation, *etc.*); and facilitating presentation to B-cells and/or T-cells.

"Fragment" or "Portion" as used herein refers to a polypeptide consisting of only a part of the intact full-length polypeptide sequence and structure as found in nature. For instance, a fragment can include a C-terminal deletion and/or an N-terminal deletion of a protein.

A "recombinant" protein is a protein which has been prepared by recombinant DNA techniques as described herein. In general, the gene of interest is cloned and then expressed in transformed organisms, as described further below. The host organism expressed the foreign gene to produce the protein under expression conditions.

By "isolated" is meant, when referring to a polypeptide, that the indicated molecule is separate and discrete from the whole organism with which the molecule is found in nature or, when the polypeptide is not found in nature, is sufficiently free of other biological macromolecules so that the polypeptide can be used for its intended purpose. The polypeptides of the invention are preferably isolated polypeptides.

An "immunogenic composition" as used herein refers to a composition that comprises an antigenic molecule where administration of the composition to a subject results in the development in the subject of a humoral and/or a cellular immune response to the antigenic molecule of interest. The immunogenic composition can be introduced directly into a recipient subject, such as by injection, inhalation, oral, intranasal or any other parenteral, mucosal or transdermal (*e.g.*, intra-rectally or intra-vaginally) route of administration.

The term "derived from" is used to identify the source of molecule (*e.g.*, a molecule can be derived from a polynucleotide, polypeptide, an immortalized cell line can be derived from any tissue, *etc.*).

A first polypeptide is "derived from" a second polypeptide if it is (i) encoded by a first polynucleotide derived from a second polynucleotide, or (ii) displays sequence identity to the second polypeptides as described above. Thus, a polypeptide (protein) is "derived from" a particular SARS virus if it is (i) encoded by an open reading frame of a polynucleotide of that SARS virus, or (ii) displays sequence identity, as described above, to polypeptides of that SARS virus.

Both polynucleotide and polypeptide molecules can be physically derived from a SARS virus or produced recombinantly or synthetically, for example, based on known sequences.

The immunogenic compositions of the invention may further comprise one or more adjuvants.

The immunogenic compositions of the invention may be administered mucosally. Suitable routes of mucosal administration include oral, intranasal, intragastric, pulmonary, intestinal, rectal, ocular and vaginal routes. The immunogenic composition may be adapted for mucosal administration. For instance, where the composition is for oral administration, it may be in the form of tablets or capsules, optionally enteric-coated, liquid, transgenic plants, *etc.* Where the composition is for intranasal administration, it may be in the form of a nasal spray, nasal drops, gel or powder.

The immunogenic compositions of the disclosure may be administered parenterally. Suitable routes of parenteral administration include intramuscular (IM), subcutaneous, intravenous, intraperitoneal, intradermal, transcutaneous, and transdermal (*see e.g.*, International patent application WO 98/20734) routes, as well as delivery to the interstitial space of a tissue. The immunogenic composition may be adapted for parenteral administration, for instance in the form of an injectable that may be sterile and pyrogen free.

Vaccines of the disclosure may be administered in conjunction with other immunoregulatory agents. In particular, compositions will usually include an adjuvant. Preferred further adjuvants include, but are not limited to, one or more of the following set forth below:

### A. Mineral Containing, Compositions

Mineral containing compositions suitable for use as adjuvants in the disclosure include mineral salts, such as aluminium salts.and calcium salts. The disclosure includes mineral salts such as hydroxides (*e.g.* oxyhydroxides), phosphates (*e.g.* hydroxyphoshpates, orthophosphates), sulphates, *etc.* (*e.g.* see chapters 8 & 9 of Vaccine design:the subunit and adjuvant approach (1995) Powell & Newman. ISBN 0-306-44867-X.), or mixtures of different mineral compounds, with the compounds taking any suitable form (*e.g.* gel, crystalline, amorphous, *etc.*), and with adsorption being preferred. The mineral containing compositions may also be formulated as a particle of metal salt. See WO00/23105.

### B. Oil-Emulsions

Oil-emulsion compositions suitable for use as adjuvants in the disclosure include squalene-water emulsions, such as MF59 (5% Squalene, 0.5% Tween 80, and 0.5% Span 85, formulated into submicron particles using a microfluidizer). See WO90/14837. See also, Frey et al., "Comparison of the safety, tolerability, and immunogenicity of a MF59-adjuvanted influenza vaccine and a non-adjuvanted influenza vaccine in non-elderly adults", Vaccine (2003) 21:4234-4237.

Particularly preferred adjuvants for use in the compositions are submicron oil-inwater emulsions. Preferred submicron oil-in-water emulsions for use herein are squalene/water emulsions optionally containing varying amounts of MTP-PE, such as a submicron oil-in-water emulsion containing 4-5% w/v squalene, 0.25-1.0% w/v Tween 80™ (polyoxyelthylenesorbitan monooleate), and/or 0.25-1.0% Span 85™ (sorbitan trioleate), and, optionally, N-acetylmuramyl-L-alanyl-D-isogluatminyl-L-alanine-2-(1'-2'-dipalmitoyl-*sn*-glycero-3-huydroxyphosphophoryloxy)-ethylamine (MTP-PE), for example, the submicron oil-in-water emulsion known as "MF59" (International Publication No. WO 90/14837; US Patent Nos. 6,299,884 and 6,451,325; and Ott et al., "MF59 - Design and Evaluation of a Safe and Potent Adjuvant for Human Vaccines" in Vaccine Design: The Subunit and Adjuvant Approach (Powell, M.F. and Newman, M.J. eds.) Plenum Press, New York, 1995, pp. 277-296). MF59 contains 4-5% w/v Squalene (*e.g.*, 4.3%), 0.25-0.5% w/v Tween 80™, and 0.5% w/v Span 85™ and optionally contains various amounts of MTP-PE, formulated into submicron particles using a microfluidizer such as Model 110Y microfluidizer (Microfluidics, Newton, MA). For example, MTP-PE may be present in an amount of about 0-500 ng/dose, more preferably 0-250 µg/dose and most preferably, 0-100 µg/dose. As used herein, the term "MF59-0" refers to the above submicron oil-in-water emulsion lacking MTP-PE, while the term MF59-MTP denotes a formulation that contains MTP-PE. For instance, "MF59-100" contains 100 µg MTP-PE per dose, and so on. MF69, another submicron oil-in-water emulsion for use herein, contains 4.3% w/v squalene, 0.25% w/v Tween 80™, and 0.75% w/v Span 85™ and optionally MTP-PE. Yet another submicron oil-in-water emulsion is MF75, also known as SAF, containing 10% squalene, 0.4% Tween 80™, 5% pluronic-blocked polymer L121, and thr-MDP, also microfluidized into a submicron emulsion. MF75-MTP denotes an MF75 formulation that includes MTP, such as from 100-400 µg MTP-PE per dose.

Submicron oil-in-water emulsions, methods of making the same and immunostimulating agents, such as muramyl peptides, for use in the compositions, are described in detail in International Publication No. WO 90114837 and US Patent Nos. 6,299,884 and 6,45 1,325.

Complete Freund's adjuvant (CFA) and incomplete Freund's adjuvant (IFA) may also be used as adjuvants in the disclosure.

### C. Saponin Formulations

Saponin formulations, may also be used as adjuvants in the disclosure. Saponins are a heterologous group of sterol glycosides and triterpenoid glycosides that are found in the bark, leaves, stems, roots and even flowers of a wide range of plant species. Saponin from the bark of the *Quillaia saponaria* Molina tree have been widely studied as adjuvants. Saponin can also be commercially obtained from *Smilax ornata* (sarsaprilla), *Gypsophilla paniculata* (brides veil), and *Saponaria officianalis* (soap root). Saponin adjuvant formulations include purified formulations, such as QS21, as well as lipid formulations, such as ISCOMs.

Saponin compositions have been purified using High Performance Thin Layer Chromatography (HP-LC) and Reversed Phase High Performance Liquid Chromatography (RP-HPLC). Specific purified fractions using these techniques have been identified, including QS7, QS17, QS18, QS21, QH-A, QH-B and QH-C. Preferably, the saponin is QS21. A method of production of QS21 is disclosed in US Patent No. 5,057,540. Saponin formulations may also comprise a sterol, such as cholesterol (see WO 96/33739).

Combinations of saponins and cholesterols can be used to form unique particles called Immunostimulating Complexs (ISCOMs). ISCOMs typically also include a phospholipid such as phosphatidylethanolamine or phosphatidylcholine. Any known saponin can be used in ISCOMs. Preferably, the ISCOM includes one or more of Quil A, QHA and QHC. ISCOMs are further described in EP 0 109 942, WO 96/11711 and WO 96/33739. Optionally, the ISCOMS may be devoid of additional detergent. See WO00/07621.

A review of the development of saponin based adjuvants can be found at Barr, et al., "ISCOMs and other saponin based adjuvants", Advanced Drug Delivery Reviews (1998) 32:247-271. See also Sjolander, et al., "Uptake and adjuvant activity of orally delivered saponin and ISCOM vaccines", Advanced Drug Delivery Reviews (1998) 32:321-338.

### D. Bacterial or Microbial Derivatives

Adjuvants suitable for use in the disclosure include bacterial or microbial derivatives such as:

### (1) Non-toxic derivatives of enterobacterial lipopolysaccharide (LPS)

Such derivatives include Monophosphoryl lipid A (MPL) and 3-O-deacylated MPL (3dMPL). 3dMPL is a mixture of 3 De-O-acylated monophosphoryl lipid A with 4, 5 or 6 acylated chains. A preferred "small particle" form of 3 De-O-acylated monophosphoryl lipid A is disclosed in EP 0 689 454. Such "small particles" of 3dMPL are small enough to be sterile filtered through a 0.22 micron membrane (see EP 0 689 454). Other non-toxic LPS derivatives include monophosphoryl lipid A mimics, such as aminoalkyl glucosaminide phosphate derivatives *e.g.* RC-529. See Johnson et al. (1999) Bioorg Med Chem Lett 9:2273-2278.

### (2) Lipid A Derivatives

Lipid A derivatives include derivatives of lipid A from *Escherichia coli* such as OM-174. OM-174 is described for example in Meraldi et al., "OM-174, a New Adjuvant with a Potential for Human Use, Induces a Protective Response with Administered with the Synthetic C-Terminal Fragment 242-310 from the circumsporozoite protein of Plasmodium berghei", Vaccine (2003) 21:2485-2491; and Pajak, et al., "The Adjuvant OM-174 induces both the migration and maturation of murine dendritic cells in vivo", Vaccine (2003) 21:836-842.

### (3) Immunostimulatory oligonucleotides

Immunostimulatory oligonucleotides suitable for use as adjuvants in the disclosure include nucleotide sequences containing a CpG motif (a sequence containing an unmethylated cytosine followed by guanosine and linked by a phosphate bond). Bacterial double stranded RNA or oligonucleotides containing palindromic or poly(dG) sequences have also been shown to be immunostimulatory.

The CpG's can include nucleotide modifications/analogs such as phosphorothioate modifications and can be double-stranded or single-stranded. Optionally, the guanosine may be replaced with an analog such as 2'-deoxy-7-deazaguanosine. See Kandimalla, et al., "Divergent synthetic nucleotide motif recognition pattern: design and development of potent immunomodulatory oligodeoxyribonucleotide agents with distinct cytokine induction profiles", Nucleic Acids Research (2003) 31(9): 2393-2400; WO 02/26757 and WO 99/62923 for examples of possible analog substitutions. The adjuvant effect of CpG oligonucleotides is further discussed in Krieg, "CpG motifs: the active ingredient in bacterial extracts?", Nature Medicine (2003) 9(7): 831-835; McCluskie, et al., "Parenteral and mucosal prime-boost immunization strategies in mice with hepatitis B surface antigen and CpG DNA", FEMS Immunology and Medical Microbiology (2002) 32:179-185; WO 98/40100; US Patent No. 6,207,646; US Patent No. 6,239,116 and US Patent No. 6,429,199.

The CpG sequence may be directed to TLR9, such as the motif GTCGTT or TTCGTT. See Kandimalla, et al., "Toll-like receptor 9: modulation of recognition and cytokine induction by novel synthetic CpG DNAs", Biochemical Society Transactions (2003) 31 (part 3): 654-658. The CpG sequence may be specific for inducing a Th1 immune response, such as a CpG-A ODN, or it may be more specific for inducing a B cell response, such a CpG-B ODN. CpG-A and CpG-B ODNs are discussed in Blackwell, et al., "CpG-A-Induced Monocyte IFN-gamma-Inducible Protein-10 Production is Regulated by Plasmacytoid Dendritic Cell Derived IFN-alpha", J. Immunol. (2003) 170(8):4061-4068; Krieg, "From A to Z on CpG", TRENDS in Immunology (2002) 23(2): 64-65 and WO 01/95935. Preferably, the CpG is a CpG-A ODN.

Preferably, the CpG oligonucleotide is constructed so that the 5' end is accessible for receptor recognition. Optionally, two CpG oligonucleotide sequences may be attached at their 3' ends to form "immunomers". See, for example, Kandimalla, et al., "Secondary structures in CpG oligonucleotides affect immunostimulatory activity", BBRC (2003) 306:948-953; Kandimalla, et al., "Toll-like receptor 9: modulation of recognition and cytokine induction by novel synthetic GpG DNAs", Biochemical Society Transactions (2003) 31(part 3):664-658; Bhagat et al., "CpG penta- and hexadeoxyribonucleotides as potent immunomodulatory agents" BBRC (2003) 300:853-861 and WO 03/035836.

### (4) ADP-ribosylaling toxins and detoxified derivatives thereof.

Bacterial ADP-ribosylating toxins and detoxified derivatives thereof may be used as adjuvants in the disclosure Preferably, the protein is derived from *E. coli* (*i.e.*, E. coli heat labile enterotoxin "LT), cholera ("CT"), or pertussis ("PT"). The use of detoxified ADP-ribosylating toxins as mucosal adjuvants is described in WO 95/17211 and as parenteral adjuvants in WO 98/42375. Preferably, the adjuvant is a detoxified LT mutant such as LT-K63, LT-R72, and LTR192G. The use of ADP-ribosylating toxins and detoxified derivaties thereof, particularly LT-K63 and LT-R72, as adjuvants can be found in the following references. Beignon, et al., "The LTR72 Mutant of Heat-Labile Enterotoxin of Escherichia coli Enahnces the Ability of Peptide Antigens to Elicit CD4+ T Cells and Secrete Gamma Interferon after Coapplication onto Bare Skin", Infection and Immunity (2002) 70(6):3012-3019; Pizza, et al., "Mucosal vaccines: non-toxic derivatives of LT and CT as mucosal adjuvants", Vaccine (2001) 19:2534-2541; Pizza, et al., "LTK63 and LTR72, two mucosal adjuvants ready for clinical trials" Int. J. Med. Microbiol (2000) 290(4-5):455-461; Scharton-Kersten et al., "Transcutaneous Immunization with Bacterial ADP-Ribosylating Exotoxins, Subunits and Unrelated Adjuvants", Infection and Immunity (2000) 68(9):5306-5313; Ryan et al., "Mutants of Escherichia coli Heat-Labile Toxin Act as Effective Mucosal Adjuvants for Nasal Delivery of an Acellular Pertussis Vaccine: Differential Effects of the Nontoxic AB Complex and Enzyme Activity on Th1 and Th2 Cells" Infection and Immunity (1999) 67(12):6270-6280; Partidos et al., "Heat-labile enterotoxin of Escherichia coli and its site-directed mutant LTK63 enhance the proliferative and cytotoxic T-cell responses to intranasally co-immunized synthetic peptides", Immunol. Lett. (1999) 67(3):209-216; Peppoloni et al., "Mutants of the Escherichia coli heat-labile enterotoxin as safe and strong adjuvants for intranasal delivery of vaccines". Vaccines (2003) 2(2):285-293; and Pine et al., (2002) "Intranasal immunization with influenza vaccine and a detoxified mutant of heat labile enterotoxin from Escherichia coli (LTK63)" J. Control Release (2002) 85(1-3):263-270. Numerical reference for amino acid substitutions is preferably based on the alignments of the A and B subunits of ADP-ribosylating toxins set forth in Domenighini et al., Mol. Microbiol (1995) 15(6):1165-1167.

### E. Human Immunomodulators

Human immunomodulators suitable for use as adjuvants in the disclosure include cytokines, such as interleukins (*e.g.* IL-1, IL-2, IL-4, IL-5, IL-6, IL-7, IL-12, *etc.*), interferons (*e.g.* interferon-γ), macrophage colony stimulating factor, and tumor necrosis factor.

### F. Bioadhesives and Mucoadhesives

Bioadhesives and mucoadhesives may also be used as adjuvants in the disclosure. Suitable bioadhesives include esterified hyaluronic acid microspheres (Singh et al. (2001) J. Cont. Rele. 70:267-276) or mucoadhesives such as cross-linked derivatives of poly(acrylic acid), polyvinyl alcohol, polyvinyl pyrollidone, polysaccharides and carboxymethylcellulose. Chitosan and derivatives thereof may also be used as adjuvants in the disclosure. *E.g.*, WO99/27960.

### G. Microparticles

Microparticles may also be used as adjuvants in the disclosure. Microparticles (*i.e.* a particle of ~100nm to ~150µm in diameter, more preferably ~200nm to ~30µm in diameter, and most preferably ~500nm to ~10µm in diameter) formed from materials that are biodegradable and non-toxic (*e.g.* a poly(α-hydroxy acid), a polyhydroxybutyric acid, a polyorthoester, a polyanhydride, a polycaprolactone, *etc.*), with poly(lactide-co-glycolide) are preferred, optionally treated to have a negatively-charged surface (*e.g.* with SDS) or a positively-charged surface (*e.g*. with a cationic detergent, such as CTAB).

### H. Liposomes

Examples of liposome formulations suitable for use as adjuvants are described in US Patent No. 6,090,406, US Patent No. 5,916,588, and EP 0 626 169.

### I. Polyoxyethylene ether and Polyoxyethylene Ester Formulations

Adjuvants suitable for use in the disclosure include polyoxyethylene ethers and polyoxyethylene esters. WO99/52549. Such formulations further include polyoxyethylene sorbitan ester surfactants in combination with an octoxynol (WO01/21207) as well as polyoxyethylene alkyl ethers or ester surfactants in combination with at least one additional non-ionic surfactant such as an octoxynol (WO01/21152).

Preferred polyoxyethylene ethers are selected from the following group: polyoxyethylene-9-lauryl ether (laureth 9), polyoxyethylene-9-steoryl ether, polyoxytheylene-8-steoryl ether, polyoxyethylene-4-lauryl ether, polyoxyethylene-35-lauryl ether, and polyoxyethylene-23-lauryl ether.

### J. Polyphosphazene (PCPP)

PCPP formulations are described, for example, in Andrianov et al., "Preparation of hydrogel microspheres by coacervation of aqueous polyphophazene solutions", Biomaterials (1998) 19(1-3):109-115 and Payne et al., "Protein Release from Polyphosphazene Matrices", Adv. Drug. Delivery Review (1998) 31(3):185-196.

### K. Muramyl peptides

Examples of muramyl peptides suitable for use as adjuvants in the disclosure include N-acetyl-muramyl-L-threonyl-D-isoglutamine (thr-MDP), N-acetyl-normuramyl-L-alanyl-D-isoglutamine (nor-MDP), and N-acetylmuramyl-L-alanyl-D-isoglutaminyl-L-alanine-2-(1'-2'-dipalmitoyl-*sn*-glycero-3-hydroxyphosphoryloxy)-ethylamine MTP-PE).

### L. Imidazoquinolone Compounds.

Examples of imidazoquinolone compounds suitable for use adjuvants in the disclosure include Imiquamod and its homologues, described further in Stanley, "Imiquimod and the imidazoquinolones: mechanism of action and therapeutic potential" Clin Exp Dermatol (2002) 27(7):571-577 and Jones, "Resiquimod 3M", Curr Opin Investig Drugs (2003) 4(2):214-218. M. Virosomes and Virus Like Particles (VLPs)

Virosomes and Virus Like Particles (VLPs) can also be used as adjuvants in the disclosure These structures generally contain one or more proteins from a virus optionally combined or formulated with a phospholipid. They are generally non-pathogenic, non-replicating and generally do not contain any of the native viral genome. The viral proteins may be recombinantly produced or isolated from whole viruses. These viral proteins suitable for use in virosomes or VLPs include proteins derived from influenza virus (such as HA or NA), Hepatitis B virus (such as core or capsid proteins), Hepatitis E virus, measles virus, Sindbis virus, Rotavirus, Foot-and-Mouth Disease virus, Retrovirus, Norwalk virus, human Papilloma virus, HIV, RNA-phages, Qß-phage (such as coat proteins), GA-phage, fr-phage, AP205 phage, and Ty (such as retrotransposon Ty protein p1). VLPs are discussed further in WO 03/024480, WO 03/024481, and Niikura et al., "Chimeric Recombinant Hepatitis E Virus-Like Particles as an Oral Vaccine Vehicle Presenting Foreign Epitopes", Virology (2002) 293:273-280; Lenz et al., "Papillomarivurs-Like Particles Induce Acute Activation of Dendritic Cells", Journal of Immunology (2001) 5246-5355; Pinto, et al., "Cellular Immune Responses to Human Papillomavirus (HPV)-16 L1 Healthy Volunteers Immunized with Recombinant HPV-16 L1 Virus-Like Particles", Journal of Infectious Diseases (2003) 188:327-338; and Gerber et al., "Human Papillomavrisu Virus-Like Particles Are Efficient Oral Immunogens when Coadministered with Escherichia coli Heat-Labile Entertoxin Mutant R192G or CpG", Journal of Virology (2001) 75(10):4752-4760. Virosomes are discussed further in, for example, Gluck et al., "New Technology Platforms in the Development of Vaccines for the Future", Vaccine (2002) 20:B10-B16.

The disclosure may also comprise combinations of aspects of one or more of the adjuvants identified above. For example, the following adjuvant compositions may be used in the disclosure:
(1) a saponin and an oil-in-water emulsion (WO99/11241);
(2) a saponin (*e.g.*, QS21) + a non-toxic LPS derivative (*e.g.,* 3dMPL) (see WO 94/00153);
(3) a saponin (*e.g.*, QS21) + a non-toxic LPS derivative (*e.g.*, 3dMPL) + a cholesterol;
(4) a saponin (*e.g.* QS21) + 3dMPL + IL-12 (optionally + a sterol) (WO98/57659);
(5) combinations of 3dMPL with, for example, QS21 and/or oil-in-water emulsions (See European patent applications 0835318, 0735898 and 0761231);
(6) SAF, containing 10% Squalane, 0.4% Tween 80,5% pluronic-block polymer L121, and thr-MDP, either microfluidized into a submicron emulsion or vortexed to generate a larger particle size emulsion.
(7) Ribi™ adjuvant system (RAS), (Ribi Immunochem) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); and
(8) one or more mineral salts (such as an aluminum salt) + a non-toxic derivative of LPS (such as 3dPML).

Aluminium salts and MF59 are preferred adjuvants for parenteral immunisation. Mutant bacterial toxins are preferred mucosal adjuvants.

As mentioned above, adjuvants suitable for use in the disclosure may also include one or more of the following:
- *E.coli* heat-labile enterotoxin ("LT"), or detoxified mutants thereof, such as the K63 or R72 mutants;
- cholera toxin ("CT"), or detoxified mutants thereof;
- microparticles (i.e., a particle of ~100mm to ~150µm in diameter, more preferably ~200nm to ~30µm in diameter, and most preferably ~500nm to ~10µm in diameter) formed from materials that are biodegradable and non-toxic (e.g. a poly(α-hydroxy acid), a polyhydroxybutyric acid, a polyorthoester, a polyanhydride, a polycaprolactone *etc.*);
- a polyoxyethylene ether or a polyoxyethylene ester (see International patent application WO 99/52549);
- a polyoxyethylene sorbitan ester surfactant in combination with an octoxynol (see International patent application WO 01/21207) or a polyoxyethylene alkyl ether or ester surfactant in combination with at least one additional non-ionic surfactant such as an octoxynol (see International patent application WO 01/21152);
- chitosan (e.g. International patent application WO 99/27960)
- an immunostimulatory oligonucleotide (e.g. a CpG oligonucleotide) and a saponin (see International patent application WO 00/62800)
- immunostimulatory double stranded RNA.
- aluminum compounds (e.g. aluminum hydroxide, aluminum phosphate, aluminum hydroxyphosphate, oxyhydroxide, orthophosphate, sulfate *etc.* (*e.g.* see chapters 8 & 9 of Vaccine design: the subunit and adjuvant aproach, eds. Powell & Newman, Plenum Press 1995 (ISBN 0-306-44867-X) (hereinafter "Vaccine design"), or mixtures of different aluminum compounds, with the compounds taking any suitable form (e.g. gel, crystalline, amorphous *etc*.), and with adsorption being preferred;
- MF59 (5% Squalene, 0.5% Tween 80, and 0.5% Span 85, formulated into submicron particles using a microfluidizer) (see Chapter 10 of Vaccine design; see also International patent application WO 90/14837);
- liposomes (see Chapters 13 and 14 of *Vaccine design*);
- ISCOMs (see Chapter 23 of *Vaccine design*);
- SAF, containing 10% Squalane, 0.4% Tween 80, 5% pluronic-block polymer L121, and thr-MDP, either microfluidized into a submicron emulsion or vortexed to generate a larger particle size emulsion (see Chapter 12 of *Vaccine design*);
- Ribi™ adjuvant system (RAS), (Ribi Immunochem) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™);
- saponin adjuvants, such as QuilA or QS21 (see Chapter 22 of *Vaccines design*), also known as Stimulon™;
- ISCOMs, which may be devoid of additional detergent (WO 00/07621);
- complete Freund's adjuvant (CFA) and incomplete Freund's adjuvant (IFA);
- cytokines, such as interleukins (e.g. IL-1, IL-2, IL-4, IL-5, EL-6, IL-7, IL-12, etc.), interferons (e.g. interferon-γ), macrophage colony stimulating factor, tumor necrosis factor, etc. (see Chapters 27 & 28 of *Vaccine design*);
- monophosphoryl lipid A (MPL) or 3-O-deacylated MPL (3dMPL) (e.g. chapter 21 of *Vaccine design*);
- combinations of 3dMPL with, for example, QS21 and/or oil-in-water emulsions (European patent applications 0835318,0735898 and 0761231);
- oligonucleotides comprising CpG motifs (see Krieg (2000) Vaccine, 19:618-622; Krieg (2001) Curr. Opin. Mol. Ther., 2001, 3:15-24; WO 96/02555, WO 98/16247, WO 98/18810, WO 98/40100, WO 98/55495, WO 98/37919 and WO 98/52581, etc.) *i.e.* containing at least one CG dinucleotide,
- a polyoxyethylene ether or a polyoxyethylene ester (International patent application WO99/52549);
- a polyoxyethylene sorbitan ester surfactant in combination with an octoxynol (International patent application WO 01/21207) or a polyoxyethylene alkyl ether or ester surfactant in combination with at least one additional non-ionic surfactant such as an octoxynol (WO 01/21152);
- an immunostimulatory oligonucleotide (e.g. a CpG oligonucleotide) and a saponin (WO00/62800);
- an immunostimulant and a particle of metal salt (International patent application WO00/23105);
- a saponin and an oil-in-water emulsion (WO 99/11241);
- a saponin (e.g. QS21) + 3dMPL + IL-12 (optionally + a sterol) (WO 98/57659).

Other adjuvants suitable for mucosal or parenteral administration are also available (*e.g.* see chapter 7 of Vaccine design: the subunit and adjuvant aproach, eds. Powell & Newman, Plenum Press 1995 (ISBN 0-306-44867-X).

Mutants of LT are preferred mucosal adjuvants, in particular the "K63" and "R72" mutants (*e.g.* see International patent application WO 98/18928), as these result in an enhanced immune response.

Microparticles are also preferred mucosal adjuvants. These are preferably derived from a poly(α-hydroxy acid), in particular, from a poly(lactide) ("PLA"), a copolymer of D,L-lactide and glycolide or glycolic acid, such as a poly(D,L-lactide-co-glycolide) ("PLG" or "PLGA"), or a copolymer of D,L-lactide and caprolactone. The microparticles may be derived from any of various polymeric starting materials which have a variety of molecular weights and, in the case of the copolymers such as PLG, a variety of lactide:glycolide ratios, the selection of which will be largely a matter of choice, depending in part on the coadministered antigen.

### EXAMPLES

### Example 1-EXAMPLE of a SARS VIRUS ISOLATE

A SARS virus was isolated from clinical specimens of a patient in Frankfurt, Germany (FRA). The isolate was grown in Vero cells. RNA of the SARS virus was extracted and amplified by RT-PCR. Nucleotide sequence of the viral genome was determined by direct sequencing of the PCR product. Computer analysis was used to predict the features of the genome, to compare it to previously known coronaviruses and to the sequence of different SARS virus isolates.

More specifically, isolation and sequence was performed as follows. After the third passage of the SARS virus in Vero cells, viral particles were purified by ultra centrifugation from 3x10⁷ cells supernatant. Viral RNA was extracted by Triazol method (Gibco-BRL). Viral RNA (200 ng) was transcribed into cDNA with avian RNaseH- thermostable reverse transcriptase following the instructions of the manufacturer (ThermoScript RT System, Invitrogen). Briefly, either 50 pmoles of oligo (dT)₂₀ or 25 ng of random hexamers were used to prime the RT reaction in a 20 µl final volume. Amplification and sequencing of the SARS genome were accomplished by direct sequencing of PCR products obtained with: i) specific primers from conserved regions of homology found through multiple alignment among known coronaviruses; ii) oligonucleotides designed around short sequences of SARS isolates available on the Web through WHO network laboratories; iii) degenerate primers to amplify the cDNA mixture with multiple overlapping fragments as end products. Gap closure was realized by long distance PCR with high fidelity Taq (Expand High Fidelity system, Roche) using primers designed on selected fragments. Sequence was collected by primer walking using a BigDye terminator chemistry (Applied Biosystems) and an automated DNA sequencer (3700 capillary model, Applied Biosystems). After obtaining a first pass of the entire genome, a set of both forward and reverse primers were used to amplify and sequence *de novo* the genome using as a template DNA segments of 2 kb on average. Readings from overlapping fragments were automatically assembled by AutoAssembler (Applied Biosystems) and the 29,740 bp contiguous edited manually.

Computer analysis of the sequence was performed as follows. The GCG Wisconsin Package suite (version 10.0) was used for computer analysis of gene and protein sequences. The PSORT program (*http:*//*psort.nibb.ac.jp*/) was used for localization predictions. For secondary structure analysis, the PHD software available on the Web at *http:*//*cubic.bioc.columbia.edu*/*predictprotein*/ was applied. The PSI-BLAST algorithm was used for homology searches (*http:*//*www.ncbi.nlm.nih.gov*/*blast*) using the non-redundant protein database. ClustalW was applied to obtain multiple sequence alignments of gene and protein sequences. The LearnCoil-VMF program was used to predict coiled-coil regions in the spike proteins (*http:*//*learncoil-vmf.lcs.mit.edu*/*cgi-bin*/*vmf*). Leucine zippers were predicted with the program 2ZIP, available at *http:*//*2Zip.molgen.mpg.de.*

Phylogenetic analysis was performed using the neighbor-joining algorithm as implemented in the program NEIGHBOR within the Phylogeny Inference Package (Phylip) (Felsenstein J 1993, program distributed by the author). Bootstrap analysis was always performed with 100 replicates using the program Seqboot. Trees were handled and displayed using TreeView. The program HMMER was used to generate sequence profiles from multiple sequence alignments of the S1 domains of spike proteins. Subsequently, the HMMPFAM program was used to compare the S1 domain of SARS spike to the profiles.

The genome of this SARS virus isolate is 29,740 bases long and the overall structure of the genome is similar to that of the three known groups of coronaviruses. Starting from the 5' end a leader sequence, an untranslated region (UTR) and two overlapping open reading frames coding for one polyprotein containing the enzymes necessary for replication can be identified. They are followed by a region coding for the spike (S), envelope (E), matrix (M), nucleocapsid (N) structural proteins and eight additional ORFs specific for the SARS virus. At the 3'-end of the genome a UTR with a poly(A) is located. The overall homology to coronaviruses groups 1, 2 and 3 is low and therefore the SARS virus belongs to a new group (group 4) of coronavirus. More detailed analysis of the spike protein amino acid sequence shows that the SARS virus isolate is more closely related to coronavirus group 2.

The complete genome sequence of the SARS virus isolate is 29,740 bp in length. The sequence is available on Genbank and has a GC content of 40.8%, comparable with that of known viruses of the same family. Genome structure is similar to that of other coronaviruses. 14 open reading frames have been predicted. The principal features of the genome and gene products are illustrated reported in Figure 10 and Table 10. The comparison between the SARS genome and those of group 1,2 and 3 coronaviruses is reported in Figure 11.

Nucleotides 1-73 contain a predicted RNA leader sequence followed by an untranslated region (UTR) of 197 nucleotides. The UTR is followed by two overlapping open reading frames (ORF1a, ORF1b), which encompass two-thirds of the genome (nucleotides 265-21485). They encode for a large polyprotein, which is predicted to be processed by viral proteases to generate the replicase complex. The 3' part of the.genome contains the genes coding for the four structural proteins (S, spike protein, E, envelope protein, M, matrix glycoprotein, and N, nucleocapsid protein), and eight predicted ORFs of unknown function (Figure 10). Finally, at the 3' end of the genome, we found a second UTR of 340 bases followed by a poly(A) tract. We identified a putative intergenic (IG) sequence also referred to as transcription-associated sequence (TAS), which is a typical feature for coronaviruses. The IG sequence is characterized by 6-18 nucleotides present at the 3' end of the leader and can be found in front of each gene. The IG sequence plays a key role in RNA transcription and its regulation. The IG sequence of the SARS virus is present nine times in the genome (Figure 10). The sequence of the leader and IG are peculiar for each coronavirus and represent a specific signature for the virus.

### The Structural Region

Analysis of the nucleotide sequence at the 3' part of the SARS genome identified 12 predicted open reading frames. They are coded within 8.2 kb and comprise the four structural proteins S, E, M and N, common for all coronaviruses and eight predicted ORFs, which are specific for this virus (Figure 11). SARS-specific IG sequences upstream of most ORFs (Figures 10 & 11) suggest that most genes are likely to be transcribed independently. Interestingly, sequences identical to the group 2 IG are also present at the end of the RNA leader and in front of the Matrix encoding gene and of ORF 10.

The spike is a type I glycoprotein, which forms the large spikes on the surface of the virion and is responsible for receptor-binding and membrane fusion. (Gallagher (2001) Adv Exp Med Biol 494: 183-92). The protein is 1255 residues long with 17 predicted N-glycosylation sites. It has a 13aa leader peptide and a 17 aa C-terminal membrane anchoring sequence (1202-1218).

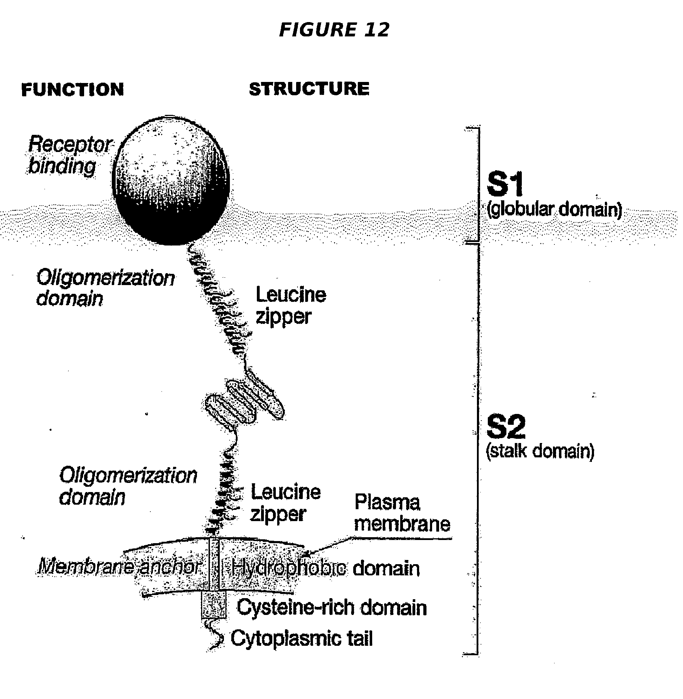

Some (MHV, HCoV-OC43, AIBV and BCoV), but not all (TGV, FIPV, HCoV-229E) coronavirus spike proteins are proteolytically cleaved in two subunits, S1 and S2. S1 is supposed to form the bulbous head, which stays non-covalently linked to the C-terminal membrane anchor. Cleavage is mediated by a basic aminoacid sequence, which resembles the consensus sequence for a furin cleavage site. (Garten et.al., Biochimie 1994; 76(3-4): 217-225). However, in case of this SARS virus isolate, we were not able to identify such a sequence, implicating that the S protein of this SARS virus isolate is unlikely to be cleaved during maturation. Secondary structure predictions indicated that the global architecture of the spike protein is conserved within all known coronaviruses. The S1 domain is mainly formed by beta sheets and likely adopts a globular fold, while in the S2 domain extensive alpha helical regions are predicted. In addition, the LearnCoil-VMF program, specifically designed to identify coiled-coil-like regions in viral membrane-fusion proteins, predicts two coiled-coils within S2, spanning aminoacids 900-1005 and 1151-1185, respectively (Figure 12). Both coiled-coil regions contain a leucine-zipper motif, which is also present in the spikes of all coronaviruses. Leucine zippers are known to promote protein oligomerization; since the spike proteins of TGV and MHV form hetero-trimers, (Delmas et al, J Virol 1990; 64(11):5367-5375) (Godeke, et al., J Virology 2000; 74(3):1566-1571) it is conceivable that in SARS leucine zippers play a role in promoting and/or stabilizing a similar quaternary structure. The spike protein plays a major role in the biology of coronaviruses because the S1 domain contains the receptor-binding domain and the virus neutralizing epitopes, while the S2 domain is involved in the process of membrane fusion, which is essential for virus infectivity. As expected, multiple sequence alignment of different spike proteins showed a major degree of variability within the S1 domain, whereas S2 is more conserved.

The envelope protein E is a very short polypeptide of 76 aa, involved in the morphogenesis of the virion envelope. (Godet et al., Virology 1992; 188(2):666-675). Computer analysis predicts a long transmembrane domain close to the N-terminus and two N-glycosylation sites. The level of aminoacid similarity with other coronaviruses is very low and the best homology is with the small envelope protein of the transmissible gastroenteritis virus (TGV).

The matrix glycoprotein (M) is a 221-residue polypeptide with a predicted molecular weight of 25 kDa. Computer analysis predicts a topology consisting of a short aminoterminal ectodomain, three transmembrane segments and a carboxyl terminus located at the interior side of the viral envelope. In analogy with the matrix glycoprotein of TGV, that of the avian infective bronchitis virus (AIBV) and that of the hypervirulent MHV-2 strain the SARS M glycoprotein is N-glycosylated at the N-terminus. SARS M protein shows highest similarity to group 2 viruses (Table 11).

Finally, the nucleocapsid protein N is a 397-residue-long phosphoprotein that interacts with viral genomic RNA to form the nucleocapsid. The level of conservation with other coronaviruses is low, ranging from 26,9% identity with the HCoV-229E to 37,4% identity to the Bovine coronavirus (BcoV) (Table 11). Epitope analysis of the nucleocapsid protein has been carried out (Li et al. (2003) Geno Prot & Bioinfo 1:198-206) in which the epitope site at the C terminus of the protein was located.

In addition to the above fundamental proteins, many viruses express a set of other peptides, which are generally dispensable for viability, but can influence the infectivity potential of the virus. (de Haan et al., Virology 2002; 296(1):177-189). These proteins are generally conserved within members of the same serogroup, but differ profoundly among the groups. For this reason, they are generally referred to as group-specific proteins. Members of the group 1, represented here by HcoV-229E, have two group-specific genes located between the S and E genes and sometimes one or two ORFs downstream of the N gene, preceding the 3' UTR region of the genome. Viruses of the group 2, with MHV as prototype, have two group-specific genes (2a and HE) between ORFlb and S, as well as other two between S and E genes. Finally, the group 3 viruses, represented by the prototype AIBV, have two group-specific genes between S and E and other two between the M and N genes.

With the exception of the hemagglutinin esterase HE, for which hemagglutinating and acetyl-esterase enzymatic activities have been demonstrated, all the other group-specific ORFs encode proteins whose role has not yet been established.

Interestingly, the arrangement of specific genes in the SARS genome is peculiar and the predicted ORFs do not display any significant homology with ORFs present in the other coronaviruses, nor with any other known protein from different organisms. Like viruses of the group 1 and 3, SARS lacks the HE hemagglutinin and does not contain ORFs between the ORFlb and the S gene. Furthermore, two predicted ORFs (ORF3 and ORF4) are encoded in the region between S and E, and superimpose for most of their length. ORF3 has an IG sequence 2 bp upstream of the ATG start codon. In contrast to the other groups, SARS contains five predicted ORFs in the region between M and N genes. ORF7 is located 10 bases downstream of the stop codon of M gene, and has an IG sequence 155 nucleotides upstream from the ATG start codon. Similarly, ORF8 and ORF10 present an 10 right upstream of their ATG start codons. On the other hand, the 5' ends of ORF9 and ORF11 shortly superimpose with the flanking genes, and for this reason they do not need an IG to activate transcription. ORF12 totally superimposes with the N gene and shares very low homology with a 22kDa protein of the MHV virus, coded in the corresponding region.

Despite the absence of indications of possible localization and function deriving from sequence similarity, ORF3, ORF7 and ORF8 contain hydrophobic segments, suggesting association with membrane structures. In addition, ORF3, the longest among the SARS specific gene, is the only one that encodes for a peptide containing a high number of predicted O-glycosylation sites (Table 11). Predicted N-glycosylation sites have been identified in ORF3, ORF11 and ORF12.

Two shorter ORFs in the non-structural regions are present.

### Phylogenetic analysis

The substitution frequency within 922 conserved bases from the *pol* gene of eleven coronaviruses from the three different serogroups has been used in the past to show that the variability within members of each serogroup is much smaller than between members of different serogroups, confirming the previously described serological groupings. (Stephensen et al., Virus Res 1999; 60(2):181-9). We used the 922 bp region of the *pol* gene of SARS and aligned it with the same fragment from other 12 coronaviruses. The tree obtained showed that the SARS virus is distinct from the other three groups of coronaviruses (Figure 13). Similar results were obtained using the full-length aminoacid sequences of *pol*, 3CL-protease and helicase from the replicase region and those of the spike and the matrix glycoproteins from the structural region (data not shown). These data confirmed that the entire genome of the SARS virus clusters in a new group (group 4) of coronavirus.

To gain more resolution for possible evolutionary relationships we performed the analysis using consensus sequences of predicted domains of the proteins. In particular, we generated consensus sequences of the S1 domain of the spike protein from the group 1 and group 2 and then we compared them to the S1 domain of the SARS spike. No consensus could be generated from the group 3 since only the spike protein of AIBV is know. Interestingly, the tree constructed from the alignment of SARS S1 with the consensus generated from the two groups of spike proteins was different from that in Figure 13, and showed a much closer relationship between SARS and group 2 coronaviruses (Figure 14A). Further analysis showed that 19 out of the 20 cysteines present in the SARS S1 domain are spatially conserved with the group 2 consensus sequence, while only five are maintained either within the group 1 and group 3 sequences (Figure 14B). Given the fundamental role played by cysteines in protein folding, it is likely that the S1 domain of SARS and group 2 coronaviruses share a similar spatial organization.

### Sequence variability between SARS coronaviruses

We compared the FRA sequence to the four complete SARS genomes available on the Web. A total of 30 mutations were detected. Nine of these mutations were silent while 21 resulted in aminoacid substitutions (Table 12). Within ORF1a, three silent and seven productive mutations were detected. In ORF1b, there were five silent and three productive mutations. One of the productive mutations was caused by two nucleotide substitutions resulting in a single aminoacid change. Five changes were located in the spike protein, four of these were productive and one silent. Two productive mutations were in ORF3 and in the matrix glycoprotein M. One productive mutation each was in ORF10 and in the nucleocapsid protein N.

The overall difference between FRA and TOR2 was of nine nucleotides resulting in two silent mutations and seven aminoacid changes. The difference between FRA and Urbani is 12 nucleotides, which result in five silent mutations and seven aminoacid changes. For CUHK 16 nucleotides were different, five of which were silent mutations. For FRA and HKU 14 nucleotide changes resulted in four silent and nine productive mutations.

### EXAMPLE 2 - Mouse Immunization with Inactivated SARS Virus (for reference)

Mice were immunized subcutaneously on days 0, 14, and 28 with 5 µg BPL-inactivated SARS-CoV particles (BPL-SARS-CoV), either alone or together with Alum or MF59 as adjuvants. Serum was collected on days 0 (pre-immunization), 13 (post 1st immunization), 28 (post 2nd), and 35 (1 week post 3rd immunization). Neutralizing antibodies were assessed for blocking SARS-CoV infection of Vero cells *in vitro.* After 3 immunizations, neutralization titers were in the range 1:100-1:1000, which are levels similar to those present in the serum of SARS convalescent patients. As shown in the following table, the non-adjuvanted vaccine induced neutralizing antibody after the third immunization, and potency of this vaccine was enhanced significantly by including the adjuvants, with neutralizing antibody appearing after then 2nd immunization and overall titers increasing after then 3rd immunization:

| | **Neutralization Titer** | | | |
|---|---|---|---|---|
| **Immunogen** | **pre** | **post 1st** | **post 2nd** | **post 3rd** |
| BPL-SARS-CoV+MF59 (5 *µ*g) | < 1:20 | < 1:20 | 1:158 | 1:630 |
| BPL-SARS-CoV+Alum (5 *µ*g) | < 1:20 | < 1:20 | 1:67 | 1:612 |
| BPL-SARS-CoV (5 *µ*g) | < 1:20 | < 1:20 | < 1:20 | 1:71 |
| PBS | < 1:20 | < 1:20 | < 1:20 | < 1:20 |

### EXAMPLE 3 - Balb/cMouse Immunization with Inactivated SARS Virus (for reference)

A Balb/c mouse model for SARS infection has been developed (Subbarao et al. (2004), J.Virol., 78:3572-77. In this model, Balb/c mice are inoculated intranasally with 10⁴ TCID₅₀ of virus. At 48 hours post-inoculation, a 2-log increase in the TCID₅₀ virus titer can be detected in the lungs of infected mice. While virus replication is readily detected, the mice do not show any SARS disease symptoms and spontaneously clear the virus one week after inoculation. A decrease in virus titer in previously-immunized animals as compared to control animals demonstrates a protective effect of the vaccine being evaluated.

In this example, four Balb/c mice per group are immunized three times with 5 µg BPL inactivated SARS-CoV (days 0,14, 28) either alone or in combination with MF59 and challenged with 10⁴ TCID₅₀ of SARS-CoV on day 43. Two days following virus challenge the mice are euthanized and SARS-CoV is quantified from nasal turbinates (NT) and lungs and the mean virus titer for each mouse is measured. Control groups received PBS alone, or an influenza virus vaccine (FLU) with or without MF59 adjuvant. Data were as follows (see also Figure 43), where four mice were tested per group and virus titers are expressed as log₁₀ TCID₅₀ per gram of tissue:

| | **Virus replication in lungs of challenged mice** | | **Virus replication in nasal turbinates of challenged mice** | |
|---|---|---|---|---|
| **Immomogen** | **# infected/ # tested** | **Mean (± SE) virus titer** | **# infected/ # tested** | **Mean (± SE) virus titer** |
| PBS | 4/4 | 6.3 ± 0.3 | 3/4 | 2.8 ± 0.35 |
| MF-59 alone | 4/4 | 6.1 ± 0.13 | 3/4 | 3.0 ± 0.38 |
| FLU vaccine (5 *µ*g) | 4/4 | 6.3 ± 0.07 | 3/4 | 2.9 ± 0.36 |
| FLU vaccine (5 *µ*g) + MF-59 | 4/4 | 6.0 ± 0.19 | 4/4 | 3.0 ± 0.11 |
| BPL-SARS-CoV (5 *µ*g) | ¼ | 1.6 ± 0.13 * | 0/4 | Not detected ** |
| BPL-SARS-CoV (5 *µ*g) + MF-59 | 0/4 | Not detected * | 0/4 | Not detected ** |

| | | | | |
|---|---|---|---|---|
| Two-tailed Student's t-test, compared to PBS-immunized mice, showed: * P<0.00001 or ** P=0.025 | | | | |

As shown, virus could not be detected in the BPL-SARS-CoV immunized mice. The lower limit of detection of infectious virus in a 10% w/v suspension of lung homogenate was 1.5 log₁₀TCID₅₀/gm, and in a 5% w/v suspension of nasal turbinates the limit was 1.8 log₁₀TCID₅₀/gm. Viral titers in the immunized mammals were thus below these threshold values.

Thus the inactivated SARS-CoV vaccine was very efficient at preventing virus infection, as only one of eight mice immunized with the vaccine, either with or without MF59 adjuvant, was infected. Similar protection was not observed in control groups of PBS diluent, MF59 adjuvant, or influenza virus vaccine with or without adjuvant.

Neutralization titers of sera taken from the animals in the challenge study were assessed at two weeks post-1st, one week post-2nd, and one week post-3rd immunization. Mice immunized with the vaccine with MF59 adjuvant had already developed a neutralization titer of 1:71 after the 2nd immunization, which increased to 1:588 after the 3rd immunization, whereas mice receiving the unadjuvanted vaccine did not have any neutralizing activity post-2nd and a neutralization titer of 1:64 post-3rd immunization. Sera from mice in each of the control groups did not show any neutralization activity. These data clearly demonstrate not only the ability of the inactivated SARS-CoV vaccine to induce protective levels of SARS neutralizing antibodies, but also a beneficial effect of formulating the vaccine with adjuvant for elevated neutralization titers.

### EXAMPLE 4 - Preparation of OMV comprising SARS viral antigens (for reference)

*E.coli* were transfected with a plasmid of interest (encoding a SARS viral antigen). Single colonies harbouring the plasmid of interest were grown overnight at 37°C in 20 ml of LB/Amp (100 µg/ml) liquid culture. Bacteria were diluted 1:30 in 1.0 L of fresh medium and grown at either 30°C or 37°C until the OD₅₅₀ reached 0.6-0.8. Expression of recombinant protein was induced with IPTG at a final concentration of 1.0 mM. After incubation for 3 hours, bacteria were harvested by centrifugation at 8 000 *x g* for 15 minutes at 4°C and resuspended in 20 ml of 20 mM Tris-HCl (pH 7.5) and complete protease inhibitors (Boehringer-Mannheim™). All subsequent procedures were performed at 4°C or on ice.

Cells were disrupted by sonication using a Branson Sonifier 450 and centrifuged at 5 000 x g for 20 min to sediment unbroken cells and inclusion bodies. The supernatant, containing membranes and cellular debris, was centrifuged at 50000g (Beckman Ti50, 29 000 rpm) for 75 min, washed with 20 mM Bis-tris propane (pH 6.5), 1.0 M NaCl, 10% (v/v) glycerol and sedimented again at 50000g for 75 minutes. The pellet was resuspended in 20mM Tris-HCl (pH 7.5), 2.0% (v/v) Sarkosyl, complete protease inhibitor (1.0 mM EDTA, final concentration) and incubated for 20 minutes to dissolve inner membrane. Cellular debris was pelleted by centrifugation at 5000g for 10 min and the supernatant centrifuged at 75000g for 75 minutes (Beckman Ti50,33000 rpm). Outer membrane vesicles were washed with 20 mM Tris-HCl (pH 7.5) and centrifuged at 75 000 x g for 75 minutes or overnight. The OMV was finally resuspended in 500 µl of 20 mM Tris-HCl (pH 7.5), 10% v/v glycerol. Protein concentration was estimated by standard Bradford Assay (Bio-Rad), while protein concentration of inner membrane fraction was determined with the DC protein assay (Bio-Rad). Various fractions from the isolation procedure were assayed by SDS-PAGE.

### EXAMPLE 5 - Tinmunogenicity, dose and route schedule for recombinant Spike protein in mice

The immunogenicity, route and dosing of the recombinant spike proteins of the invention in mice may be assessed using the below detailed protocol. Preferably, the administered antigen will elicit neutralizing antibody titers at least in the range of 1/100-1/1000. Increasing doses of antigen can be tested in the range from 5 to 20 µg of recombinant Spike antigen alone or mixed with an equal volume of MF59-citrate, administered SC or IM to anesthetized mice in 100 µl of inoculum. Groups of BALB/c mice, 6 per treatment are primed at day 0 and boosted at day 14 and 28.

| **Group** | **Treatment** | **Dose/Route** | **Sampling interval** | **Number of mice** |
|---|---|---|---|---|
| 1-3 | Rec-Spike protein | 20, 10, 5 *µ*g/SC | 7, 21, 35, 42 d | 6 per dose level |
| 4-6 | Rec-Spike protein | 20, 10, 5 *µ*g/SC | 7 | 6 per dose level |
| 7-9 | Rec-Spike protein | 20, 10, 5 *µ*g/IM | 7, 21, 35, 42 d | 6 per dose level |
| 10-12 | Rec-Spike protein | 20, 10, 5 *µ*g/IM | 7 | 6 per dose level |
| 13-15 | Rec-Spike - MF59 | 20, 10, 5 *µ*g/SC | 7, 21, 35, 42 d | 6 per dose level |
| 16-18 | Rec-Spike - MF59 | 20, 10, 5 *µ*g/SC | 7 | 6 per dose level |
| 19-21 | Rec-Spike - MF59 | 20, 10, 5 *µ*g/IM | 7, 21, 35, 42 d | 6 per dose level |
| 22-24 | Rec-Spike - MF59 | 20, 10, 5 *µ*g/IM | 7 | 6 per dose level |
| 25 | MF59 | NA/SC | 7, 21, 35, 42 d | 6 + 6 (sac d 7 and 42) |
| 27 | MF59 | NA/IM | 7, 21, 35, 42 d | 6 + 6 (sac d 7 and 42) |
| 29 | Saline | NA/SC | 7, 21, 35, 42 d | 6 + 6 (sac d 7 and 42) |
| 31 | Saline | NA/IM | 7, 21, 35, 42 d | 6 + 6 (sac d 7 and 42) |

This protocol can also be used to assess the Th1/Th2 profile of the specific immune response elicited by the recombinant Spike protein. Neutralizing and Spike-specific antibody titers will be assessed at days 7, 21, and 35; IgG2a *vs* IgG1 isotype of the Spike-specific antibodies will be determined at days 21 and 35; *in vitro* proliferation of lymph node and splenic T cells against the recombinant Spike protein will be determined at days 7 and 42, respectively; IFN-γ and IL-4 production by splenic T cell against the recombinant Spike protein from SARS-CoV will be assessed at day 42. Peripheral blood will be collected at days 7, 21, 35; lymph nodes cells at day 7, and spleen cells at day 42. Neutralizing and Spike-specific antibody titers and isotypes will be determined by inhibition of SARS-CoV infection of Vero cells and by ELISA, respectively. Proliferation of lymph node and splenic cells will be determined by ³[H]-Thymidine uptake. Frequencies of splenic IFN-γ and IL-4 producing T lymphocytes, will be determined by ELISPOT and FACS.

### EXAMPLE 6 -Immunogenicity, dosing and route schedule for Spike proteins in rabbits

The immunogenicity, route and dosing of the recombinant spike proteins of the invention in rabbits may be assessed using the below detailed protocol. Increasing doses can be tested in the range from 5 to 40 µg of recombinant Spike antigen alone or mixed with an equal volume of MF59-citrate, administered SC or IM to anesthetized animals in 200µl of inoculum. Groups of New Zealand white female rabbits, 10 per treatment, will be immunized as shown in the table below. The animals will be primed at day 0 and boosted at days 14 and 28. Peripheral blood will be collected at days 7,21, and 35. Neutralizing and Spike-specific antibody titers will be determined by inhibition of SARS-CoV infection of Vero cells and by ELISA, respectively.

| **Group** | **Treatment** | **Dose/Route** | **Sampling interval** | **Number of rabbits** |
|---|---|---|---|---|
| 1-4 | Full-length Spike protein | 40, 20, 10, 5*µ*g/SC | 7, 21, 35 d | 10 per dose level |
| 5-8 | Full-length Spike protein | 40, 20, 10, 5*µ*g/IM | 7, 21, 35 d | 10 per dose level |
| 9-12 | Truncated Spike protein | 40, 20, 10, 5*µ*g/SC | 7, 21, 35 d | 10 per dose level |
| 13-16 | Truncated Spike protein | 40, 20, 10, 5*µ*g/IM | 7, 21, 35 d | 10 per dose level |
| 17-20 | Full-length Spike protein - MF59 | 40, 20, 10, 5*µ*g/SC | 7, 21, 35 d | 10 per dose level |
| 21-24 | Full-length Spike protein - MF59 | 40, 20, 10, 5*µ*g/IM | 7, 21, 35 d | 10 per dose level |
| 25-28 | Truncated Spike protein - MF59 | 40, 20, 10, 5*µ*g/SC | 7, 21, 35 d | 10 per dose level |
| 29-32 | Truncated Spike protein - MF59 | 40, 20, 10, 5*µ*g/IM | 7, 21, 35 d | 10 per dose level |
| 33 | MF59 | NA/SC | 7, 21, 35 d | 10 |
| 34 | MF59 | NA/IM | 7, 21, 35 d | 10 |
| 35 | Saline | NA/SC | 7, 21, 35 d | 10 |
| 36 | Saline | NA/IM | 7, 21, 35 d | 10 |

### EXAMPLE 7 - Immunogenicity and dose schedule for recombinant Spike in ferrets

The immunogenicity and dosing of the recombinant spike proteins of the invention in ferrets may be assessed using the below detailed protocol. Three groups of ferrets, 6 for treatment, will be immunized with recombinant SARS-CoV Spike protein from CHO cell lines, alone or mixed with an equal volume of MF59-citrate, administered SC to anesthetized animals in 200µl of inoculum. The recombinant Spike protein vaccine will be tested at the dose eliciting the highest neutralizing antibody titers in mice at day 35 after the second boost. The animals will be primed at day 0 and boosted at day 14 and 28. Peripheral blood will be collected at days 7, 21, and 35. Neutralizing and Spike-specific antibodies titers will be determined by inhibition of SARS-CoV infection of Vero cells and by ELISA, respectively.

| **Groups** | **Treatment** | **Dose/Route** | **Sampling interval** | **Number of ferrets** |
|---|---|---|---|---|
| 1 & 2 | Rec-Spike protein | Y *µ*g or 2Y *µ*g /SC | 7, 21, 35 d | 6 |
| 3 & 4 | Rec-Spike protein + MF59 | Y *µ*g or 2Y *µ*g/SC | 7, 21, 35 d | 6 |
| 5 | Saline | NA/SC | 7, 21, 35 d | 6 |

The 3 groups of ferrets, 6 animals per group, used for the immunogenicity studies above can then be used to assess efficacy of the recombinant Spike protein in protecting vaccinated animals from infection and/or disease. Anestethized animals will be challenged two weks after the last boost intratracheally with 10⁶ median tissue culture infectious dose unit (TCID₅₀) of the SARS-CoV Utah strain. Infection by SARS-CoV will be assessed by taking nasal, faringeal and rectal swabs from animals for 20 days after challenge as described (12). The presence of SARS-CoV in sample materials will be assessed by RT-PCR and infection assay of Vero cells. Animals will be monitored for clinical signs of SARS disease by assessing sleeping time, temperature, respiratory symptoms, diarrhea, body weight and survival. Protection will be determined by the magnitude and duration of virus shedding and by duration and severity of disease symptoms and percentages of surviving animals.

### EXAMPLE 8: Expression of Spike protein for vaccination

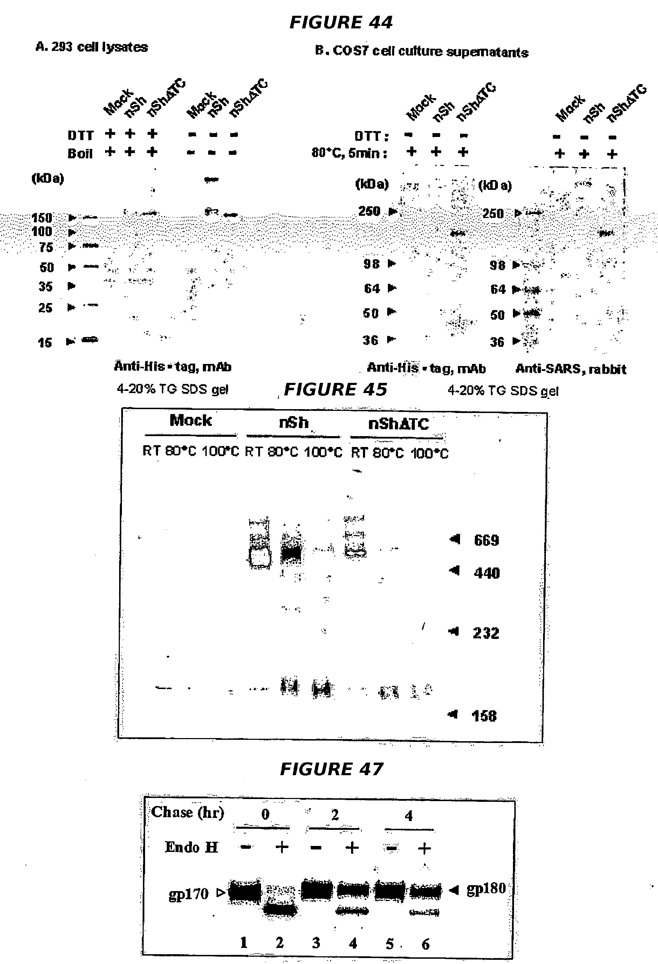

The SARS-CoV Spike glycoprotein was expressed in both full-length and truncated forms, using the nSh and nShΔTC pCMVIII constructs described above, both with hexahistidine tags. The vector constructs were evaluated for expression 48 hr after transfection into 293 cells and COS7 cells. The full-length Spike protein (nSh) was detected by western blot only in cell lysate, but not in culture media (Figure 44).

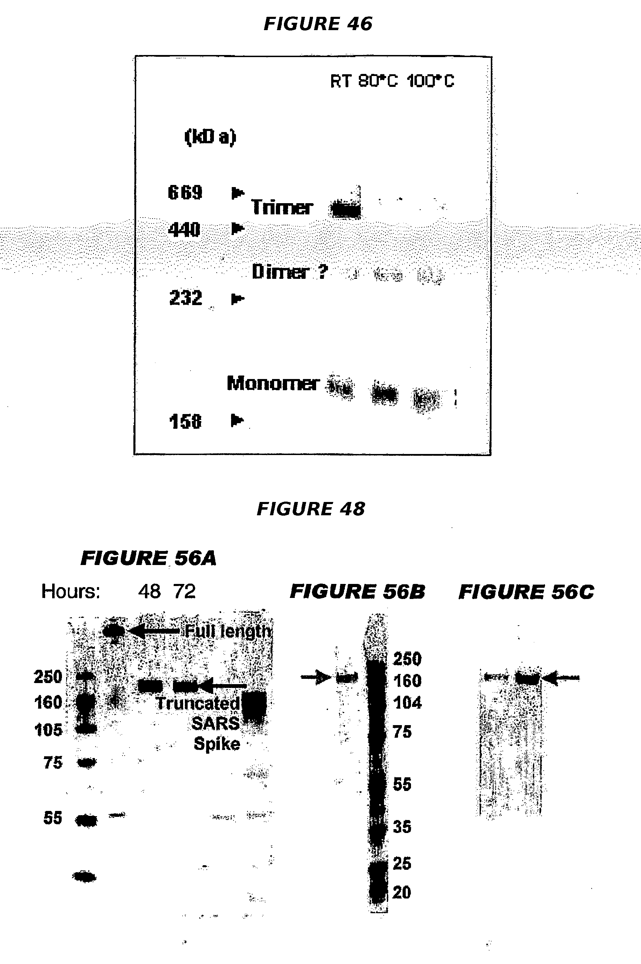

The majority of SARS-CoV full-length Spike protein was expressed in transiently-transfected COS7 cells as a high molecular glycoprotein which ran at 540 kDa in non-reducing gels (Figure 45). The gp540 is heat labile as indicated by the complete dissociation into monomeric forms (gp170 & gp180) by boiling, but it was resistant to DTT treatment. These data suggest that the recombinant Spike protein is noncovalently associated into a homotrimer (gp540). The presence of Spike protein in homotrimeric association also was confirmed in inactivated, purified SARS-CoV virion particles. Analysis of virion proteins by western blot under the same condition used for the characterization of recombinant Spike protein generated essentially identical results (Figure 46).

### EXAMPLE 9: Spike protein processing

In order to characterize Spike protein processing, BHK-21 cells were infected with alphavirus replicon particles expressing the SARS-CoV full-length Spike. At 6 hoursr post-infection with an MOI of 5, infected cells were labeled for 1 hr with L-[³⁵S]methionine/cysteine and chased for up to 4 hours. The [³⁵S]-labeled spike protein was immunoprecipitated by anti-SARS rabbit serum and digested with Endo-H. Both digested and undigested proteins were analyzed by SDS-PAGE (4% polyacrylamide). As shown in Figure 47, the full-length spike protein is synthesized as an Endo-H sensitive high-mannose glycoprotein (gp170, an ER form) that undergoes modification to an Endo-H resistant glycoprotein with complex oligosaccharides (gp180, a Golgi form). The conversion of gp170 into the gp180 form takes place within 2 hours (Figure 48).

### EXAMPLE 10: Selection of CHO cell lines for Spike protein expression

Methods for the derivation of Chinese Hamster Ovary (CHO) cell lines that stably express viral envelope glycoproteins that are conformationally intact, appropriately glycosylated and efficiently bind neutralizing antibodies are well established for HIV and HCV (Srivastava et al. (2002) J Virol 76:2835-47; Srivastava et al. (2003) J Virol 77:11244-259; Heile et al. (2000) J Virol 74:6885-92). The same techniques can be applied to SARS-CoV, to generate two different stable CHOK-1 cell lines producing either full-length or truncated SARS Spike proteins. The Spike proteins can be expressed using the constructs described herein, but without the hexa-His tags. These proteins can compared for their ability to produce neutralizing antibodies in immunized animals as well as for their expression levels in CHOK-1 cells.

A pCMV3 vector expressing Spike can be used for the derivation of stable CHOK-1 cell lines, containing the CMV enhancer/promoter, ampicillin resistance, and a fused DHFR and attenuated neomycin gene for selection purposes. Stable cell lines can produced using the neomycin selection system in CHOK-1 cells. Clones can be sequenced to verify the integrity of the insert, and transient transfections can be performed using Trans-LT1 polyamine transfection reagent (PanVera Corp., Madison, WI) to assess the expression level and also the integrity of the expressed protein by ELISA and western blot analysis.

Initial CHO cells will be selected to be free from TSEBSE contaminants and risks according to relevant regulatory standards. To construct cell lines, procedures involve transfection, primary screening with selective medium, followed by subcloning to assure purity of cell lines. Cell supernatants can be assayed using an antigen capture ELISA to quantify expression levels at all stages of selection and amplification. For full-length Spike expression, methanol fixed cells can be screened for internal expression by immunofluorescent staining using a rabbit anti-SARS antibody. Successive measurements at the T75-flask stage of expansion canbe employed to assure stability of expression levels. The molecular mass and integrity of the expressed proteins can be checked by PAGE both under native and reducing and denaturing conditions, followed by immunoprobing.

The pCMV3 vectors expressing SARS-CoV Spike proteins in either full-length or truncated forms can be introduced into CHOK-1 cells using the Trans-LT-1 reagent and non-selective media. 24-48 hours post-transfection, depending on cell density, cells are split at a 1:5 ratio and the medium can be changed to selective media containing neomycin at 500µg/ml. Any bovine serum used in these procedures will be from TSE-free sources that meet regulatory standards. Ten to fourteen days later, individual colonies can be picked and transferred to 96 well plates and cultured in complete non-selective medium. When approximately 80% of the wells are confluent, 24 hour supernatants can be screened by Spike capture ELISA. For initial expression of full length Spike protein, cells can be fixed with methanol and screened by immunofluorescent staining using a rabbit anti-SARS antibody. After low-expressing cell lines have been eliminated and there are fewer than 20-30 cell lines, capture ELISA and western blots can then be used to determine the expression level after cell lysis. A portion of each cell line can be pelleted, weighed and lysed in 1% Triton lysis buffer for determination of expression levels. Three to four clones producing the highest levels of spike protein in correct structure and conformation can be expanded to three-liter bioreactors and adapted to low serum suspension culture conditions for scale-up.

The antigen capture ELISA assay for the SARS spike protein can be performed using 96 well flat-bottom plates coated with 250ng per well of purified immunoglobulin obtained from rabbit sera that were immunized with inactivated SARS virus. Supernatant or lysate samples are added and incubated for 2 hours at 37°C. Bound antigen is reacted against pooled SARS^{+ve} serum or high affinity monoclonal antibody either human or mouse against SARS spike protein and detected using appropriate species-specific peroxidase-conjugated second antibody. The plates are developed using TMB substrate (Pierce, Rockford, IL), read at a wavelength of 450nm, and the concentration of protein per ml sample is derived from a standard curve (OD *vs*. protein concentration) based on serial dilutions of a known concentration of recombinant spike protein.

The immunoprobing analysis will also be performed following the standard methods described by Srivastava *et al.* (2002) *supra*. Briefly, 10-20µl of the sample is analyzed on 4-20% SDS PAGE under non-reducing/denaturing conditions with mild heating. The proteins are then transferred onto nitrocellulose membranes and reacted against polyclonal anti-Spike rabbit serum, followed by anti-rabbit Ig conjugated to Alexa 688 (Molecular Probes, Oregon). The blots are scanned using an infrared imaging system.

The highest expressing candidate cell lines will be screened for Spike protein expression and stability in small-scale (3 liter) perfusion bioreactors. The candidate clones will be further evaluated for level of expression as well as integrity of expressed protein, and subsequently tested for expression stability in the absence of selection. The selected clones also will be tested for maintenance of the DNA sequence integrity of the integrated SARS spike protein gene. To quickly monitor the expression levels in small flasks and in the three liter evaluation cultures, a lectin-based process (Gluvanthus Nivalis lectin) has been developed to isolate SARS spike protein to a degree of purity that allows semi-quantitation and characterization of the protein in CHO supernatant. Full-length Spike protein will be obtained from Triton X-100 detergent extracted cells and then captured on GNA lectin, followed by hydroxyapatite and SP chromatograph. Eluted protein is then characterized by: (1) polyacrylamide gel electrophoresis (PAGE) and Coomassie staining, (2) immunoprobing with anti-SARS rabbit sera, (3) structural characterization using size exclusion chromatography (SEC), as well as mass spec analysis using MALDI-TOF.

Productivity from the CHO cell line expressing SARS spike protein should be at least 2 mg/L and for full-length Spike protein will be 3mg/100gm of cells, at steady-state cell density. Yield from one 45 day, 2.5-liter bioreactor will be -1000 mg crude protein.

### EXAMPLE 11: Purification of spike protein for Human vaccines

To purify SARS spike protein for the purpose of producing GMP grade material for human use, the following basic process is used, with all steps being performed at 2-8°C: the starting material, concentrated CHO cell culture supernatant (20-30X) is thawed and filtered through a 0.45µm membrane; this material is heavily contaminated proteins from culture, as well as DNA; the first purification step is affinity chromatography using Gluvanthus Nivalis (GNA), a lectin that preferentially recognizes terminal mannose containing carbohydrates; glycosylated proteins, including SARS spike protein are captured and non-glycosylated proteins, as well as DNA, do not bind to this column; the GNA column is followed by two chromatographic steps operated in the flow through mode; the anion exchanger, DEAE, and ceramic hydroxyapatite (cHAP); DEAE binds some contaminating supernatant proteins and DNA, whereas cHAP binds any contaminating serum proteins; full-length Spike protein is purified from the cell pellet; the cells are lysed with Triton X-100 and full-length Spike protein is then captured on GNA lectin, followed by hydroxyapatite and SP chromatography.

The purified SARS spike can be further treated to remove adventitious viruses: viral inactivation at pH 3.5 for 1 hour; the sample is then concentrated and diafiltered into a buffer at pH 4 and finally captured the purified protein using SP resin; the spike protein binds to this resin and many viruses flow through.

The spike protein is eluted, concentrated and diafiltered into formulation buffer. This formulated bulk product is then filtered through a DV50 viral removal membrane followed by filtration through a 0.2 µm membrane. The formulated bulk is filled into suitable containers *e.g.* into 3.0 ml vials, in a class 100 laminar flow hood.

In process testing at each step of the purification includes protein concentration, endotoxin (LAL), bioburden, and recovery.

Prior to human administration, a test for potency will evaluate the specific ability of the vaccine in an *in vitro* or *in vivo* test to effect a given response. The *in vivo* immunogenicity will be determined by dosing groups of 10 mice with various doses of the protein antigen. Sera will be analyzed for the presence of IgG antibodies using an ELISA. The criterion for passing will be based upon the number of vaccine treated animals that are seropositive compared to a reference standard. Other tests include General Safety, sterility, purity, identity of the vaccine (using an ELISA specific for Spike protein), and quantity & protein concentration (UV spectrophotometric absorbance procedure based on the molar absorbance of the aromatic amino acids).

Stability testing will be performed on the bulk drug substance and on the final container product. Bulk product will be evaluated at temperatures of -60°C (recommended storage condition), 25 ±2 °C and 40 ±2 °C protected from light, at time points of 0, 3, 6, 9, 12 months. Final container product will be tested at temperatures of -60 °C, and inverted at 5 ±3 °C, 25 ±2 °C, and 40 ±2 °C at time points of 0, 3, 6, 9, 12 months. Stability-indicating assays may include appearance, pH, protein content, SDS-PAGE, size exclusion HPLC, and container/closure integrity, performed on single samples of bulk and triplicate vials of final container material.

The protein purified in this way can be evaluated in mice, rabbits and ferrets as described in, and based on the results of, examples 4, 5, 8 and 9 above.

Initial experiments will be performed in mice to determine optimal dose and schedule of the GMP Spike protein required to elicit the highest levels of neutralizing antibody, with titers at least in the range of 1/100 -1/1000. Spike protein will be tested in the range from 5 to 40 µg, alone or mixed with an equal volume of MF59-citrate, to anesthetized mice in 100µl of inoculum. Groups of BALB/c mice, 10 per treatment, will be immunized. The animals will be primed at day 0 and boosted at days 14 and 28. Secondary endpoints will be to compare the kinetics of neutralizing *vs*. Spike-specific antibody titers and to assess the Th1/Th2 profile of the specific immune response. Neutralizing and Spike-specific antibody titers will be assessed at days 7, 21, and 35 and at 2, 3, 4, and 5 months after priming; the IgG2a and IgG1 titers of Spike-specific antibodies will be determined at days 21 and 35, and at 2, 3,4, and 5 months after priming; proliferation and IFN-γ and IL-4 production by splenic T cell against the recombinant Spike protein from SARS-CoV will be assessed at day 42 and at the end of the 5th month. Peripheral blood will be collected at days 7, 21, and 35 and at 2, 3, 4, and 5 months after priming; spleen cells at day 42 and at the end of the 5th month. Neutralizing and Spike-specific antibody titers and isotypes will be determined by inhibition of SARS-CoV infection of Vero cells and by ELISA, respectively. Proliferation of splenic cells will be determined by ³[H]-thymidine uptake. Frequencies of splenic IFN-γ and IL-4 producing CD4+ T lymphocytes, will be determined by ELISPOT and FACS analysis.

Next, the optimal dosing and schedule for recombinant Spike vaccine will be determined in ferrets. Based on the mouse results, the Spike vaccine eliciting the highest antibody neutralizing titers will be tested against a two-fold higher dose of recombinant Spike protein given in the same formulation. Three groups of ferrets, 6 per treatment, will be immunized SC under anesthesia with 200µl of inoculum. The animals will be primed at day 0 and boosted at days 14 and 28. Peripheral blood will be collected at days 7, 21, and 35. Neutralizing and Spike-specific antibodies titers will be determined by inhibition of SARS-CoV infection of Vero cells and by ELISA, respectively. Similar to the previous ferret studies, each group of animals will be used to assess efficacy of the vaccine in protecting immunized animals from infection and/or disease.

Immunogenicity and efficacy of the candidate vaccine also will be evaluated in nonhuman primates. Three groups of adult cynomolgus macaques, 4 per treatment, will be immunized with recombinant SARS-CoV Spike protein, alone or mixed with an equal volume of MF59-citrate, administered SC to anesthetized animals in 500 µl of inoculum. The Spike protein vaccine will be tested at the dose eliciting the highest neutralizing antibody titers in ferrets at day 35. The animals will be primed at day 0 and boosted at 3 and 6 weeks. Peripheral blood will be collected at weeks 1,4, and 7. A secondary endpoint will be to assess the Th1/Th2 profile of the specific immune response, as described above (neutralizing and Spike-specific antibody titers, frequencies of peripheral blood CD4+ T cells producing IFN-γ and IL-4 in response to the recombinant Spike protein, assessed at at weeks 1, 4, and 7).

Finally, human phase I, placebo-controlled, dose-escalation, safety/ immunogenicity trials will be performed for the IM recombinant SARS vaccine with MF59 adjuvant. The trial will evaluate safety and immune responses in healthy adults following immunization with escalating doses of SARS recombinant vaccine with MF59 adjuvant, administered intramuscularly. Three/four immunizations will be given at 0, 1, 6 months. The trial will be observer blind and placebo controlled. Subjects will be randomized into each dose level. Immune response parameters to be measured include serum neutralizing antibodies, ELISA antibodies and peripheral blood IFN-γ-producing CD4+ T cells by intracellular cytokine staining:

| **Group** | **Vaccine Antigen dose (µg)** | **Administration schedule** | **No. of treated subjects** | **No. of subjects with placebo (MF59)** | **Sampling interval** |
|---|---|---|---|---|---|
| A1 | 50 | 0,1,6 months | 18 | 6 | 0, 1, 2, 6, 7 months |
| A2 | 100 | 0,1,6 months | 18 | 6 | 0, 1, 2, 6, 7 months |

### EXAMPLE 12: Comparison of inactivated virus and purified Spike protein

Immunogenicity and efficacy of the inactivated virus vaccine and the purified Spike protein can be compared in non-human primates. Three groups of adult cynomolgus macaques, 4 for treatment, will be immunized with recombinant SARS-CoV Spike protein from CHO cell lines or with BPL-SARS-COV, given in the dose and formulation eliciting the highest neutralizing antibody titers in previous immunogenicity challenge experiments, administered SC to anesthetized animals in 500µl of inoculum. The animals will be primed at day 0 and boosted at 3 and 6 weeks. Peripheral blood will be collected at weeks 1,4, 7. A secondary endpoint will be to assess the Th1/Th2 profile of the specific immune response, as described above.

| **Group** | **Treatment** | **Dose/Route** | **Sampling interval** | **No. of macaques** |
|---|---|---|---|---|
| 1 | Rec-Spike protein + or - MF59 | Y *µ*g /SC | 1,4, 7 w | 4 |
| 2 | BPL-SARS-CoV + or - MF59 | Y *µ*g/SC | 1,4, 7 w | 4 |
| 3 | Saline | NA/SC | 1,4, 7 w | 4 |

### EXAMPLE 13: Expression in yeast

Yeast is a useful and inexpensive eukaryotic expression system. Yeast-expressed proteins are used in recombinant hepatitis B virus vaccines, and recombinant SARS antigens may also be expressed in yeast for vaccine purposes. Yeast-expression is also convenient for the production of antigens for preparing monoclonal and polyclonal antitobodies, or for use in serological assays.

The nucleocapsid protein (N) and two different versions of the spike glycoprotein (S) from SARS coronavirus FRA strain (AY310120) were cloned for expression in *S.cerevisiae:*
SARS N: aa 1 - 422 (coordinates 28120-29388 of AY310120 strain) Fig.57
SARS spike: aa 14 -1195 (transmembrane domain and cytoplasmic tail deleted) Fig.58
SARS spike: aa 14 - 662 (S1 domain)

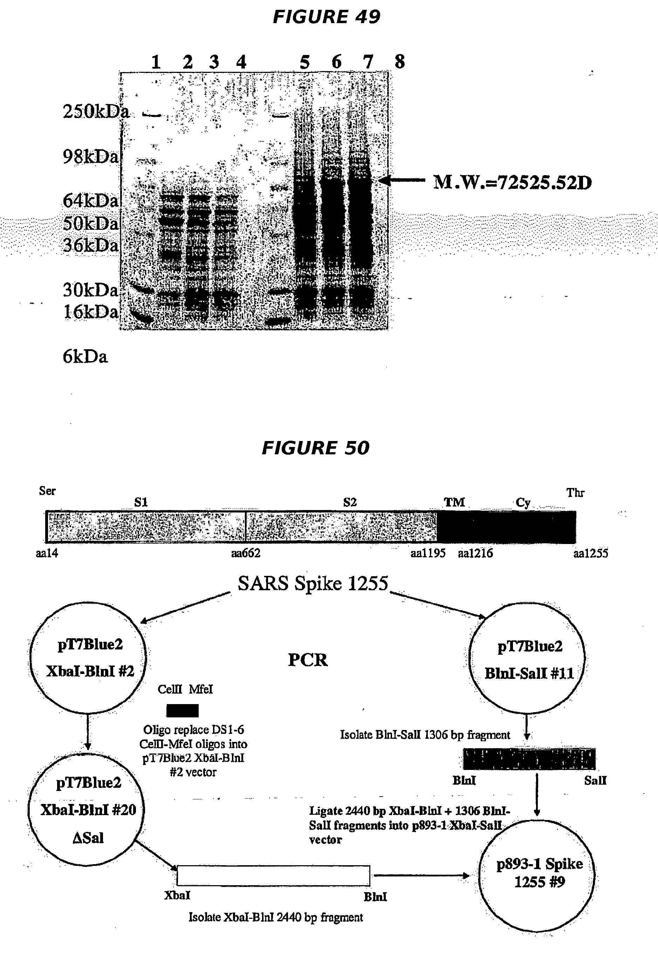

To make the S1 construct, a XhoI-NotI fragment of approximately 3733bp encoding the full-length spike glycoprotein was the starting point. PCR was used to amplify the full-length gene in two pieces: XbaI-BlnI of 2440bp and BlnI-SalI of 1306bp. These fragments were subcloned into commercial vectors (Novagen): pT7Blue2 XbaI-BlnI (5' end of spike glycoprotein) and pT7Blue2 BlnI-SalI (3' end of spike glycoprotein; Figure 50), respectively. The following primers were used in the subsequent PCR reactions: Spk-1 (5') SEQ ID NO: 9785; Spk-2 (5') SEQ ID NO: 9786; Spk-3 (5') SEQ ID NO: 9787; Spk-4 (5') SEQ ID NO: 9788.

*E. coli* HB101 competent cells were transformed with the PCR ligation product and plated on Luria agar plates, containing 100µg/ml ampicillin. The desired clones were identified using miniscreen DNA analysis. After sequence verification and plasmid amplification of the desired subclones, it was desirable to eliminate the internal SalI site present in the XbaI-BlnI portion of the spike sequence in order to facilitate future cloning into the yeast expression vector (BamHI-SalI). Therefore, we prepared a CelII-MfeI vector from the pT7Blue2 XbaI-BlnI (5' end Spike) subclone to eliminated a 143bp sequence containing the SalI site. Kinased oligos DS1-6 (SEQ ID NOS: 9789-9794) were then ligated into the CelII-MfeI vector to replace the 143bp that were removed to mutate the SalI site (no aa changes), creating pT7Blue2.XbaI-BlnIΔsal.

The 5' XbaI-BlnI (from pT7Blue2.XbaI-BlnI ΔSal) and the 3' BlnI-SalI (from pT7Blue2 BlnI-SalI) spike glycoprotein inserts were gel-purified and ligated them into the p893-1 XbaI-SalI vector (a vector derived from pLitmus 38 (New England Biolabs) with the alpha-factor leader sequence cloned into the BamHI -SalI sites of the MCS). The resulting full-length SARS Spike coding sequence was named p893-1.SARS Spike 1255 #9 (Figure 50).

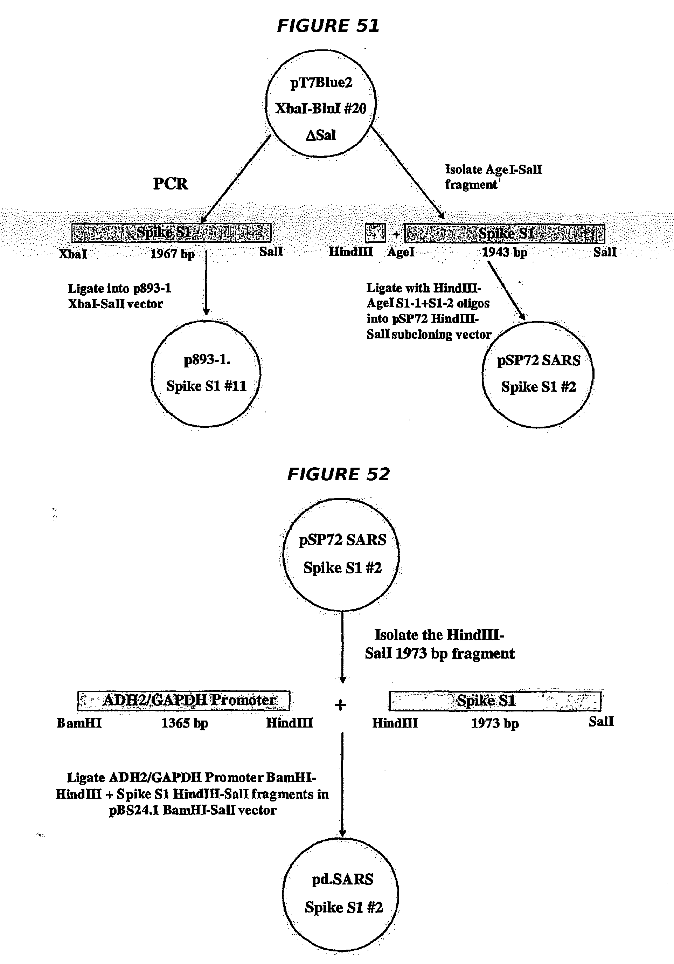

*E.coli* HB101 competent cells were transformed with the oligo replacement ligation product and plated on Luria agar plates, containing 100µg/ml ampicillin. The desired clones were identified using miniscreen DNA analysis. After sequence verification of the positive clones, pT7Blue2 Xba-Bln ΔSal was chosen for use as a template for PCR reactions to amplify the Spike S1 1967 bp Xba-Sal fragment. The fragment was then subcloned into the p893-1 Xba-Sal vector, sequence verified, and named it p893-1.Spike S1 #11 (Figure 51).

In order to clone into the *S.cerevisiae* expression vector, pBS24.1, the 5' end of the S1 sequence had to be modified from XbaI to HindIII to allow ligation with the 3' HindIII end of the ADH2/GAPDH BamHI-HindIII promoter fragment. From pT7Blue2 Xba-BlnΔSal (described above) an AgeI-SalI 1943bp fragment was gel-purified. This fragment was ligated along with a synthetic pair of HindIII-AgeI 30bp kinased oligos (S1-1+S1-2 creating the necessary 5'HindIII site) into the pSP72 HindIII-SalI commercial subcloning vector (named pSP72.SARS Spike S1 #2; Figure 51). S1-1 had SEQ ID NO: 9795 and S 1-2 has SEQ ID NO: 9796.

After sequence verification of the positive clone from miniscreen DNA analysis, the HindTII-SalI fragment was gel purified. The 1365 bp BamHI-HindIII ADH2/GAPDH promoter fragment was ligated along with the 1973 bp HindIII-SalI S1 fragment into the pBS24.1 BamHI-SalI vector creating the genetically engineered pd.SARS Spike S1 #2 expression plasmid (Figure 52).

*S.cerevisiae* strain AD3 was transformed with pd.SARS Spike S1 #2 and single transformants were checked for expression after depletion of glucose in the medium. The recombinant protein was expressed at high levels in yeast, as detected by Coomassie blue staining. In particular, yeast cells were transformed with the SARS S1 expression plasmid using the Invitrogen S.c. EasyComp™ Transformation Kit. Expression in shown in Figure 49.

To express Spike 1195 protein, which does not contain the trans-membrane (TM) region or cytoplasmic tail that are present in the full-length SARS construct, the following series of genetic manipulations was performed:

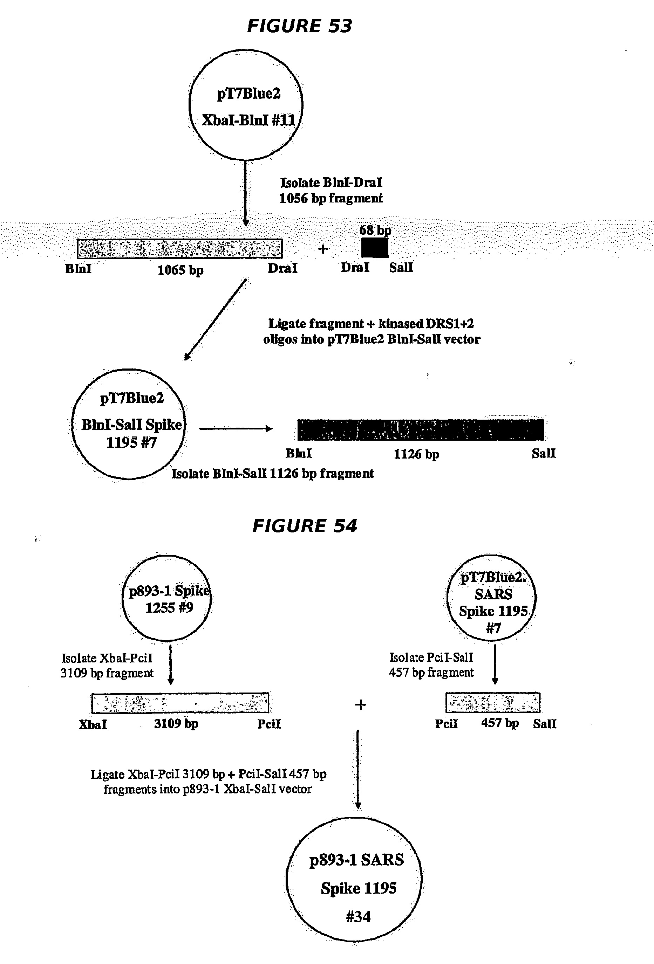

From pT7Blue2 BlnI-SalI #11 (described above) a BlnI-DraI 1056bp fragment was gel purified. This fragment was ligated with a synthetic pair of 68bp DraI-SalI kinased oligos (DRS1+2; SEQ ID NOS: 9797 & 9798) into a pT7Blue2 BlnI-SalI vector (Figure 53). *E.Coli* HB101 competent cells were transformed with the oligo replacement ligation product and plated on Luria agar plates, containing 100µg/ml ampicillin. The desired clones were identified using miniscreen DNA analysis. After sequence confirmation the clone was named pT7Blue2 BlnI-Sal Spike 1195 #7. The 1126bp BlnI-SalI fragment encoding the 3' end of the Spike 1195 was gel purified (Fig.53).

In order to generate the XbaI-SalI Spike 1195 fragment, the 3109bp XbaI-PciI fragment was isolated from the p893-1.SARS Spike 1255 #9 (described above) and a 457bp PciI-SalI fragment from pT7Blue2.SARS Spike 1195 #7 (described above). The two fragments were cloned into the p893-1 XbaI-SalI vector, creating the p893-1.SARS Spike 1195 #34 plasmid (Figure 54).

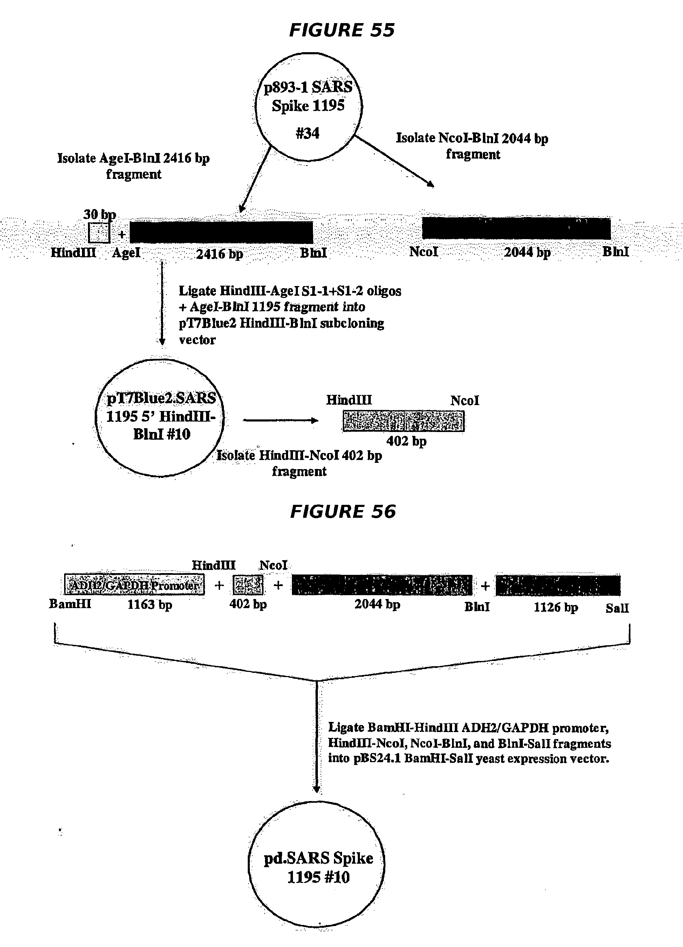

To clone SARS Spike 1195 into the pBS24.1 Saccharomyces cerevisisae expression vector, it was necessary to modify the 5' end of the SARS Spike 1195 from XbaI to HindIII, as done for the Spike S1 expression clone described above. To begin, the 2416bp AgeI-BlnI fragment was isolated from p893-1.SARS Spike 1195 #34. This fragment was ligated with the synthetic HindIII-AgeI 30bp oligos (described above to generate the S1 protein for expression in *S.cerevisiae*) into the pT7Blue2 HindIII-BlnI vector. E. coli HB101 competent cells were transformed with the oligo replacement ligation product and plated on Luria agar plates, containing 100µg/ml ampicillin. The desired clones were identified using miniscreen DNA analysis. After sequence verification of the positive clone and plasmid amplification of pT7Blue2.SARS 1195 5' HindIII-BlnI #10 (Figure 55), we isolated a 402bp HindIII-NcoI fragment and the 2044bp Ncoi-BlnI fragment (Figure 55). It was necessary for the HindIII-BlnI isolation to be done in two steps to avoid cloning issues related to the internal HindIII site located at nucleotide number 1319 of the spike 1195 protein.

To assemble the BamHI-SalI-expression cassette of Spike 1195 into the pBS24.1 vector *E.coli* HB101 competent cells were transformed with the the BamHI-HindIII (ADH2/GAPDH promoter), HmdIII-NcoI 402bp fragment, NcoI-BlnI 2044bp and the BlnI-SalI 1126bp fragments into the pBS24.1 BamHI SalI vector. The samples were plated on Luria agar plates, containing 100µg/ml ampicillin. The desired clone was identified using miniscreen DNA analysis, thus creating the genetically engineered pd.SARS Spike 1195 #10 (Figure 56).

*S.cerevisiae* strain AD3 was transformed with pd.SARS Spike 1195 #10 and single transformants were checked for expression after depletion of glucose in the medium. The recombinant protein was detected by Coomassie blue staining. In particular, yeast cells were transformed with the SARS 1195 expression plasmid using the Invitrogen S.c. EasyCompT™ Transformation Kit.

### 14:EXAMPLE 14: Expression in mammalian cell lines

cDNA fragments containing the S protein ORF of 1255 amino acids were amplified by RT-PCR from SARS viral RNA (Frankfurt isolate) grown in Vero cells. The amplified PCR fragments were cloned into pBlueScript vector, sequenced, and consensus spike sequence was assembled to create a full-length SARS spike clone, pBSnSh. *In vitro* transcription of pBSnSh followed by translation in a rabbit reticulocyte lysate resulted in the production of single polypeptide with an estimated molecular mass of -140 kDa.

The insert of this plasmid was recloned via XhoI and Not I into a mammlian expression vector pCMVIII (Srivastava et al. (2003) J. Virol. 77:11244-11259) to create a construct, nSh (Fig. 66A). A PCR fragment containing a spike protein of 1195 amino acid, which was deleted for transmembrane (TM) domain and cystein-rich cytoplasmic tail (Cy) was amplified and cloned pCMVIII vector to generate the contstruct nShΔTC (Figure.66B). Both constructs were tagged with six histidine residues at the C-terminus in order to aid in their characterization. The Xho I/Not I fragment without a histidine tag also was subcloned into the alphavirus replicon vector backbone pVCRchim2.1 for use in the production of an alphavirus replicon particle chimera that expresses S protein. Production and characterization of the replication defective alphavirus vector particles was performed essentially as described previously (Perri et al. (2003) J. Virol. 77:10394-10403; Polo et al. (1999) PNAS USA. 96:4598-4603). The resultant alphavirus vector particles were named as VEE/SIN.

COS7 cells and BHK-21 cells were maintained in Dulbecco's modified Eagle's medium supplemented with 10% fetal bovine serum at 37°C and 5% CO₂ in air. COS7 cells were transfected with expression plasmids (nSh, nShΔTC) using a transfection kit (TransIt-COS, Mirus) following the manufacturer's protocol. The cells were washed once with ice-cold PBS and lysed with 1x Lysis buffer (20mM MOPS, 10mM NaCl, 1.5mM MgCl₂, and 1% Triton X-100) containing complete mini protease inhibitor (Roche). After a 30-min incubation on ice, the debris was cleared by centrifugation. The cleared lysate was either purified or used directly in western blotting.

To purify secreted spike proteins, medium from transfected cells was collected and subjected to centrifugation at 12,000 rpm for 10 min to remove cellular debris. The cleared medium was applied to a ConA-agarose column (Vector Lab). The column was washed extensively with 20mM sodium phosphate buffer, and then the bound proteins were eluted with 1M methyl α-D-mannopyranoside (MMP), 1M NaCl in 20mM sodium phosphate buffer. Column fractions containing SARS-CoV spike proteins were applied to MagneHis Protein purification system (Promega) following the protocol suggested by the manufacturer.

For western blot analysis, proteins were separated by 4-20% SDS-PAGE and then transferred electrophoretically to nitrocellulose membrane (Invitrogen). Membrane was blocked in blocking buffer (5% skim milk and 0.1% Tween 20 in PBS) and incubated with indicated antibody at room temperature for 1 hr, washed and probed with horseradish peroxidase (HRP)-conjugated secondary antibody (Biosource) followed by chemiluminescence (ECL system, Amersham) and exposed by X-ray films. The antibodies used were a mouse monoclonal anti-histidine antibody (anti-His•tag Mab, Novagen), a rabbit polyclonlal antipeptide antibody against SARS-CoV spike proten (SmPab, Abgent), or rabbit anti-SARS sera (2BE) obtained by immunization of rabbits with purified SARS-CoV virion. The latter has a cell culture neutralizing titer of 1/2,500. Unless stated otherwise, antibody was used at 1/1,000 for anti-histidine antibody and SmPab and 1/10,000 for anti-SARS rabbit sera.

Some spike proteins were treated with Peptide-N glycosidase F (PNGase F). Cell lysates were diluted in 0.5% SDS and 1% β-mercaptoethanol and denatured at 100°C for 10 min. After 2-fold dilution with 1% NP-40 in 50mM sodium phosphate (pH 7.5), the samples were treated with PNGase F (NEB) at 37°C for 1 hr. Enzyme-treated samples were analyzed by 4-12% SDS-PAGE in reducing condition. For a partial digestion with the PNGase, the cell lysates were diluted with 50mM sodium phosphate (pH 6.0) containing 0.75% Triton-X and treated with PNGase F (Calbiochem) at 37°C for 3 hr. Enzyme-treated samples were analyzed by 4-20% SDS-PAGE in nonreducing condition.

Western blots of cells 48-hours after transfection are shown in Figure 67. The S protein was detected in cell lysates as a doublet with estimated molecular weight of -170 -180 kDa, when the lysate was boiled and analysed under reducing SDS-PAGE conditions (Fig. 67A, lane 3). This doublet appears to result from differential glycosylation of one polypeptide product since pre-treatment of the cell lysate with PNGase F reduced the doublet to a single species of -140 kDa (Fig. 67A, lane 4). This is the expected size predicted from the aa sequence for a full-length, intact polypeptide product. This experiment indicates that the full length SARS-CoV S is expressed in mammalian cells as a single, uncleaved polypeptide, but in two differentially glycosylated forms, gp170 and gp180 respectively. Unlike the two S glycoforms encoded by the full-length sequence, none of which were secreted, the SΔ protein product was detected both in cell lysates (Fig. 67A, lane 5) as well as in the cell culture medium (Fig. 67B, lane3) as a single species of ~ 160 kDa.

In order to further characterize the intracellular processing of the S protein, and as described above, BHK21 cells were infected with defective alphavirus particles expressing the full-length S. At 6 hr post infection with a MOI of 5, infected cells were pulse labeled for 1 hr with L-[³⁵S] methionine/cysteine and chased for 2 or 4 hours. The [³⁵S]-labeled S protein was immuno-precipitated using the rabbit antiserum raised against inactivated, purified virus and then digested with Endo H. The Endo H treatment involved dilution with a sample buffer (50mM sodium phosphate, 0.1% SDS, 50 mM DTT, pH 6.0) and boiling for 5 min. After denaturation, the samples were further diluted with 0.75% Triton-X 100 and treated with endoglycosidase H (Endo H) following manufacturer's protocol (Calbiochem) for 3 hr at 37°C. Enzyme-treated samples were added with gel loading buffer containing 0.1% SDS and DTT and analyzed by 8% SDS-PAGE.

Both digested and undigested proteins were boiled in SDS and analysed by reducing SDS-PAGE (Figure 47). After a 1-hr pulse, the S protein was apparent as a single gp170 component that was Endo H sensitive (lanes 1 and 2). After a 2-hr chase, a new species (gp180) was present along with gp170 in approximately equal proportions (lane3). After a 4-hr chase, the gp180 species was the dominant S protein component (lane 5) that was now Endo H resistant (lanes 5 and 6). This data is consistent with gp170 being an ER-resident glycoprotein containing high mannose chains and with gp180 corresponding with a Golgi-processed glycoprotein containing Endo H-resistant complex oligosaccharides.

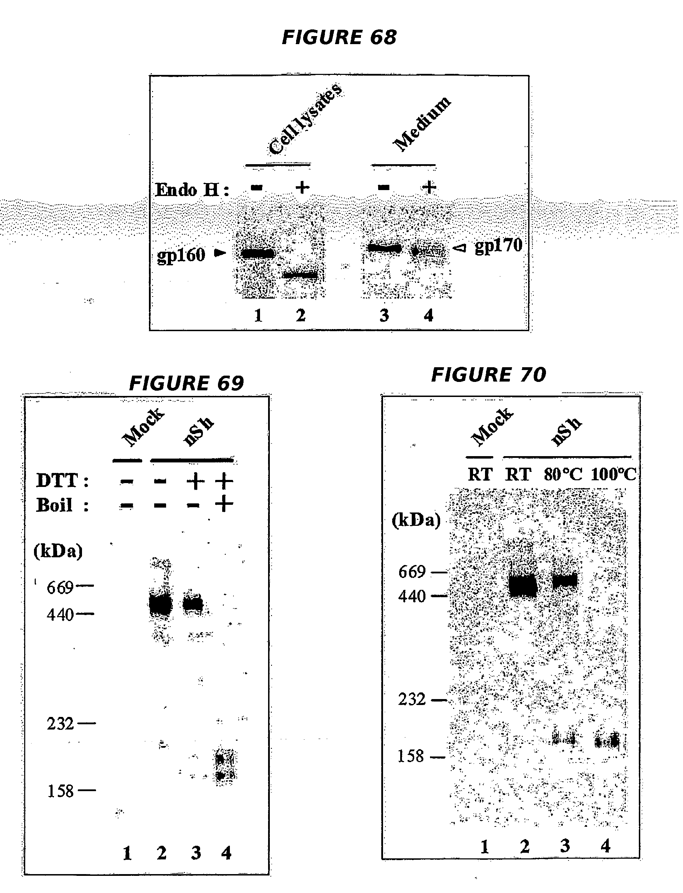

The Endo H sensitivity of the C-terminus deleted SΔ protein purified from cell culture media ws also tested. As shown in Figure 68, the SΔ observed within cell lysates was found to be Endo H sensitive (lanes 1 and 2), while the secreted SΔ in cell culture media was Endo H resistant (lanes 3 and 4). This result is consistent with this glycoprotein being synthesized in an immature form in the ER prior to transfer to the Golgi where the complex carbohydrate is added and the protein then secreted.

As already described, the S protein expressed in COS7 cells was detected as a gp170/gp180 doublet in western blot analyses of cell lysates that were fully denatured by boiling in the presence of DTT. However, the majority of S protein was detected as a high molecular glycoprotein in the 440-669 kDa range when the same cell lysate was not heat-denatured prior to western blot analysis using SDS-PAGE (Fig. 69, lane 1). The ~500 kDa species was resistant to 10 mM DTT treatment (lane 3) and not dissociated into the monomeric form unless the lysate was first heat-denatured at 100°C (lane 4). In contrast, oligomeric form of a test protein (Thyroglobulin) of which quaternary structure is held by disulfide-linkage was converted into subunit form by the 10 mM DTT treatment. These data suggest that the -500 kDa oligomeric form of S protein is not disulfide-linked and is heat labile. To confirm the heat-sensitivity of the ~500 kDa species of S protein, the heat-denaturation experiment was repeated but without DTT. As shown in Figure 70, heat denaturation of -500 kDa protein at 100°C alone was sufficient to convert it into gp170/180 monomeric forms (lane 4). Using a 80°C heat-denaturation step, both the ~500 kDa and monomeric forms were detectable in similar proportion (lane 3).

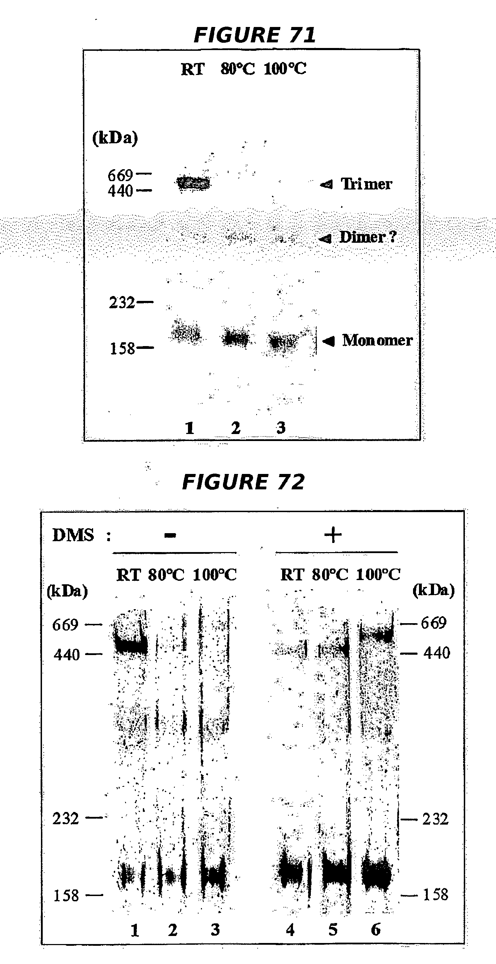

In order to investigate further whether this -500 kDa species represents an S protein oligomer in native conformation, comparative analyses with virion-derived S glycoprotein derived from Vero cell cultures was performed. The purified virions were solubilised in 1% SDS prior to Western blot analyses after SDS PAGE. The presence of the -500 kDa spike protein oligomer was confirmed in virion particles (Fig. 71, lane 1). In addition, heat denaturation of solubilised virions produced the same oligomer-to-monomer conversion as seen with the full-length recombinant S (lanes 2,3). The oligomeric nature of virion S was further analysed in a cross-linking experiment. Aliquots of inactivated virion from sucrose gradient fractions were treated with 10% SDS at 1% final concentration and diluted 2-fold with 0.2M Triethanolamine-HCl (pH 8, Sigma); Dimethyl suberimidate (DMS; Pierce Chemical Co.) was then added from a freshly prepared solution (10mg/ml in 0.2M Triethanolamine-HCl) at 3.3mg/ml final concentration. After 2 hr at room temperature, samples were concentrated with Centricon-30 and analyzed by silver staining after electrophoresis on a 4% polyacrylamide gel. Both untreated and DMS cross-linked virion proteins were heat-denatured, and the heat effect on the maintenance of oligomer structure was analysed by SDS-PAGE and silver staining (Figure 72). In the absence of cross-linking, heat denaturation resulted in the replacement of the ∼500 kD spike protein species with the monomer species. In contrast, in the cross-linked proteins, the levels of the -500 kD and monomer species did not change significantly after heating. These data support the fact that the -500 kD protein is an oligomer of S monomer proteins that are bound non-covalently. After cross-linking and boiling, the -500 kDa species migrated as a somewhat slower diffuse form than the untreated form. This mobility shift is probably due to a structural change resulting from boiling. In addition, a minor protein species of -300 kD, which may represent a non-dissociated S dimer, could be seen.

To estimate more precisely the size of the recombinant ∼500 kDa S species expressed in COS7 cells, a COS7 cell lysate containing the S protein oligomer was fractionated using size-exclusion column chromatography. The major portion of the -500 kDa oligomer co-eluted with a 572 kDa marker protein. Taken together, these experiments suggest that the -500 kDa S species seen in COS7 cell lysates is probably a homotrimer of the S protein monomer.

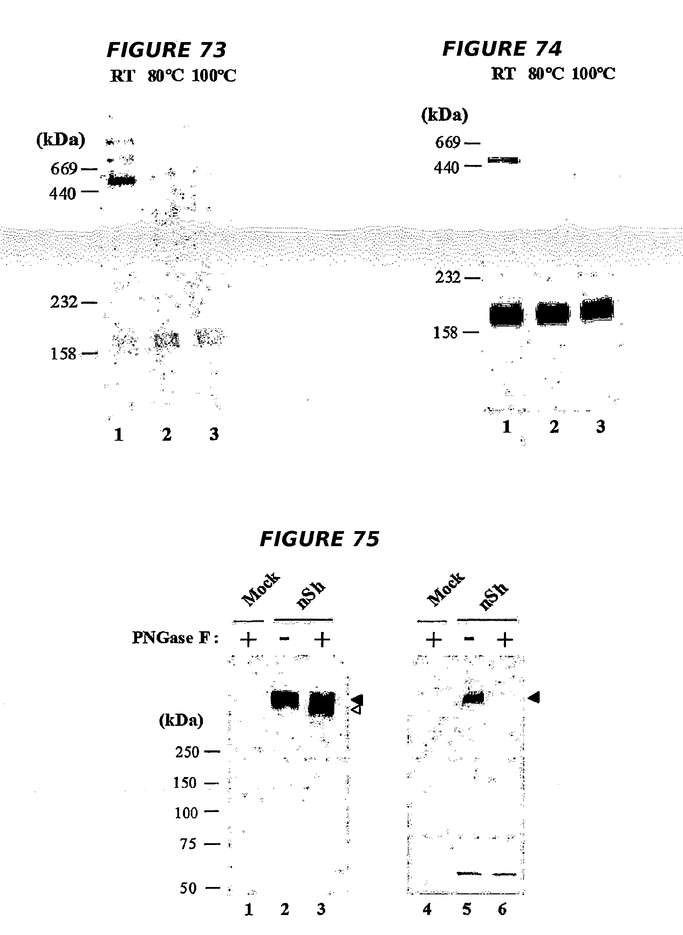

The oligomeric status of the SΔ spike protein was also examined after expression in COS7 cells. As shown in Figure 73, the recombinant SΔ proteins present in cell lysates were also detected in high molecular weight forms of -500 kDa range when the lysate was not heated prior to SDS-PAGE and Western blot analysis (lane 1). However, the efficiency of oligomerization by intracellular SΔ protein appears to be much less (<10%) compared to that of full-length S protein under the same western analysis conditions. A heat-sensitivity test on this -500 kDa protein showed that the SΔ oligomer was more heat labile than that of the full-length S oligomer, as demonstrated by the >90% conversion of all of the -500 kDa species into monomeric Sd forms at 80°C (lane 2). Also (Figure 74), the majority of the secreted SΔ protein was found in monomeric form with the -500 kDa species barely detectable (and only detectable when the protein was loaded in excess for Western analysis) (lane 1). At a temperature above 80°C, all secreted SΔ proteins were detected as monomers (lanes 2, 3).

The ∼500kDa protein is glycosylated, and the effect of deglycosylation on its antibody binding was investigated. The recombinant COS7 lysate was treated with PNGase F under non-denaturing condition (as described above) and analysed by western blot. As shown in Figure 75, deglycosylation did not affect the binding of anti-histidine Mab antibody to the treated S oligomer (lanes 2,3). However, it compromised the reactivity with the rabbit antisera raised against purified virus (lane 6). This antiserum binds to virion-derived S in western blot analyses only when DTT is omitted from the sample for SDS-PAGE indicating that it recognizes primarily a discontinuous, conformational epitope(s). This antisera has also been shown to have a high-titer of viral neutralizing antibodies. Its lack of binding to deglycosylated, recombinant S suggests that the carbohydrate actively contributes to the higher order, native structure of the S polypeptide oligomer.

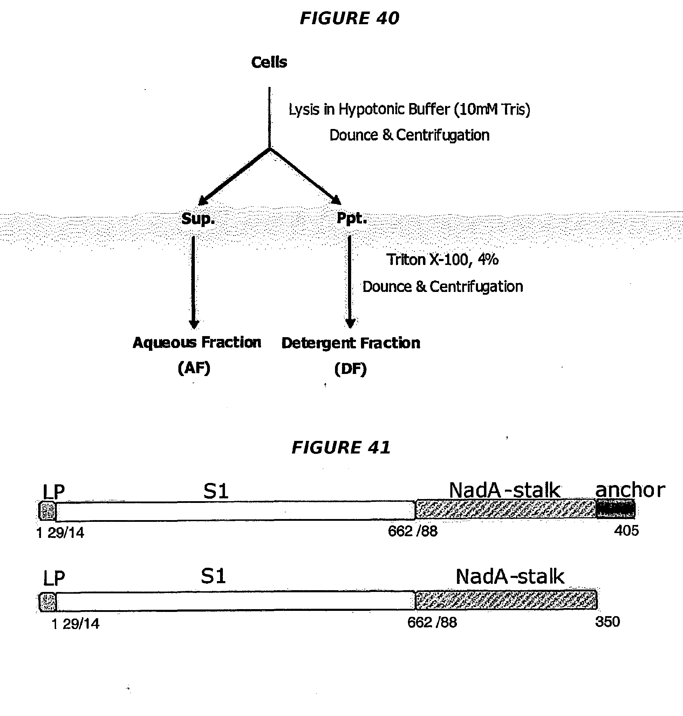

The difference between the recombinant S and SΔ protein is the presence or absence of the TM-and Cys-rich domains at the C-terminus. This difference predicts that full-length S would be found associated with the membrane fraction while Sd would be in the soluble fraction upon lysis of transfected cells. Therefore, nSh- or nShΔTC-transfected cells were lysed under hypotonic conditions and the soluble cytosol fraction was separated from the insoluble membrane fraction by centrifugation (Figure 40). As shown in Figure 76, the S protein was found in the membrane fraction (DF) both as a ~500 kDa and 180/170 kDa species (lane 4) but was not detectable in the soluble cytosol fraction (AF) (lane 3). However, the truncated SΔ protein was found as a monomeric species (gp170) in both fractions (lanes 5,6). This indicates that the C-terminal TM and Cys-rich domains are required for the anchorage of the S protein to cell membrane.

The cellular location of the S and SΔ proteins in COS7 cells was analyzed by indirect immunofluorescence microscopy. At 48 hr post-transfection, cells were directly fixed with 2% paraformaldehyde without detergent for cell surface staining or treated with detergent followed by Cytofix/Cytoperm solution for intracellular staining. Fixed cells were then stained with rabbit anti-SARS sera (2BE) and FITC-conjugated antibody. The nSh-transfected cells showed foci of S protein indicative of Golgi-localisation (Figure 77A), while the nShΔTC-transfected cells displayed a uniform distribution of SΔ protein throughout the cytoplasm indicative of ER localisation (Figure 77B). While the complete S protein was also observed on the surface of transfected cells in unfixed cells (Figure 77D), the SΔ was undetectable on the cell surface (Figure 77E). These results indicate the role played by the TM-and Cys-rich domains in anchoring the S protein to the plasma membrane. Although the TM-region alone is likely responsible for membrane anchorage, the potential role played by the Cys-rich region remains to be determined.

The SARS recombinant full-length S protein is thus an N-linked glycoprotein with an estimated molecular weight of 170-180,000 kDa. Deglycosylation with PNGase F resulted in a polypeptide of the expected size for the uncleaved, encoded polypeptide (140kDa). Both transient and stable expression of the full-length SARS-CoV S gene in a variety of mammalian cells, including COS7, 293, BHK21, and Huh7 cell lines, consistently produced a S protein doublet (gp170/180) as detected in western blot analyses. Pulse-chase analyses of transfected cells demonstrated that the SARS CoV S protein was initially synthesized as an Endo H sensitive gp170 species followed by the gradual appearance of an Endo H resistant gp180 form, presumably as a result of the addition of complex carbohydrate within the Golgi apparatus.

The recombinant S protein was not secreted into the cell culture medium unless the C-terminal 60 amino acids containing the TM-region and the Cys-rich tail were deleted.

The quaternary structure of the full-length recombinant S protein was investigated using cross-linking treatment, heat-denaturation, and size fractionation analyses. The results data are consistent with the recombinant S protein existing as a homotrimer of ~500kDa. Similar analyses of virion-derived S yielded the same results. Such a trimeric structure has been reported for other enveloped RNA viruses: the hemagglutinin HA of influenza virus, the E1-E2 heterodimer of alphaviruses and the G protein of vesicular stomatitis virus. Incubations under reducing conditions indicate that the SARS-CoV S trimeric structure is non-covalently associated, and is very stable. S oligomers present in the cell lysate were shown to be resistant to reduction by 10 mM DTT, detergent treatment with 1% SDS, and heat denaturation at up to 60°C. Incubation at a temperature higher than >80°C resulted in the dissociation of the trimeric complex as evidenced by the decrease in trimer with the concomitant increase in the monomer bands. The temperature-induced appearance of the high-mannosylated gp170 (ER monomer form) as well as the complex-glycosylated gp180 (Golgi monomer form) suggests that trimerization can occur before the transport of the monomer spike protein to the medial Golgi apparatus. This is consistent with other reports for TGEV, influenza virus HA, and vesicular stomatitis virus G proteins. With these proteins, trimerization was reported to take place before addition of complex oligosaccharides in the Golgi apparatus.

The C-terminally truncated form of S was found in the cell lysate in both oligomeric and monomeric forms at a frequency of 10% and 90%, respectively. The truncated protein secreted into medium was found fully glysosylated and it was essentially all in monomeric form. We conclude that the C-terminal 60 amino acids of the S glycoprotein contains a membrane anchor region that affects the efficiency of trimerization. In S protein trimerization, it is possible that the C-terminal region is required to initiate the event and the triple-stranded coiled coil structures in the S2 stalk domain provide further stabilizing force as seen in HA oligomer of influenza virus.

### EXAMPLE 15: CHO cells for Spike protein expression

CHO cell lines that stably express either the full-length or truncated SARS-CoV spike proteins were prepared. Several stably transfected CHO cell lines were obtained, and Figure 65 shows western blot data from a panel of representative clones.

### EXAMPLE 16: Expression in E.coli

All SARS-CoV ORFs (Figure 10, Table 10) were cloned in the pET vector and expressed as C-terminal His-Tag fusion proteins in *E. coli.* The proteins smaller than 16KD were also expressed as N-terminal GST (Glutathione S-transferase) fusion proteins using pGEX vector.

Nsp1 and Nsp2, the two SARS-CoV proteins with proteolytic activity, were not expressed as full length proteins due to toxicity in *E*.*coli*. The respective genes were instead cloned in different portions in order to separate the catalytic residues (Cys833/ His994 for Nsp1; His41/Cys145 for Nsp2) in the resulting recombinant proteins: Nsp1A from nucleotides 2719-5214 of AY310120; Nsp1B from nucleotides 5218-7371; NsplC from nucleotide 7372-9984; Nsp2A from nucleotide 9985-10416; Nsp2B from nucleotide 10476-10902.

Nsp9 was divided into two portions: Nsp9A from nucleotide 13371-14756; Nsp9B from nucleotide 14757-16166.

Matrix (M), ORF3 and ORF7 contain respectively three, two and one transmembrane domains. These proteins were expressed as deletion proteins excluding the first 100 amino acids (M and ORF3) or the first 18 amino acids (ORF7) that include the hydrophobic regions.

The cloned sequences are shown in Table 26.

A two-step strategy was used to amplify the cloned sequences. In the first step, amplification of DNA fragments containing more than one gene or single gene used sequenced cDNA as template. Eleven cDNA sequences were amplified: (1) a fragment, named amplC1, including genes coding for protein E, protein M, orf 7-8-9-10; (2) a fragment, named amplC2, including genes coding for orf 3-4; (3) a fragment, named amplC5, including genes coding for proteins Nsp12 and Nsp13; (4) Nsp11gene; (5) P28 and P65 genes; (6) Nsp1B and NsplC genes portion; (7) a fragment, named amplC9, including genes coding for proteins Nsp2 and Nsp3; (8) a fragment, named amplNsp4-7, including genes coding for proteins Nsp4, Nsp5, Nsp6, Nsp7 and for amplification of Nsp9A gene portion; (9) Nsp 9B gene portion and Nsp10 gene; (10) a fragment, named amplCO, including genes coding for proteins Orf11, Nucleocapsid (N) and Orf12; (11) Nsp1A gene portion. The primers used in this first step are given in Table 27:

In the second step, amplification of single genes was performed using DNA fragments from the first amplification step as templates. The primers are shown in Table 28.

Of the proteins where expression was seen, it was either in inclusion bodies (insoluble) or in a soluble form. Purification proceeded on appropriate material. Table 29 shows the molecular weight of the expressed fragments of SARS-CoV ORFs, whether they were cloned (+ or -), whether the cloned fragment was seen to be expressed (+ or -) and the form of protein which was chosen for purification.

Where a protein was a soluble His-tagged product, a single colony was streaked and grown overnight at 37°C on a LB/Amp (100 µg/ml) agar plate. An isolated colony from this plate was inoculated into 20ml of LB/Amp (100 µg/ml) liquid medium and grown overnight at 37°C with shaking. The overnight culture was diluted 1:30 into 1.0 L LB/Amp (100 µg/ml) liquid medium and allowed to grow at the optimal temperature (30 or 37°C) until the OD550nm reached 0.6-0.8. Expression of recombinant protein was induced by addition of IPTG (final concentration 1.0 mM) and the culture incubated for a further 3 hours. Bacteria were harvested by centrifugation at 8000 *x g* for 15 min at 4°C. The bacterial pellet was resuspended in 10 ml of cold buffer A (300 mM NaCl, 50 mM phosphate buffer, 10 mM imidazole, pH 8.0). Cells were disrupted by sonication (or French Press) on ice four times for 30 sec at 40W using a Branson sonifier 450 and centrifuged at 13 000xg for 30 min at 4°C. Supernatants were mixed with 150µl Ni²⁺-resin (previously equilibrated with buffer A) and incubated at room temperature with gentle agitation for 30 min. The resin was Chelating Sepharose Fast Flow (Pharmacia), prepared according to the manufacturer's protocol. The batch-wise preparation was centrifuged at 700 x g for 5 min at 4°C and the supernatant discarded. The resin was washed twice (batch-wise) with 10ml buffer A for 10 min, resuspended in 1.0 ml buffer A and loaded onto a disposable column. The resin continued to be washed with buffer A at 4°C until the OD280nm of the flow-through reached 0.02-0.01. The resin was further washed with cold buffer B(300 mM NaCl, 50 mM phosphate buffer, 20 mM imidazole, pH 8.0) until the the OD280nm of the flow-through reached 0.02-0.01. The His-fusion protein was eluted by addition of 700µl of cold elution buffer C (300 mM NaCl, 50mM phosphate buffer, 250 mM imidazole, pH 8.0) and fractions collected until the OD₂₈₀ₙₘ indicated all the recombinant protein was obtained. 20µl aliquots of each elution fraction were analyzed by SDS-PAGE. Protein concentrations were estimated using the Bradford assay.

Where a protein was seen as an insoluble product, the inclusion bodies were purified as follows: homogenize cells (5 g wet weight) in 25 ml 0.1M Tris HCl pH 7, 1mM EDTA, at 4°C using an ultraturrax (10000 rpm); add 1.5mg lysozyme per gram cells; mix shortly with an ultraturrax and incubate at 4°C for 30'; use sonication or high-pressure homogenization to disrupt the cells; to digest DNA, add MgCl₂ to a final concentration of 3mM and DNase to a final concentration of 10ug/ml and incubate 30' at 25°C. add 0.5 vol of 60mM EDTA, 6% Triton x-100, 1.5M NaCl pH 7.0 to the solution, and incubate for 30' at 4°C; centrifugation at 31000 g for 10' at 4°C; re-suspend pellet in 40ml of 0.1M Tris HCl pH 7.0, 20 mM EDTA using ultraturrax; centrifugation at 31000 g for 10' a 4°C; store the IB pellet at - 20°C.

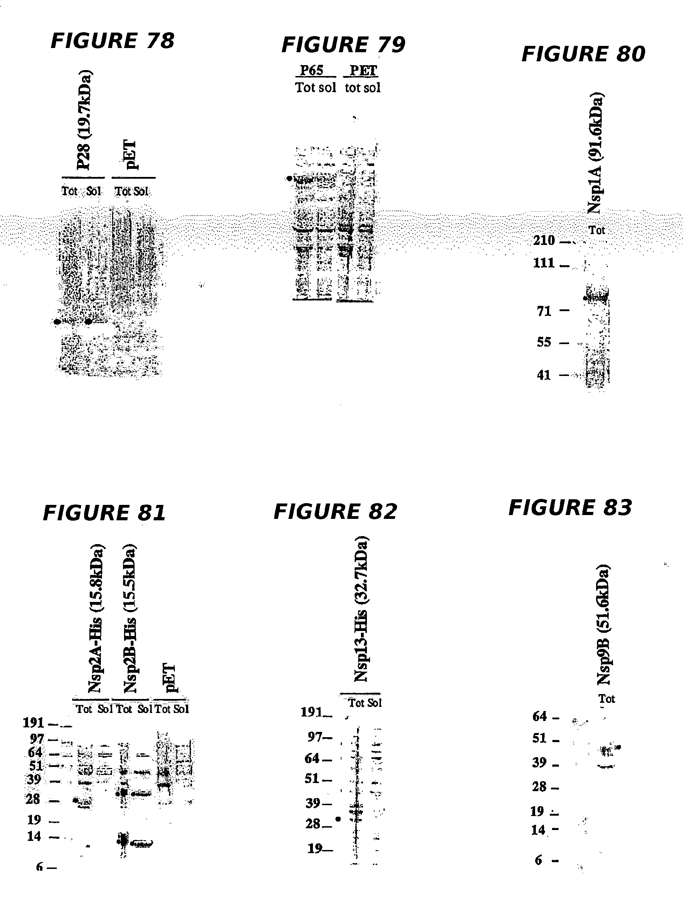

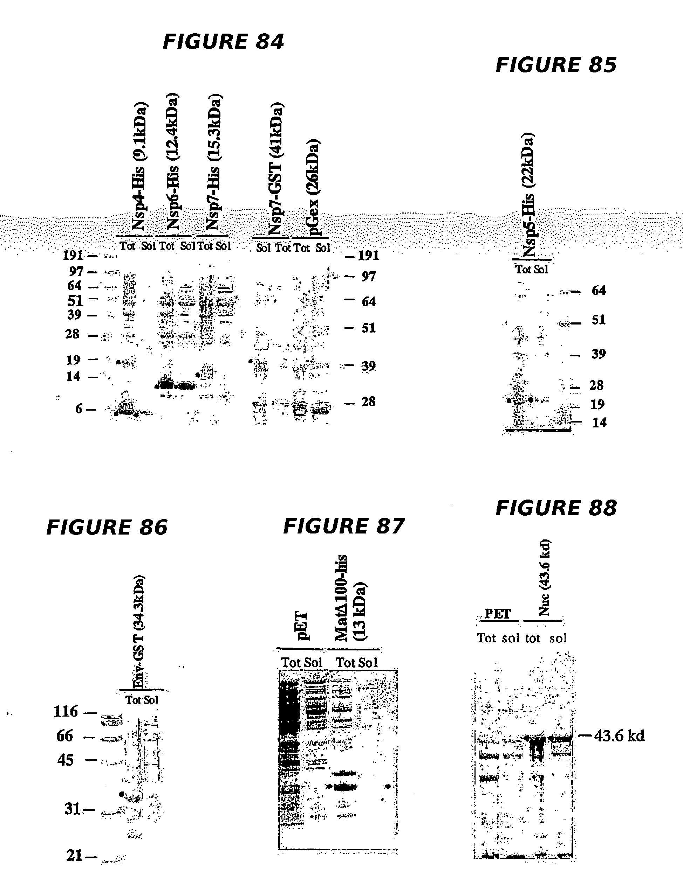

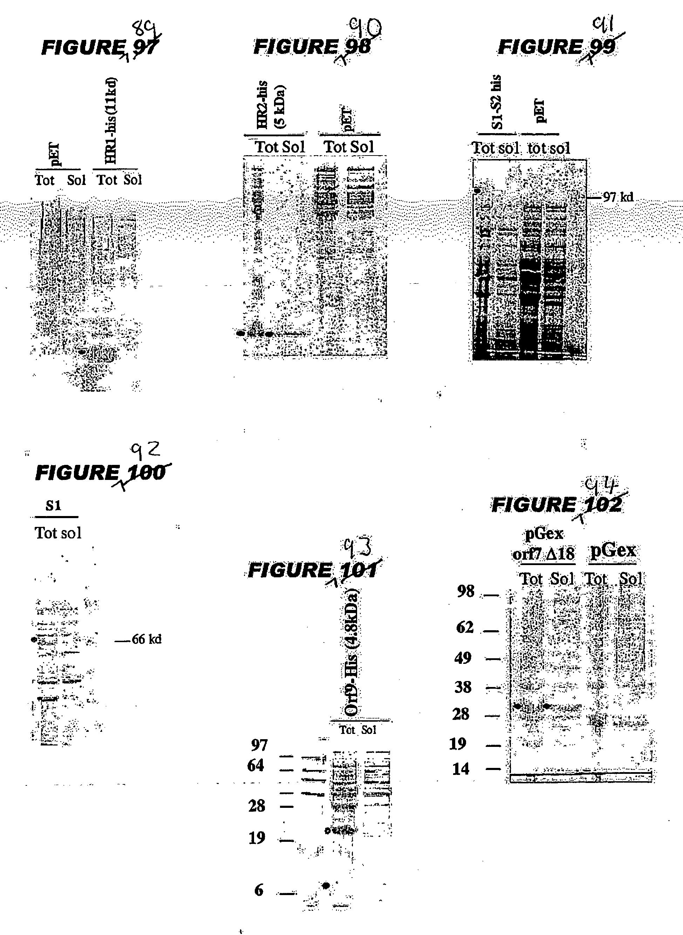

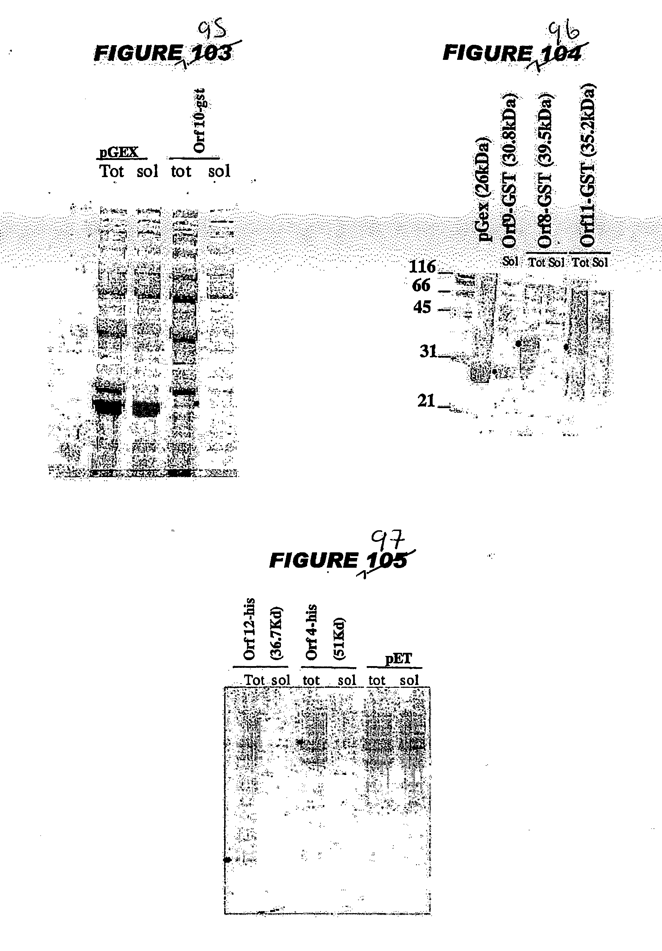

The results of expression are shown in Figures 78 to 97. Examples of purity and yield are given in Table 30.

### EXAMPLE 17: Retention of critical epitope on truncated Spike antigen

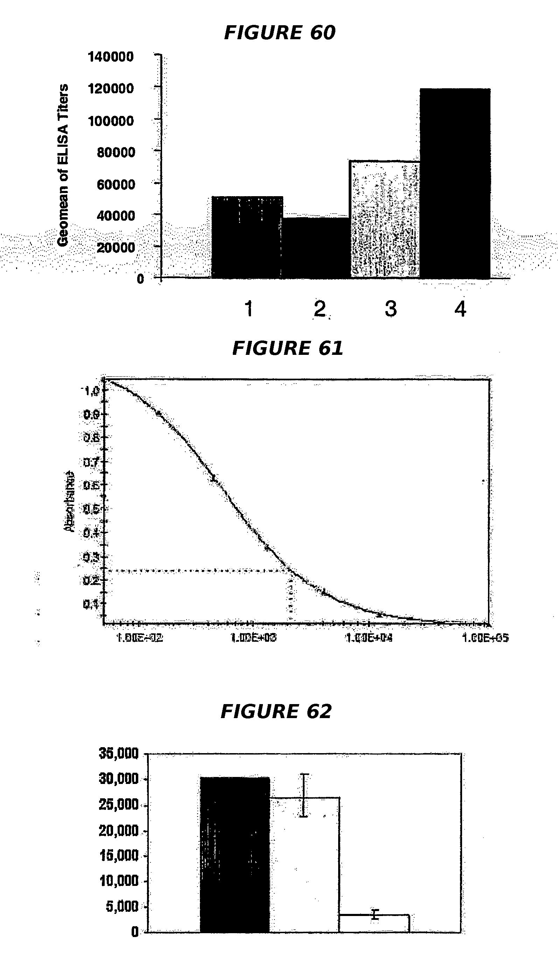

A human monoclonal antibody having neutralizing activity was tested in an ELISA assay for reactivity with the purified truncated Spike protein. Briefly, ELISA plates were coated with truncated form of the spike protein at a concentration of 1µg/ml (100µl/ well) by incubating the plates overnight at 4°C. The plates were washed, non-specific binding sites were blocked and then different dilutions of the antibody were added and plates were incubated for 1 hour at room temperature. At the end of incubation, the plates were washed and bound antibody was detected by using anti-human IgG conjugated to horse radish peroxidase (HRP) and an appropriate substrate. The optical density of each well was recorded at 405 mm using an ELISA reader. The data are shown in Figure 61 and clearly demonstrate that the neutralizing epitope recognized by the mAb is preserved and exposed on the recombinant truncated Spike protein.

### EXAMPLE 18: Different Spike vaccines

Purified truncated spike protein was used to immunize mice and the level of binding antibodies induced against the truncated spike protein was determined by ELISA assay. Briefly a group of 10 mice were immunized with 3µg of truncated spike protein adjuvanted in MF59 at 0, 4 and 8 weeks intervals. Sera samples were collected from these animals and assayed for antibodies induced by truncated spike protein in an ELISA assay. An additional group of 8 mice was immunized with 75 µg of DNA encoding the truncated form of the spike protein on PLG particles at 0, 4 and 13 weeks intervals, the sera were collected and analyzed as above for anti-spike antibodies as above

The profile of binding antibodies induced in each group was plotted as geometric mean titer (GMT). Compared to a plasmid DNA vaccine expressing truncated spike antigen and delivered using a PLG microparticle formulation, the purified truncated spike protein was significantly more potent for inducing strong antibody responses. Further comparison with the antibody responses induced by inactivated BPL-SARS-CoV (already shown protective) in the same mouse strain revealed that the magnitude of antibody responses induced by purified truncated spike protein and the inactivated virus vaccine are in the same range (Figure 62).

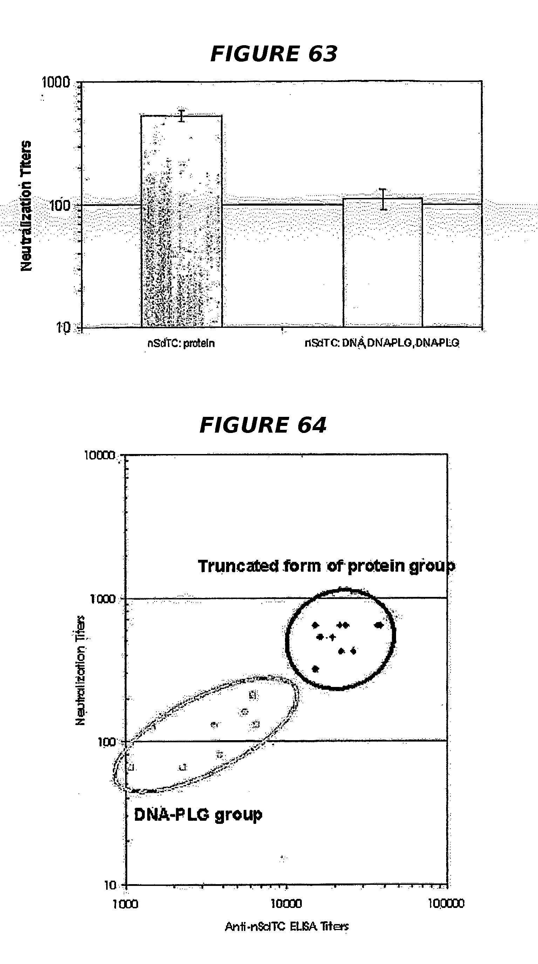

The neutralization potential of antibodies induced by the recombinant truncated spike protein, or plasmid DNA expressing the same spike antigen, were also evaluated. The GMT values obtained in both groups are shown in Figure 63. From these data, it appears that the purified protein is significantly more effective at inducing neutralizing antibody responses against the SARS-CoV spike. Furthermore, the neutralization titers typically induced by the purified truncated spike protein are comparable to neutralization titers induced by an inactivated SARS-CoV vaccine.

Figure 64 shows a comparison of antibody binding levels (ELISA, X-axis) with neutralization titers (Y-axis). In general there is a very good correlation between the binding and neutralizing antibodies. The bottom-left grouping shows ratios 2 weeks post-3rd immunization with the DNA vaccine; the top-right grouping shows ratios 2 weeks post-2nd immunization with the protein vaccine. Both forms of vaccine show a consistent correlation.

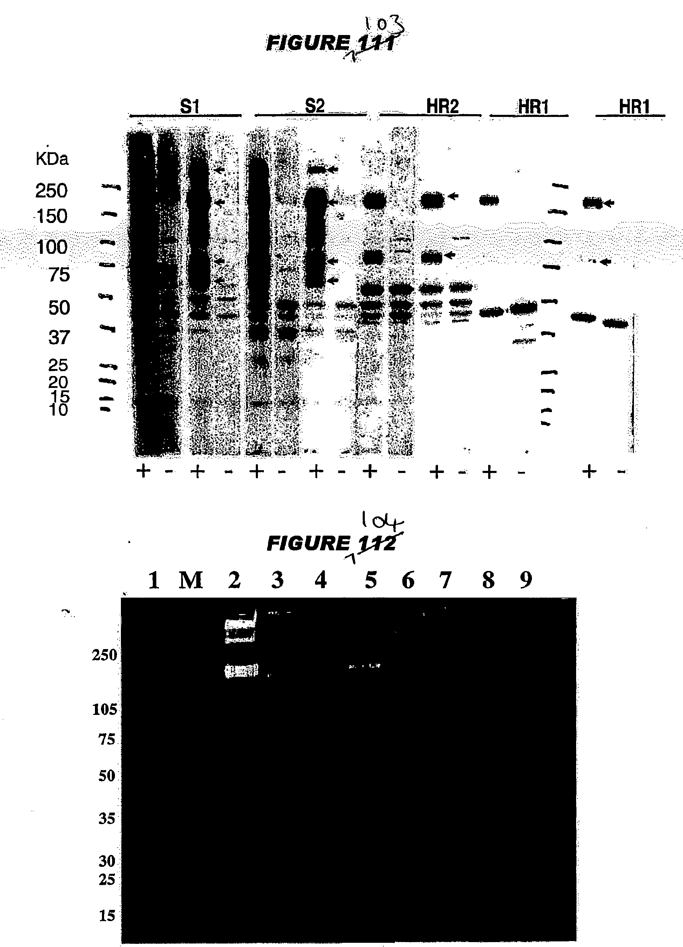

In further experiments, the ability of a DNA vaccine to invoke an immune response in mice was studied. Mice were immunized with pCMV-nSdTC plasmid, either free or with PLG microparticles. Serum from the mice was then used as the staining antibody against cultured 293 cells that had been transfected with spike, either full-length or truncated. The cells were centrifuged prior to testing and the pellet was lysed. The antibody was tested against the culture supernatant and against the cell lysate. As shown in Figure 104, the mouse serum was able to detect spike protein in the lysate of cells that expressed full-length spike and in the supernatant of cells that expressed the truncated spike protein. Results were comparable to the staining seen when using rabbit serum that had been obtained after immunization with whole killed virus. Thus anti-spike antibodies can be induced by the use of DNA vaccination.

### EXAMELE 19: Expression cassettes in pCMV

The sequence of plasmid pCMVKm2 is given as SEQ ID NO: 9923. Genes encoding the spike protein either in full-length form (pCMVKm2 SARS Spike nS; SEQ ID NO: 9921) or in its ΔTC form (pCMVKm2 SARS Spike nSΔTC; SEQ ID NO: 9922) were inserted into this basic vector.

Mice were immunized with these vectors, and with similar vectors encoding the N, M or E proteins. Vectors encoding the same proteins but with optimized codon usage were also prepared. Codons were optimized for efficient human expression starting from the FRA sequence (GenBank: AY310120).

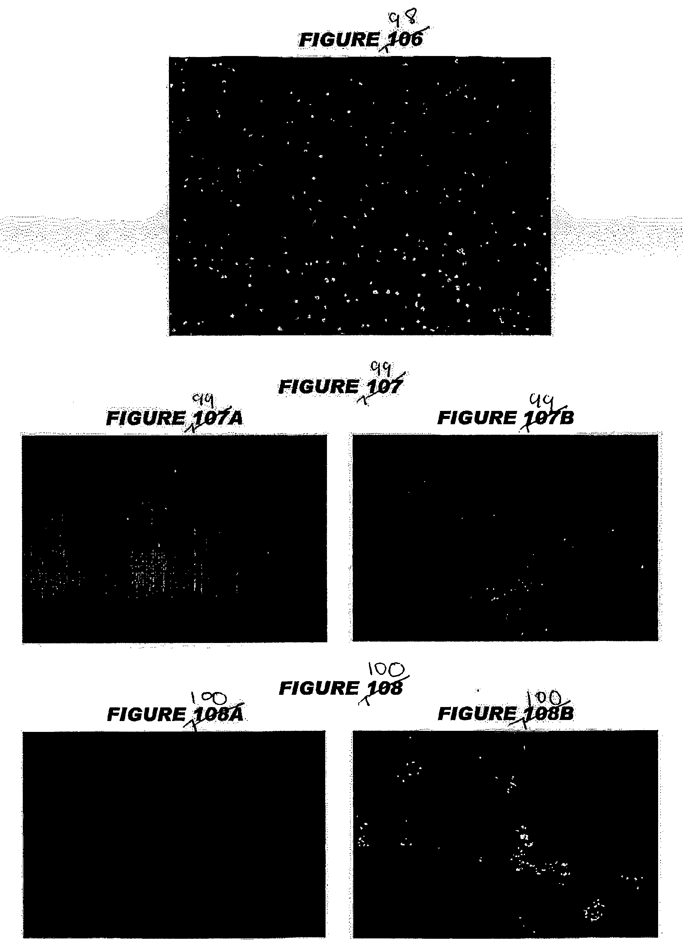

After administration, expression of proteins could be detected by immunofluorescence in all cases. For example, Figure 98 shows immunofluorescence (using anti-SARS rabbit serum) results after administration of the vector encoding optimsed N antigen, revealing high level expression. Mice receiving the control vector alone showed no fluorescence.

Figure 99 compares immunofluorescence (using Abgent anti-M antibody) of the native M sequence (99A) or the codon-optimsed M sequence (107B). Similarly, Figure 100 compares immunofluorescence (using Abgent anti-E antibody) of the native E sequence (100A) or the codon-optimsed E sequence (100B).

Four groups of mice (8 mice per group) were immunized with: (1) SARS nS Spike, nSdTC truncated Spike, and N proteins; (2) pCMV-SARS-nSdTC: DNA+DNA-PLG at weeks 0,4 and 13 wks; (3) CMV-nS: DNA+DNA-PLG+VEE/SIN Rep at 0,4 and 9 wks; (4) VEE/SIN Rep-SARS-nS three times at 0, 4 and 13 wks. Sera from all groups recognized SARS nS and nSdTC proteins, and also showed virus binding and neutralization activity.

### EXAMPLE 20: Spike protein cleavage

To investigate the effect of proteolytic cleavage on SARS-CoV Spike protein, it was expressed in various forms in *E.coli,* including: (1) full-length S1-S2; (2) S1 alone; (3) HR1 heptad; and (4) HR2 heptad. The expressed proteins were used to raise immune rabbit sera, which were then used for visualizing western blots of Vero cells, either infected or not infected with SARS-CoV.

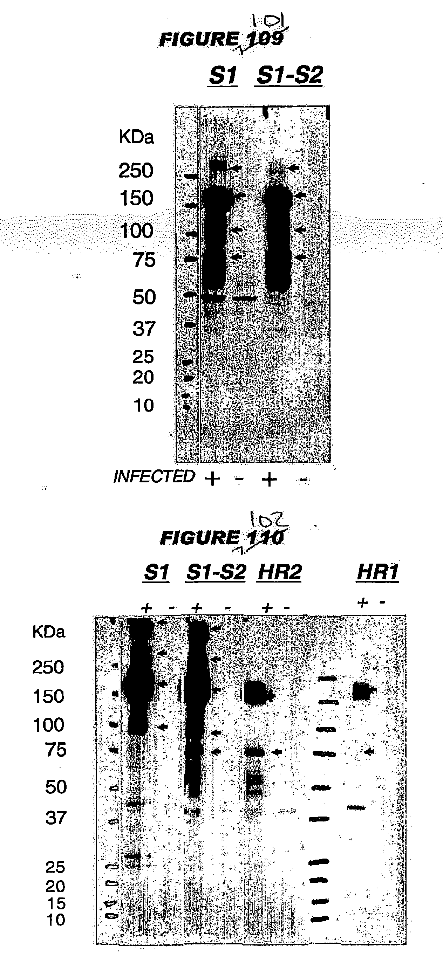

Figure 101 shows a western blot using a 1:10000 dilution of antibody raised against either the S1 domain or the uncleaved S 1-S2 domains. Figure 102 shows a western blot using a 1:10000 dilution of antibody raised against each of the four proteins. The difference in antigen reactivity is readily apparent.

Figure 103 shows similar data. Each serum was tested against four lanes, with those gour lanes being from left to right: (a) serum at 1:500 dilution, SARS-CoV-infected cells; (b) serum at 1:500 dilution, non-infected cells; (c) serum at 1:2500 dilution, SARS-CoV-infected cells; (d) serum at 1:2500 dilution, non-infected cells. Again, the difference in antigen reactivity is readily apparent.

Figures 101-103 show that the Spike protein exists in various forms in infected Vero cells, with sizes of approx. 75kDa, 90kDa, 180kDa and >250kDa. The Spike protein is cleaved (at least partially) either intracellulary or after release of the particles.

If enzymatic cleavage of the mouse hepatitis coronavirus spike protein is inhibited then cell-cell fusion (syncytia formation) is also inhibited, but virus-cell fusion is not (de Haan *et al.* (2004) *J Virol*). Syncytia are observed *in vivo* in the lungs of SARS-infected patients, but are not seen in Vero cell cultures of the SARS-CoV. Inhibition of Spike protein cleavage may thus be used to prevent syncytia formation and related pathology, even though viral infectivity may not be blocked.

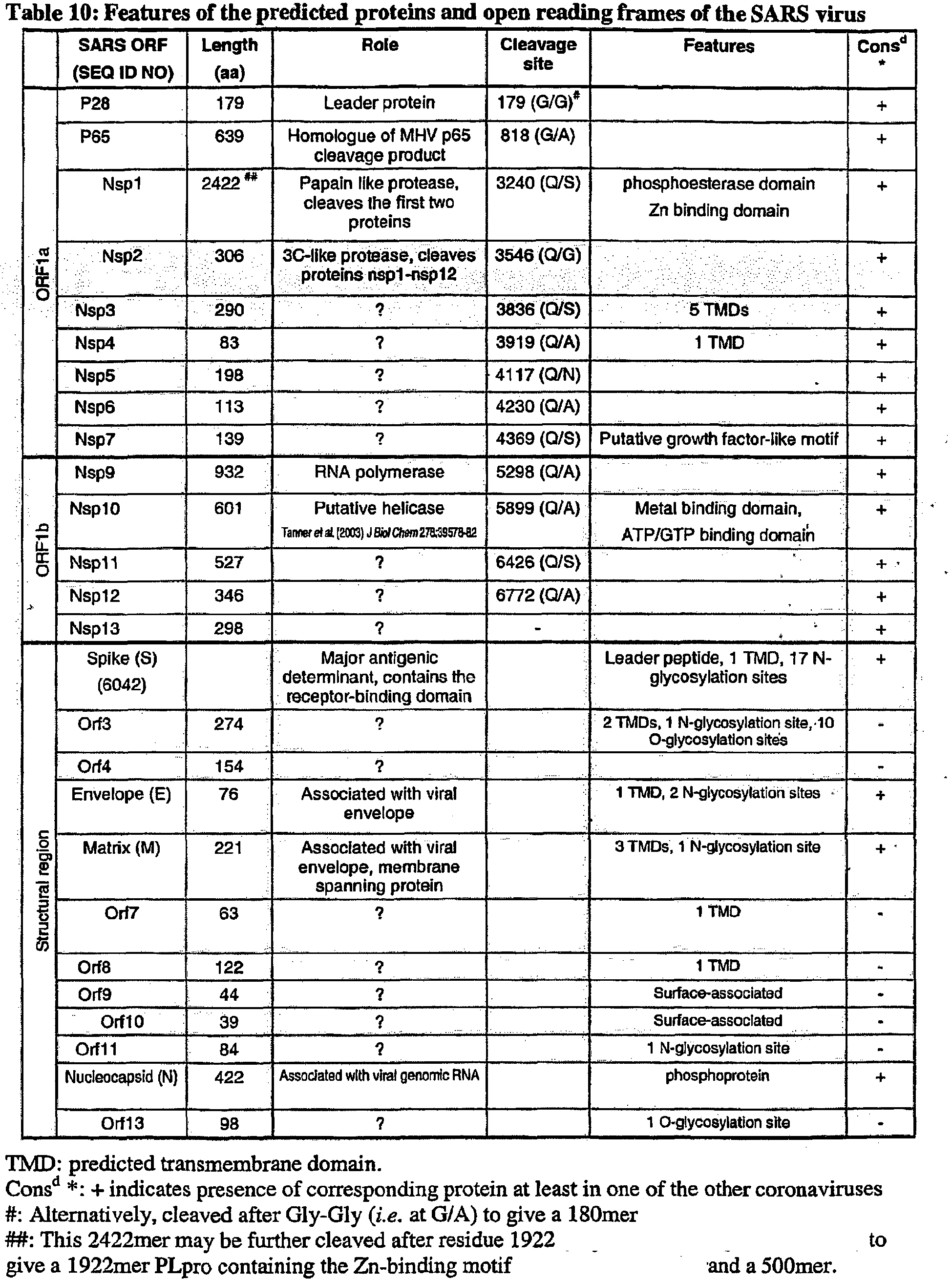

**Table 11: Protein homologies between SARS and other coronaviruses**

| Numbers indicate percentage of aminoacid identity between SARS proteins and corresponding gene products of other coronaviruses. More conserved pairs are in bold; more variable pairs are underlined. | | | | | | |
|---|---|---|---|---|---|---|
| | group 1 | | | group 2 | | group 3 |
| Proteins | 229E | TGV | PEDV | MHV | BCoV | AIBV |
| **REPLICASE REGION** | | | | | | |
| leader protein p28 | <20 | <20 | <20 | **27** | <20 | <20 |
| p65 homologue | <20 | **23** | 23 | <20 | 20 | <20 |
| nsp1 (PLP protease) | 25.5 | 25.8 | 25.4 | 29 | **30** | 25 |
| nsp2 (3CL protease) | 40.4 | 43.8 | 44.6 | **50** | 48.4 | 41 |
| nsp3 | 30 | 27 | 29.4 | 34.2 | **35.5** | 28.5 |
| nsp4 | 38.6 | 42.2 | 39.8 | **47.5** | 46.1 | 37.3 |
| nsp5 | **48.2** | 42.9 | 43.9 | 46.8 | 47.3 | 38.7 |
| nsp6 | 45.1 | 38.9 | 45.1 | 45.1 | **46.9** | 39.8 |
| nsp7 | 53.8 | 54.5 | 56.1 | 56.2 | 55.4 | **58.3** |
| nsp9 (polymerase) | 59.8 | 59.6 | 60 | **67.3** | 66.9 | 62.4 |
| nsp10 (helicase) | 60.7 | 62 | 62.3 | 67.2 | 68.6 | 58.9 |
| nsp11 | 52.3 | 53.7 | 52.3 | **57.6** | 57.6 | 52 |
| nsp12 | 43.1 | 43 | 45.4 | **45.9** | 45 | 40.2 |
| nsp13 | 56.4 | 54.4 | 55.3 | 63 | **65** | 53.4 |

| **STRUCTURAL REGION** | | | | | | |
|---|---|---|---|---|---|---|
| Spike (S) | 28.8 | 31.6* | 30.3 | 31.1 | 31 | 32.7* |
| Envelope (E) | 33* | **27.9** | 20 | 23 | 26.5 | 23.2 |
| Matrix glycoprotein (M) | 30.6 | 32.5 | 34.8 | 40.8 | **41.9** | 32.5 |
| Nucleocapsid (N) | 26.9 | 30.1 | 29.5 | 37.3 | **37.4** | 31.5 |

| | | | | | | |
|---|---|---|---|---|---|---|
| * These three alignments were obtained only on a fragment of the whole protein. | | | | | | |

**Table 12: Nucleotide and aminoacid differences between five SARS isolates**

| | | **FRA*** | **TOR2*** | **Urbani*** | **CUHK*** | **HKU*** |
|---|---|---|---|---|---|---|
| | position° | base/aminoacid | base/aminoacid | base/aminoacid | base/aminoacid | base/aminoacid |
| | | | | | | |
| **ORF1a** | 2557 | A/Thr | G/Ala | G/Ala | G/Ala | G/Ala |
| | 2601 | T/Val | | | | C |
| | 7746 | G/Pro | | | T | |
| | 7919 | C/Ala | | T/Val | | |
| | 7930 | G/Asp | | | | A/Asn |
| | 8387 | G/Ser | | | | C/Thr |
| | 8416 | G/Arg | | | | C/Thr |
| | 9404 | T/Val | | | C/Ala | |
| | 9479 | T/Val | | | C/Ala | |
| | 11448 | T/Ile | C | C | C | C |
| | | | | | | |
| **ORF1b** | 13494 | GT/Val | | | | AG/Ser |
| | 16622 | C/Ala | | T | | |
| | 17564 | T/Asp | | | C/Glu | |
| | 17846 | C/Arg | | | T | |
| | 18065 | G/Lys | | | | A |
| | 18965 | A/Ile | T | T | T | T |
| | 19064 | A/Glu | | G | G | |
| | 19084 | T/Ile | C/Thr | C/Thr | C/Thr | C/Thr |
| | | | | | | |
| **spike** | 21721 | G/Gly | | | A/Asp | |
| | 22222 | T/Ile | | | C/Thr | |
| | 23220 | T/Ser | G/Ala | | | |
| | 24872 | T/Leu | | C | | |
| | 24933 | T/Phe | C/Leu | C/Leu | C/Leu | C/Leu |
| | | | | | | |
| **ORF3** | 25298 | G/Gly | A/Arg | | | |
| | 25569 | T/Met | | | | A/Lys |
| | | | | | | |
| **matrix** | 26600 | T/Val | C/Ala | C/Ala | C/Ala | |
| | 26857 | T/Ser | | C/Pro | | |
| | | | | | | |
| **ORF10** | 27827 | T/Cys | | | C/Arg | |
| | | | | | | |
| **nucleocapsid** | 28268 | T/Ile | C/Thr | C/Thr | C/Thr | C/Thr |

| | | | | | | |
|---|---|---|---|---|---|---|
| * SARS coronavirus FRA (accession number AY310120) SARS coronavirus TOR2 (accession number AY274119) SARS coronavirus Urbani (accession number AY278741) SARS coronavirus CUHK-W1 (accession number AY278554) SARS coronavirus HKU-39849 (accession number AY278491) ° The position is based on the FRA sequence. | | | | | | |

**TABLES 13-25: T-epitope predictions for SEQ ID NO: 6042** Epitope predictions were performed at *http:*//*www.mpiib-berlin.mpg.de*/*MAPPP*/*binding.html* using a minimum score of 0.5 and the BIMAS matrix, with a maximum of 20 results being selected. The analysis revealed 9mer and 10mer epitopes.

**Table 16: Epitopes for SEQ ID NO: 6042**

| **HLA A1 - 9 mers** | | | | |
|---|---|---|---|---|
| Maximum possible score using this molecule type | | | | 5625 |
| **Rank** | **Start position** | **Sequence** | **% of max. score** | **Score** |
| 1 | 846 | SEQ ID NO: 8041 | 2.22 % | 125 |
| 2 | 798 | SEQ ID NO: 8042 | 1.6 % | 90 |
| 3 | 787 | SEQ ID NO: 8043 | 0.88 % | 50 |
| 4 | 1178 | SEQ ID NO: 8044 | 0.8 % | 50 |
| 5 | 637 | SEQ ID NO: 8045 | 0.8 % | 45 |
| 6 | 557 | SEQ ID NO: 8046 | 0.44 % | 25 |
| 7 | 1020 | SEQ ID NO: 8047 | 0.44 % | 25 |
| 8 | 282 | SEQ ID NO: 8048 | 0.32 % | 18 |
| 9 | 1241 | SEQ ID NO: 8049 | 0.24 % | 13.5 |
| 10 | 466 | SEQ ID NO: 8050 | 0.22 % | 12.5 |
| 11 | 727 | SEQ ID NO: 8051 | 0.2 % | 11.25 |
| 12 | 706 | SEQ ID NO: 8052 | 0.17 % | 10 |
| 13 | 324 | SEQ ID NO: 8053 | 0.16 % | 9 |
| 14 | 752 | SEQ ID NO: 8054 | 0.16 % | 9 |
| 15 | 54 | SEQ ID NO: 8055 | 0.13 % | 7.5 |
| 16 | 554 | SEQ ID NO: 8056 | 0.13 % | 7.5 |
| 17 | 590 | SEQ ID NO: 8057 | 0.12 % | 6.75 |
| 18 | 569 | SEQ ID NO: 8058 | 0.08 % | 5 |
| 19 | 613 | SEQ ID NO: 8059 | 0.08 % | 5 |
| 20 | 90 | SEQ ID NO: 8060 | 0.08 % | 4.5 |
| | | | | |

| **HLA A1 - 10 mers** | | | | |
|---|---|---|---|---|
| Maximum possible score using this molecule type | | | | 5625 |
| **Rank** | **Start position** | **Sequence** | **% of max. score** | **Score** |
| 1 | 1241 | SEQ ID NO: 8061 | 4.8 % | 270 |
| 2 | 967 | SEQ ID NO: 8062 | 0.8 % | 45 |
| 3 | 1010 | SEQ ID NO: 8063 | 0.48 % | 27 |
| 4 | 426 | SEQ ID NO: 8064 | 0.44 % | 25 |
| 5 | 809 | SEQ ID NO: 8065 | 0.44 % | 25 |
| 6 | 1178 | SEQ ID NO: 8066 | 0.44 % | 25 |
| 7 | 787 | SEQ ID NO: 8067 | 0.22 % | 12.5 |
| 8 | 958 | SEQ ID NO: 8068 | 0.22 % | 12.5 |
| 9 | 727 | SEQ ID NO: 8069 | 0.2 % | 11.25 |
| 10 | 610 | SEQ ID NO: 8070 | 0.17 % | 10 |
| 11 | 12 | SEQ ID NO: 8071 | 0.13 % | 7.5 |
| 12 | 1181 | SEQ ID NO: 8072 | 0.12 % | 6.75 |
| 13 | 373 | SEQ ID NO: 8073 | 0.11 % | 6.25 |
| 14 | 602 | SEQ ID NO: 8074 | 0.11 % | 6.25 |
| 15 | 20 | SEQ ID NO: 8075 | 0.04 % | 2.5 |
| 16 | 32 | SEQ ID NO: 8076 | 0.04 % | 2.5 |
| 17 | 53 | SEQ ID NO: 8077 | 0.04 % | 2.5 |
| 18 | 400 | SEQ ID NO: 8078 | 0.04 % | 2.5 |
| 19 | 557 | SEQ ID NO: 8079 | 0.04 % | 2.5 |
| 20 | 667 | SEQ ID NO: 8080 | 0.04 % | 2.5 |
| | | | | |

| **HLA A3 - 9 mers** | | | | |
|---|---|---|---|---|
| Maximum possible score using this molecule type | | | | 12150 |
| **Rank** | **Start position** | **Sequence** | **% of max. score** | **Score** |
| 1 | 768 | SEQ ID NO: 8081 | 0.82 % | 100 |
| 2 | 808 | SEQ ID NO: 8082 | 0.49 % | 60 |
| 3 | 85 | SEQ ID NO: 8083 | 0.24 % | 30 |
| 4 | 663 | SEQ ID NO: 8084 | 0.24 % | 30 |
| 5 | 1245 | SEQ ID NO: 8085 | 0.14 % | 18 |
| 6 | 288 | SEQ ID NO: 8086 | 0.09 % | 12 |
| 7 | 50 | SEQ ID NO: 8087 | 0.08 % | 10 |
| 8 | 320 | SEQ ID NO: 8088 | 0.07 % | 9 |
| 9 | 402 | SEQ ID NO: 8089 | 0.07 % | 9 |
| 10 | 798 | SEQ ID NO: 8090 | 0.07 % | 9 |
| 11 | 902 | SEQ ID NO: 8091 | 0.06 % | 8.1 |
| 12 | 364 | SEQ ID NO: 8092 | 0.05 % | 6.75 |
| 13 | 297 | SEQ ID NO: 8093 | 0.04 % | 6 |
| 14 | 992 | SEQ ID NO: 8094 | 0.04 % | 6 |
| 15 | 38 | SEQ ID NO: 8095 | 0.03 % | 4.5 |
| 16 | 249 | SEQ ID NO: 8096 | 0.03 % | 4.5 |
| 17 | 706 | SEQ ID NO: 8097 | 0.03 % | 4.05 |
| 18 | 1204 | SEQ ID NO: 8098 | 0.03 % | 4.05 |
| 19 | 1178 | SEQ ID NO: 8099 | 0.03 % | 4 |
| 20 | 343 | SEQ ID NO: 8100 | 0.02 % | 3.6 |
| | | | | |

| **HLA A3 - 10 mers** | | | | |
|---|---|---|---|---|
| Maximum possible score using this molecule type | | | | 12150 |
| **Rank** | **Start position** | **Sequence** | **% of max. score** | **Score** |
| 1 | 255 | SEQ ID NO: 8101 | 1.48 % | 180 |
| 2 | 180 | SEQ ID NO: 8102 | 0.55 % | 67.5 |
| 3 | 768 | SEQ ID NO: 8103 | 0.49 % | 60 |
| 4 | 1177 | SEQ ID NO: 8104 | 0.49 % | 60 |
| 5 | 380 | SEQ ID NO: 8105 | 0.24 % | 30 |
| 6 | 100 | SEQ ID NO: 8106 | 0.18 % | 22.5 |
| 7 | 786 | SEQ ID NO: 8107 | 0.16 % | 20 |
| 8 | 1217 | SEQ ID NO: 8108 | 0.16 % | 20 |
| 9 | 207 | SEQ ID NO: 8109 | 0.14 % | 18 |
| 10 | 1183 | SEQ ID NO: 8110 | 0.14 % | 18 |
| 11 | 38 | SEQ ID NO: 8111 | 0.09 % | 12 |
| 12 | 52 | SEQ ID NO: 8112 | 0.09 % | 12 |
| 13 | 8 | SEQ ID NO: 8113 | 0.06 % | 8 |
| 14 | 679 | SEQ ID NO: 8114 | 0.06 % | 8 |
| 15 | 73 | SEQ ID NO: 8115 | 0.05 % | 6.75 |
| 16 | 1204 | SEQ ID NO: 8116 | 0.05 % | 6.075 |
| 17 | 50 | SEQ ID NO: 8117 | 0.04 % | 6 |
| 18 | 774 | SEQ ID NO: 8118 | 0.04 % | 6 |
| 19 | 845 | SEQ ID NO: 8119 | 0.04 % | 6 |
| 20 | 214 | SEQ ID NO: 8120 | 0.04 % | 5.4 |
| | | | | |

| **HLA A24 - 9 mers** | | | | |
|---|---|---|---|---|
| Maximum possible score using this molecule type | | | | 1596.672 |

| **Rank** | **Start position** | **Sequence** | **% of max**. **score** | **Score** |
|---|---|---|---|---|
| 1 | 1118 | SEQ ID NO: 8121 | 19.84 % | 316.8 |
| 2 | 51 | SEQ ID NO: 8122 | 18.78 % | 300 |
| 3 | 161 | SEQ ID NO: 8123 | 18.78 % | 300 |
| 4 | 434 | SEQ ID NO: 8124 | 18.78 % | 300 |
| 5 | 365 | SEQ ID NO: 8125 | 13.77 % | 220 |
| 6 | 736 | SEQ ID NO: 8126 | 12.52 % | 200 |
| 7 | 620 | SEQ ID NO: 8127 | 7.51 % | 120 |
| 8 | 1068 | SEQ ID NO: 8128 | 7.51 % | 120 |
| 9 | 817 | SEQ ID NO: 8129 | 3.75 % | 60 |
| 10 | 336 | SEQ ID NO: 8130 | 3.44 % | 55 |
| 11 | 687 | SEQ ID NO: 8131 | 3.13 % | 50 |
| 12 | 254 | SEQ ID NO: 8132 | 2.34 % | 37.5 |
| 13 | 627 | SEQ ID NO: 8133 | 1.87 % | 30 |
| 14 | 950 | SEQ ID NO: 8134 | 1.75 % | 28 |
| 15 | 28 | SEQ ID NO: 8135 | 1.56 % | 25 |
| 16 | 408 | SEQ ID NO: 8136 | 1.56 % | 25 |
| 17 | 159 | SEQ ID NO: 8137 | 1.31 % | 21 |
| 18 | 1166 | SEQ ID NO: 8138 | 1.26 % | 20.16 |
| 19 | 45 | SEQ ID NO: 8139 | 1.25 % | 20 |
| 20 | 185 | SEQ ID NO: 8140 | 1.25 % | 20 |
| | | | | |

| **HLA A24** - **10 mers** | | | | |
|---|---|---|---|---|
| Maximum possible score using this molecule type | | | | 1596.672 |
| **Rank** | **Start position** | **Sequence** | **% of max. score** | **Score** |
| 1 | 438 | SEQ ID NO: 8141 | 27.55 % | 440 |
| 2 | 489 | SEQ ID NO: 8142 | 22.54 % | 360 |
| 3 | 254 | SEQ ID NO: 8143 | 18.78 % | 300 |
| 4 | 354 | SEQ ID NO: 8144 | 11.27 % | 180 |
| 5 | 406 | SEQ ID NO: 8145 | 11.27 % | 180 |
| 6 | 1047 | SEQ ID NO: 8146 | 11.27 % | 180 |
| 7 | 473 | SEQ ID NO: 8147 | 7.51 % | 120 |
| 8 | 350 | SEQ ID NO: 8148 | 6.26 % | 100 |
| 9 | 769 | SEQ ID NO: 8149 | 6.26 % | 100 |
| 10 | 193 | SEQ ID NO: 8150 | 5.63 % | 90 |
| 11 | 479 | SEQ ID NO: 8151 | 3.13 % | 50 |
| 12 | 0 | SEQ ID NO: 8152 | 2.70 % | 43.2 |
| 13 | 813 | SEQ ID NO: 8153 | 1.87 % | 30 |
| 14 | 739 | SEQ ID NO: 8154 | 1.50 % | 24 |
| 15 | 782 | SEQ ID NO: 8155 | 1.50 % | 24 |
| 16 | 1186 | SEQ ID NO: 8156 | 1.31 % | 21 |
| 17 | 910 | SEQ ID NO: 8157 | 1.05 % | 16.8 |
| 18 | 128 | SEQ ID NO: 8158 | 0.93 % | 15 |
| 19 | 183 | SEQ ID NO: 8159 | 0.93 % | 15 |
| 20 | 1069 | SEQ ID NO: 8160 | 0.93 % | 15 |
| | | | | |

| **HLA A 0201 - 9 mers** | | | | |
|---|---|---|---|---|
| Maximum possible score using this molecule type | | | | 3925227.1 |
| **Rank** | **Start position** | **Sequence** | **% of max. score** | **Score** |
| 1 | 1041 | SEQ ID NO: 8161 | 0.01 % | 484.2379773 |
| 2 | 981 | SEQ ID NO: 8162 | 0.00 % | 382.536 |
| 3 | 957 | SEQ ID NO: 8163 | 0.00 % | 342.4606344 |
| 4 | 896 | SEQ ID NO: 8164 | 0.00 % | 232.6931712 |
| 5 | 1173 | SEQ ID NO: 8165 | 0.00 % | 201.447432 |
| 6 | 733 | SEQ ID NO: 8166 | 0.00 % | 171.86796 |
| 7 | 410 | SEQ ID NO: 8167 | 0.00 % | 135.45252 |
| 8 | 786 | SEQ ID NO: 8168 | 0.00 % | 119.463012 |
| 9 | 150 | SEQ ID NO: 8169 | 0.00 % | 102.17550222 |
| 10 | 1 | SEQ ID NO: 8170 | 0.00 % | 94.98737754 |
| 11 | 595 | SEQ ID NO: 8171 | 0.00 % | 93.239424 |
| 12 | 1095 | SEQ ID NO: 8172 | 0.00 % | 89.41779 |
| 13 | 1166 | SEQ ID NO: 8173 | 0.00 % | 87.58584 |
| 14 | 845 | SEQ ID NO: 8174 | 0.00 % | 79.642008 |
| 15 | 734 | SEQ ID NO: 8175 | 0.00 % | 73.47672 |
| 16 | 802 | SEQ ID NO: 8176 | 0.00 % | 71.872056 |
| 17 | 1213 | SEQ ID NO: 8177 | 0.00 % | 71.872056 |
| 18 | 105 | SEQ ID NO: 8178 | 0.00 % | 50.232 |
| 19 | 939 | SEQ ID NO: 8179 | 0.00 % | 49.13352 |
| 20 | 130 | SEQ ID NO: 8180 | 0.00 % | 48.732354 |
| | | | | |

| **HLA A 0201 - 10 mers** | | | | |
|---|---|---|---|---|
| Maximum possible score using this molecule type | | | | 3925227.1 |
| **Rank** | **Start position** | **Sequence** | **% of max. score** | **Score** |
| 1 | 372 | SEQ ID NO: 8181 | 0.04 % | 1896.33528 |
| 2 | 410 | SEQ ID NO: 8182 | 0.02 % | 1134.00849744 |
| 3 | 162 | SEQ ID NO: 8183 | 0.01 % | 685.3897512 |
| 4 | 1076 | SEQ ID NO: 8184 | 0.01 % | 640.90320525 |
| 5 | 1196 | SEQ ID NO: 8185 | 0.01 % | 623.742666372 |
| 6 | 353 | SEQ ID NO: 8186 | 0.01 % | 446.7384576 |
| 7 | 50 | SEQ ID NO: 8187 | 0.00 % | 375.97824 |
| 8 | 733 | SEQ ID NO: 8188 | 0.00 % | 271.863864 |
| 9 | 130 | SEQ ID NO: 8189 | 0.00 % | 235.6873848 |
| 10 | 415 | SEQ ID NO: 8190 | 0.00 % | 185.679 |
| 11 | 297 | SEQ ID NO: 8191 | 0.00 % | 177.496704 |
| 12 | 1 | SEQ ID NO: 8192 | 0.00 % | 152.42160582 |
| 13 | 56 | SEQ ID NO: 8193 | 0.00 % | 110.013876 |
| 14 | 732 | SEQ ID NO: 8194 | 0.00 % | 101.0988 |
| 15 | 6 | SEQ ID NO: 8195 | 0.00 % | 98.26704 |
| 16 | 261 | SEQ ID NO: 8196 | 0.00 % | 91.60164 |
| 17 | 1040 | SEQ ID NO: 8197 | 0.00 % | 76.98537 |
| 18 | 928 | SEQ ID NO: 8198 | 0.00 % | 71.2908 |
| 19 | 1188 | SEQ ID NO: 8199 | 0.00 % | 69.81282 |
| 20 | 1094 | SEQ ID NO: 8200 | 0.00 % | 52.5987 |
| | | | | |

| **HLA A 1101 - 9 mers** | | | | |
|---|---|---|---|---|
| Maximum possible score using this molecule type | | | | 36 |
| **Rank** | **Start position** | **Sequence** | **% of max.** | **Score** |
| 1 | 402 | SEQ ID NO: 8201 | 25 % | 9 |
| 2 | 902 | SEQ ID NO: 8202 | 22.5 % | 8.1 |
| 3 | 288 | SEQ ID NO: 8203 | 11.11 % | 4 |
| 4 | 85 | SEQ ID NO: 8204 | 6.66 % | 2.4 |
| 5 | 706 | SEQ ID NO: 8205 | 6.66 % | 2.4 |
| 6 | 456 | SEQ ID NO: 8206 | 5.55 % | 2 |
| 7 | 920 | SEQ ID NO: 8207 | 5.55 % | 2 |
| 8 | 535 | SEQ ID NO: 8208 | 5 % | 1.8 |
| 9 | 364 | SEQ ID NO: 8209 | 3.33 % | 1.2 |
| 10 | 438 | SEQ ID NO: 8210 | 3.33 % | 1.2 |
| 11 | 798 | SEQ ID NO: 8211 | 3.33 % | 1.2 |
| 12 | 808 | SEQ ID NO: 8212 | 3.33 % | 1.2 |
| 13 | 937 | SEQ ID NO: 8213 | 3.33 % | 1.2 |
| 14 | 956 | SEQ ID NO: 8214 | 3.33 % | 1.2 |
| 15 | 557 | SEQ ID NO: 8215 | 2.77 % | 1 |
| 16 | 1218 | SEQ ID NO: 8216 | 2.77 % | 1 |
| 17 | 784 | SEQ ID NO: 8217 | 2.5 % | 0.9 |
| 18 | 249 | SEQ ID NO: 8218 | 2.22 % | 0.8 |
| 19 | 768 | SEQ ID NO: 8219 | 2.22 % | 0.8 |
| 20 | 1178 | SEQ ID NO: 8220 | 2.22 % | 0.8 |

| **HLA A 1101 - 10 mers** | | | | |
|---|---|---|---|---|
| Maximum possible score using this molecule type | | | | 36 |
| **Rank** | **Start position** | **Sequence** | **% of max. score** | **Score** |
| 1 | 38 | SEQ ID NO: 8221 | 13.33 % | 4.8 |
| 2 | 807 | SEQ ID NO: 8222 | 12.5 % | 4.5 |
| 3 | 100 | SEQ ID NO: 8223 | 11.11 % | 4 |
| 4 | 380 | SEQ ID NO: 8224 | 11.11 % | 4 |
| 5 | 767 | SEQ ID NO: 8225 | 10 % | 3.6 |
| 6 | 533 | SEQ ID NO: 8226 | 8.33 % | 3 |
| 7 | 967 | SEQ ID NO: 8227 | 6.66 % | 2.4 |
| 8 | 919 | SEQ ID NO: 8228 | 5.55 % | 2 |
| 9 | 305 | SEQ ID NO: 8229 | 5 % | 1.8 |
| 10 | 211 | SEQ ID NO: 8230 | 3.33 % | 1.2 |
| 11 | 511 | SEQ ID NO: 8231 | 3.33 % | 1.2 |
| 12 | 1177 | SEQ ID NO: 8232 | 3.33 % | 1.2 |
| 13 | 429 | SEQ ID NO: 8233 | 2.77 % | 1 |
| 14 | 758 | SEQ ID NO: 8234 | 2.77 % | 1 |
| 15 | 797 | SEQ ID NO: 8235 | 2.5 % | 0.9 |
| 16 | 255 | SEQ ID NO: 8236 | 2.22 % | 0.8 |
| 17 | 986 | SEQ ID NO: 8237 | 2.22 % | 0.8 |
| 18 | 1157 | SEQ ID NO: 8238 | 2.22 % | 0.8 |
| 19 | 170 | SEQ ID NO: 8239 | 1.66 % | 0.6 |
| 20 | 893 | SEQ ID NO: 8240 | 1.66 % | 0.6 |
| | | | | |

| **HLA B7 - 9 mers** | | | | |
|---|---|---|---|---|
| Maximum possible score using this molecule type | | | | 5400 |
| **Rank** | **Start position** | **Sequence** | **% of max. score** | **Score** |
| 1 | 200 | SEQ ID NO: 8241 | 1.48 % | 80 |
| 2 | 1243 | SEQ ID NO: 8242 | 1.48 % | 80 |
| 3 | 123 | SEQ ID NO: 8243 | 0.74 % | 40 |
| 4 | 248 | SEQ ID NO: 8244 | 0.66 % | 36 |
| 5 | 1036 | SEQ ID NO: 8245 | 0.66 % | 36 |
| 6 | 494 | SEQ ID NO: 8246 | 0.37 % | 20 |
| 7 | 495 | SEQ ID NO: 8247 | 0.37 % | 20 |
| 8 | 523 | SEQ ID NO: 8248 | 0.37 % | 20 |
| 9 | 842 | SEQ ID NO: 8249 | 0.37 % | 20 |
| 10 | 932 | SEQ ID NO: 8250 | 0.37 % | 20 |
| 11 | 274 | SEQ ID NO: 8251 | 0.33 % | 18 |
| 12 | 588 | SEQ ID NO: 8252 | 0.22 % | 12 |
| 13 | 656 | SEQ ID NO: 8253 | 0.22 % | 12 |
| 14 | 657 | SEQ ID NO: 8254 | 0.22 % | 12 |
| 15 | 767 | SEQ ID NO: 8255 | 0.22 % | 12 |
| 16 | 911 | SEQ ID NO: 8256 | 0.22 % | 12 |
| 17 | 939 | SEQ ID NO: 8257 | 0.22 % | 12 |
| 18 | 1007 | SEQ ID NO: 8258 | 0.22 % | 12 |
| 19 | 1170 | SEQ ID NO: 8259 | 0.22 % | 12 |
| 20 | 1206 | SEQ ID NO: 8260 | 0.22 % | 12 |
| | | | | |

| **HLA B7 - 10 mers** | | | | |
|---|---|---|---|---|
| Maximum possible score using this molecule type | | | | 5400 |
| **Rank** | **Start position** | **Sequence** | **% of max. score** | **Score** |
| 1 | 505 | SEQ ID NO: 8261 | 4.44 % | 240 |
| 2 | 312 | SEQ ID NO: 8262 | 3.70 % | 200 |
| 3 | 141 | SEQ ID NO: 8263 | 1.11 % | 60 |
| 4 | 1006 | SEQ ID NO: 8264 | 0.66 % | 36 |
| 5 | 411 | SEQ ID NO: 3265 | 0.44 % | 24 |
| 6 | 122 | SEQ ID NO: 8266 | 0.37 % | 20 |
| 7 | 134 | SEQ ID NO: 8267 | 0.37 % | 20 |
| 8 | 184 | SEQ ID NO: 8268 | 0.37 % | 20 |
| 9 | 367 | SEQ ID NO: 8269 | 0.37 % | 20 |
| 10 | 402 | SEQ ID NO: 8270 | 0.37 % | 20 |
| 11 | 494 | SEQ ID NO: 8271 | 0.37 % | 20 |
| 12 | 560 | SEQ ID NO: 8272 | 0.37 % | 20 |
| 13 | 626 | SEQ ID NO: 8273 | 0.37 % | 20 |
| 14 | 931 | SEQ ID NO: 8274 | 0.37 % | 20 |
| 15 | 956 | SEQ ID NO: 8275 | 0.37 % | 20 |
| 16 | 1117 | SEQ ID NO: 8276 | 0.37 % | 20 |
| 17 | 1169 | SEQ ID NO: 8277 | 0.37 % | 20 |
| 18 | 1196 | SEQ ID NO: 8278 | 0.37 % | 20 |
| 19 | 247 | SEQ ID NO: 8279 | 0.22 % | 12 |
| 20 | 273 | SEQ ID NO: 8280 | 0.22 % | 12 |

**TABLE 26: Cloned sequences for E.coli expression**

| **ORF** | **DNA length bp** | **Cloning** | |
|---|---|---|---|
| | | **pET** | **pGEX** |
| **P28** | 537 | NdeI / XhoI | |
| **P65** | 1917 | NheI / HindIII | |
| **Nsp1A** | 2495 | NheI / XhoI | |
| **Nsp1B** | 2153 | NdeI / XhoI | |
| **Nsp1C** | 2612 | NdeI / XhoI | |
| **Nsp2A** | 431 | NdeI / XhoI | BamHI / XhoI |
| **Nsp2B** | 426 | NdeI / XhoI | BamHI / XhoI |
| **Nsp3** | 870 | NdeI / XhoI | |
| **Nsp4** | 249 | NdeI / XhoI | BamHI / XhoI |
| **Nsp5** | 594 | NheI / XhoI | |
| **Nsp6** | 339 | NdeI / XhoI | BamHI / XhoI |
| **Nsp7** | 417 | NdeI / XhoI | BamHI / XhoI |
| **Nsp9A** | 1385 | NheI /XhoI | |
| **Nsp9B** | 1409 | NdeI / XhoI | |
| **Nsp10** | 1803 | Nhel / XhoI | |
| **Nsp11** | 1581 | NdeI / XhoI | |
| **Nsp12** | 1038 | NdeI / HindIII | |
| **Nsp1A3** | 897 | NdeI / XhoI | |
| **Spike (S1)** | 1946 | NdeI / XhoI | |
| **Spike (S2)** | 1598 | NdeI / XhoI | |
| **Spike (S1-S2)** | 3545 | NdeI / XhoI | |
| **HR1** | 287 | NdeI / XhoI | BamHI / XhoI |
| **HR2** | 146 | NdeI / XhoI | BamHI / XhoI |
| **ORF30100** | 525 | NdeI / XhoI | |
| **ORF4** | 465 | NdeI / XhoI | |
| **Envelope (E)** | 231 | NdeI / XhoI | BamHI / XhoI |
| **Matrix (M)**Δ**100** | 366 | NdeI / XhoI | BamHI / XhoI |
| **ORF7Δ18** | 137 | NdeI / XhoI | BamHI / XhoI |
| **ORF8** | 369 | NdeI / XhoI | BamHI / XhoI |
| **ORF9** | 135 | NdeI / XhoI | BamHI / XhoI |
| **ORF10** | 120 | NheI / XhoI | BamHI / XhoI |
| **ORF11** | 255 | NdeI / XhoI | BamHI / XhoI |
| **Nucleocapsid (N)** | 1269 | NdeI / EcoRI | |
| **ORF12** | 297 | NdeI / EcoRI | BamHI / EcoRI |

**TABLE 29: Cloning, purification and expression in E.coli**

| **SARS CoV ORFs** | **M.W Kd** | **cloning** | **Expr.** | **purification as** |
|---|---|---|---|---|
| **P28** | **19,7** | **+** | **+** | **his sol** |
| **P65** | **70,3** | **+** | **+** | **his sol** |
| **Nsp1A (N-term)** | **91,6** | **+** | **+** | **his ins** |
| **Nsp1B (core)** | **80,8** | **+** | **-** | |
| **Nsp1C (C-term)** | **95,3** | **+** | **-** | |
| **Nsp2A (N-term)** | **15,8** | **+** | **+** | **his ins** |
| **Nsp2B (C-term)** | **15,5** | **+** | **+** | **his sol** |
| **Nsp3** | **31,9** | **+** | **+** | |
| **Nsp4** | **9,1** | **+** | **+** | **his sol** |
| **Nsp5** | **21,8** | **+** | **+** | **his sol** |
| **Nsp6** | **12,4** | **+** | **+** | **his sol** |
| **Nsp9A (N-term)** | **15,3** | **+** | **-** | |
| **Nsp9B (C-term)** | **51,6** | **+** | **+** | **his ins** |
| **Nsp10** | **66** | - | | |
| **Nsp11** | **58** | **-** | **-** | |
| **Nsp12** | **38** | **-** | | |
| **Nsp13** | **32,7** | **+** | **+** | **his ins** |
| **Spike (S1-his)** | **71,3** | **+** | **+** | **his ins** |
| **Spike (S2-his)** | **58,6** | **+** | **-** | |
| **Spike (S1S2-his)** | **130** | **+** | **+** | **his ins** |
| **HR1** | **11** | **+** | **+** | **his ins** |
| **HR2** | **5,4** | **+** | **+** | **his sol** |
| **ORF3 Δ100¹** | **19,1** | **+** | **-** | |
| **ORF4** | **16,9** | **+** | **+** | **his ins (trimer)** |
| **Envelope (E)** | **34,3** | **+** | **+** | **qst ins (IB)** |
| **Matrix (M) Δ100** | **13,3** | **+** | **+** | **his ins** |
| **ORF7Δ18²** | **31** | **+** | **+** | **gst sol** |
| **ORF8** | **39,5** | **+** | | **gst ins (IB)** |
| **ORF9** | **30,8** | **+** | **+** | **gst sol** |
| **ORF10** | **30,3** | **+** | **+** | **gst ins (IB)** |
| **ORF11** | **35,2** | **+** | **+** | **gst ins (IB)** |
| **Nucleocapsid (N)** | **43,6** | **+** | **+** | **his ins** |
| **ORF12** | **36,7** | **+** | **+** | **his ins** |

**TABLE 30: E.coli expression, purity and yield**

| **Protein** | **Tag** | **Purity (%)** | **Yield (mg/l)** |
|---|---|---|---|
| Nsp2A (N-term) | His | 95 | 1.7 |
| Nsp2B (C-term) | His | 95 | 4.1 |
| Nsp4 | His | 95 | 12.6 |
| Nsp5 | His | 95 | 5.88 |
| Nsp6 | His | 95 | 8.1 |
| P28 | His | 95 | 1 |
| P65 | His | 80 | 0.553 |
| HR2 | His | 95 | 11.9 |
| HR1 | His | 80 | 2.64 |
| Nsp1A | His | 95 | 0.267 |
| Spike S1-S2 | His | 80 | 0.381 |
| Matrix M | His | 85 | 12.4 |
| ORF7 | GST | 85 | 4.9 |

### BRIEF DESCRIPTION OF SEQUENCE LISTING

| | |
|---|---|
| 7188-7189 | Component sequences of Figure 25 |
| 7194 | Amino acids 901-1005 of SEQ ID NO: 6042 |
| 7196 | Amino acids 1144-1196 of SEQ ID NO: 6042 |
| 7197-7199 | Membrane fusion peptide regions |
| 7207-7223 | N-glycosylation sites within SEQ ID NO: 6042 |
| 7307-7308 | Fragments of SEQ ID NO: 6042 |
| 7398-7399 | Antigenic fragments of SEQ ID NO: 6042 |
| 9785-9798 | Oligonucleotides used for *S.cerevisiae* expression |
| 9921-9923 | pCMVKm2 vectors |
| 9962 | Spike variant |

## Claims

1. An immunogenic composition comprising an isolated or purified polypeptide comprising
a) the Severe Acute Respiratory Syndrome (SARS) virus Spike polypeptide amino acid sequence SEQ ID NO: 6042, or
b) an amino acid sequence having >80% sequence identity to SEQ ID NO: 6042, or
c) a fragment of at least 10 consecutive amino acids of SEQ ID NO: 6042;
with the proviso that the polypeptide is not MFIFLLFLTLTSGSDLDRCTTFDDVQAP.

2. An immunogenic composition comprising an isolated or purified polypeptide comprising
a) amino acid sequence SEQ ID NO: 6042, or
b) an amino acid sequence having >80% sequence identity to SEQ ID NO: 6042, or
c) a fragment of at least 10 consecutive amino acids of SEQ ID NO: 6042;
and further comprising an adjuvant.

3. The composition of claim 1 or claim 2, wherein the polypeptide comprises: (a) an amino acid sequence having >85%, or >90%, or >95% sequence identity to SEQ ID NO: 6042.

4. The composition of claim 1 or claim 2, wherein the polypeptide comprises a fragment of at least 25, or at least 40, or at least 100 consecutive amino acids of SEQ ID NO: 6042.

5. The composition of any preceding claim, wherein the polypeptide is isolated and purified from the SARS virus.

6. The composition of any preceding claim, wherein the polypeptide is produced by recombinant expression.

7. The composition of claim 6, wherein the fragment comprises any of the polypeptide sequences given in SEQ ID NOs: 7197, 7198, 7199 and 8041-8280.

8. The composition of claim 6 wherein the polypeptide is a fusion protein.

9. The composition of claim 8, wherein the fusion protein comprises one or more of the following:
a) the S1 domain (SEQ ID NO: 7307),
b) S2 domain (SEQ ID NO: 7308),
c) the receptor binding region of the S1 domain,
d) the oligomerization domain regions of the S2 domain,
e) the leucine zipper region of the S2 domain,
f) the membrane anchor region of the S2 domain,
g) the hydrophobic domain region of the S2 domain,
h) the cytoplasmic tail region of the S2 domain, and/or
i) any of the polypeptide sequences given in SEQ ID NOs: 7193-7194, 7196-7199, 7207-7223, 7398, 7399 and 8041-8280.

10. The composition of any preceding claim wherein the polypeptide is in oligomeric form.

11. The composition of claim 10, wherein the oligomer is in trimeric form.

12. The composition of any one of claims 8 or 9, wherein the fusion protein comprises
a) a tag sequence,
b) a second SARS virus protein,
c) a non-SARS virus protein,
d) a bacterial protein and/or
e) an adjuvant.

13. The composition of claim 12, wherein the non-SARS virus protein is derived from a coronavirus, influenza virus, rhinovirus, parainfluenza virus (PIV), respiratory syncytial virus (RSV), adenovirus, and/or metapneumovirus.

14. The composition of claim 12, wherein the bacterial protein is a bacterial adhesion protein or fragment thereof, such as NadA, YadA, UspA2 or a NadA-like protein.

15. The composition of claim 2, or of claims 3-14 when dependent from claim 2, wherein the adjuvant is selected from: mineral salts, oil emulsions, saponin formulations, bacterial or microbial derivatives, human immunomodulators, bioadhesives or mucoadhesives, microparticels, liposomes, polyethylene ether and polyethylene ester formuations, PCPP, muramyl peptides, immidazoquinolone compounds, and/or virosomes or virus-like particles.

16. The composition of claim 15, wherein the adjuvant is a detoxified bacterial ADP-ribosylating toxin, a non-toxic double mutant form of Bordella pertussis toxoids, chitosan, MF59, aluminum, an aluminum salt or a small molecule immunomodulator (SMIP).

## Patentansprüche

1. Immunogene Zusammensetzung, umfassend ein isoliertes oder aufgereinigtes Polypeptid umfassend
a) das Spike-Polypeptid des das schwere akute Atemwegssyndrom (Severe Acute Respiratory Syndrome, SARS) auslösenden Virus mit der Aminosäuresequenz SEQ ID NR: 6042 oder
b) eine Aminosäuresequenz mit >80% Sequenzidentität zu SEQ ID NR: 6042 oder
c) ein Fragment von mindestens 10 aufeinanderfolgenden Aminosäuren von SEQ ID NR: 6042;
mit der Maßgabe, dass es sich bei dem Polypeptid nicht um MFIFLLFLTLTSGSDLDRCTTFDDVQAP handelt.

2. Immunogene Zusammensetzung, umfassend ein isoliertes oder aufgereinigtes Polypeptid umfassend
a) die Aminosäuresequenz SEQ ID NR: 6042 oder
b) eine Aminosäuresequenz mit >80% Sequenzidentität zu SEQ ID NR: 6042 oder
c) ein Fragment von mindestens 10 aufeinanderfolgenden Aminosäuren von SEQ ID NR: 6042,
und weiterhin umfassend ein Adjuvans.

3. Zusammensetzung nach Anspruch 1 oder 2, wobei das Polypeptid Folgendes umfasst: (a) eine Aminosäuresequenz mit >85% oder >90% oder >95% Sequenzidentität zu SEQ ID NR: 6042.

4. Zusammensetzung nach Anspruch 1 oder 2, wobei das Polypeptid ein Fragment von mindestens 25 oder mindestens 40 oder mindestens 100 aufeinanderfolgenden Aminosäuren von SEQ ID NR: 6042 umfasst.

5. Zusammensetzung nach einem der vorhergehenden Ansprüche, wobei das Polypeptid aus dem SARS-Virus isoliert und aufgereinigt wird.

6. Zusammensetzung nach einem der vorhergehenden Ansprüche, wobei das Polypeptid durch rekombinante Expression produziert wird.

7. Zusammensetzung nach Anspruch 6, wobei das Fragment eine der in den SEQ ID NR: 7197, 7198, 7199 und 8041-8280 angegebenen Polypeptidsequenzen umfasst.

8. Zusammensetzung nach Anspruch 6, wobei es sich bei dem Polypeptid um ein Fusionsprotein handelt.

9. Zusammensetzung nach Anspruch 8, wobei das Fusionsprotein ein oder mehrere der Folgenden umfasst:
a) die S1-Domäne (SEQ ID NR: 7307),
b) die S2-Domäne (SEQ ID NR: 7308),
c) die rezeptorbindende Region der S1-Domäne,
d) die Regionen der Oligomerisierungsdomäne der S2-Domäne,
e) die Leucin-Zipper-Region der S2-Domäne,
f) die Membranankerregion der S2-Domäne,
g) die Region der hydrophoben Domäne der S2-Domäne,
h) die zytoplasmatische Schwanzregion der S2-Domäne und/oder
i) eine der in SEQ ID NR: 7193-7194, 7196-7199, 7207-7223, 7398, 7399 und 8041-8280 angeführten Polypeptidsequenzen.

10. Zusammensetzung nach einem der vorhergehenden Ansprüche, wobei das Polypeptid in oligomerer Form vorliegt.

11. Zusammensetzung nach Anspruch 10, wobei das Oligomer in trimerer Form vorliegt.

12. Zusammensetzung nach Anspruch 8 oder 9, wobei das Fusionsprotein Folgendes umfasst:
a) eine Tag-Sequenz,
b) ein zweites SARS-Virusprotein,
c) ein Nicht-SARS-Virusprotein,
d) ein bakterielles Protein und/oder
e) ein Adjuvans.

13. Zusammensetzung nach Anspruch 12, wobei das Nicht-SARS-Virusprotein von einem Coronavirus, einem Grippevirus, einem Rhinovirus, einem Parainfluenzavirus (PIV), einem Respiratory-Syncytial-Virus (RSV), einem Adenovirus und/oder einem Metapneumovirus stammt.

14. Zusammensetzung nach Anspruch 12, wobei es sich bei dem bakteriellen Protein um ein bakterielles Adhäsionsprotein oder ein Fragment davon, wie NadA, YadA, UspA2 oder ein NadA-ähnliches Protein, handelt.

15. Zusammensetzung nach Anspruch 2 oder den Ansprüchen 3-14 in Abhängigkeit von Anspruch 2, wobei das Adjuvans ausgewählt ist aus: Mineralsalzen, Ölemulsionen, Saponinformulierungen, bakteriellen oder mikrobiellen Derivaten, humanen Immunmodulatoren, Bioadhäsiven oder Mukoadhäsiven, Mikropartikeln, Liposomen, Polyethylenether- und Polyethylenesterformulierungen, PCPP, Muramylpeptiden, Imidazochinolonverbindungen und/oder Virosomen oder virusähnlichen Partikeln.

16. Zusammensetzung nach Anspruch 15, wobei es sich bei dem Adjuvans um ein detoxifiziertes bakterielles ADP-ribosylierendes Toxin, eine nichttoxische Doppelmutantenform von Bordellapertussis-Toxoiden, Chitosan, MF59, Aluminium, ein Aluminiumsalz oder einen niedermolekularen Immunmodulator (Small Molecule Immunomodulator, SMIP) handelt.

## Revendications

1. Composition immunogène comprenant un polypeptide isolé ou purifié comprenant
a) la séquence d'acides aminés SEQ ID NO:6042 du polypeptide Spike du virus du Syndrome Respiratoire Aigu Sévère (SRAS), ou
b) une séquence d'acides aminés ayant une identité de séquence > 80 % avec SEQ ID NO:6042, ou
c) un fragment d'au moins 10 acides aminés consécutifs de SEQ ID NO:6042 ;
à la condition que le polypeptide ne soit pas MFIFLLFLTLTSGSDLDRCTTFDDVQAP.

2. Composition immunogène comprenant un polypeptide isolé ou purifié, comprenant
a) la séquence d'acides aminés SEQ ID NO:6042, ou
b) une séquence d'acides aminés ayant une identité de séquence > 80 % avec SEQ ID NO:6042, ou
c) un fragment d'au moins 10 acides aminés consécutifs de SEQ ID NO:6042 ;
et comprenant en outre un adjuvant.

3. Composition selon la revendication 1 ou 2, dans laquelle le polypeptide comprend (a) une séquence d'acides aminés ayant une identité de séquence > 85 % ou > 90 %, ou > 95 % avec SEQ ID NO: 6042.

4. Composition selon la revendication 1 ou 2, dans laquelle le polypeptide comprend un fragment d'au moins 25, ou d'au moins 40, ou d'au moins 100 acides aminés consécutifs de SEQ ID NO:6042.

5. Composition selon l'une quelconque des revendications précédentes, dans laquelle le polypeptide est isolé et purifié à partir du virus SRAS.

6. Composition selon l'une quelconque des revendications précédentes, dans laquelle le peptide est produit par expression recombinante.

7. Composition selon la revendication 6, dans laquelle le fragment comprend l'une quelconque des séquences polypeptidiques présentées dans SEQ ID NO:7197, 7198, 7199 et 8041-8280.

8. Composition selon la revendication 6, dans laquelle le polypeptide est une protéine de fusion.

9. Composition selon la revendication 8, dans laquelle la protéine de fusion comprend un ou plusieurs des éléments suivants :
a) le domaine S1 (SEQ ID NO:7307),
b) un domaine S2 (SEQ ID NO:7308),
c) la région de lésion au récepteur du domaine S1,
d) les régions domaines d'oligomérisation du domaine S2,
e) la région glissière à leucine du domaine S2,
f) la région ancre membranaire du domaine S2,
g) la région domaine hydrophobe du domaine S2,
h) la région queue cytoplasmique du domaine S2, et/ou
i) l'une quelconque des séquences polypeptides présentées dans SEQ ID NO:7193-7194, 7196-7199, 7207-7223, 7398, 7399 et 8041-8280.

10. Composition selon l'une quelconque des revendications précédentes, dans laquelle le polypeptide se présente sous forme oligomère.

11. Composition selon la revendication 10, dans laquelle l'oligomère se présente sous forme trimère.

12. Composition selon l'une quelconque des revendications 8 ou 9, dans laquelle la protéine de fusion comprend :
a) une séquence étiquette,
b) une deuxième protéine du virus SRAS,
c) une protéine d'un virus non SRAS,
d) une protéine bactérienne et/ou
c) un adjuvant.

13. Composition selon la revendication 12, dans laquelle la protéine d'un virus non SRAS dérive d'un coronavirus, d'un virus de la grippe, d'un rhinovirus, d'un virus parainfluenza (PIV), du virus syncytial respiratoire (RSV), d'un adénovirus et/ou d'un métapneumovirus.

14. Composition selon la revendication 12, dans laquelle la protéine bactérienne est une protéine d'adhérence bactérienne, ou un fragment de celle-ci, telle qu'une protéine NadA, YadA, UspA2 ou NadA-like.

15. Composition selon la revendication 2, ou selon les revendications 3-14 quand elles dépendent de la revendication 2, dans laquelle l'adjuvant est choisi parmi les sels minéraux, les émulsions huileuses, les formulations de saponine, les dérivés bactériens ou microbiens, les immunomodulateurs humains, les bioadhésifs ou les mucoadhésifs, les microparticules, les liposomes, les formulations d'éthers et d'esters du polyéthylène, le PCPP, les muramylpeptides, les composés imidazoquinolones et/ou les virosomes ou les particules virus-like.

16. Composition selon la revendication 15, dans laquelle l'adjuvant est une toxine d'ADP-ribosylation bactérienne détoxifiée, une forme double mutante non toxique des toxoïdes de Bordella pertussis, le chitosan, le MF59, l'aluminium, un sel d'aluminium ou un immunomodulateur à petite molécule (SMIP).


<210> 6042
   <211> 1255
   <212> PRT
   <213> SARS coronavirus
<400> 6042
   Met Phe Ile Phe Leu Leu Phe Leu Thr Leu Thr Ser Gly Ser Asp Leu
   1               5                   10                  15
   Asp Arg Cys Thr Thr Phe Asp Asp Val Gln Ala Pro Asn Tyr Thr Gln
   20 25 30
   His Thr Ser Ser Met Arg Gly Val Tyr Tyr Pro Asp Glu Ile Phe Arg
   35 40 45
   Ser Asp Thr Leu Tyr Leu Thr Gln Asp Leu Phe Leu Pro Phe Tyr Ser
   50 55 60
   Asn Val Thr Gly Phe His Thr Ile Asn His Thr Phe Gly Asn Pro Val
   65 70 75 80
   Ile Pro Phe Lys Asp Gly Ile Tyr Phe Ala Ala Thr Glu Lys Ser Asn
   85 90 95
   Val Val Arg Gly Trp Val Phe Gly Ser Thr Met Asn Asn Lys Ser Gln
   100 105 110
   Ser Val Ile Ile Ile Asn Asn Ser Thr Asn Val Val Ile Arg Ala Cys
   115 120 125
   Asn Phe Glu Leu Cys Asp Asn Pro Phe Phe Ala Val Ser Lys Pro Met
   130 135 140
   Gly Thr Gln Thr His Thr Met Ile Phe Asp Asn Ala Phe Asn Cys Thr
   145 150 155 160
   Phe Glu Tyr Ile Ser Asp Ala Phe Ser Leu Asp Val Ser Glu Lys Ser
   165 170 175
   Gly Asn Phe Lys His Leu Arg Glu Phe Val Phe Lys Asn Lys Asp Gly
   180 185 190
   Phe Leu Tyr Val Tyr Lys Gly Tyr Gln Pro Ile Asp Val Val Arg Asp
   195 200 205
   Leu Pro Ser Gly Phe Asn Thr Leu Lys Pro Ile Phe Lys Leu Pro Leu
   210 215 220
   Gly Ile Asn Ile Thr Asn Phe Arg Ala Ile Leu Thr Ala Phe Ser Pro
   225 230 235 240
   Ala Gln Asp Ile Trp Gly Thr Ser Ala Ala Ala Tyr Phe Val Gly Tyr
   245 250 255
   Leu Lys Pro Thr Thr Phe Met Leu Lys Tyr Asp Glu Asn Gly Thr Ile
   260 265 270
   Thr Asp Ala Val Asp Cys Ser Gln Asn Pro Leu Ala Glu Leu Lys Cys
   275 280 285
   Ser Val Lys Ser Phe Glu Ile Asp Lys Gly Ile Tyr Gln Thr Ser Asn
   290 295 300
   Phe Arg Val Val Pro Ser Gly Asp Val Val Arg Phe Pro Asn Ile Thr
   305 310 315 320
   Asn Leu Cys Pro Phe Gly Glu Val Phe Asn Ala Thr Lys Phe Pro Ser
   325 330 335
   Val Tyr Ala Trp Glu Arg Lys Lys Ile Ser Asn Cys Val Ala Asp Tyr
   340 345 350
   Ser Val Leu Tyr Asn Ser Thr Phe Phe Ser Thr Phe Lys Cys Tyr Gly
   355 360 365
   Val Ser Ala Thr Lys Leu Asn Asp Leu Cys Phe Ser Asn Val Tyr Ala
   370 375 380
   Asp Ser Phe Val Val Lys Gly Asp Asp Val Arg Gln Ile Ala Pro Gly
   385 390 395 400
   Gln Thr Gly Val Ile Ala Asp Tyr Asn Tyr Lys Leu Pro Asp Asp Phe
   405 410 415
   Met Gly Cys Val Leu Ala Trp Asn Thr Arg Asn Ile Asp Ala Thr Ser
   420 425 430
   Thr Gly Asn Tyr Asn Tyr Lys Tyr Arg Tyr Leu Arg His Gly Lys Leu
   435 440 445
   Arg Pro Phe Glu Arg Asp Ile Ser Asn Val Pro Phe Ser Pro Asp Gly
   450 455 460
   Lys Pro Cys Thr Pro Pro Ala Leu Asn Cys Tyr Trp Pro Leu Asn Asp
   465 470 475 480
   Tyr Gly Phe Tyr Thr Thr Thr Gly Ile Gly Tyr Gln Pro Tyr Arg Val
   485 490 495
   Val Val Leu Ser Phe Glu Leu Leu Asn Ala Pro Ala Thr Val Cys Gly
   500 505 510
   Pro Lys Leu Ser Thr Asp Leu Ile Lys Asn Gln Cys Val Asn Phe Asn
   515 520 525
   Phe Asn Gly Leu Thr Gly Thr Gly Val Leu Thr Pro Ser Ser Lys Arg
   530 535 540
   Phe Gln Pro Phe Gln Gln Phe Gly Arg Asp Val Ser Asp Phe Thr Asp
   545 550 555 560
   Ser Val Arg Asp Pro Lys Thr Ser Glu Ile Leu Asp Ile Ser Pro Cys
   565 570 575
   Ala Phe Gly Gly Val Ser Val Ile Thr Pro Gly Thr Asn Ala Ser Ser
   580 585 590
   Glu Val Ala Val Leu Tyr Gln Asp Val Asn Cys Thr Asp Val Ser Thr
   595 600 605
   Ala Ile His Ala Asp Gln Leu Thr Pro Ala Trp Arg Ile Tyr Ser Thr
   610 615 620
   Gly Asn Asn Val Phe Gln Thr Gln Ala Gly Cys Leu Ile Gly Ala Glu
   625 630 635 640
   His Val Asp Thr Ser Tyr Glu Cys Asp Ile Pro Ile Gly Ala Gly Ile
   645 650 655
   Cys Ala Ser Tyr His Thr Val Ser Leu Leu Arg Ser Thr Ser Gln Lys
   660 665 670
   Ser Ile Val Ala Tyr Thr Met Ser Leu Gly Ala Asp Ser Ser Ile Ala
   675 680 685
   Tyr Ser Asn Asn Thr Ile Ala Ile Pro Thr Asn Phe Ser Ile Ser Ile
   690 695 700
   Thr Thr Glu Val Met Pro Val Ser Met Ala Lys Thr Ser Val Asp Cys
   705 710 715 720
   Asn Met Tyr Ile Cys Gly Asp Ser Thr Glu Cys Ala Asn Leu Leu Leu
   725 730 735
   Gln Tyr Gly Ser Phe Cys Thr Gln Leu Asn Arg Ala Leu Ser Gly Ile
   740 745 750
   Ala Ala Glu Gln Asp Arg Asn Thr Arg Glu Val Phe Ala Gln Val Lys
   755 760 765
   Gln Met Tyr Lys Thr Pro Thr Leu Lys Tyr Phe Gly Gly Phe Asn Phe
   770 775 780
   Ser Gln Ile Leu Pro Asp Pro Leu Lys Pro Thr Lys Arg Ser Phe Ile
   785 790 795 800
   Glu Asp Leu Leu Phe Asn Lys Val Thr Leu Ala Asp Ala Gly Phe Met
   805 810 815
   Lys Gln Tyr Gly Glu Cys Leu Gly Asp Ile Asn Ala Arg Asp Leu Ile
   820 825 830
   Cys Ala Gln Lys Phe Asn Gly Leu Thr Val Leu Pro Pro Leu Leu Thr
   835 840 845
   Asp Asp Met Ile Ala Ala Tyr Thr Ala Ala Leu Val Ser Gly Thr Ala
   850 855 860
   Thr Ala Gly Trp Thr Phe Gly Ala Gly Ala Ala Leu Gln Ile Pro Phe
   865 870 875 880
   Ala Met Gln Met Ala Tyr Arg Phe Asn Gly Ile Gly Val Thr Gln Asn
   885 890 895
   Val Leu Tyr Glu Asn Gln Lys Gln Ile Ala Asn Gln Phe Asn Lys Ala
   900 905 910
   Ile Ser Gln Ile Gln Glu Ser Leu Thr Thr Thr Ser Thr Ala Leu Gly
   915 920 925
   Lys Leu Gln Asp Val Val Asn Gln Asn Ala Gln Ala Leu Asn Thr Leu
   930 935 940
   Val Lys Gln Leu Ser Ser Asn Phe Gly Ala Ile Ser Ser Val Leu Asn
   945 950 955 960
   Asp Ile Leu Ser Arg Leu Asp Lys Val Glu Ala Glu Val Gln Ile Asp
   965 970 975
   Arg Leu Ile Thr Gly Arg Leu Gln Ser Leu Gln Thr Tyr Val Thr Gln
   980 985 990
   Gln Leu Ile Arg Ala Ala Glu Ile Arg Ala Ser Ala Asn Leu Ala Ala
   995 1000 1005
   Thr Lys Met Ser Glu Cys Val Leu Gly Gln Ser Lys Arg Val Asp Phe
   1010 1015 1020
   Cys Gly Lys Gly Tyr His Leu Met Ser Phe Pro Gln Ala Ala Pro His
   1025 1030 1035 1040
   Gly Val Val Phe Leu His Val Thr Tyr Val Pro Ser Gln Glu Arg Asn
   1045 1050 1055
   Phe Thr Thr Ala Pro Ala Ile Cys His Glu Gly Lys Ala Tyr Phe Pro
   1060 1065 1070
   Arg Glu Gly Val Phe Val Phe Asn Gly Thr Ser Trp Phe Ile Thr Gln
   1075 1080 1085
   Arg Asn Phe Phe Ser Pro Gln Ile Ile Thr Thr Asp Asn Thr Phe Val
   1090 1095 1100
   Ser Gly Asn Cys Asp Val Val Ile Gly Ile Ile Asn Asn Thr Val Tyr
   1105 1110 1115 1120
   Asp Pro Leu Gln Pro Glu Leu Asp Ser Phe Lys Glu Glu Leu Asp Lys
   1125 1130 1135
   Tyr Phe Lys Asn His Thr Ser Pro Asp Val Asp Leu Gly Asp Ile Ser
   1140 1145 1150
   Gly Ile Asn Ala Ser Val Val Asn Ile Gln Lys Glu Ile Asp Arg Leu
   1155 1160 1165
   Asn Glu Val Ala Lys Asn Leu Asn Glu Ser Leu Ile Asp Leu Gln Glu
   1170 1175 1180
   Leu Gly Lys Tyr Glu Gln Tyr Ile Lys Trp Pro Trp Tyr Val Trp Leu
   1185 1190 1195 1200
   Gly Phe Ile Ala Gly Leu Ile Ala Ile Val Met Val Thr Ile Leu Leu
   1205 1210 1215
   Cys Cys Met Thr Ser Cys Cys Ser Cys Leu Lys Gly Ala Cys Ser Cys
   1220 1225 1230
   Gly Ser Cys Cys Lys Phe Asp Glu Asp Asp Ser Glu Pro Val Leu Lys
   1235 1240 1245
   Gly Val Lys Leu His Tyr Thr
   1250 1255
<210> 7188
   <211> 2712
   <212> PRT
   <213> SARS coronavirus
<400> 7188
   Pro Thr Pro Arg Thr Leu Asp Ala Val Cys Gly Cys Ile Asn Val Phe
   1 5 10 15
   Lys Arg Val Cys Gly Val Ser Ala Ala Arg Leu Thr Pro Cys Gly Thr
   20 25 30
   Gly Thr Ser Thr Asp Val Val Tyr Arg Ala Phe Asp Ile Tyr Asn Glu
   35 40 45
   Lys Val Ala Gly Phe Ala Lys Phe Leu Lys Thr Asn Cys Cys Arg Phe
   50 55 60
   Gln Glu Lys Asp Glu Glu Gly Asn Leu Leu Asp Ser Tyr Phe Val Val
   65 70 75 80
   Lys Arg His Thr Met Ser Asn Tyr Gln His Glu Glu Thr Ile Tyr Asn
   85 90 95
   Leu Val Lys Asp Cys Pro Ala Val Ala Val His Asp Phe Phe Lys Phe
   100 105 110
   Arg Val Asp Gly Asp Met Val Pro His Ile Ser Arg Gln Arg Leu Thr
   115 120 125
   Lys Tyr Thr Met Ala Asp Leu Val Tyr Ala Leu Arg His Phe Asp Glu
   130 135 140
   Gly Asn Cys Asp Thr Leu Lys Glu Ile Leu Val Thr Tyr Asn Cys Cys
   145 150 155 160
   Asp Asp Asp Tyr Phe Asn Lys Lys Asp Trp Tyr Asp Phe Val Glu Asn
   165 170 175
   Pro Asp Ile Leu Arg Val Tyr Ala Asn Leu Gly Glu Arg Val Arg Gln
   180 185 190
   Ser Leu Leu Lys Thr Val Gln Phe Cys Asp Ala Met Arg Asp Ala Gly
   195 200 205
   Ile Val Gly Val Leu Thr Leu Asp Asn Gln Asp Leu Asn Gly Asn Trp
   210 215 220
   Tyr Asp Phe Gly Asp Phe Val Gln Val Ala Pro Gly Cys Gly Val Pro
   225 230 235 240
   Ile Val Asp Ser Tyr Tyr Ser Leu Leu Met Pro Ile Leu Thr Leu Thr
   245 250 255
   Arg Ala Leu Ala Ala Glu Ser His Met Asp Ala Asp Leu Ala Lys Pro
   260 265 270
   Leu Ile Lys Trp Asp Leu Leu Lys Tyr Asp Phe Thr Glu Glu Arg Leu
   275 280 285
   Cys Leu Phe Asp Arg Tyr Phe Lys Tyr Trp Asp Gln Thr Tyr His Pro
   290 295 300
   Asn Cys Ile Asn Cys Leu Asp Asp Arg Cys Ile Leu His Cys Ala Asn
   305 310 315 320
   Phe Asn Val Leu Phe Ser Thr Val Phe Pro Pro Thr Ser Phe Gly Pro
   325 330 335
   Leu Val Arg Lys Ile Phe Val Asp Gly Val Pro Phe Val Val Ser Thr
   340 345 350
   Gly Tyr His Phe Arg Glu Leu Gly Val Val His Asn Gln Asp Val Asn
   355 360 365
   Leu His Ser Ser Arg Leu Ser Phe Lys Glu Leu Leu Val Tyr Ala Ala
   370 375 380
   Asp Pro Ala Met His Ala Ala Ser Gly Asn Leu Leu Leu Asp Lys Arg
   385 390 395 400
   Thr Thr Cys Phe Ser Val Ala Ala Leu Thr Asn Asn Val Ala Phe Gln
   405 410 415
   Thr Val Lys Pro Gly Asn Phe Asn Lys Asp Phe Tyr Asp Phe Ala Val
   420 425 430
   Ser Lys Gly Phe Phe Lys Glu Gly Ser Ser Val Glu Leu Lys His Phe
   435 440 445
   Phe Phe Ala Gln Asp Gly Asn Ala Ala Ile Ser Asp Tyr Asp Tyr Tyr
   450 455 460
   Arg Tyr Asn Leu Pro Thr Met Cys Asp Ile Arg Gln Leu Leu Phe Val
   465 470 475 480
   Val Glu Val Val Asp Lys Tyr Phe Asp Cys Tyr Asp Gly Gly Cys Ile
   485 490 495
   Asn Ala Asn Gln Val Ile Val Asn Asn Leu Asp Lys Ser Ala Gly Phe
   500 505 510
   Pro Phe Asn Lys Trp Gly Lys Ala Arg Leu Tyr Tyr Asp Ser Met Ser
   515 520 525
   Tyr Glu Asp Gln Asp Ala Leu Phe Ala Tyr Thr Lys Arg Asn Val Ile
   530 535 540
   Pro Thr Ile Thr Gln Met Asn Leu Lys Tyr Ala Ile Ser Ala Lys Asn
   545 550 555 560
   Arg Ala Arg Thr Val Ala Gly Val Ser Ile Cys Ser Thr Met Thr Asn
   565 570 575
   Arg Gln Phe His Gln Lys Leu Leu Lys Ser Ile Ala Ala Thr Arg Gly
   580 585 590
   Ala Thr Val Val Ile Gly Thr Ser Lys Phe Tyr Gly Gly Trp His Asn
   595 600 605
   Met Leu Lys Thr Val Tyr Ser Asp Val Glu Thr Pro His Leu Met Gly
   610 615 620
   Trp Asp Tyr Pro Lys Cys Asp Arg Ala Met Pro Asn Met Leu Arg Ile
   625 630 635 640
   Met Ala Ser Leu Val Leu Ala Arg Lys His Asn Thr Cys Cys Asn Leu
   645 650 655
   Ser His Arg Phe Tyr Arg Leu Ala Asn Glu Cys Ala Gln Val Leu Ser
   660 665 670
   Glu Met Val Met Cys Gly Gly Ser Leu Tyr Val Lys Pro Gly Gly Thr
   675 680 685
   Ser Ser Gly Asp Ala Thr Thr Ala Tyr Ala Asn Ser Val Phe Asn Ile
   690 695 700
   Cys Gln Ala Val Thr Ala Asn Val Asn Ala Leu Leu Ser Thr Asp Gly
   705 710 715 720
   Asn Lys Ile Ala Asp Lys Tyr Val Arg Asn Leu Gln His Arg Leu Tyr
   725 730 735
   Glu Cys Leu Tyr Arg Asn Arg Asp Val Asp His Glu Phe Val Asp Glu
   740 745 750
   Phe Tyr Ala Tyr Leu Arg Lys His Phe Ser Met Met Ile Leu Ser Asp
   755 760 765
   Asp Ala Val Val Cys Tyr Asn Ser Asn Tyr Ala Ala Gln Gly Leu Val
   770 775 780
   Ala Ser Ile Lys Asn Phe Lys Ala Val Leu Tyr Tyr Gln Asn Asn Val
   785 790 795 800
   Phe Met Ser Glu Ala Lys Cys Trp Thr Glu Thr Asp Leu Thr Lys Gly
   805 810 815
   Pro His Glu Phe Cys Ser Gln His Thr Met Leu Val Lys Gln Gly Asp
   820 825 830
   Asp Tyr Val Tyr Leu Pro Tyr Pro Asp Pro Ser Arg Ile Leu Gly Ala
   835 840 845
   Gly Cys Phe Val Asp Asp Ile Val Lys Thr Asp Gly Thr Leu Met Ile
   850 855 860
   Glu Arg Phe Val Ser Leu Ala Ile Asp Ala Tyr Pro Leu Thr Lys His
   865 870 875 880
   Pro Asn Gln Glu Tyr Ala Asp Val Phe His Leu Tyr Leu Gln Tyr Ile
   885 890 895
   Arg Lys Leu His Asp Glu Leu Thr Gly His Met Leu Asp Met Tyr Ser
   900 905 910
   Val Met Leu Thr Asn Asp Asn Thr Ser Arg Tyr Trp Glu Pro Glu Phe
   915 920 925
   Tyr Glu Ala Met Tyr Thr Pro His Thr Val Leu Gln Ala Val Gly Ala
   930 935 940
   Cys Val Leu Cys Asn Ser Gln Thr Ser Leu Arg Cys Gly Ala Cys Ile
   945 950 955 960
   Arg Arg Pro Phe Leu Cys Cys Lys Cys Cys Tyr Asp His Val Ile Ser
   965 970 975
   Thr Ser His Lys Leu Val Leu Ser Val Asn Pro Tyr Val Cys Asn Ala
   980 985 990
   Pro Gly Cys Asp Val Thr Asp Val Thr Gln Leu Tyr Leu Gly Gly Met
   995 1000 1005
   Ser Tyr Tyr Cys Lys Ser His Lys Pro Pro Ile Ser Phe Pro Leu Cys
   1010 1015 1020
   Ala Asn Gly Gln Val Phe Gly Leu Tyr Lys Asn Thr Cys Val Gly Ser
   1025 1030 1035 1040
   Asp Asn Val Thr Asp Phe Asn Ala Ile Ala Thr Cys Asp Trp Thr Asn
   1045 1050 1055
   Ala Gly Asp Tyr Ile Leu Ala Asn Thr Cys Thr Glu Arg Leu Lys Leu
   1060 1065 1070
   Phe Ala Ala Glu Thr Leu Lys Ala Thr Glu Glu Thr Phe Lys Leu Ser
   1075 1080 1085
   Tyr Gly Ile Ala Thr Val Arg Glu Val Leu Ser Asp Arg Glu Leu His
   1090 1095 1100
   Leu Ser Trp Glu Val Gly Lys Pro Arg Pro Pro Leu Asn Arg Asn Tyr
   1105 1110 1115 1120
   Val Phe Thr Gly Tyr Arg Val Thr Lys Asn Ser Lys Val Gln Ile Gly
   1125 1130 1135
   Glu Tyr Thr Phe Glu Lys Gly Asp Tyr Gly Asp Ala Val Val Tyr Arg
   1140 1145 1150
   Gly Thr Thr Thr Tyr Lys Leu Asn Val Gly Asp Tyr Phe Val Leu Thr
   1155 1160 1165
   Ser His Thr Val Met Pro Leu Ser Ala Pro Thr Leu Val Pro Gln Glu
   1170 1175 1180
   His Tyr Val Arg Ile Thr Gly Leu Tyr Pro Thr Leu Asn Ile Ser Asp
   1185 1190 1195 1200
   Glu Phe Ser Ser Asn Val Ala Asn Tyr Gln Lys Val Gly Met Gln Lys
   1205 1210 1215
   Tyr Ser Thr Leu Gln Gly Pro Pro Gly Thr Gly Lys Ser His Phe Ala
   1220 1225 1230
   Ile Gly Leu Ala Leu Tyr Tyr Pro Ser Ala Arg Ile Val Tyr Thr Ala
   1235 1240 1245
   Cys Ser His Ala Ala Val Asp Ala Leu Cys Glu Lys Ala Leu Lys Tyr
   1250 1255 1260
   Leu Pro Ile Asp Lys Cys Ser Arg Ile Ile Pro Ala Arg Ala Arg Val
   1265 1270 1275 1280
   Glu Cys Phe Asp Lys Phe Lys Val Asn Ser Thr Leu Glu Gln Tyr Val
   1285 1290 1295
   Phe Cys Thr Val Asn Ala Leu Pro Glu Thr Thr Ala Asp Ile Val Val
   1300 1305 1310
   Phe Asp Glu Ile Ser Met Ala Thr Asn Tyr Asp Leu Ser Val Val Asn
   1315 1320 1325
   Ala Arg Leu Arg Ala Lys His Tyr Val Tyr Ile Gly Asp Pro Ala Gln
   1330 1335 1340
   Leu Pro Ala Pro Arg Thr Leu Leu Thr Lys Gly Thr Leu Glu Pro Glu
   1345 1350 1355 1360
   Tyr Phe Asn Ser Val Cys Arg Leu Met Lys Thr Ile Gly Pro Asp Met
   1365 1370 1375
   Phe Leu Gly Thr Cys Arg Arg Cys Pro Ala Glu Ile Val Asp Thr Val
   1380 1385 1390
   Ser Ala Leu Val Tyr Asp Asn Lys Leu Lys Ala His Lys Asp Lys Ser
   1395 1400 1405
   Ala Gln Cys Phe Lys Met Phe Tyr Lys Gly Val Ile Thr His Asp Val
   1410 1415 1420
   Ser Ser Ala Ile Asn Arg Pro Gln Ile Gly Val Val Arg Glu Phe Leu
   1425 1430 1435 1440
   Thr Arg Asn Pro Ala Trp Arg Lys Ala Val Phe Ile Ser Pro Tyr Asn
   1445 1450 1455
   Ser Gln Asn Ala Val Ala Ser Lys Ile Leu Gly Leu Pro Thr Gln Thr
   1460 1465 1470
   Val Asp Ser Ser Gln Gly Ser Glu Tyr Asp Tyr Val Ile Phe Thr Gln
   1475 1480 1485
   Thr Thr Glu Thr Ala His Ser Cys Asn Val Asn Arg Phe Asn Val Ala
   1490 1495 1500
   Ile Thr Arg Ala Lys Ile Gly Ile Leu Cys Ile Met Ser Asp Arg Asp
   1505 1510 1515 1520
   Leu Tyr Asp Lys Leu Gln Phe Thr Ser Leu Glu Ile Pro Arg Arg Asn
   1525 1530 1535
   Val Ala Thr Leu Gln Ala Glu Asn Val Thr Gly Leu Phe Lys Asp Cys
   1540 1545 1550
   Ser Lys Ile Ile Thr Gly Leu His Pro Thr Gln Ala Pro Thr His Leu
   1555 1560 1565
   Ser Val Asp Ile Lys Phe Lys Thr Glu Gly Leu Cys Val Asp Ile Pro
   1570 1575 1580
   Gly Ile Pro Lys Asp Met Thr Tyr Arg Arg Leu Ile Ser Met Met Gly
   1585 1590 1595 1600
   Phe Lys Met Asn Tyr Gln Val Asn Gly Tyr Pro Asn Met Phe Ile Thr
   1605 1610 1615
   Arg Glu Glu Ala Ile Arg His Val Arg Ala Trp Ile Gly Phe Asp Val
   1620 1625 1630
   Glu Gly Cys His Ala Thr Arg Asp Ala Val Gly Thr Asn Leu Pro Leu
   1635 1640 1645
   Gln Leu Gly Phe Ser Thr Gly Val Asn Leu Val Ala Val Pro Thr Gly
   1650 1655 1660
   Tyr Val Asp Thr Glu Asn Asn Thr Glu Phe Thr Arg Val Asn Ala Lys
   1665 1670 1675 1680
   Pro Pro Pro Gly Asp Gln Phe Lys His Leu Ile Pro Leu Met Tyr Lys
   1685 1690 1695
   Gly Leu Pro Trp Asn Val Val Arg Ile Lys Ile Val Gln Met Leu Ser
   1700 1705 1710
   Asp Thr Leu Lys Gly Leu Ser Asp Arg Val Val Phe Val Leu Trp Ala
   1715 1720 1725
   His Gly Phe Glu Leu Thr Ser Met Lys Tyr Phe Val Lys Ile Gly Pro
   1730 1735 1740
   Glu Arg Thr Cys Cys Leu Cys Asp Lys Arg Ala Thr Cys Phe Ser Thr
   1745 1750 1755 1760
   Ser Ser Asp Thr Tyr Ala Cys Trp Asn His Ser Val Gly Phe Asp Tyr
   1765 1770 1775
   Val Tyr Asn Pro Phe Met Ile Asp Val Gln Gln Trp Gly Phe Thr Gly
   1780 1785 1790
   Asn Leu Gln Ser Asn His Asp Gln His Cys Gln Val His Gly Asn Ala
   1795 1800 1805
   His Val Ala Ser Cys Asp Ala Ile Met Thr Arg Cys Leu Ala Val His
   1810 1815 1820
   Glu Cys Phe Val Lys Arg Val Asp Trp Ser Val Glu Tyr Pro Ile Ile
   1825 1830 1835 1840
   Gly Asp Glu Leu Arg Val Asn Ser Ala Cys Arg Lys Val Gln His Met
   1845 1850 1855
   Val Val Lys Ser Ala Leu Leu Ala Asp Lys Phe Pro Val Leu His Asp
   1860 1865 1870
   Ile Gly Asn Pro Lys Ala Ile Lys Cys Val Pro Gln Ala Glu Val Glu
   1875 1880 1885
   Trp Lys Phe Tyr Asp Ala Gln Pro Cys Ser Asp Lys Ala Tyr Lys Ile
   1890 1895 1900
   Glu Glu Leu Phe Tyr Ser Tyr Ala Thr His His Asp Lys Phe Thr Asp
   1905 1910 1915 1920
   Gly Val Cys Leu Phe Trp Asn Cys Asn Val Asp Arg Tyr Pro Ala Asn
   1925 1930 1935
   Ala Ile Val Cys Arg Phe Asp Thr Arg Val Leu Ser Asn Leu Asn Leu
   1940 1945 1950
   Pro Gly Cys Asp Gly Gly Ser Leu Tyr Val Asn Lys His Ala Phe His
   1955 1960 1965
   Thr Pro Ala Phe Asp Lys Ser Ala Phe Thr Asn Leu Lys Gln Leu Pro
   1970 1975 1980
   Phe Phe Tyr Tyr Ser Asp Ser Pro Cys Glu Ser His Gly Lys Gln Val
   1985 1990 1995 2000
   Val Ser Asp Ile Asp Tyr Val Pro Leu Lys Ser Ala Thr Cys Ile Thr
   2005 2010 2015
   Arg Cys Asn Leu Gly Gly Ala Val Cys Arg His His Ala Asn Glu Tyr
   2020 2025 2030
   Arg Gln Tyr Leu Asp Ala Tyr Asn Met Met Ile Ser Ala Gly Phe Ser
   2035 2040 2045
   Leu Trp Ile Tyr Lys Gln Phe Asp Thr Tyr Asn Leu Trp Asn Thr Phe
   2050 2055 2060
   Thr Arg Leu Gln Ser Leu Glu Asn Val Ala Tyr Asn Val Val Asn Lys
   2065 2070 2075 2080
   Gly His Phe Asp Gly His Ala Gly Glu Ala Pro Val Ser Ile Ile Asn
   2085 2090 2095
   Asn Ala Val Tyr Thr Lys Val Asp Gly Ile Asp Val Glu Ile Phe Glu
   2100 2105 2110
   Asn Lys Thr Thr Leu Pro Val Asn Val Ala Phe Glu Leu Trp Ala Lys
   2115 2120 2125
   Arg Asn Ile Lys Pro Val Pro Glu Ile Lys Ile Leu Asn Asn Leu Gly
   2130 2135 2140
   Val Asp Ile Ala Ala Asn Thr Val Ile Trp Asp Tyr Lys Arg Glu Ala
   2145 2150 2155 2160
   Pro Ala His Val Ser Thr Ile Gly Val Cys Thr Met Thr Asp Ile Ala
   2165 2170 2175
   Lys Lys Pro Thr Glu Ser Ala Cys Ser Ser Leu Thr Val Leu Phe Asp
   2180 2185 2190
   Gly Arg Val Glu Gly Gln Val Asp Leu Phe Arg Asn Ala Arg Asn Gly
   2195 2200 2205
   Val Leu Ile Thr Glu Gly Ser Val Lys Gly Leu Thr Pro Ser Lys Gly
   2210 2215 2220
   Pro Ala Gln Ala Ser Val Asn Gly Val Thr Leu Ile Gly Glu Ser Val
   2225 2230 2235 2240
   Lys Thr Gln Phe Asn Tyr Phe Lys Lys Val Asp Gly Ile Ile Gln Gln
   2245 2250 2255
   Leu Pro Glu Thr Tyr Phe Thr Gln Ser Arg Asp Leu Glu Asp Phe Lys
   2260 2265 2270
   Pro Arg Ser Gln Met Glu Thr Asp Phe Leu Glu Leu Ala Met Asp Glu
   2275 2280 2285
   Phe Ile Gln Arg Tyr Lys Leu Glu Gly Tyr Ala Phe Glu His Ile Val
   2290 2295 2300
   Tyr Gly Asp Phe Ser His Gly Gln Leu Gly Gly Leu His Leu Met Ile
   2305 2310 2315 2320
   Gly Leu Ala Lys Arg Ser Gln Asp Ser Pro Leu Lys Leu Glu Asp Phe
   2325 2330 2335
   Ile Pro Met Asp Ser Thr Val Lys Asn Tyr Phe Ile Thr Asp Ala Gln
   2340 2345 2350
   Thr Gly Ser Ser Lys Cys Val Cys Ser Val Ile Asp Leu Leu Leu Asp
   2355 2360 2365
   Asp Phe Val Glu Ile Ile Lys Ser Gln Asp Leu Ser Val Ile Ser Lys
   2370 2375 2380
   Val Val Lys Val Thr Ile Asp Tyr Ala Glu Ile Ser Phe Met Leu Trp
   2385 2390 2395 2400
   Cys Lys Asp Gly His Val Glu Thr Phe Tyr Pro Lys Leu Gln Ala Ser
   2405 2410 2415
   Arg Ala Trp Gln Pro Gly Val Ala Met Pro Asn Leu Tyr Lys Met Gln
   2420 2425 2430
   Arg Met Leu Leu Glu Lys Cys Asp Leu Gln Asn Tyr Gly Glu Asn Ala
   2435 2440 2445
   Val Ile Pro Lys Gly Ile Met Met Asn Val Ala Lys Tyr Thr Gln Leu
   2450 2455 2460
   Cys Gln Tyr Leu Asn Thr Leu Thr Leu Ala Val Pro Tyr Asn Met Arg
   2465 2470 2475 2480
   Val Ile His Phe Gly Ala Gly Ser Asp Lys Gly Val Ala Pro Gly Thr
   2485 2490 2495
   Ala Val Leu Arg Gln Trp Leu Pro Thr Gly Thr Leu Leu Val Asp Ser
   2500 2505 2510
   Asp Leu Asn Asp Phe Val Ser Asp Ala Tyr Ser Thr Leu Ile Gly Asp
   2515 2520 2525
   Cys Ala Thr Val His Thr Ala Asn Lys Trp Asp Leu Ile Ile Ser Asp
   2530 2535 2540
   Met Tyr Asp Pro Arg Thr Lys His Val Thr Lys Glu Asn Asp Ser Lys
   2545 2550 2555 2560
   Glu Gly Phe Phe Thr Tyr Leu Cys Gly Phe Ile Lys Gln Lys Leu Ala
   2565 2570 2575
   Leu Gly Gly Ser Ile Ala Val Lys Ile Thr Glu His Ser Trp Asn Ala
   2580 2585 2590
   Asp Leu Tyr Lys Leu Met Gly His Phe Ser Trp Trp Thr Ala Phe Val
   2595 2600 2605
   Thr Asn Val Asn Ala Ser Ser Ser Glu Ala Phe Leu Ile Gly Ala Asn
   2610 2615 2620
   Tyr Leu Gly Lys Pro Lys Glu Gln Ile Asp Gly Tyr Thr Met His Ala
   2625 2630 2635 2640
   Asn Tyr Ile Phe Trp Arg Asn Thr Asn Pro Ile Gln Leu Ser Ser Tyr
   2645 2650 2655
   Ser Leu Phe Asp Met Ser Lys Phe Pro Leu Lys Leu Arg Gly Thr Ala
   2660 2665 2670
   Val Met Ser Leu Lys Glu Asn Gln Ile Asn Asp Met Ile Tyr Ser Leu
   2675 2680 2685
   Leu Glu Lys Gly Arg Leu Ile Ile Arg Glu Asn Asn Arg Val Val Val
   2690 2695 2700
   Ser Ser Asp Ile Leu Val Asn Asn
   2705 2710
<210> 7189
   <211> 1257
   <212> PRT
   <213> SARS coronavirus
<400> 7189
   Thr Asn Met Phe Ile Phe Leu Leu Phe Leu Thr Leu Thr Ser Gly Ser
   1 5 10 15
   Asp Leu Asp Arg Cys Thr Thr Phe Asp Asp Val Gln Ala Pro Asn Tyr
   20 25 30
   Thr Gln His Thr Ser Ser Met Arg Gly Val Tyr Tyr Pro Asp Glu Ile
   35 40 45
   Phe Arg Ser Asp Thr Leu Tyr Leu Thr Gln Asp Leu Phe Leu Pro Phe
   50 55 60
   Tyr Ser Asn Val Thr Gly Phe His Thr Ile Asn His Thr Phe Gly Asn
   65 70 75 80
   Pro Val Ile Pro Phe Lys Asp Gly Ile Tyr Phe Ala Ala Thr Glu Lys
   85 90 95
   Ser Asn Val Val Arg Gly Trp Val Phe Gly Ser Thr Met Asn Asn Lys
   100 105 110
   Ser Gln Ser Val Ile Ile Ile Asn Asn Ser Thr Asn Val Val Ile Arg
   115 120 125
   Ala Cys Asn Phe Glu Leu Cys Asp Asn Pro Phe Phe Ala Val Ser Lys
   130 135 140
   Pro Met Gly Thr Gln Thr His Thr Met Ile Phe Asp Asn Ala Phe Asn
   145 150 155 160
   Cys Thr Phe Glu Tyr Ile Ser Asp Ala Phe Ser Leu Asp Val Ser Glu
   165 170 175
   Lys Ser Gly Asn Phe Lys His Leu Arg Glu Phe Val Phe Lys Asn Lys
   180 185 190
   Asp Gly Phe Leu Tyr Val Tyr Lys Gly Tyr Gln Pro Ile Asp Val Val
   195 200 205
   Arg Asp Leu Pro Ser Gly Phe Asn Thr Leu Lys Pro Ile Phe Lys Leu
   210 215 220
   Pro Leu Gly Ile Asn Ile Thr Asn Phe Arg Ala Ile Leu Thr Ala Phe
   225 230 235 240
   Ser Pro Ala Gln Asp Ile Trp Gly Thr Ser Ala Ala Ala Tyr Phe Val
   245 250 255
   Gly Tyr Leu Lys Pro Thr Thr Phe Met Leu Lys Tyr Asp Glu Asn Gly
   260 265 270
   Thr Ile Thr Asp Ala Val Asp Cys Ser Gln Asn Pro Leu Ala Glu Leu
   275 280 285
   Lys Cys Ser Val Lys Ser Phe Glu Ile Asp Lys Gly Ile Tyr Gln Thr
   290 295 300
   Ser Asn Phe Arg Val Val Pro Ser Gly Asp Val Val Arg Phe Pro Asn
   305 310 315 320
   Ile Thr Asn Leu Cys Pro Phe Gly Glu Val Phe Asn Ala Thr Lys Phe
   325 330 335
   Pro Ser Val Tyr Ala Trp Glu Arg Lys Lys Ile Ser Asn Cys Val Ala
   340 345 350
   Asp Tyr Ser Val Leu Tyr Asn Ser Thr Phe Phe Ser Thr Phe Lys Cys
   355 360 365
   Tyr Gly Val Ser Ala Thr Lys Leu Asn Asp Leu Cys Phe Ser Asn Val
   370 375 380
   Tyr Ala Asp Ser Phe Val Val Lys Gly Asp Asp Val Arg Gln Ile Ala
   385 390 395 400
   Pro Gly Gln Thr Gly Val Ile Ala Asp Tyr Asn Tyr Lys Leu Pro Asp
   405 410 415
   Asp Phe Met Gly Cys Val Leu Ala Trp Asn Thr Arg Asn Ile Asp Ala
   420 425 430
   Thr Ser Thr Gly Asn Tyr Asn Tyr Lys Tyr Arg Tyr Leu Arg His Gly
   435 440 445
   Lys Leu Arg Pro Phe Glu Arg Asp Ile Ser Asn Val Pro Phe Ser Pro
   450 455 460
   Asp Gly Lys Pro Cys Thr Pro Pro Ala Leu Asn Cys Tyr Trp Pro Leu
   465 470 475 480
   Asn Asp Tyr Gly Phe Tyr Thr Thr Thr Gly Ile Gly Tyr Gln Pro Tyr
   485 490 495
   Arg Val Val Val Leu Ser Phe Glu Leu Leu Asn Ala Pro Ala Thr Val
   500 505 510
   Cys Gly Pro Lys Leu Ser Thr Asp Leu Ile Lys Asn Gln Cys Val Asn
   515 520 525
   Phe Asn Phe Asn Gly Leu Thr Gly Thr Gly Val Leu Thr Pro Ser Ser
   530 535 540
   Lys Arg Phe Gln Pro Phe Gln Gln Phe Gly Arg Asp Val Ser Asp Phe
   545 550 555 560
   Thr Asp Ser Val Arg Asp Pro Lys Thr Ser Glu Ile Leu Asp Ile Ser
   565 570 575
   Pro Cys Ala Phe Gly Gly Val Ser Val Ile Thr Pro Gly Thr Asn Ala
   580 585 590
   Ser Ser Glu Val Ala Val Leu Tyr Gln Asp Val Asn Cys Thr Asp Val
   595 600 605
   Ser Thr Ala Ile His Ala Asp Gln Leu Thr Pro Ala Trp Arg Ile Tyr
   610 615 620
   Ser Thr Gly Asn Asn Val Phe Gln Thr Gln Ala Gly Cys Leu Ile Gly
   625 630 635 640
   Ala Glu His Val Asp Thr Ser Tyr Glu Cys Asp Ile Pro Ile Gly Ala
   645 650 655
   Gly Ile Cys Ala Ser Tyr His Thr Val Ser Leu Leu Arg Ser Thr Ser
   660 665 670
   Gln Lys Ser Ile Val Ala Tyr Thr Met Ser Leu Gly Ala Asp Ser Ser
   675 680 685
   Ile Ala Tyr Ser Asn Asn Thr Ile Ala Ile Pro Thr Asn Phe Ser Ile
   690 695 700
   Ser Ile Thr Thr Glu Val Met Pro Val Ser Met Ala Lys Thr Ser Val
   705 710 715 720
   Asp Cys Asn Met Tyr Ile Cys Gly Asp Ser Thr Glu Cys Ala Asn Leu
   725 730 735
   Leu Leu Gln Tyr Gly Ser Phe Cys Thr Gln Leu Asn Arg Ala Leu Ser
   740 745 750
   Gly Ile Ala Ala Glu Gln Asp Arg Asn Thr Arg Glu Val Phe Ala Gln
   755 760 765
   Val Lys Gln Met Tyr Lys Thr Pro Thr Leu Lys Tyr Phe Gly Gly Phe
   770 775 780
   Asn Phe Ser Gln Ile Leu Pro Asp Pro Leu Lys Pro Thr Lys Arg Ser
   785 790 795 800
   Phe Ile Glu Asp Leu Leu Phe Asn Lys Val Thr Leu Ala Asp Ala Gly
   805 810 815
   Phe Met Lys Gln Tyr Gly Glu Cys Leu Gly Asp Ile Asn Ala Arg Asp
   820 825 830
   Leu Ile Cys Ala Gln Lys Phe Asn Gly Leu Thr Val Leu Pro Pro Leu
   835 840 845
   Leu Thr Asp Asp Met Ile Ala Ala Tyr Thr Ala Ala Leu Val Ser Gly
   850 855 860
   Thr Ala Thr Ala Gly Trp Thr Phe Gly Ala Gly Ala Ala Leu Gln Ile
   865 870 875 880
   Pro Phe Ala Met Gln Met Ala Tyr Arg Phe Asn Gly Ile Gly Val Thr
   885 890 895
   Gln Asn Val Leu Tyr Glu Asn Gln Lys Gln Ile Ala Asn Gln Phe Asn
   900 905 910
   Lys Ala Ile Ser Gln Ile Gln Glu Ser Leu Thr Thr Thr Ser Thr Ala
   915 920 925
   Leu Gly Lys Leu Gln Asp Val Val Asn Gln Asn Ala Gln Ala Leu Asn
   930 935 940
   Thr Leu Val Lys Gln Leu Ser Ser Asn Phe Gly Ala Ile Ser Ser Val
   945 950 955 960
   Leu Asn Asp Ile Leu Ser Arg Leu Asp Lys Val Glu Ala Glu Val Gln
   965 970 975
   Ile Asp Arg Leu Ile Thr Gly Arg Leu Gln Ser Leu Gln Thr Tyr Val
   980 985 990
   Thr Gln Gln Leu Ile Arg Ala Ala Glu Ile Arg Ala Ser Ala Asn Leu
   995 1000 1005
   Ala Ala Thr Lys Met Ser Glu Cys Val Leu Gly Gln Ser Lys Arg Val
   1010 1015 1020
   Asp Phe Cys Gly Lys Gly Tyr His Leu Met Ser Phe Pro Gln Ala Ala
   1025 1030 1035 1040
   Pro His Gly Val Val Phe Leu His Val Thr Tyr Val Pro Ser Gln Glu
   1045 1050 1055
   Arg Asn Phe Thr Thr Ala Pro Ala Ile Cys His Glu Gly Lys Ala Tyr
   1060 1065 1070
   Phe Pro Arg Glu Gly Val Phe Val Phe Asn Gly Thr Ser Trp Phe Ile
   1075 1080 1085
   Thr Gln Arg Asn Phe Phe Ser Pro Gln Ile Ile Thr Thr Asp Asn Thr
   1090 1095 1100
   Phe Val Ser Gly Asn Cys Asp Val Val Ile Gly Ile Ile Asn Asn Thr
   1105 1110 1115 1120
   Val Tyr Asp Pro Leu Gln Pro Glu Leu Asp Ser Phe Lys Glu Glu Leu
   1125 1130 1135
   Asp Lys Tyr Phe Lys Asn His Thr Ser Pro Asp Val Asp Leu Gly Asp
   1140 1145 1150
   Ile Ser Gly Ile Asn Ala Ser Val Val Asn Ile Gln Lys Glu Ile Asp
   1155 1160 1165
   Arg Leu Asn Glu Val Ala Lys Asn Leu Asn Glu Ser Leu Ile Asp Leu
   1170 1175 1180
   Gln Glu Leu Gly Lys Tyr Glu Gln Tyr Ile Lys Trp Pro Trp Tyr Val
   1185 1190 1195 1200
   Trp Leu Gly Phe Ile Ala Gly Leu Ile Ala Ile Val Met Val Thr Ile
   1205 1210 1215
   Leu Leu Cys Cys Met Thr Ser Cys Cys Ser Cys Leu Lys Gly Ala Cys
   1220 1225 1230
   Ser Cys Gly Ser Cys Cys Lys Phe Asp Glu Asp Asp Ser Glu Pro Val
   1235 1240 1245
   Leu Lys Gly Val Lys Leu His Tyr Thr
   1250 1255
<210> 7193
   <211> 102
   <212> PRT
   <213> SARS cor onavi r us
<400> 7193 Ile Asp Arg Leu Ile Thr 100
<210> 7194
   <211> 105
   <212> PRT
   <213> SARS coronavi rus
<400> 7194
   Asn Gln Lys Gln Ile Ala Asn Gln Phe Asn Lys Ala Ile Ser Gln Ile
   1 5 10 15
   Gln Glu Ser Leu Thr Thr Thr Ser Thr Ala Leu Gly Lys Leu Gln Asp
   20 25 30
   Val Val Asn Gln Asn Ala Gln Ala Leu Asn Thr Leu Val Lys Gln Leu
   35 40 45
   Ser Ser Asn Phe Gly Ala Ile Ser Ser Val Leu Asn Asp Ile Leu Ser
   50 55 60
   Arg Leu Asp Lys Val Glu Ala Glu Val Gln Ile Asp Arg Leu Ile Thr
   65 70 75 80
   Gly Arg Leu Gln Ser Leu Gln Thr Tyr Val Thr Gln Gln Leu Ile Arg
   85 90 95
   Ala Ala Glu Ile Arg Ala Ser Ala Asn
   100 105
<210> 7196
   <211> 52
   <212> PRT
   <213> SARS cor onavi r us
<400> 7196
   Pro Asp Val Asp Leu Gly Asp Ile Ser Gly Ile Asn Ala Ser Val Val
   1 5 10 15
   Asn Ile Gln Lys Glu Ile Asp Arg Leu Asn Glu Val Ala Lys Asn Leu
   20 25 30
   Asn Glu Ser Leu Ile Asp Leu Gln Glu Leu Gly Lys Tyr Glu Gln Tyr
   35 40 45
   Ile Lys Trp Pro
   50
<210> 7197
   <211> 19
   <212> PRT
   <213> SARS coronavirus
<400> 7197
   Lys Gln Ile Ala Asn Gln Phe Asn Lys Ala Ile Ser Gln Ile Gln Glu
   1 5 10 15
   Ser Leu Thr
<210> 7198
   <211> 17
   <212> PRT
   <213> SARS coronavirus
<400> 7198
   His Leu Met Ser Phe Pro Gln Ala Ala Pro His Gly Val Val Phe Leu
   1 5 10 15
   His
<210> 7199
   <211> 20
   <212> PRT
   <213> SARS coronavirus
<400> 7199
   Ala Ala Leu Val Ser Gly Thr Ala Thr Ala Gly Trp Thr Phe Gly Ala
   1 5 10 15
   Gly Ala Ala Leu
   20
<210> 7207
   <211> 4
   <212> PRT
   <213> SARS coronavirus
<400> 7207
   Asn Tyr Thr Gln
   1
<210> 7208
   <211> 4
   <212> PRT
   <213> SARS coronavirus
<400> 7208
   Asn Val Thr Gly
   1
<210> 7209
   <211> 4
   <212> PRT
   <213> SARS coronavirus
<400> 7209
   Asn Lys Ser Gln
   1
<210> 7210
   <211> 4
   <212> PRT
   <213> SARS coronavirus
<400> 7210
   Asn Ser Thr Asn
   1
<210> 7211
   <211> 4
   <212> PRT
   <213> SARS coronavirus
<400> 7211
   Asn Cys Thr Phe
   1
<210> 7212
   <211> 4
   <212> PRT
   <213> SARS cor onavi r us
<400> 7212
   Asn Ile Thr Asn
   1
<210> 7213
   <211> 4
   <212> PRT
   <213> SARS cor onavi rus
<400> 7213
   Asn Gly Thr Ile
   1
<210> 7214
   <211> 4
   <212> PRT
   <213> SARS coronavirus
<400> 7214
   Asn Ile Thr Asn
   1
<210> 7215
   <211> 4
   <212> PRT
   <213> SARS coronavirus
<400> 7215
   Asn Ala Thr Lys
   1
<210> 7216
   <211> 4
   <212> PRT
   <213> SARS coronavirus
<400> 7216
   Asn Ser Thr Phe
   1
<210> 7217
   <211> 4
   <212> PRT
   <213> SARS coronavirus
<400> 7217
   Asn Ala Ser Ser
   1
<210> 7218
   <211> 4
   <212> PRT
   <213> SARS coronavirus
<400> 7218
   Asn Cys Thr Asp
   1
<210> 7219
   <211> 4
   <212> PRT
   <213> SARS coronavirus
<400> 7219
   Asn Phe Ser Ile
   1
<210> 7220
   <211> 4
   <212> PRT
   <213> SARS coronavirus
<400> 7220
   Asn Phe Ser Gln
   1
<210> 7221
   <211> 4
   <212> PRT
   <213> SARS coronavirus
<400> 7221
   Asn Gly Thr Ser
   1
<210> 7222
   <211> 4
   <212> PRT
   <213> SARS coronavirus
<400> 7222
   Asn Asn Thr Val
   1
<210> 7223
   <211> 4
   <212> PRT
   <213> SARS coronavirus
<400> 7223
   Asn Glu Ser Leu
   1
<210> 7307
   <211> 649
   <212> PRT
   <213> SARS coronavirus
<400> 7307
   Ser Asp Leu Asp Arg Cys Thr Thr Phe Asp Asp Val Gln Ala Pro Asn
   1               5                   10                  15
   Tyr Thr Gln His Thr Ser Ser Met Arg Gly Val Tyr Tyr Pro Asp Glu
               20                  25                  30
   Ile Phe Arg Ser Asp Thr Leu Tyr Leu Thr Gln Asp Leu Phe Leu Pro
           35                  40                  45
   Phe Tyr Ser Asn Val Thr Gly Phe His Thr Ile Asn His Thr Phe Gly
       50                  55                  60
   Asn Pro Val Ile Pro Phe Lys Asp Gly Ile Tyr Phe Ala Ala Thr Glu
   65 70 75 80
   Lys Ser Asn Val Val Arg Gly Trp Val Phe Gly Ser Thr Met Asn Asn
   85 90 95
   Lys Ser Gln Ser Val Ile Ile Ile Asn Asn Ser Thr Asn Val Val Ile
   100 105 110
   Arg Ala Cys Asn Phe Glu Leu Cys Asp Asn Pro Phe Phe Ala Val Ser
   115 120 125
   Lys Pro Met Gly Thr Gln Thr His Thr Met Ile Phe Asp Asn Ala Phe
   130 135 140
   Asn Cys Thr Phe Glu Tyr Ile Ser Asp Ala Phe Ser Leu Asp Val Ser
   145 150 155 160
   Glu Lys Ser Gly Asn Phe Lys His Leu Arg Glu Phe Val Phe Lys Asn
   165 170 175
   Lys Asp Gly Phe Leu Tyr Val Tyr Lys Gly Tyr Gln Pro Ile Asp Val
   180 185 190
   Val Arg Asp Leu Pro Ser Gly Phe Asn Thr Leu Lys Pro Ile Phe Lys
   195 200 205
   Leu Pro Leu Gly Ile Asn Ile Thr Asn Phe Arg Ala Ile Leu Thr Ala
   210 215 220
   Phe Ser Pro Ala Gln Asp Ile Trp Gly Thr Ser Ala Ala Ala Tyr Phe
   225 230 235 240
   Val Gly Tyr Leu Lys Pro Thr Thr Phe Met Leu Lys Tyr Asp Glu Asn
   245 250 255
   Gly Thr Ile Thr Asp Ala Val Asp Cys Ser Gln Asn Pro Leu Ala Glu
   260 265 270
   Leu Lys Cys Ser Val Lys Ser Phe Glu Ile Asp Lys Gly Ile Tyr Gln
   275 280 285
   Thr Ser Asn Phe Arg Val Val Pro Ser Gly Asp Val Val Arg Phe Pro
   290 295 300
   Asn Ile Thr Asn Leu Cys Pro Phe Gly Glu Val Phe Asn Ala Thr Lys
   305 310 315 320
   Phe Pro Ser Val Tyr Ala Trp Glu Arg Lys Lys Ile Ser Asn Cys Val
   325 330 335
   Ala Asp Tyr Ser Val Leu Tyr Asn Ser Thr Phe Phe Ser Thr Phe Lys
   340 345 350
   Cys Tyr Gly Val Ser Ala Thr Lys Leu Asn Asp Leu Cys Phe Ser Asn
   355 360 365
   Val Tyr Ala Asp Ser Phe Val Val Lys Gly Asp Asp Val Arg Gln Ile
   370 375 380
   Ala Pro Gly Gln Thr Gly Val Ile Ala Asp Tyr Asn Tyr Lys Leu Pro
   385 390 395 400
   Asp Asp Phe Met Gly Cys Val Leu Ala Trp Asn Thr Arg Asn Ile Asp
   405 410 415
   Ala Thr Ser Thr Gly Asn Tyr Asn Tyr Lys Tyr Arg Tyr Leu Arg His
   420 425 430
   Gly Lys Leu Arg Pro Phe Glu Arg Asp Ile Ser Asn Val Pro Phe Ser
   435 440 445
   Pro Asp Gly Lys Pro Cys Thr Pro Pro Ala Leu Asn Cys Tyr Trp Pro
   450 455 460
   Leu Asn Asp Tyr Gly Phe Tyr Thr Thr Thr Gly Ile Gly Tyr Gln Pro
   465 470 475 480
   Tyr Arg Val Val Val Leu Ser Phe Glu Leu Leu Asn Ala Pro Ala Thr
   485 490 495
   Val Cys Gly Pro Lys Leu Ser Thr Asp Leu Ile Lys Asn Gln Cys Val
   500 505 510
   Asn Phe Asn Phe Asn Gly Leu Thr Gly Thr Gly Val Leu Thr Pro Ser
   515 520 525
   Ser Lys Arg Phe Gln Pro Phe Gln Gln Phe Gly Arg Asp Val Ser Asp
   530 535 540
   Phe Thr Asp Ser Val Arg Asp Pro Lys Thr Ser Glu Ile Leu Asp Ile
   545 550 555 560
   Ser Pro Cys Ala Phe Gly Gly Val Ser Val Ile Thr Pro Gly Thr Asn
   565 570 575
   Ala Ser Ser Glu Val Ala Val Leu Tyr Gln Asp Val Asn Cys Thr Asp
   580 585 590
   Val Ser Thr Ala Ile His Ala Asp Gln Leu Thr Pro Ala Trp Arg Ile
   595 600 605
   Tyr Ser Thr Gly Asn Asn Val Phe Gln Thr Gln Ala Gly Cys Leu Ile
   610 615 620
   Gly Ala Glu His Val Asp Thr Ser Tyr Glu Cys Asp Ile Pro Ile Gly
   625 630 635 640
   Ala Gly Ile Cys Ala Ser Tyr His Thr
   645
<210> 7308
   <211> 533
   <212> PRT
   <213> SARS coronavirus
<400> 7308
   Val Ser Leu Leu Arg Ser Thr Ser Gln Lys Ser Ile Val Ala Tyr Thr
   1               5                   10                  15
   Met Ser Leu Gly Ala Asp Ser Ser Ile Ala Tyr Ser Asn Asn Thr Ile
               20                  25                  30
   Ala Ile Pro Thr Asn Phe Ser Ile Ser Ile Thr Thr Glu Val Met Pro
           35                  40                  45
   Val Ser Met Ala Lys Thr Ser Val Asp Cys Asn Met Tyr Ile Cys Gly
       50                  55                  60
   Asp Ser Thr Glu Cys Ala Asn Leu Leu Leu Gln Tyr Gly Ser Phe Cys
   65                  70                  75                  80
   Thr Gln Leu Asn Arg Ala Leu Ser Gly Ile Ala Ala Glu Gln Asp Arg
                   85                  90                  95
   Asn Thr Arg Glu Val Phe Ala Gln Val Lys Gln Met Tyr Lys Thr Pro
               100                 105                 110
   Thr Leu Lys Tyr Phe Gly Gly Phe Asn Phe Ser Gln Ile Leu Pro Asp
   115 120 125
   Pro Leu Lys Pro Thr Lys Arg Ser Phe Ile Glu Asp Leu Leu Phe Asn
   130 135 140
   Lys Val Thr Leu Ala Asp Ala Gly Phe Met Lys Gln Tyr Gly Glu Cys
   145 150 155 160
   Leu Gly Asp Ile Asn Ala Arg Asp Leu Ile Cys Ala Gln Lys Phe Asn
   165 170 175
   Gly Leu Thr Val Leu Pro Pro Leu Leu Thr Asp Asp Met Ile Ala Ala
   180 185 190
   Tyr Thr Ala Ala Leu Val Ser Gly Thr Ala Thr Ala Gly Trp Thr Phe
   195 200 205
   Gly Ala Gly Ala Ala Leu Gln Ile Pro Phe Ala Met Gln Met Ala Tyr
   210 215 220
   Arg Phe Asn Gly Ile Gly Val Thr Gln Asn Val Leu Tyr Glu Asn Gln
   225 230 235 240
   Lys Gln Ile Ala Asn Gln Phe Asn Lys Ala Ile Ser Gln Ile Gln Glu
   245 250 255
   Ser Leu Thr Thr Thr Ser Thr Ala Leu Gly Lys Leu Gln Asp Val Val
   260 265 270
   Asn Gln Asn Ala Gln Ala Leu Asn Thr Leu Val Lys Gln Leu Ser Ser
   275 280 285
   Asn Phe Gly Ala Ile Ser Ser Val Leu Asn Asp Ile Leu Ser Arg Leu
   290 295 300
   Asp Lys Val Glu Ala Glu Val Gln Ile Asp Arg Leu Ile Thr Gly Arg
   305 310 315 320
   Leu Gln Ser Leu Gln Thr Tyr Val Thr Gln Gln Leu Ile Arg Ala Ala
   325 330 335
   Glu Ile Arg Ala Ser Ala Asn Leu Ala Ala Thr Lys Met Ser Glu Cys
   340 345 350
   Val Leu Gly Gln Ser Lys Arg Val Asp Phe Cys Gly Lys Gly Tyr His
   355 360 365
   Leu Met Ser Phe Pro Gln Ala Ala Pro His Gly Val Val Phe Leu His
   370 375 380
   Val Thr Tyr Val Pro Ser Gln Glu Arg Asn Phe Thr Thr Ala Pro Ala
   385 390 395 400
   Ile Cys His Glu Gly Lys Ala Tyr Phe Pro Arg Glu Gly Val Phe Val
   405 410 415
   Phe Asn Gly Thr Ser Trp Phe Ile Thr Gln Arg Asn Phe Phe Ser Pro
   420 425 430
   Gln Ile Ile Thr Thr Asp Asn Thr Phe Val Ser Gly Asn Cys Asp Val
   435 440 445
   Val Ile Gly Ile Ile Asn Asn Thr Val Tyr Asp Pro Leu Gln Pro Glu
   450 455 460
   Leu Asp Ser Phe Lys Glu Glu Leu Asp Lys Tyr Phe Lys Asn His Thr
   465 470 475 480
   Ser Pro Asp Val Asp Leu Gly Asp Ile Ser Gly Ile Asn Ala Ser Val
   485 490 495
   Val Asn Ile Gln Lys Glu Ile Asp Arg Leu Asn Glu Val Ala Lys Asn
   500 505 510
   Leu Asn Glu Ser Leu Ile Asp Leu Gln Glu Leu Gly Lys Tyr Glu Gln
   515 520 525
   Tyr Ile Lys Trp Pro
   530
<210> 7398
   <211> 16
   <212> PRT
   <213> SARS coronavirus
<400> 7398
   Asp Val Gln Ala Pro Asn Tyr Thr Gln His Thr Ser Ser Met Arg Gly
   1 5 10 15
<210> 7399
   <211> 15
   <212> PRT
   <213> SARS coronavirus
<400> 7399
   Pro Ser Ser Lys Arg Phe Gln Pro Phe Gln Gln Phe Gly Arg Asp
   1 5 10 15
<210> 8041
   <211> 9
   <212> PRT
   <213> SARS coronavirus
<400> 8041
   Leu Thr Asp Asp Met Ile Ala Ala Tyr
   1 5
<210> 8042
   <211> 9
   <212> PRT
   <213> SARS coronavirus
<400> 8042
   Phe Ile Glu Asp Leu Leu Phe Asn Lys
   1 5
<210> 8043
   <211> 9
   <212> PRT
   <213> SARS coronavirus
<400> 8043
   Leu Pro Asp Pro Leu Lys Pro Thr Lys
   1 5
<210> 8044
   <211> 9
   <212> PRT
   <213> SARS coronavirus
<400> 8044
   Leu Ile Asp Leu Gln Glu Leu Gly Lys
   1 5
<210> 8045
   <211> 9
   <212> PRT
   <213> SARS coronavirus
<400> 8045
   Gly Ala Glu His Val Asp Thr Ser Tyr
   1 5
<210> 8046
   <211> 9
   <212> PRT
   <213> SARS coronavirus
<400> 8046
   Phe Thr Asp Ser Val Arg Asp Pro Lys
   1 5
<210> 8047
   <211> 9
   <212> PRT
   <213> SARS coronavirus
<400> 8047
   Arg Val Asp Phe Cys Gly Lys Gly Tyr
   1 5
<210> 8048
   <211> 9
   <212> PRT
   <213> SARS coronavirus
<400> 8048
   Leu Ala Glu Leu Lys Cys Ser Val Lys
   1 5
<210> 8049
   <211> 9
   <212> PRT
   <213> SARS coronavirus
<400> 8049
   Asp Ser Glu Pro Val Leu Lys Gly Val
   1 5
<210> 8050
   <211> 9
   <212> PRT
   <213> SARS coronavirus
<400> 8050
   Cys Thr Pro Pro Ala Leu Asn Cys Tyr
   1 5
<210> 8051
   <211> 9
   <212> PRT
   <213> SARS coronavirus
<400> 8051
   Ser Thr Glu Cys Ala Asn Leu Leu Leu
   1 5
<210> 8052
   <211> 9
   <212> PRT
   <213> SARS cor onavi r us
<400> 8052
   Glu Val Met Pro Val Ser Met Ala Lys
   1 5
<210> 8053
   <211> 9
   <212> PRT
   <213> SARS cor onavi r us
<400> 8053
   Phe Gly Glu Val Phe Asn Ala Thr Lys
   1 5
<210> 8054
   <211> 9
   <212> PRT
   <213> SARS coronavirus
<400> 8054
   Ala Ala Glu Gln Asp Arg Asn Thr Arg
   1 5
<210> 8055
   <211> 9
   <212> PRT
   <213> SARS coronavirus
<400> 8055
   Thr Gln Asp Leu Phe Leu Pro Phe Tyr
   1 5
<210> 8056
   <211> 9
   <212> PRT
   <213> SARS coronavirus
<400> 8056
   Val Ser Asp Phe Thr Asp Ser Val Arg
   1 5
<210> 8057
   <211> 9
   <212> PRT
   <213> SARS cor onavi r us
<400> 8057
   Ser Ser Glu Val Ala Val Leu Tyr Gln
   1 5
<210> 8058
   <211> 9
   <212> PRT
   <213> SARS coronavirus
<400> 8058
   Ile Leu Asp Ile Ser Pro Cys Ala Phe
   1 5
<210> 8059
   <211> 9
   <212> PRT
   <213> SARS coronavirus
<400> 8059
   Gln Leu Thr Pro Ala Trp Arg Ile Tyr
   1 5
<210> 8060
   <211> 9
   <212> PRT
   <213> SARS cor onavi r us
<400> 8060
   Ala Thr Glu Lys Ser Asn Val Val Arg
   1 5
<210> 8061
   <211> 10
   <212> PRT
   <213> SARS coronavirus
<400> 8061
   Asp Ser Glu Pro Val Leu Lys Gly Val Lys
   1 5 10
<210> 8062
   <211> 10
   <212> PRT
   <213> SARS coronavirus
<400> 8062
   Lys Val Glu Ala Glu Val Gln Ile Asp Arg
   1 5 10
<210> 8063
   <211> 10
   <212> PRT
   <213> SARS coronavirus
<400> 8063
   Met Ser Glu Cys Val Leu Gly Gln Ser Lys
   1 5 10
<210> 8064
   <211> 10
   <212> PRT
   <213> SARS coronavirus
<400> 8064
   Asn Ile Asp Ala Thr Ser Thr Gly Asn Tyr
   1 5 10
<210> 8065
   <211> 10
   <212> PRT
   <213> SARS cor onavi r us
<400> 8065
   Leu Ala Asp Ala Gly Phe Met Lys Gln Tyr
   1 5 10
<210> 8066
   <211> 10
   <212> PRT
   <213> SARS coronavirus
<400> 8066
   Leu Ile Asp Leu Gln Glu Leu Gly Lys Tyr
   1 5 10
<210> 8067
   <211> 10
   <212> PRT
   <213> SARS cor onavi r us
<400> 8067
   Leu Pro Asp Pro Leu Lys Pro Thr Lys Arg
   1 5 10
<210> 8068
   <211> 10
   <212> PRT
   <213> SARS coronavirus
<400> 8068
   Leu Asn Asp Ile Leu Ser Arg Leu Asp Lys
   1 5 10
<210> 8069
   <211> 10
   <212> PRT
   <213> SARS coronavirus
<400> 8069
   Ser Thr Glu Cys Ala Asn Leu Leu Leu Gln
   1 5 10
<210> 8070
   <211> 10
   <212> PRT
   <213> SARS coronavirus
<400> 8070
   His Ala Asp Gln Leu Thr Pro Ala Trp Arg
   1 5 10
<210> 8071
   <211> 10
   <212> PRT
   <213> SARS coronavirus
<400> 8071
   Gly Ser Asp Leu Asp Arg Cys Thr Thr Phe
   1 5 10
<210> 8072
   <211> 10
   <212> PRT
   <213> SARS coronavirus
<400> 8072
   Leu Gln Glu Leu Gly Lys Tyr Glu Gln Tyr
   1 5 10
<210> 8073
   <211> 10
   <212> PRT
   <213> SARS coronavirus
<400> 8073
   Leu Asn Asp Leu Cys Phe Ser Asn Val Tyr
   1 5 10
<210> 8074
   <211> 10
   <212> PRT
   <213> SARS coronavirus
<400> 8074
   Cys Thr Asp Val Ser Thr Ala Ile His Ala
   1 5 10
<210> 8075
   <211> 10
   <212> PRT
   <213> SARS coronavirus
<400> 8075
   Thr Phe Asp Asp Val Gln Ala Pro Asn Tyr
   1 5 10
<210> 8076
   <211> 10
   <212> PRT
   <213> SARS coronavirus
<400> 8076
   His Thr Ser Ser Met Arg Gly Val Tyr Tyr
   1 5 10
<210> 8077
   <211> 10
   <212> PRT
   <213> SARS coronavirus
<400> 8077
   Leu Thr Gln Asp Leu Phe Leu Pro Phe Tyr
   1 5 10
<210> 8078
   <211> 10
   <212> PRT
   <213> SARS coronavirus
<400> 8078
   Gln Thr Gly Val Ile Ala Asp Tyr Asn Tyr
   1 5 10
<210> 8079
   <211> 10
   <212> PRT
   <213> SARS coronavirus
<400> 8079
   Phe Thr Asp Ser Val Arg Asp Pro Lys Thr
   1 5 10
<210> 8080
   <211> 10
   <212> PRT
   <213> SARS coronavirus
<400> 8080
   Ser Thr Ser Gln Lys Ser Ile Val Ala Tyr
   1 5 10
<210> 8081
   <211> 9
   <212> PRT
   <213> SARS coronavirus
<400> 8081
   Gln Met Tyr Lys Thr Pro Thr Leu Lys
   1 5
<210> 8082
   <211> 9
   <212> PRT
   <213> SARS coronavirus
<400> 8082
   Thr Leu Ala Asp Ala Gly Phe Met Lys
   1 5
<210> 8083
   <211> 9
   <212> PRT
   <213> SARS coronavirus
<400> 8083
   Gly Ile Tyr Phe Ala Ala Thr Glu Lys
   1 5
<210> 8084
   <211> 9
   <212> PRT
   <213> SARS coronavirus
<400> 8084
   Ser Leu Leu Arg Ser Thr Ser Gln Lys
   1 5
<210> 8085
   <211> 9
   <212> PRT
   <213> SARS coronavirus
<400> 8085
   Val Leu Lys Gly Val Lys Leu His Tyr
   1 5
<210> 8086
   <211> 9
   <212> PRT
   <213> SARS coronavirus
<400> 8086
   Ser Val Lys Ser Phe Glu Ile Asp Lys
   1 5
<210> 8087
   <211> 9
   <212> PRT
   <213> SARS coronavirus
<400> 8087
   Thr Leu Tyr Leu Thr Gln Asp Leu Phe
   1 5
<210> 8088
   <211> 9
   <212> PRT
   <213> SARS coronavirus
<400> 8088
   Asn Leu Cys Pro Phe Gly Glu Val Phe
   1 5
<210> 8089
   <211> 9
   <212> PRT
   <213> SARS coronavirus
<400> 8089
   Gly Val Ile Ala Asp Tyr Asn Tyr Lys
   1 5
<210> 8090
   <211> 9
   <212> PRT
   <213> SARS coronavirus
<400> 8090
   Phe Ile Glu Asp Leu Leu Phe Asn Lys
   1 5
<210> 8091
   <211> 9
   <212> PRT
   <213> SARS coronavirus
<400> 8091
   Lys Gln Ile Ala Asn Gln Phe Asn Lys
   1 5
<210> 8092
   <211> 9
   <212> PRT
   <213> SARS cor onavi r us
<400> 8092
   Lys Cys Tyr Gly Val Ser Ala Thr Lys
   1 5
<210> 8093
   <211> 9
   <212> PRT
   <213> SARS cor onavi r us
<400> 8093
   Gly Ile Tyr Gln Thr Ser Asn Phe Arg
   1 5
<210> 8094
   <211> 9
   <212> PRT
   <213> SARS coronavirus
<400> 8094
   Gln Leu Ile Arg Ala Ala Glu Ile Arg
   1 5
<210> 8095
   <211> 9
   <212> PRT
   <213> SARS cor onavi r us
<400> 8095
<210> 8096
   <211> 9
   <212> PRT
   <213> SARS cor onavi r us
<400> 8096
<210> 8097
   <211> 9
   <212> PRT
   <213> SARS cor onavi r us
<400> 8097
<210> 8098
   <211> 9
   <212> PRT
   <213> SARS cor onavi r us
<400> 8098
<210> 8099
   <211> 9
   <212> PRT
   <213> SARS coronavirus
<400> 8099
<210> 8100
   <211> 9
   <212> PRT
   <213> SARS cor onavi r us
<400> 8100
<210> 8101
   <211> 10
   <212> PRT
   <213> SARS coronavirus
<400> 8101
   Tyr Leu Lys Pro Thr Thr Phe Met Leu Lys
   1 5 10
<210> 8102
   <211> 10
   <212> PRT
   <213> SARS coronavirus
<400> 8102
   His Leu Arg Glu Phe Val Phe Lys Asn Lys
   1 5 10
<210> 8103
   <211> 10
   <212> PRT
   <213> SARS cor onavi r us
<400> 8103
   Gln Met Tyr Lys Thr Pro Thr Leu Lys Tyr
   1 5 10
<210> 8104
   <211> 10
   <212> PRT
   <213> SARS coronavirus
<400> 8104
   Ser Leu Ile Asp Leu Gln Glu Leu Gly Lys
   1 5 10
<210> 8105
   <211> 10
   <212> PRT
   <213> SARS cor onavi r us
<400> 8105
   Asn Val Tyr Ala Asp Ser Phe Val Val Lys
   1 5 10
<210> 8106
   <211> 10
   <212> PRT
   <213> SARS cor onavi r us
<400> 8106
   Trp Val Phe Gly Ser Thr Met Asn Asn Lys
   1 5 10
<210> 8107
   <211> 10
   <212> PRT
   <213> SARS cor onavi r us
<400> 8107
   Ile Leu Pro Asp Pro Leu Lys Pro Thr Lys
   1 5 10
<210> 8108
   <211> 10
   <212> PRT
   <213> SARS coronavirus
<400> 8108
   Cys Met Thr Ser Cys Cys Ser Cys Leu Lys
   1 5 10
<210> 8109
   <211> 10
   <212> PRT
   <213> SARS coronavirus
<400> 8109
   Asp Leu Pro Ser Gly Phe Asn Thr Leu Lys
   1 5 10
<210> 8110
   <211> 10
   <212> PRT
   <213> SARS coronavirus
<400> 8110
   Glu Leu Gly Lys Tyr Glu Gln Tyr Ile Lys
   1 5 10
<210> 8111
   <211> 10
   <212> PRT
   <213> SARS coronavirus
<400> 8111
   Gly Val Tyr Tyr Pro Asp Glu Ile Phe Arg
   1 5 10
<210> 8112
   <211> 10
   <212> PRT
   <213> SARS coronavirus
<400> 8112
   Tyr Leu Thr Gln Asp Leu Phe Leu Pro Phe
   1 5 10
<210> 8113
   <211> 10
   <212> PRT
   <213> SARS coronavirus
<400> 8113
   Thr Leu Thr Ser Gly Ser Asp Leu Asp Arg
   1 5 10
<210> 8114
   <211> 10
   <212> PRT
   <213> SARS coronavirus
<400> 8114
   Ser Leu Gly Ala Asp Ser Ser Ile Ala Tyr
   1 5 10
<210> 8115
   <211> 10
   <212> PRT
   <213> SARS coronavirus
<400> 8115
   His Thr Phe Gly Asn Pro Val Ile Pro Phe
   1 5 10
<210> 8116
   <211> 10
   <212> PRT
   <213> SARS coronavirus
<400> 8116
   Gly Leu Ile Ala Ile Val Met Val Thr Ile
   1 5 10
<210> 8117
   <211> 10
   <212> PRT
   <213> SARS coronavirus
<400> 8117
   Thr Leu Tyr Leu Thr Gln Asp Leu Phe Leu
   1 5 10
<210> 8118
   <211> 10
   <212> PRT
   <213> SARS cor onavi r us
<400> 8118
   Thr Leu Lys Tyr Phe Gly Gly Phe Asn Phe
   1 5 10
<210> 8119
   <211> 10
   <212> PRT
   <213> SARS cor onavi r us
<400> 8119
   Leu Leu Thr Asp Asp Met Ile Ala Ala Tyr
   1 5 10
<210> 8120
   <211> 10
   <212> PRT
   <213> SARS cor onavi r us
<400> 8120
   Thr Leu Lys Pro Ile Phe Lys Leu Pro Leu
   1 5 10
<210> 8121
   <211> 9
   <212> PRT
   <213> SARS cor onavi r us
<400> 8121
   Val Tyr Asp Pro Leu Gln Pro Glu Leu
   1 5
<210> 8122
   <211> 9
   <212> PRT
   <213> SARS coronavirus
<400> 8122
   Leu Tyr Leu Thr Gln Asp Leu Phe Leu
   1 5
<210> 8123
   <211> 9
   <212> PRT
   <213> SARS coronavirus
<400> 8123
   Glu Tyr Ile Ser Asp Ala Phe Ser Leu
   1 5
<210> 8124
   <211> 9
   <212> PRT
   <213> SARS coronavirus
<400> 8124
   Asn Tyr Asn Tyr Lys Tyr Arg Tyr Leu
   1 5
<210> 8125
   <211> 9
   <212> PRT
   <213> SARS coronavirus
<400> 8125
   Cys Tyr Gly Val Ser Ala Thr Lys Leu
   1 5
<210> 8126
   <211> 9
   <212> PRT
   <213> SARS coronavirus
<400> 8126
   Gln Tyr Gly Ser Phe Cys Thr Gln Leu
   1 5
<210> 8127
   <211> 9
   <212> PRT
   <213> SARS coronavirus
<400> 8127
   Ile Tyr Ser Thr Gly Asn Asn Val Phe
   1 5
<210> 8128
   <211> 9
   <212> PRT
   <213> SARS coronavirus
<400> 8128
   Ala Tyr Phe Pro Arg Glu Gly Val Phe
   1 5
<210> 8129
   <211> 9
   <212> PRT
   <213> SARS coronavi rus
<400> 8129
   Gln Tyr Gly Glu Cys Leu Gly Asp Ile
   1 5
<210> 8130
   <211> 9
   <212> PRT
   <213> SARS coronavirus
<400> 8130
   Val Tyr Ala Trp Glu Arg Lys Lys Ile
   1 5
<210> 8131
   <211> 9
   <212> PRT
   <213> SARS coronavirus
<400> 8131
   Ala Tyr Ser Asn Asn Thr Ile Ala Ile
   1 5
<210> 8132
   <211> 9
   <212> PRT
   <213> SARS coronavirus
<400> 8132
   Gly Tyr Leu Lys Pro Thr Thr Phe Met
   1 5
<210> 8133
   <211> 9
   <212> PRT
   <213> SARS coronavirus
<400> 8133
   Val Phe Gln Thr Gln Ala Gly Cys Leu
   1 5
<210> 8134
   <211> 9
   <212> PRT
   <213> SARS cor onavi r us
<400> 8134
   Asn Phe Gly Ala Ile Ser Ser Val Leu
   1 5
<210> 8135
   <211> 9
   <212> PRT
   <213> SARS coronavi rus
<400> 8135
   Asn Tyr Thr Gln His Thr Ser Ser Met
   1 5
<210> 8136
   <211> 9
   <212> PRT
   <213> SARS coronavi rus
<400> 8136
   Asn Tyr Lys Leu Pro Asp Asp Phe Met
   1 5
<210> 8137
   <211> 9
   <212> PRT
   <213> SARS coronavi rus
<400> 8137
   Thr Phe Glu Tyr Ile Ser Asp Ala Phe
   1 5
<210> 8138
   <211> 9
   <212> PRT
   <213> SARS coronavirus
<400> 8138
   Arg Leu Asn Glu Val Ala Lys Asn Leu
   1 5
<210> 8139
   <211> 9
   <212> PRT
   <213> SARS coronavi rus
<400> 8139
   Ile Phe Arg Ser Asp Thr Leu Tyr Leu
   1 5
<210> 8140
   <211> 9
   <212> PRT
   <213> SARS coronavi rus
<400> 8140
   Val Phe Lys Asn Lys Asp Gly Phe Leu
   1 5
<210> 8141
   <211> 10
   <212> PRT
   <213> SARS coronavirus
<400> 8141
   Lys Tyr Arg Tyr Leu Arg His Gly Lys Leu
   1 5 10
<210> 8142
   <211> 10
   <212> PRT
   <213> SARS coronavirus
<400> 8142
   Gly Tyr Gln Pro Tyr Arg Val Val Val Leu
   1 5 10
<210> 8143
   <211> 10
   <212> PRT
   <213> SARS coronavirus
<400> 8143
   Gly Tyr Leu Lys Pro Thr Thr Phe Met Leu
   1 5 10
<210> 8144
   <211> 10
   <212> PRT
   <213> SARS coronavi rus
<400> 8144
   Leu Tyr Asn Ser Thr Phe Phe Ser Thr Phe
   1 5 10
<210> 8145
   <211> 10
   <212> PRT
   <213> SARS cor onavi r us
<400> 8145
   Asp Tyr Asn Tyr Lys Leu Pro Asp Asp Phe
   1 5 10
<210> 8146
   <211> 10
   <212> PRT
   <213> SARS cor onavi r us
<400> 8146
   Thr Tyr Val Pro Ser Gln Glu Arg Asn Phe
   1 5 10
<210> 8147
   <211> 10
   <212> PRT
   <213> SARS coronavirus
<400> 8147
   Cys Tyr Trp Pro Leu Asn Asp Tyr Gly Phe
   1 5 10
<210> 8148
   <211> 10
   <212> PRT
   <213> SARS coronavirus
<400> 8148
   Asp Tyr Ser Val Leu Tyr Asn Ser Thr Phe
   1 5 10
<210> 8149
   <211> 10
   <212> PRT
   <213> SARS coronavirus
<400> 8149
   Met Tyr Lys Thr Pro Thr Leu Lys Tyr Phe
   1 5 10
<210> 8150
   <211> 10
   <212> PRT
   <213> SARS coronavirus
<400> 8150
   Leu Tyr Val Tyr Lys Gly Tyr Gln Pro Ile
   1 5 10
<210> 8151
   <211> 10
   <212> PRT
   <213> SARS cor onavi r us
<400> 8151
   Asp Tyr Gly Phe Tyr Thr Thr Thr Gly Ile
   1 5 10
<210> 8152
   <211> 10
   <212> PRT
   <213> SARS coronavirus
<400> 8152
   Met Phe Ile Phe Leu Leu Phe Leu Thr Leu
   1 5 10
<210> 8153
   <211> 10
   <212> PRT
   <213> SARS coronavirus
<400> 8153
   Gly Phe Met Lys Gln Tyr Gly Glu Cys Leu
   1 5 10
<210> 8154
   <211> 10
   <212> PRT
   <213> SARS coronavirus
<400> 8154
   Ser Phe Cys Thr Gln Leu Asn Arg Ala Leu
   1 5 10
<210> 8155
   <211> 10
   <212> PRT
   <213> SARS cor onavi r us
<400> 8155
   Asn Phe Ser Gln Ile Leu Pro Asp Pro Leu
   1 5 10
<210> 8156
   <211> 10
   <212> PRT
   <213> SARS coronavirus
<400> 8156
   Lys Tyr Glu Gln Tyr Ile Lys Trp Pro Trp
   1 5 10
<210> 8157
   <211> 10
   <212> PRT
   <213> SARS coronavirus
<400> 8157
   Lys Ala Ile Ser Gln Ile Gln Glu Ser Leu
   1 5 10
<210> 8158
   <211> 10
   <212> PRT
   <213> SARS coronavirus
<400> 8158
   Asn Phe Glu Leu Cys Asp Asn Pro Phe Phe
   1 5 10
<210> 8159
   <211> 10
   <212> PRT
   <213> SARS coronavirus
<400> 8159
   Glu Phe Val Phe Lys Asn Lys Asp Gly Phe
   1 5 10
<210> 8160
   <211> 10
   <212> PRT
   <213> SARS coronavirus
<400> 8160
   Tyr Phe Pro Arg Glu Gly Val Phe Val Phe
   1 5 10
<210> 8161
   <211> 9
   <212> PRT
   <213> SARS coronavirus
<400> 8161
   Val Val Phe Leu His Val Thr Tyr Val
   1 5
<210> 8162
   <211> 9
   <212> PRT
   <213> SARS coronavirus
<400> 8162
   Arg Leu Gln Ser Leu Gln Thr Tyr Val
   1 5
<210> 8163
   <211> 9
   <212> PRT
   <213> SARS cor onavi r us
<400> 8163
   Val Leu Asn Asp Ile Leu Ser Arg Leu
   1 5
<210> 8164
   <211> 9
   <212> PRT
   <213> SARS cor onavi r us
<400> 8164
   Val Leu Tyr Glu Asn Gln Lys Gln Ile
   1 5
<210> 8165
   <211> 9
   <212> PRT
   <213> SARS coronavirus
<400> 8165
   Asn Leu Asn Glu Ser Leu Ile Asp Leu
   1 5
<210> 8166
   <211> 9
   <212> PRT
   <213> SARS coronavirus
<400> 8166
   Leu Leu Leu Gln Tyr Gly Ser Phe Cys
   1 5
<210> 8167
   <211> 9
   <212> PRT
   <213> SARS coronavi rus
<400> 8167
   Lys Leu Pro Asp Asp Phe Met Gly Cys
   1 5
<210> 8168
   <211> 9
   <212> PRT
   <213> SARS coronavirus
<400> 8168
   Ile Leu Pro Asp Pro Leu Lys Pro Thr
   1 5
<210> 8169
   <211> 9
   <212> PRT
   <213> SARS coronavirus
<400> 8169
   Met Ile Phe Asp Asn Ala Phe Asn Cys
   1 5
<210> 8170
   <211> 9
   <212> PRT
   <213> SARS coronavirus
<400> 8170
   Phe Ile Phe Leu Leu Phe Leu Thr Leu
   1 5
<210> 8171
   <211> 9
   <212> PRT
   <213> SARS coronavi rus
<400> 8171
   Val Leu Tyr Gln Asp Val Asn Cys Thr
   1 5
<210> 8172
   <211> 9
   <212> PRT
   <213> SARS coronavirus
<400> 8172
   Ile Ile Thr Thr Asp Asn Thr Phe Val
   1 5
<210> 8173
   <211> 9
   <212> PRT
   <213> SARS cor onavi r us
<400> 8173
   Arg Leu Asn Glu Val Ala Lys Asn Leu
   1 5
<210> 8174
   <211> 9
   <212> PRT
   <213> SARS coronavi rus
<400> 8174
   Leu Leu Thr Asp Asp Met Ile Ala Ala
   1 5
<210> 8175
   <211> 9
   <212> PRT
   <213> SARS coronavi rus
<400> 8175
   Leu Leu Gln Tyr Gly Ser Phe Cys Thr
   1 5
<210> 8176
   <211> 9
   <212> PRT
   <213> SARS coronavirus
<400> 8176
   Leu Leu Phe Asn Lys Val Thr Leu Ala
   1 5
<210> 8177
   <211> 9
   <212> PRT
   <213> SARS coronavirus
<400> 8177
   Ile Leu Leu Cys Cys Met Thr Ser Cys
   1 5
<210> 8178
   <211> 9
   <212> PRT
   <213> SARS coronavirus
<400> 8178
   Thr Met Asn Asn Lys Ser Gln Ser Val
   1 5
<210> 8179
   <211> 9
   <212> PRT
   <213> SARS cor onavi r us
<400> 8179
   Ala Leu Asn Thr Leu Val Lys Gln Leu
   1 5
<210> 8180
   <211> 9
   <212> PRT
   <213> SARS coronavi rus
<400> 8180
   Glu Leu Cys Asp Asn Pro Phe Phe Ala
   1 5
<210> 8181
   <211> 10
   <212> PRT
   <213> SARS coronavirus
<400> 8181
   Lys Leu Asn Asp Leu Cys Phe Ser Asn Val
   1 5 10
<210> 8182
   <211> 10
   <212> PRT
   <213> SARS coronavi rus
<400> 8182
   Lys Leu Pro Asp Asp Phe Met Gly Cys Val
   1 5 10
<210> 8183
   <211> 10
   <212> PRT
   <213> SARS coronavirus
<400> 8183
   Tyr Ile Ser Asp Ala Phe Ser Leu Asp Val
   1 5 10
<210> 8184
   <211> 10
   <212> PRT
   <213> SARS coronavirus
<400> 8184
   Phe Val Phe Asn Gly Thr Ser Trp Phe Ile
   1 5 10
<210> 8185
   <211> 10
   <212> PRT
   <213> SARS coronavirus
<400> 8185
   Tyr Val Trp Leu Gly Phe Ile Ala Gly Leu
   1 5 10
<210> 8186
   <211> 10
   <212> PRT
   <213> SARS coronavirus
<400> 8186
   Val Leu Tyr Asn Ser Thr Phe Phe Ser Thr
   1 5 10
<210> 8187
   <211> 10
   <212> PRT
   <213> SARS coronavirus
<400> 8187
   Thr Leu Tyr Leu Thr Gln Asp Leu Phe Leu
   1 5 10
<210> 8188
   <211> 10
   <212> PRT
   <213> SARS coronavirus
<400> 8188
   Leu Leu Leu Gln Tyr Gly Ser Phe Cys Thr
   1 5 10
<210> 8189
   <211> 10
   <212> PRT
   <213> SARS coronavirus
<400> 8189
   Glu Leu Cys Asp Asn Pro Phe Phe Ala Val
   1 5 10
<210> 8190
   <211> 10
   <212> PRT
   <213> SARS coronavirus
<400> 8190
   Phe Met Gly Cys Val Leu Ala Trp Asn Thr
   1 5 10
<210> 8191
   <211> 10
   <212> PRT
   <213> SARS cor onavi r us
<400> 8191
   Gly Ile Tyr Gln Thr Ser Asn Phe Arg Val
   1 5 10
<210> 8192
   <211> 10
   <212> PRT
   <213> SARS coronavi rus
<400> 8192
   Phe Ile Phe Leu Leu Phe Leu Thr Leu Thr
   1 5 10
<210> 8193
   <211> 10
   <212> PRT
   <213> SARS coronavirus
<400> 8193
   Asp Leu Phe Leu Pro Phe Tyr Ser Asn Val
   1 5 10
<210> 8194
   <211> 10
   <212> PRT
   <213> SARS coronavirus
<400> 8194
   Asn Leu Leu Leu Gln Tyr Gly Ser Phe Cys
   1 5 10
<210> 8195
   <211> 10
   <212> PRT
   <213> SARS coronavirus
<400> 8195
   Phe Leu Thr Leu Thr Ser Gly Ser Asp Leu
   1 5 10
<210> 8196
   <211> 10
   <212> PRT
   <213> SARS coronavirus
<400> 8196
   Phe Met Leu Lys Tyr Asp Glu Asn Gly Thr
   1 5 10
<210> 8197
   <211> 10
   <212> PRT
   <213> SARS coronavirus
<400> 8197
   Gly Val Val Phe Leu His Val Thr Tyr Val
   1 5 10
<210> 8198
   <211> 10
   <212> PRT
   <213> SARS cor onavi r us
<400> 8198
   Lys Leu Gln Asp Val Val Asn Gln Asn Ala
   1               5                   10
<210> 8199
   <211> 10
   <212> PRT
   <213> SARS coronavirus
<400> 8199
   Glu Gln Tyr Ile Lys Trp Pro Trp Tyr Val
   1               5                   10
<210> 8200
   <211> 10
   <212> PRT
   <213> SARS cor onavi r us
<400> 8200
   Gln Ile Ile Thr Thr Asp Asn Thr Phe Val
   1               5                   10
<210> 8201
   <211> 9
   <212> PRT
   <213> SARS cor onavi r us
<400> 8201
   Gly Val Ile Ala Asp Tyr Asn Tyr Lys
   1               5
<210> 8202
   <211> 9
   <212> PRT
   <213> SARS cor onavi r us
<400> 8202
   Lys Gln Ile Ala Asn Gln Phe Asn Lys
   1               5
<210> 8203
   <211> 9
   <212> PRT
   <213> SARS coronavirus
<400> 8203
   Ser Val Lys Ser Phe Glu Ile Asp Lys
   1               5
<210> 8204
   <211> 9
   <212> PRT
   <213> SARS coronavirus
<400> 8204
   Gly Ile Tyr Phe Ala Ala Thr Glu Lys
   1 5
<210> 8205
   <211> 9
   <212> PRT
   <213> SARS coronavirus
<400> 8205
   Glu Val Met Pro Val Ser Met Ala Lys
   1 5
<210> 8206
   <211> 9
   <212> PRT
   <213> SARS cor onavi r us
<400> 8206
   Asn Val Pro Phe Ser Pro Asp Gly Lys
   1 5
<210> 8207
   <211> 9
   <212> PRT
   <213> SARS coronavirus
<400> 8207
   Thr Thr Thr Ser Thr Ala Leu Gly Lys
   1 5
<210> 8208
   <211> 9
   <212> PRT
   <213> SARS coronavirus
<400> 8208
   Gly Val Leu Thr Pro Ser Ser Lys Arg
   1 5
<210> 8209
   <211> 9
   <212> PRT
   <213> SARS coronavirus
<400> 8209
   Lys Cys Tyr Gly Val Ser Ala Thr Lys
   1 5
<210> 8210
   <211> 9
   <212> PRT
   <213> SARS coronavirus
<400> 8210
   Lys Tyr Arg Tyr Leu Arg His Gly Lys
   1 5
<210> 8211
   <211> 9
   <212> PRT
   <213> SARS coronavirus
<400> 8211
   Phe Ile Glu Asp Leu Leu Phe Asn Lys
   1 5
<210> 8212
   <211> 9
   <212> PRT
   <213> SARS coronavirus
<400> 8212
   Thr Leu Ala Asp Ala Gly Phe Met Lys
   1 5
<210> 8213
   <211> 9
   <212> PRT
   <213> SARS coronavirus
<400> 8213
   Ala Gln Ala Leu Asn Thr Leu Val Lys
   1 5
<210> 8214
   <211> 9
   <212> PRT
   <213> SARS cor onavi r us
<400> 8214
   Ser Val Leu Asn Asp Ile Leu Ser Arg
   1 5
<210> 8215
   <211> 9
   <212> PRT
   <213> SARS coronavirus
<400> 8215
   Phe Thr Asp Ser Val Arg Asp Pro Lys
   1 5
<210> 8216
   <211> 9
   <212> PRT
   <213> SARS coronavirus
<400> 8216
   Met Thr Ser Cys Cys Ser Cys Leu Lys
   1 5
<210> 8217
   <211> 9
   <212> PRT
   <213> SARS coronavirus
<400> 8217
   Ser Gln Ile Leu Pro Asp Pro Leu Lys
   1 5
<210> 8218
   <211> 9
   <212> PRT
   <213> SARS coronavirus
<400> 8218
   Ala Ala Tyr Phe Val Gly Tyr Leu Lys
   1 5
<210> 8219
   <211> 9
   <212> PRT
   <213> SARS coronavirus
<400> 8219
   Gln Met Tyr Lys Thr Pro Thr Leu Lys
   1 5
<210> 8220
   <211> 9
   <212> PRT
   <213> SARS coronavirus
<400> 8220
   Leu Ile Asp Leu Gln Glu Leu Gly Lys
   1 5
<210> 8221
   <211> 10
   <212> PRT
   <213> SARS coronavi rus
<400> 8221
   Gly Val Tyr Tyr Pro Asp Glu Ile Phe Arg
   1 5 10
<210> 8222
   <211> 10
   <212> PRT
   <213> SARS coronavirus
<400> 8222
   Val Thr Leu Ala Asp Ala Gly Phe Met Lys
   1 5 10
<210> 8223
   <211> 10
   <212> PRT
   <213> SARS coronavirus
<400> 8223
   Trp Val Phe Gly Ser Thr Met Asn Asn Lys
   1 5 10
<210> 8224
   <211> 10
   <212> PRT
   <213> SARS cor onavi r us
<400> 8224
   Asn Val Tyr Ala Asp Ser Phe Val Val Lys
   1 5 10
<210> 8225
   <211> 10
   <212> PRT
   <213> SARS coronavirus
<400> 8225
   Lys Gln Met Tyr Lys Thr Pro Thr Leu Lys
   1 5 10
<210> 8226
   <211> 10
   <212> PRT
   <213> SARS coronavirus
<400> 8226
   Gly Thr Gly Val Leu Thr Pro Ser Ser Lys
   1 5 10
<210> 8227
   <211> 10
   <212> PRT
   <213> SARS coronavirus
<400> 8227
   Lys Val Glu Ala Glu Val Gln Ile Asp Arg
   1 5 10
<210> 8228
   <211> 10
   <212> PRT
   <213> SARS coronavirus
<400> 8228
   Leu Thr Thr Thr Ser Thr Ala Leu Gly Lys
   1 5 10
<210> 8229
   <211> 10
   <212> PRT
   <213> SARS coronavirus
<400> 8229
   Arg Val Val Pro Ser Gly Asp Val Val Arg
   1 5 10
<210> 8230
   <211> 10
   <212> PRT
   <213> SARS coronavirus
<400> 8230
   Gly Phe Asn Thr Leu Lys Pro Ile Phe Lys
   1 5 10
<210> 8231
   <211> 10
   <212> PRT
   <213> SARS coronavirus
<400> 8231
   Gly Pro Lys Leu Ser Thr Asp Leu Ile Lys
   1 5 10
<210> 8232
   <211> 10
   <212> PRT
   <213> SARS coronavirus
<400> 8232
   Ser Leu Ile Asp Leu Gln Glu Leu Gly Lys
   1 5 10
<210> 8233
   <211> 10
   <212> PRT
   <213> SARS coronavirus
<400> 8233
   Ala Thr Ser Thr Gly Asn Tyr Asn Tyr Lys
   1 5 10
<210> 8234
   <211> 10
   <212> PRT
   <213> SARS coronavirus
<400> 8234
   Asn Thr Arg Glu Val Phe Ala Gln Val Lys
   1 5 10
<210> 8235
   <211> 10
   <212> PRT
   <213> SARS coronavirus
<400> 8235
   Ser Phe Ile Glu Asp Leu Leu Phe Asn Lys
   1 5 10
<210> 8236
   <211> 10
   <212> PRT
   <213> SARS coronavirus
<400> 8236
   Tyr Leu Lys Pro Thr Thr Phe Met Leu Lys
   1 5 10
<210> 8237
   <211> 10
   <212> PRT
   <213> SARS cor onavi r us
<400> 8237
   Gln Thr Tyr Val Thr Gln Gln Leu Ile Arg
   1 5 10
<210> 8238
   <211> 10
   <212> PRT
   <213> SARS cor onavi r us
<400> 8238
   Val Val Asn Ile Gln Lys Glu Ile Asp Arg
   1 5 10
<210> 8239
   <211> 10
   <212> PRT
   <213> SARS coronavirus
<400> 8239
   Asp Val Ser Glu Lys Ser Gly Asn Phe Lys
   1 5 10
<210> 8240
   <211> 10
   <212> PRT
   <213> SARS coronavirus
<400> 8240
   Thr Gln Asn Val Leu Tyr Glu Asn Gln Lys
   1 5 10
<210> 8241
   <211> 9
   <212> PRT
   <213> SARS coronavirus
<400> 8241
   Gln Pro Ile Asp Val Val Arg Asp Leu
   1 5
<210> 8242
   <211> 9
   <212> PRT
   <213> SARS coronavirus
<400> 8242
   Glu Pro Val Leu Lys Gly Val Lys Leu
   1 5
<210> 8243
   <211> 9
   <212> PRT
   <213> SARS coronavirus
<400> 8243
   Val Ile Arg Ala Cys Asn Phe Glu Leu
   1 5
<210> 8244
   <211> 9
   <212> PRT
   <213> SARS coronavirus
<400> 8244
   Ala Ala Ala Tyr Phe Val Gly Tyr Leu
   1 5
<210> 8245
   <211> 9
   <212> PRT
   <213> SARS coronavirus
<400> 8245
   Ala Ala Pro His Gly Val Val Phe Leu
   1 5
<210> 8246
   <211> 9
   <212> PRT
   <213> SARS coronavirus
<400> 8246
   Arg Val Val Val Leu Ser Phe Glu Leu
   1 5
<210> 8247
   <211> 9
   <212> PRT
   <213> SARS coronavirus
<400> 8247
   Val Val Val Leu Ser Phe Glu Leu Leu
   1 5
<210> 8248
   <211> 9
   <212> PRT
   <213> SARS coronavirus
<400> 8248
   Cys Val Asn Phe Asn Phe Asn Gly Leu
   1 5
<210> 8249
   <211> 9
   <212> PRT
   <213> SARS coronavirus
<400> 8249
   Leu Pro Pro Leu Leu Thr Asp Asp Met
   1 5
<210> 8250
   <211> 9
   <212> PRT
   <213> SARS coronavirus
<400> 8250
   Val Val Asn Gln Asn Ala Gln Ala Leu
   1 5
<210> 8251
   <211> 9
   <212> PRT
   <213> SARS coronavirus
<400> 8251
   Ala Val Asp Cys Ser Gln Asn Pro Leu
   1 5
<210> 8252
   <211> 9
   <212> PRT
   <213> SARS coronavirus
<400> 8252
   Asn Ala Ser Ser Glu Val Ala Val Leu
   1 5
<210> 8253
   <211> 9
   <212> PRT
   <213> SARS coronavirus
<400> 8253
   Cys Ala Ser Tyr His Thr Val Ser Leu
   1 5
<210> 8254
   <211> 9
   <212> PRT
   <213> SARS cor onavi r us
<400> 8254
   Ala Ser Tyr His Thr Val Ser Leu Leu
   1 5
<210> 8255
   <211> 9
   <212> PRT
   <213> SARS coronavirus
<400> 8255
   Lys Gln Met Tyr Lys Thr Pro Thr Leu
   1 5
<210> 8256
   <211> 9
   <212> PRT
   <213> SARS coronavirus
<400> 8256
   Ala Ile Ser Gln Ile Gln Glu Ser Leu
   1 5
<210> 8257
   <211> 9
   <212> PRT
   <213> SARS coronavi rus
<400> 8257
   Ala Leu Asn Thr Leu Val Lys Gln Leu
   1 5
<210> 8258
   <211> 9
   <212> PRT
   <213> SARS coronavirus
<400> 8258
   Ala Thr Lys Met Ser Glu Cys Val Leu
   1 5
<210> 8259
   <211> 9
   <212> PRT
   <213> SARS coronavirus
<400> 8259
   Val Ala Lys Asn Leu Asn Glu Ser Leu
   1 5
<210> 8260
   <211> 9
   <212> PRT
   <213> SARS coronavirus
<400> 8260
   Ile Ala Ile Val Met Val Thr Ile Leu
   1 5
<210> 8261
   <211> 10
   <212> PRT
   <213> SARS coronavirus
<400> 8261
   Ala Pro Ala Thr Val Cys Gly Pro Lys Leu
   1 5 10
<210> 8262
   <211> 10
   <212> PRT
   <213> SARS coronavirus
<400> 8262
   Val Val Arg Phe Pro Asn Ile Thr Asn Leu
   1 5 10
<210> 8263
   <211> 10
   <212> PRT
   <213> SARS coronavirus
<400> 8263
   Lys Pro Met Gly Thr Gln Thr His Thr Met
   1 5 10
<210> 8264
   <211> 10
   <212> PRT
   <213> SARS coronavirus
<400> 8264
   Ala Ala Thr Lys Met Ser Glu Cys Val Leu
   1 5 10
<210> 8265
   <211> 10
   <212> PRT
   <213> SARS coronavirus
<400> 8265
   Leu Pro Asp Asp Phe Met Gly Cys Val Leu
   1 5 10
<210> 8266
   <211> 10
   <212> PRT
   <213> SARS coronavirus
<400> 8266
   Val Val Ile Arg Ala Cys Asn Phe Glu Leu
   1 5 10
<210> 8267
   <211> 10
   <212> PRT
   <213> SARS coronavirus
<400> 8267
   Asn Pro Phe Phe Ala Val Ser Lys Pro Met
   1 5 10
<210> 8268
   <211> 10
   <212> PRT
   <213> SARS coronavirus
<400> 8268
   Phe Val Phe Lys Asn Lys Asp Gly Phe Leu
   1 5 10
<210> 8269
   <211> 10
   <212> PRT
   <213> SARS coronavirus
<400> 8269
   Gly Val Ser Ala Thr Lys Leu Asn Asp Leu
   1 5 10
<210> 8270
   <211> 10
   <212> PRT
   <213> SARS coronavirus
<400> 8270
   Gly Val Ile Ala Asp Tyr Asn Tyr Lys Leu
   1 5 10
<210> 8271
   <211> 10
   <212> PRT
   <213> SARS coronavirus
<400> 8271
   Arg Val Val Val Leu Ser Phe Glu Leu Leu
   1 5 10
<210> 8272
   <211> 10
   <212> PRT
   <213> SARS coronavirus
<400> 8272
   Ser Val Arg Asp Pro Lys Thr Ser Glu Ile
   1 5 10
<210> 8273
   <211> 10
   <212> PRT
   <213> SARS coronavirus
<400> 8273
   Asn Val Phe Gln Thr Gln Ala Gly Cys Leu
   1 5 10
<210> 8274
   <211> 10
   <212> PRT
   <213> SARS coronavirus
<400> 8274
   Asp Val Val Asn Gln Asn Ala Gln Ala Leu
   1 5 10
<210> 8275
   <211> 10
   <212> PRT
   <213> SARS coronavirus
<400> 8275
   Ser Val Leu Asn Asp Ile Leu Ser Arg Leu
   1 5 10
<210> 8276
   <211> 10
   <212> PRT
   <213> SARS coronavi rus
<400> 8276
   Thr Val Tyr Asp Pro Leu Gln Pro Glu Leu
   1 5 10
<210> 8277
   <211> 10
   <212> PRT
   <213> SARS coronavirus
<400> 8277
   Glu Val Ala Lys Asn Leu Asn Glu Ser Leu
   1 5 10
<210> 8278
   <211> 10
   <212> PRT
   <213> SARS coronavi rus
<400> 8278
   Tyr Val Trp Leu Gly Phe Ile Ala Gly Leu
   1 5 10
<210> 8279
   <211> 10
   <212> PRT
   <213> SARS coronavirus
<400> 8279
   Ser Ala Ala Ala Tyr Phe Val Gly Tyr Leu
   1 5 10
<210> 8280
   <211> 10
   <212> PRT
   <213> SARS coronavirus
<400> 8280
   Asp Ala Val Asp Cys Ser Gln Asn Pro Leu
   1 5 10
<210> 9785
   <211> 42
   <212> DNA
   <213> Artificial Sequence
<220>
   <223> Primer
<400> 9785
   agatcttcta gataaaagaa gtgaccttga ccggt gcacc ac 42
<210> 9786
   <211> 50
   <212> DNA
   <213> Artificial Sequence
<220>
   <223> Primer
<400> 9786
   agatctccta ggcattcgcc atattgcttc atgaagccag catcagcgag 50
<210> 9787
   <211> 49
   <212> DNA
   <213> Artificial Sequence
<220>
   <223> Primer
<400> 9787
   agat ct cct a ggtgatatta atgctaggga cctcatttgt gcgcagaag 49
<210> 9788
   <211> 45
   <212> DNA
   <213> Artificial Sequence
<220>
   <223> Primer
<400> 9788
   agatctgtcg act cat t at g tgtaatgtaa tttgacaccc t t gag 45
<210> 9789
   <211> 44
   <212> DNA
   <213> Artificial Sequence
<220>
   <223> Oligonucleotide
<400> 9789
   tgagcatgtt gatacttctt t at gagt gcga cat t cct at t ggag 44
<210> 9790
   <211> 39
   <212> DNA
   <213> Artificial Sequence
<220>
   <223> Oligonucleotide
<400> 9790
   ctggcatttg tgctagttac catacagttt ctttattac 39
<210> 9791
   <211> 58
   <212> DNA
   <213> Artificial Sequence
<220>
   <223> Oligonucleotide
<400> 9791
   gt agt act ag ccaaaaat ct attgtggctt atactatgtc tttaggtgct gat agt t c 58
<210> 9792
   <211> 52
   <212> DNA
   <213> Artificial Sequence
<220>
   <223> Oligonucleotide
<400> 9792
   aattgaacta tcagcaccta aagacat agt at aagccaca atagattttt gg 52
<210> 9793
   <211> 37
   <212> DNA
   <213> Artificial Sequence
<220>
   <223> Oligonucleotide
<400> 9793
   ct agt act ac gt aat aaaga aact gt at gg t aact ag 37
<210> 9794
   <211> 53
   <212> DNA
   <213> Artificial Sequence
<220>
   <223> Oligonucleotide
<400> 9794
   cacaaat gcc agctccaata ggaatgtcgc act cat aaga agt at caaca t gc 53
<210> 9795
   <211> 29
   <212> DNA
   <213> Artificial Sequence
<220>
   <223> Oligonucleotide
<400> 9795
   agcttacaaa acaaaat gag t gacct t ga 29
<210> 9796
   <211> 29
   <212> DNA
   <213> Artificial Sequence
<220>
   <223> Oligonucleotide
<400> 9796
   ccggt caagg t cact cat t t t gt t t t g t a 29
<210> 9797
   <211> 68
   <212> DNA
   <213> Artificial Sequence
<220>
   <223> Oligonucleotide
<400> 9797
   aaat gaat ca ct cat t gacc t t caagaat t gggaaaat at gagcaat at a t t aaat ggcc 60
   t t aat gag 68
<210> 9798
   <211> 72
   <212> DNA
   <213> Artificial Sequence
<220>
   <223> Oligonucleotide
<400> 9798
   t cgact cat t aaggccat t t aat at at t gc t cat at t t t c ccaat t ct t g aaggt caat g 60
   agt gat t cat t t 72
<210> 9921
   <211> 8098
   <212> DNA
   <213> Artificial Sequence
<220>
   <223> pCMVKm2 vect or
<400> 9921
   gccgcggaat ttcgactcta ggccattgca tacgttgtat ctatatcata atatgtacat 60
   ttatattggc tcatgtccaa tatgaccgcc atgttgacat tgattattga ctagttatta 120
   atagtaatca attacggggt cattagttca tagcccatat atggagttcc gcgttacata 180
   acttacggta aatggcccgc ctggctgacc gcccaacgac ccccgcccat tgacgtcaat 240
   aatgacgtat gttcccatag taacgccaat agggactttc cattgacgtc aatgggtgga 300
   gtatttacgg taaactgccc acttggcagt acatcaagtg tatcatatgc caagtccgcc 360
   ccctattgac gtcaatgacg gtaaatggcc cgcctggcat tatgcccagt acatgacctt 420
   acgggacttt cctacttggc agtacatcta cgtattagtc atcgctatta ccatggtgat 480
   gcggttttgg cagtacacca atgggcgtgg atagcggttt gactcacggg gatttccaag 540
   tctccacccc attgacgtca atgggagttt gttttggcac caaaatcaac gggactttcc 600
   aaaatgtcgt aataaccccg ccccgttgac gcaaatgggc ggtaggcgtg tacggtggga 660
   ggtctatata agcagagctc gtttagtgaa ccgtcagatc gcctggagac gccatccacg 720
   ctgttttgac ctccatagaa gacaccggga ccgatccagc ctccgcggcc gggaacggtg 780
   cattggaacg cggattcccc gtgccaagag tgacgtaagt accgcctata gactctatag 840
   gcacacccct ttggctctta tgcatgctat actgtttttg gcttggggcc tatacacccc 900
   cgcttcctta tgctataggt gatggtatag cttagcctat aggtgtgggt tattgaccat 960
   tattgaccac tcccctattg gtgacgatac tttccattac taatccataa catggctctt 1020
   tgccacaact atctctattg gctatatgcc aatactctgt ccttcagaga ctgacacgga 1080
   ctctgtattt ttacaggatg gggtcccatt tattatttac aaattcacat atacaacaac 1140
   gccgtccccc gtgcccgcag tttttattaa acatagcgtg ggatctccac gcgaatctcg 1200
   ggtacgtgtt ccggacatgg gctcttctcc ggtagcggcg gagcttccac atccgagccc 1260
   tggtcccatg cctccagcgg ctcatggtcg ctcggcagct ccttgctcct aacagtggag 1320
   gccagactta ggcacagcac aatgcccacc accaccagtg tgccgcacaa ggccgtggcg 1380
   gtagggtatg tgtctgaaaa tgagctcgga gattgggctc gcaccgctga cgcagatgga 1440
   agacttaagg cagcggcaga agaagatgca ggcagctgag ttgttgtatt ctgataagag 1500
   tcagaggtaa ctcccgttgc ggtgctgtta acggtggagg gcagtgtagt ctgagcagta 1560
   ctcgttgctg ccgcgcgcgc caccagacat aatagctgac agactaacag actgttcctt 1620
   tccatgggtc ttttctgcag tcaccgtcgt cgacctaaga attcgccacc atgttcatct 1680
   tcctgctgtt cctgactctc actagtggta gtgaccttga ccggtgcacc acttttgatg 1740
   atgttcaagc tcctaattac actcaacata cttcatctat gaggggggtt tactatcctg 1800
   atgaaatttt tagatcagac actctttatt taactcagga tttatttctt ccattttatt 1860
   ctaatgttac agggtttcat actattaatc atacgtttgg caaccctgtc atacctttta 1920
   aggatggtat ttattttgct gccacagaga aatcaaatgt tgtccgtggt tgggtttttg 1980
   gttctaccat gaacaacaag tcacagtcgg tgattattat taacaattct actaatgttg 2040
   ttatacgagc atgtaacttt gaattgtgtg acaacccttt ctttgctgtt tctaaaccca 2100
   tgggtacaca gacacatact atgatattcg ataatgcatt taattgcact ttcgagtaca 2160
   tatctgatgc cttttcgctt gatgtttcag aaaagtcagg taattttaaa cacttacgag 2220
   agtttgtgtt taaaaataaa gatgggtttc tctatgttta taagggctat caacctatag 2280
   atgtagttcg tgatctacct tctggtttta acactttgaa acctattttt aagttgcctc 2340
   ttggtattaa cattacaaat tttagagcca ttcttacagc cttttcacct gctcaagaca 2400
   tttggggcac gtcagctgca gcctattttg ttggctattt aaagccaact acatttatgc 2460
   tcaagtatga tgaaaatggt acaatcacag atgctgttga ttgttctcaa aatccacttg 2520
   ctgaactcaa atgctctgtt aagagctttg agattgacaa aggaatttac cagacctcta 2580
   atttcagggt tgttccctca ggagatgttg tgagattccc taatattaca aacttgtgtc 2640
   cttttggaga ggtttttaat gctactaaat tcccttctgt ctatgcatgg gagagaaaaa 2700
   aaatttctaa ttgtgttgct gattactctg tgctctacaa ctcaacattt ttttcaacct 2760
   ttaagtgcta tggcgtttct gccactaagt tgaatgatct ttgcttctcc aatgtctatg 2820
   cagattcttt tgtagtcaag ggagatgatg taagacaaat agcgccaggg caaactggtg 2880
   ttattgctga ttataattat aaattgccag atgatttcat gggttgtgtc cttgcttgga 2940
   atactaggaa cattgatgct acttcaactg gtaattataa ttataaatat aggtatctta 3000
   gacatggcaa gcttaggccc tttgagagag acatatctaa tgtgcctttc tcccctgatg 3060
   gcaaaccttg caccccacct gctcttaatt gttattggcc attaaatgat tatggttttt 3120
   acaccactac tggcattggc taccaacctt acagagttgt agtactttct tttgaacttt 3180
   taaatgcacc ggccacggtt tgtggaccaa aattatccac tgaccttatt aagaaccagt 3240
   gtgtcaattt taattttaat ggactcactg gtactggtgt gttaactcct tcttcaaaga 3300
   gatttcaacc atttcaacaa tttggccgtg atgtttctga tttcactgat tccgttcgag 3360
   atcctaaaac atctgaaata ttagacattt caccttgctc ttttgggggt gtaagtgtaa 3420
   ttacacctgg aacaaatgct tcatctgaag ttgctgttct atatcaagat gttaactgca 3480
   ctgatgtttc tacagcaatt catgcagatc aactcacacc agcttggcgc atatattcta 3540
   ctggaaacaa tgtattccag actcaagcag gctgtcttat aggagctgag catgtcgaca 3600
   cttcttatga gtgcgacatt cctattggag ctggcatttg tgctagttac catacagttt 3660
   ctttattacg tagtactagc caaaaatcta ttgtggctta tactatgtct ttaggtgctg 3720
   atagttcaat tgcttactct aataacacca ttgctatacc tactaacttt tcaattagca 3780
   ttactacaga agtaatgcct gtttctatgg ctaaaacctc cgtagattgt aatatgtaca 3840
   tctgcggaga ttctactgaa tgtgctaatt tgcttctcca atatggtagc ttttgcacac 3900
   aactaaatcg tgcactctca ggtattgctg ctgaacagga tcgcaacaca cgtgaagtgt 3960
   tcgctcaagt caaacaaatg tacaaaaccc caactttgaa atattttggt ggttttaatt 4020
   tttcacaaat attacctgac cctctaaagc caactaagag gtcttttatt gaggacttgc 4080
   tctttaataa ggtgacactc gctgatgctg gcttcatgaa gcaatatggc gaatgcctag 4140
   gtgatattaa tgctagagat ctcatttgtg cgcagaagtt caatggactt acagtgttgc 4200
   cacctctgct cactgatgat atgattgctg cctacactgc tgctctagtt agtggtactg 4260
   ccactgctgg atggacattt ggtgctggcg ctgctcttca aatacctttt gctatgcaaa 4320
   tggcatatag gttcaatggc attggagtta cccaaaatgt tctctatgag aaccaaaaac 4380
   aaatcgccaa ccaatttaac aaggcgatta gtcaaattca agaatcactt acaacaacat 4440
   caactgcatt gggcaagctg caagacgttg ttaaccagaa tgctcaagca ttaaacacac 4500
   ttgttaaaca acttagctct aattttggtg caatttcaag tgtgctaaat gatatccttt 4560
   cgcgacttga taaagtcgag gcggaggtac aaattgacag gttaattaca ggcagacttc 4620
   aaagccttca aacctatgta acacaacaac taatcagggc tgctgaaatc agggcttctg 4680
   ctaatcttgc tgctactaaa atgtctgagt gtgttcttgg acaatcaaaa agagttgact 4740
   tttgtggaaa gggctaccac cttatgtcct tcccacaagc agccccgcat ggtgttgtct 4800
   tcctacatgt cacgtatgtg ccatcccagg agaggaactt caccacagcg ccagcaattt 4860
   gtcatgaagg caaagcatac ttccctcgtg aaggtgtttt tgtgtttaat ggcacttctt 4920
   ggtttattac acagaggaac ttcttttctc cacaaataat tactacagac aatacatttg 4980
   tctcaggaaa ttgtgatgtc gttattggca tcattaacaa cacagtttat gatcctctgc 5040
   aacctgagct tgactcattc aaagaagagc tggacaagta cttcaaaaat catacatcac 5100
   cagatgttga ttttggcgac atttcaggca ttaacgcttc tgtcgtcaac attcaaaaag 5160
   aaattgaccg cctcaatgag gtcgctaaaa atttaaatga atcactcatt gaccttcaag 5220
   aattgggaaa atatgagcaa tatattaaat ggccttggta tgtttggctc ggcttcattg 5280
   ctggactaat tgccatcgtc atggttacaa tcttgctttg ttgcatgact agttgttgca 5340
   gttgcctcaa gggtgcatgc tcttgtggtt cttgctgcaa gtttgatgag gatgactctg 5400
   agccagttct caagggtgtc aaattacatt acacagccta aatctagaaa gccatggata 5460
   tcggatccac tacgcgttag agctcgctga tcagcctcga ctgtgccttc tagttgccag 5520
   ccatctgttg tttgcccctc ccccgtgcct tccttgaccc tggaaggtgc cactcccact 5580
   gtcctttcct aataaaatga ggaaattgca tcgcattgtc tgagtaggtg tcattctatt 5640
   ctggggggtg gggtggggca ggacagcaag ggggaggatt gggaagacaa tagcaggggg 5700
   gtgggcgaag aactccagca tgagatcccc gcgctggagg atcatccagc cggcgtcccg 5760
   gaaaacgatt ccgaagccca acctttcata gaaggcggcg gtggaatcga aatctcgtga 5820
   tggcaggttg ggcgtcgctt ggtcggtcat ttcgaacccc agagtcccgc tcagaagaac 5880
   tcgtcaagaa ggcgatagaa ggcgatgcgc tgcgaatcgg gagcggcgat accgtaaagc 5940
   acgaggaagc ggtcagccca ttcgccgcca agctcttcag caatatcacg ggtagccaac 6000
   gctatgtcct gatagcggtc cgccacaccc agccggccac agtcgatgaa tccagaaaag 6060
   cggccatttt ccaccatgat attcggcaag caggcatcgc catgggtcac gacgagatcc 6120
   tcgccgtcgg gcatgcgcgc cttgagcctg gcgaacagtt cggctggcgc gagcccctga 6180
   tgctcttcgt ccagatcatc ctgatcgaca agaccggctt ccatccgagt acgtgctcgc 6240
   tcgatgcgat gtttcgcttg gtggtcgaat gggcaggtag ccggatcaag cgtatgcagc 6300
   cgccgcattg catcagccat gatggatact ttctcggcag gagcaaggtg agatgacagg 6360
   agatcctgcc ccggcacttc gcccaatagc agccagtccc ttcccgcttc agtgacaacg 6420
   tcgagcacag ctgcgcaagg aacgcccgtc gtggccagcc acgatagccg cgctgcctcg 6480
   tcctgcagtt cattcagggc accggacagg tcggtcttga caaaaagaac cgggcgcccc 6540
   tgcgctgaca gccggaacac ggcggcatca gagcagccga ttgtctgttg tgcccagtca 6600
   tagccgaata gcctctccac ccaagcggcc ggagaacctg cgtgcaatcc atcttgttca 6660
   at cat gcgaa acgat cct ca t cct gt ct ct t gat cagat c t t gat cccct gcgccat cag 6720
   atccttggcg gcaagaaagc atccagttta ctttgcaggg cttcccaacc ttaccagagg 6780
   ggcgccccag ctggcaattc cggttcgctt gctgtccata aaaccgccca gtctagctat 6840
   cgccat gt aa gcccact gca agct acct gc t t t ct ct t t g cgct t gcgt t t t ccct t gt c 6900
   cagat agccc agt agct gac at t cat ccgg ggt cagcacc gt t t ct gcgg act ggct t t c 6960
   t acgt gt t cc gct t cct t t a gcagccct t g cgccct gagt gct t gcggca gcgt gaagct 7020
   aat t cat ggt t aaat t t t t g t t aaat cagc t cat t t t t t a accaat aggc cgaaat cggc 7080
   aaaat ccct t at aaat caaa agaat agccc gagat agggt t gagt gt t gt t ccagt t t gg 7140
   aacaagagtc cactattaaa gaacgtggac tccaacgtca aagggcgaaa aaccgtctat 7200
   cagggcgat g gccggat cag ct t at gcggt gt gaaat acc gcacagat gc gt aaggagaa 7260
   aat accgcat caggcgct ct t ccgct t cct cgct cact ga ct cgct gcgc t cggt cgt t c 7320
   ggct gcggcg agcggt at ca gct cact caa aggcggt aat acggt t at cc acagaat cag 7380
   gggataacgc aggaaagaac atgtgagcaa aaggccagca aaaggccagg aaccgtaaaa 7440
   aggccgcgt t gct ggcgt t t t t ccat aggc t ccgcccccc t gacgagcat cacaaaaat c 7500
   gacgctcaag tcagaggtgg cgaaacccga caggactata aagataccag gcgtttcccc 7560
   ct ggaagct c cct cgt gcgc t ct cct gt t c cgaccct gcc gct t accgga t acct gt ccg 7620
   cct t t ct ccc t t cgggaagc gt ggcgct t t ct cat agct c acgct gt agg t at ct cagt t 7680
   cggtgtaggt cgttcgctcc aagctgggct gtgtgcacga accccccgtt cagcccgacc 7740
   gct gcgcct t at ccggt aac t at cgt ct t g agt ccaaccc ggt aagacac gact t at cgc 7800
   cactggcagc agccactggt aacaggatta gcagagcgag gtatgtaggc ggtgctacag 7860
   agt t ct t gaa gt ggt ggcct aact acggct acact agaag gacagt at t t ggt at ct gcg 7920
   ctctgctgaa gccagttacc ttcggaaaaa gagttggtag ctcttgatcc ggcaaacaaa 7980
   ccaccgctgg tagcggtggt ttttttgttt gcaagcagca gattacgcgc agaaaaaaag 8040
   gat ct caaga agat cct t t g at ct t t t ct a ct gaacggt g at ccccaccg gaat t gcg 8098
<210> 9922
   <211> 7918
   <212> DNA
   <213> Artificial Sequence
<220>
   <223> pCMVKm2 vect or
<400> 9922
   gccgcggaat ttcgactcta ggccattgca tacgttgtat ctatatcata atatgtacat   60
   ttatattggc tcatgtccaa tatgaccgcc atgttgacat tgattattga ctagttatta  120
   atagtaatca attacggggt cattagttca tagcccatat atggagttcc gcgttacata  180
   acttacggta aatggcccgc ctggctgacc gcccaacgac ccccgcccat tgacgtcaat  240
   aatgacgtat gttcccatag taacgccaat agggactttc cattgacgtc aatgggtgga  300
   gtatttacgg taaactgccc acttggcagt acatcaagtg tatcatatgc caagtccgcc  360
   ccctattgac gtcaatgacg gtaaatggcc cgcctggcat tatgcccagt acatgacctt  420
   acgggacttt cctacttggc agtacatcta cgtattagtc atcgctatta ccatggtgat  480
   gcggttttgg cagtacacca atgggcgtgg atagcggttt gactcacggg gatttccaag  540
   tctccacccc attgacgtca atgggagttt gttttggcac caaaatcaac gggactttcc  600
   aaaatgtcgt aataaccccg ccccgttgac gcaaatgggc ggtaggcgtg tacggtggga  660
   ggtctatata agcagagctc gtttagtgaa ccgtcagatc gcctggagac gccatccacg  720
   ctgttttgac ctccatagaa gacaccggga ccgatccagc ctccgcggcc gggaacggtg  780
   cattggaacg cggattcccc gtgccaagag tgacgtaagt accgcctata gactctatag  840
   gcacacccct ttggctctta tgcatgctat actgtttttg gcttggggcc tatacacccc  900
   cgcttcctta tgctataggt gatggtatag cttagcctat aggtgtgggt tattgaccat  960
   tattgaccac tcccctattg gtgacgatac tttccattac taatccataa catggctctt 1020
   tgccacaact atctctattg gctatatgcc aatactctgt ccttcagaga ctgacacgga 1080
   ctctgtattt ttacaggatg gggtcccatt tattatttac aaattcacat atacaacaac 1140
   gccgtccccc gtgcccgcag tttttattaa acatagcgtg ggatctccac gcgaatctcg 1200
   ggtacgtgtt ccggacatgg gctcttctcc ggtagcggcg gagcttccac atccgagccc 1260
   tggtcccatg cctccagcgg ctcatggtcg ctcggcagct ccttgctcct aacagtggag 1320
   gccagactta ggcacagcac aatgcccacc accaccagtg tgccgcacaa ggccgtggcg 1380
   gtagggtatg tgtctgaaaa tgagctcgga gattgggctc gcaccgctga cgcagatgga 1440
   agacttaagg cagcggcaga agaagatgca ggcagctgag ttgttgtatt ctgataagag 1500
   tcagaggtaa ctcccgttgc ggtgctgtta acggtggagg gcagtgtagt ctgagcagta 1560
   ctcgttgctg ccgcgcgcgc caccagacat aatagctgac agactaacag actgttcctt 1620
   tccatgggtc ttttctgcag tcaccgtcgt cgacctaaga attcgccacc atgttcatct 1680
   tcctgctgtt cctgactctc actagtggta gtgaccttga ccggtgcacc acttttgatg 1740
   atgttcaagc tcctaattac actcaacata cttcatctat gaggggggtt tactatcctg 1800
   atgaaatttt tagatcagac actctttatt taactcagga tttatttctt ccattttatt 1860
   ctaatgttac agggtttcat actattaatc atacgtttgg caaccctgtc atacctttta 1920
   aggatggtat ttattttgct gccacagaga aatcaaatgt tgtccgtggt tgggtttttg 1980
   gttctaccat gaacaacaag tcacagtcgg tgattattat taacaattct actaatgttg 2040
   ttatacgagc atgtaacttt gaattgtgtg acaacccttt ctttgctgtt tctaaaccca 2100
   tgggtacaca gacacatact atgatattcg ataatgcatt taattgcact ttcgagtaca 2160
   tatctgatgc cttttcgctt gatgtttcag aaaagtcagg taattttaaa cacttacgag 2220
   agtttgtgtt taaaaataaa gatgggtttc tctatgttta taagggctat caacctatag 2280
   atgtagttcg tgatctacct tctggtttta acactttgaa acctattttt aagttgcctc 2340
   ttggtattaa cattacaaat tttagagcca ttcttacagc cttttcacct gctcaagaca 2400
   tttggggcac gtcagctgca gcctattttg ttggctattt aaagccaact acatttatgc 2460
   tcaagtatga tgaaaatggt acaatcacag atgctgttga ttgttctcaa aatccacttg 2520
   ctgaactcaa atgctctgtt aagagctttg agattgacaa aggaatttac cagacctcta 2580
   atttcagggt tgttccctca ggagatgttg tgagattccc taatattaca aacttgtgtc 2640
   cttttggaga ggtttttaat gctactaaat tcccttctgt ctatgcatgg gagagaaaaa 2700
   aaatttctaa ttgtgttgct gattactctg tgctctacaa ctcaacattt ttttcaacct 2760
   ttaagtgcta tggcgtttct gccactaagt tgaatgatct ttgcttctcc aatgtctatg 2820
   cagattcttt tgtagtcaag ggagatgatg taagacaaat agcgccaggg caaactggtg 2880
   ttattgctga ttataattat aaattgccag atgatttcat gggttgtgtc cttgcttgga 2940
   atactaggaa cattgatgct acttcaactg gtaattataa ttataaatat aggtatctta 3000
   gacatggcaa gcttaggccc tttgagagag acatatctaa tgtgcctttc tcccctgatg 3060
   gcaaaccttg caccccacct gctcttaatt gttattggcc attaaatgat tatggttttt 3120
   acaccactac tggcattggc taccaacctt acagagttgt agtactttct tttgaacttt 3180
   taaatgcacc ggccacggtt tgtggaccaa aattatccac tgaccttatt aagaaccagt 3240
   gtgtcaattt taattttaat ggactcactg gtactggtgt gttaactcct tcttcaaaga 3300
   gatttcaacc atttcaacaa tttggccgtg atgtttctga tttcactgat tccgttcgag 3360
   atcctaaaac atctgaaata ttagacattt caccttgctc ttttgggggt gtaagtgtaa 3420
   ttacacctgg aacaaatgct tcatctgaag ttgctgttct atatcaagat gttaactgca 3480
   ctgatgtttc tacagcaatt catgcagatc aactcacacc agcttggcgc atatattcta 3540
   ctggaaacaa tgtattccag actcaagcag gctgtcttat aggagctgag catgtcgaca 3600
   cttcttatga gtgcgacatt cctattggag ctggcatttg tgctagttac catacagttt 3660
   ctttattacg tagtactagc caaaaatcta ttgtggctta tactatgtct ttaggtgctg 3720
   atagttcaat tgcttactct aataacacca ttgctatacc tactaacttt tcaattagca 3780
   ttactacaga agtaatgcct gtttctatgg ctaaaacctc cgtagattgt aatatgtaca 3840
   tctgcggaga ttctactgaa tgtgctaatt tgcttctcca atatggtagc ttttgcacac 3900
   aactaaatcg tgcactctca ggtattgctg ctgaacagga tcgcaacaca cgtgaagtgt 3960
   tcgctcaagt caaacaaatg tacaaaaccc caactttgaa atattttggt ggttttaatt 4020
   tttcacaaat attacctgac cctctaaagc caactaagag gtcttttatt gaggacttgc 4080
   tctttaataa ggtgacactc gctgatgctg gcttcatgaa gcaatatggc gaatgcctag 4140
   gtgatattaa tgctagagat ctcatttgtg cgcagaagtt caatggactt acagtgttgc 4200
   cacctctgct cactgatgat atgattgctg cctacactgc tgctctagtt agtggtactg 4260
   ccactgctgg atggacattt ggtgctggcg ctgctcttca aatacctttt gctatgcaaa 4320
   tggcatatag gttcaatggc attggagtta cccaaaatgt tctctatgag aaccaaaaac 4380
   aaatcgccaa ccaatttaac aaggcgatta gtcaaattca agaatcactt acaacaacat 4440
   caactgcatt gggcaagctg caagacgttg ttaaccagaa tgctcaagca ttaaacacac 4500
   ttgttaaaca acttagctct aattttggtg caatttcaag tgtgctaaat gatatccttt 4560
   cgcgacttga taaagtcgag gcggaggtac aaattgacag gttaattaca ggcagacttc 4620
   aaagccttca aacctatgta acacaacaac taatcagggc tgctgaaatc agggcttctg 4680
   ctaatcttgc tgctactaaa atgtctgagt gtgttcttgg acaatcaaaa agagttgact 4740
   tttgtggaaa gggctaccac cttatgtcct tcccacaagc agccccgcat ggtgttgtct 4800
   tcctacatgt cacgtatgtg ccatcccagg agaggaactt caccacagcg ccagcaattt 4860
   gtcatgaagg caaagcatac ttccctcgtg aaggtgtttt tgtgtttaat ggcacttctt 4920
   ggtttattac acagaggaac ttcttttctc cacaaataat tactacagac aatacatttg 4980
   tctcaggaaa ttgtgatgtc gttattggca tcattaacaa cacagtttat gatcctctgc 5040
   aacctgagct tgactcattc aaagaagagc tggacaagta cttcaaaaat catacatcac 5100
   cagatgttga ttttggcgac atttcaggca ttaacgcttc tgtcgtcaac attcaaaaag 5160
   aaattgaccg cctcaatgag gtcgctaaaa atttaaatga atcactcatt gaccttcaag 5220
   aattgggaaa atatgagcaa tatattaaat ggcctgccta aatctagaaa gccatggata 5280
   tcggatccac tacgcgttag agctcgctga tcagcctcga ctgtgccttc tagttgccag 5340
   ccatctgttg tttgcccctc ccccgtgcct tccttgaccc tggaaggtgc cactcccact 5400
   gtcctttcct aataaaatga ggaaattgca tcgcattgtc tgagtaggtg tcattctatt 5460
   ctggggggtg gggtggggca ggacagcaag ggggaggatt gggaagacaa tagcaggggg 5520
   gtgggcgaag aactccagca tgagatcccc gcgctggagg atcatccagc cggcgtcccg 5580
   gaaaacgatt ccgaagccca acctttcata gaaggcggcg gtggaatcga aatctcgtga 5640
   tggcaggttg ggcgtcgctt ggtcggtcat ttcgaacccc agagtcccgc tcagaagaac 5700
   tcgtcaagaa ggcgatagaa ggcgatgcgc tgcgaatcgg gagcggcgat accgtaaagc 5760
   acgaggaagc ggtcagccca ttcgccgcca agctcttcag caatatcacg ggtagccaac 5820
   gctatgtcct gatagcggtc cgccacaccc agccggccac agtcgatgaa tccagaaaag 5880
   cggccatttt ccaccatgat attcggcaag caggcatcgc catgggtcac gacgagatcc 5940
   tcgccgtcgg gcatgcgcgc cttgagcctg gcgaacagtt cggctggcgc gagcccctga 6000
   tgctcttcgt ccagatcatc ctgatcgaca agaccggctt ccatccgagt acgtgctcgc 6060
   tcgatgcgat gtttcgcttg gtggtcgaat gggcaggtag ccggatcaag cgtatgcagc 6120
   cgccgcattg catcagccat gatggatact ttctcggcag gagcaaggtg agatgacagg 6180
   agat cct gcc ccggcact t c gcccaat agc agccagt ccc t t cccgct t c agt gacaacg 6240
   t cgagcacag ct gcgcaagg aacgcccgt c gt ggccagcc acgat agccg cgct gcct cg 6300
   tcctgcagtt cattcagggc accggacagg tcggtcttga caaaaagaac cgggcgcccc     6360
   tgcgctgaca gccggaacac ggcggcatca gagcagccga ttgtctgttg tgcccagtca     6420
   tagccgaata gcctctccac ccaagcggcc ggagaacctg cgtgcaatcc atcttgttca     6480
   at cat gcgaa acgat cct ca t cct gt ct ct t gat cagat c t t gat cccct gcgccat cag 6540
   atccttggcg gcaagaaagc catccagttt actttgcagg gcttcccaac cttaccagag      6600
   ggcgccccag ctggcaattc cggttcgctt gctgtccata aaaccgccca gtctagctat     6660
   cgccat gt aa gcccact gca agct acct gc t t t ct ct t t g cgct t gcgt t t t ccct t gt c 6720
   cagat agccc agt agct gac at t cat ccgg ggt cagcacc gt t t ct gcgg act ggct t t c 6780
   t acgt gt t cc gct t cct t t a gcagccct t g cgccct gagt gct t gcggca gcgt gaagct 6840
   aat t cat ggt t aaat t t t t g t t aaat cagc t cat t t t t t a accaat aggc cgaaat cggc 6900
   aaaat ccct t at aaat caaa agaat agccc gagat agggt t gagt gt t gt t ccagt t t gg 6960
   aacaagagtc cactattaaa gaacgtggac tccaacgtca aagggcgaaa aaccgtctat     7020
   cagggcgat g gccggat cag ct t at gcggt gt gaaat acc gcacagat gc gt aaggagaa 7080
   aat accgcat caggcgct ct t ccgct t cct cgct cact ga ct cgct gcgc t cggt cgt t c 7140
   ggct gcggcg agcggt at ca gct cact caa aggcggt aat acggt t at cc acagaat cag 7200
   gggataacgc aggaaagaac atgtgagcaa aaggccagca aaaggccagg aaccgtaaaa     7260
   aggccgcgt t gct ggcgt t t t t ccat aggc t ccgcccccc t gacgagcat cacaaaaat c 7320
   gacgctcaag tcagaggtgg cgaaacccga caggactata aagataccag gcgtttcccc     7380
   ct ggaagct c cct cgt gcgc t ct cct gt t c cgaccct gcc gct t accgga t acct gt ccg 7440
   cct t t ct ccc t t cgggaagc gt ggcgct t t ct cat agct c acgct gt agg t at ct cagt t 7500
   cggtgtaggt cgttcgctcc aagctgggct gtgtgcacga accccccgtt cagcccgacc     7560
   gct gcgcct t at ccggt aac t at cgt ct t g agt ccaaccc ggt aagacac gact t at cgc 7620
   cactggcagc agccactggt aacaggatta gcagagcgag gtatgtaggc ggtgctacag     7680
   agt t ct t gaa gt ggt ggcct aact acggct acact agaag gacagt at t t ggt at ct gcg 7740
   ctctgctgaa gccagttacc ttcggaaaaa gagttggtag ctcttgatcc ggcaaacaaa     7800
   ccaccgctgg tagcggtggt ttttttgttt gcaagcagca gattacgcgc agaaaaaaag     7860
   gat ct caaga agat cct t t g at ct t t t ct a ct gaacggt g at ccccaccg gaat t gcg 7918
<210> 9923
   <211> 4333
   <212> DNA
   <213> Artificial Sequence
<220>
   <223> pCMVKm2 vect or
<400> 9923
   gccgcggaat ttcgactcta ggccattgca tacgttgtat ctatatcata atatgtacat 60
   ttatattggc tcatgtccaa tatgaccgcc atgttgacat tgattattga ctagttatta 120
   atagtaatca attacggggt cattagttca tagcccatat atggagttcc gcgttacata 180
   acttacggta aatggcccgc ctggctgacc gcccaacgac ccccgcccat tgacgtcaat 240
   aatgacgtat gttcccatag taacgccaat agggactttc cattgacgtc aatgggtgga 300
   gtatttacgg taaactgccc acttggcagt acatcaagtg tatcatatgc caagtccgcc 360
   ccctattgac gtcaatgacg gtaaatggcc cgcctggcat tatgcccagt acatgacctt 420
   acgggacttt cctacttggc agtacatcta cgtattagtc atcgctatta ccatggtgat 480
   gcggttttgg cagtacacca atgggcgtgg atagcggttt gactcacggg gatttccaag 540
   tctccacccc attgacgtca atgggagttt gttttggcac caaaatcaac gggactttcc 600
   aaaatgtcgt aataaccccg ccccgttgac gcaaatgggc ggtaggcgtg tacggtggga 660
   ggtctatata agcagagctc gtttagtgaa ccgtcagatc gcctggagac gccatccacg 720
   ctgttttgac ctccatagaa gacaccggga ccgatccagc ctccgcggcc gggaacggtg 780
   cattggaacg cggattcccc gtgccaagag tgacgtaagt accgcctata gactctatag 840
   gcacacccct ttggctctta tgcatgctat actgtttttg gcttggggcc tatacacccc 900
   cgcttcctta tgctataggt gatggtatag cttagcctat aggtgtgggt tattgaccat 960
   tattgaccac tcccctattg gtgacgatac tttccattac taatccataa catggctctt 1020
   tgccacaact atctctattg gctatatgcc aatactctgt ccttcagaga ctgacacgga 1080
   ctctgtattt ttacaggatg gggtcccatt tattatttac aaattcacat atacaacaac 1140
   gccgtccccc gtgcccgcag tttttattaa acatagcgtg ggatctccac gcgaatctcg 1200
   ggtacgtgtt ccggacatgg gctcttctcc ggtagcggcg gagcttccac atccgagccc 1260
   tggtcccatg cctccagcgg ctcatggtcg ctcggcagct ccttgctcct aacagtggag 1320
   gccagactta ggcacagcac aatgcccacc accaccagtg tgccgcacaa ggccgtggcg 1380
   gtagggtatg tgtctgaaaa tgagctcgga gattgggctc gcaccgctga cgcagatgga 1440
   agacttaagg cagcggcaga agaagatgca ggcagctgag ttgttgtatt ctgataagag 1500
   tcagaggtaa ctcccgttgc ggtgctgtta acggtggagg gcagtgtagt ctgagcagta 1560
   ctcgttgctg ccgcgcgcgc caccagacat aatagctgac agactaacag actgttcctt 1620
   tccatgggtc ttttctgcag tcaccgtcgt cgacctaaga attcagactc gagcaagtct 1680
   agaaagccat ggatatcgga tccactacgc gttagagctc gctgatcagc ctcgactgtg 1740
   ccttctagtt gccagccatc tgttgtttgc ccctcccccg tgccttcctt gaccctggaa 1800
   ggt gccact c  ccact gt cct  t t cct aat aa  aat gaggaaa  t t gcat cgca  t t gt ct gagt    1860
   aggtgtcatt ctattctggg gggtggggtg gggcaggaca gcaaggggga ggattgggaa      1920
   gacaat agca  ggggggt ggg  cgaagaact c  cagcat gaga  t ccccgcgct  ggaggat cat    1980
   ccagccggcg tcccggaaaa cgattccgaa gcccaacctt tcatagaagg cggcggtgga      2040
   atcgaaatct cgtgatggca ggttgggcgt cgcttggtcg gtcatttcga accccagagt      2100
   cccgctcaga agaactcgtc aagaaggcga tagaaggcga tgcgctgcga atcgggagcg      2160
   gcgataccgt aaagcacgag gaagcggtca gcccattcgc cgccaagctc ttcagcaata      2220
   tcacgggtag ccaacgctat gtcctgatag cggtccgcca cacccagccg gccacagtcg      2280
   atgaatccag aaaagcggcc attttccacc atgatattcg gcaagcaggc atcgccatgg      2340
   gt cacgacga  gat cct cgcc  gt cgggcat g  cgcgcct t ga  gcct ggcgaa  cagt t cggct    2400
   ggcgcgagcc cctgatgctc ttcgtccaga tcatcctgat cgacaagacc ggcttccatc      2460
   cgagt acgt g  ct cgct cgat  gcgat gt t t c  gct t ggt ggt  cgaat gggca  ggt agccgga    2520
   tcaagcgtat gcagccgccg cattgcatca gccatgatgg atactttctc ggcaggagca      2580
   aggt gagat g  acaggagat c  ct gccccggc  act t cgccca  at agcagcca  gt ccct t ccc    2640
   gcttcagtga caacgtcgag cacagctgcg caaggaacgc ccgtcgtggc cagccacgat      2700
   agccgcgctg cctcgtcctg cagttcattc agggcaccgg acaggtcggt cttgacaaaa      2760
   agaaccgggc gcccctgcgc tgacagccgg aacacggcgg catcagagca gccgattgtc      2820
   tgttgtgccc agtcatagcc gaatagcctc tccacccaag cggccggaga acctgcgtgc      2880
   aatccatctt gttcaatcat gcgaaacgat cctcatcctg tctcttgatc agatcttgat      2940
   cccct gcgcc  at cagat cct  t ggcggcaag  aaagccat cc  agt t t act t t  gcagggct t c    3000
   ccaaccttac cagagggcgc cccagctggc aattccggtt cgcttgctgt ccataaaacc      3060
   gcccagt ct a  gct at cgcca  t gt aagccca  ct gcaagct a  cct gct t t ct  ct t t gcgct t    3120
   gcgt t t t ccc  t t gt ccagat  agcccagt ag  ct gacat t ca  t ccggggt ca  gcaccgt t t c    3180
   t gcggact gg  ct t t ct acgt  gt t ccgct t c  ct t t agcagc  cct t gcgccc  t gagt gct t g    3240
   cggcagcgt g  aagct aat t c  at ggt t aaat  t t t t gt t aaa  t cagct cat t  t t t t aaccaa    3300
   taggccgaaa tcggcaaaat cccttataaa tcaaaagaat agcccgagat agggttgagt      3360
   gt t gt t ccag  t t t ggaacaa  gagt ccact a  t t aaagaacg  t ggact ccaa  cgt caaaggg    3420
   cgaaaaaccg tctatcaggg cgatggccgg atcagcttat gcggtgtgaa ataccgcaca      3480
   gat gcgt aag  gagaaaat ac  cgcat caggc  gct ct t ccgc  t t cct cgct c  act gact cgc    3540
   tgcgctcggt cgttcggctg cggcgagcgg tatcagctca ctcaaaggcg gtaatacggt      3600
   tatccacaga atcaggggat aacgcaggaa agaacatgtg agcaaaaggc cagcaaaagg      3660
   ccaggaaccg taaaaaggcc gcgttgctgg cgtttttcca taggctccgc ccccctgacg      3720
   agcatcacaa aaatcgacgc tcaagtcaga ggtggcgaaa cccgacagga ctataaagat      3780
   accaggcgt t  t ccccct gga  agct ccct cg  t gcgct ct cc  t gt t ccgacc  ct gccgct t a    3840
   ccggat acct  gt ccgcct t t  ct ccct t cgg  gaagcgt ggc  gct t t ct cat  agct cacgct    3900
   gtaggtatct cagttcggtg taggtcgttc gctccaagct gggctgtgtg cacgaacccc      3960
   ccgt t cagcc  cgaccgct gc  gcct t at ccg  gt aact at cg  t ct t gagt cc  aacccggt aa    4020
   gacacgactt atcgccactg gcagcagcca ctggtaacag gattagcaga gcgaggtatg      4080
   taggcggtgc tacagagttc ttgaagtggt ggcctaacta cggctacact agaaggacag      4140
   t at t t ggt at  ct gcgct ct g  ct gaagccag  t t acct t cgg  aaaaagagt t  ggt agct ct t    4200
   gatccggcaa acaaaccacc gctggtagcg gtggtttttt tgtttgcaag cagcagatta      4260
   cgcgcagaaa aaaaggatct caagaagatc ctttgatctt ttctactgaa cggtgatccc      4320
   caccggaat t  gcg                                                                  4333
<210> 9962
   <211> 1255
   <212> PRT
   <213> SARS coronavirus
<400> 9962
   Met Phe Ile Phe Leu Leu Phe Leu Thr Leu Thr Ser Gly Ser Asp Leu
   1               5                   10                  15
   Asp Arg Cys Thr Thr Phe Asp Asp Val Gln Ala Pro Asn Tyr Thr Gln
               20                  25                  30
   His Thr Ser Ser Met Arg Gly Val Tyr Tyr Pro Asp Glu Ile Phe Arg
           35                  40                  45
   Ser Asp Thr Leu Tyr Leu Thr Gln Asp Leu Phe Leu Pro Phe Tyr Ser
       50                  55                  60
   Asn Val Thr Gly Phe His Thr Ile Asn His Thr Phe Gly Asn Pro Val
   65                  70                  75                  80
   Ile Pro Phe Lys Asp Gly Ile Tyr Phe Ala Ala Thr Glu Lys Ser Asn
                   85                  90                  95
   Val Val Arg Gly Trp Val Phe Gly Ser Thr Met Asn Asn Lys Ser Gln
   100 105 110
   Ser Val Ile Ile Ile Asn Asn Ser Thr Asn Val Val Ile Arg Ala Cys
   115 120 125
   Asn Phe Glu Leu Cys Asp Asn Pro Phe Phe Ala Val Ser Lys Pro Met
   130 135 140
   Gly Thr Gln Thr His Thr Met Ile Phe Asp Asn Ala Phe Asn Cys Thr
   145 150 155 160
   Phe Glu Tyr Ile Ser Asp Ala Phe Ser Leu Asp Val Ser Glu Lys Ser
   165 170 175
   Gly Asn Phe Lys His Leu Arg Glu Phe Val Phe Lys Asn Lys Asp Gly
   180 185 190
   Phe Leu Tyr Val Tyr Lys Gly Tyr Gln Pro Ile Asp Val Val Arg Asp
   195 200 205
   Leu Pro Ser Gly Phe Asn Thr Leu Lys Pro Ile Phe Lys Leu Pro Leu
   210 215 220
   Gly Ile Asn Ile Thr Asn Phe Arg Ala Ile Leu Thr Ala Phe Ser Pro
   225 230 235 240
   Ala Gln Asp Ile Trp Gly Thr Ser Ala Ala Ala Tyr Phe Val Gly Tyr
   245 250 255
   Leu Lys Pro Thr Thr Phe Met Leu Lys Tyr Asp Glu Asn Gly Thr Ile
   260 265 270
   Thr Asp Ala Val Asp Cys Ser Gln Asn Pro Leu Ala Glu Leu Lys Cys
   275 280 285
   Ser Val Lys Ser Phe Glu Ile Asp Lys Gly Ile Tyr Gln Thr Ser Asn
   290 295 300
   Phe Arg Val Val Pro Ser Gly Asp Val Val Arg Phe Pro Asn Ile Thr
   305 310 315 320
   Asn Leu Cys Pro Phe Gly Glu Val Phe Asn Ala Thr Lys Phe Pro Ser
   325 330 335
   Val Tyr Ala Trp Glu Arg Lys Lys Ile Ser Asn Cys Val Ala Asp Tyr
   340 345 350
   Ser Val Leu Tyr Asn Ser Thr Phe Phe Ser Thr Phe Lys Cys Tyr Gly
   355 360 365
   Val Ser Ala Thr Lys Leu Asn Asp Leu Cys Phe Ser Asn Val Tyr Ala
   370 375 380
   Asp Ser Phe Val Val Lys Gly Asp Asp Val Arg Gln Ile Ala Pro Gly
   385 390 395 400
   Gln Thr Gly Val Ile Ala Asp Tyr Asn Tyr Lys Leu Pro Asp Asp Phe
   405 410 415
   Met Gly Cys Val Leu Ala Trp Asn Thr Arg Asn Ile Asp Ala Thr Ser
   420 425 430
   Thr Gly Asn Tyr Asn Tyr Lys Tyr Arg Tyr Leu Arg His Gly Lys Leu
   435 440 445
   Arg Pro Phe Glu Arg Asp Ile Ser Asn Val Pro Phe Ser Pro Asp Gly
   450 455 460
   Lys Pro Cys Thr Pro Pro Ala Leu Asn Cys Tyr Trp Pro Leu Asn Asp
   465 470 475 480
   Tyr Gly Phe Tyr Thr Thr Thr Gly Ile Gly Tyr Gln Pro Tyr Arg Val
   485 490 495
   Val Val Leu Ser Phe Glu Leu Leu Asn Ala Pro Ala Thr Val Cys Gly
   500 505 510
   Pro Lys Leu Ser Thr Asp Leu Ile Lys Asn Gln Cys Val Asn Phe Asn
   515 520 525
   Phe Asn Gly Leu Thr Gly Thr Gly Val Leu Thr Pro Ser Ser Lys Arg
   530 535 540
   Phe Gln Pro Phe Gln Gln Phe Gly Arg Asp Val Ser Asp Phe Thr Asp
   545 550 555 560
   Ser Val Arg Asp Pro Lys Thr Ser Glu Ile Leu Asp Ile Ser Pro Cys
   565 570 575
   Ser Phe Gly Gly Val Ser Val Ile Thr Pro Gly Thr Asn Ala Ser Ser
   580 585 590
   Glu Val Ala Val Leu Tyr Gln Asp Val Asn Cys Thr Asp Val Ser Thr
   595 600 605
   Ala Ile His Ala Asp Gln Leu Thr Pro Ala Trp Arg Ile Tyr Ser Thr
   610 615 620
   Gly Asn Asn Val Phe Gln Thr Gln Ala Gly Cys Leu Ile Gly Ala Glu
   625 630 635 640
   His Val Asp Thr Ser Tyr Glu Cys Asp Ile Pro Ile Gly Ala Gly Ile
   645 650 655
   Cys Ala Ser Tyr His Thr Val Ser Leu Leu Arg Ser Thr Ser Gln Lys
   660 665 670
   Ser Ile Val Ala Tyr Thr Met Ser Leu Gly Ala Asp Ser Ser Ile Ala
   675 680 685
   Tyr Ser Asn Asn Thr Ile Ala Ile Pro Thr Asn Phe Ser Ile Ser Ile
   690 695 700
   Thr Thr Glu Val Met Pro Val Ser Met Ala Lys Thr Ser Val Asp Cys
   705 710 715 720
   Asn Met Tyr Ile Cys Gly Asp Ser Thr Glu Cys Ala Asn Leu Leu Leu
   725 730 735
   Gln Tyr Gly Ser Phe Cys Thr Gln Leu Asn Arg Ala Leu Ser Gly Ile
   740 745 750
   Ala Ala Glu Gln Asp Arg Asn Thr Arg Glu Val Phe Ala Gln Val Lys
   755 760 765
   Gln Met Tyr Lys Thr Pro Thr Leu Lys Tyr Phe Gly Gly Phe Asn Phe
   770 775 780
   Ser Gln Ile Leu Pro Asp Pro Leu Lys Pro Thr Lys Arg Ser Phe Ile
   785 790 795 800
   Glu Asp Leu Leu Phe Asn Lys Val Thr Leu Ala Asp Ala Gly Phe Met
   805 810 815
   Lys Gln Tyr Gly Glu Cys Leu Gly Asp Ile Asn Ala Arg Asp Leu Ile
   820 825 830
   Cys Ala Gln Lys Phe Asn Gly Leu Thr Val Leu Pro Pro Leu Leu Thr
   835 840 845
   Asp Asp Met Ile Ala Ala Tyr Thr Ala Ala Leu Val Ser Gly Thr Ala
   850 855 860
   Thr Ala Gly Trp Thr Phe Gly Ala Gly Ala Ala Leu Gln Ile Pro Phe
   865 870 875 880
   Ala Met Gln Met Ala Tyr Arg Phe Asn Gly Ile Gly Val Thr Gln Asn
   885 890 895
   Val Leu Tyr Glu Asn Gln Lys Gln Ile Ala Asn Gln Phe Asn Lys Ala
   900 905 910
   Ile Ser Gln Ile Gln Glu Ser Leu Thr Thr Thr Ser Thr Ala Leu Gly
   915 920 925
   Lys Leu Gln Asp Val Val Asn Gln Asn Ala Gln Ala Leu Asn Thr Leu
   930 935 940
   Val Lys Gln Leu Ser Ser Asn Phe Gly Ala Ile Ser Ser Val Leu Asn
   945 950 955 960
   Asp Ile Leu Ser Arg Leu Asp Lys Val Glu Ala Glu Val Gln Ile Asp
   965 970 975
   Arg Leu Ile Thr Gly Arg Leu Gln Ser Leu Gln Thr Tyr Val Thr Gln
   980 985 990
   Gln Leu Ile Arg Ala Ala Glu Ile Arg Ala Ser Ala Asn Leu Ala Ala
   995 1000 1005
   Thr Lys Met Ser Glu Cys Val Leu Gly Gln Ser Lys Arg Val Asp Phe
   1010 1015 1020
   Cys Gly Lys Gly Tyr His Leu Met Ser Phe Pro Gln Ala Ala Pro His
   1025 1030 1035 1040
   Gly Val Val Phe Leu His Val Thr Tyr Val Pro Ser Gln Glu Arg Asn
   1045 1050 1055
   Phe Thr Thr Ala Pro Ala Ile Cys His Glu Gly Lys Ala Tyr Phe Pro
   1060 1065 1070
   Arg Glu Gly Val Phe Val Phe Asn Gly Thr Ser Trp Phe Ile Thr Gln
   1075 1080 1085
   Arg Asn Phe Phe Ser Pro Gln Ile Ile Thr Thr Asp Asn Thr Phe Val
   1090 1095 1100
   Ser Gly Asn Cys Asp Val Val Ile Gly Ile Ile Asn Asn Thr Val Tyr
   1105 1110 1115 1120
   Asp Pro Leu Gln Pro Glu Leu Asp Ser Phe Lys Glu Glu Leu Asp Lys
   1125 1130 1135
   Tyr Phe Lys Asn His Thr Ser Pro Asp Val Asp Phe Gly Asp Ile Ser
   1140 1145 1150
   Gly Ile Asn Ala Ser Val Val Asn Ile Gln Lys Glu Ile Asp Arg Leu
   1155 1160 1165
   Asn Glu Val Ala Lys Asn Leu Asn Glu Ser Leu Ile Asp Leu Gln Glu
   1170 1175 1180
   Leu Gly Lys Tyr Glu Gln Tyr Ile Lys Trp Pro Trp Tyr Val Trp Leu
   1185 1190 1195 1200
   Gly Phe Ile Ala Gly Leu Ile Ala Ile Val Met Val Thr Ile Leu Leu
   1205 1210 1215
   Cys Cys Met Thr Ser Cys Cys Ser Cys Leu Lys Gly Ala Cys Ser Cys
   1220 1225 1230
   Gly Ser Cys Cys Lys Phe Asp Glu Asp Asp Ser Glu Pro Val Leu Lys
   1235 1240 1245
   Gly Val Lys Leu His Tyr Thr
   1250 1255